# FASCICULE DE BREVET EUROPEEN

(11) **EP 1 641 729 B1**
(45) Date de publication et mention de la délivrance du brevet: **07.05.2008**
(21) Numéro de dépôt: 04767564.0
(22) Date de dépôt: 02.07.2004
(51) Int. Cl.: C07B 37/12, C07B 37/04, C07D 211/16, C07B 37/02, C07C 219/10, C07C 209/08, C07C 213/02, C07C 67/03, C07C 213/08, C07C 219/12, C07C 211/63, C07C 215/44

(54) **UTILISATION DE SELS D ONIUM FONCTIONNALISES EN TANT QUE SUPP ORT SOLUBLE POUR LA SYNTHESE ORGANIQUE**
VERWENDUNG FUNKTIONALISIERTER ONIUMSALZE ALS LÖSLICHE TRÄGER FÜR DIE ORGANISCHE SYNTHESE
USE OF FUNCTIONALIZED ONIUM SALTS AS A SOLUBLE SUPPORT FOR ORGANIC SYNTHESIS

(30) Priorité: 09.07.2003 FR 0308413
(43) Date de publication de la demande: 05.04.2006
(73) Titulaire: Centre National De La Recherche Scientifique-CNRS, 75794 Paris Cedex 16 (FR); UNIVERSITE DE RENNES I, F-35065 Rennes (FR)
(72) Inventeur: VAULTIER, Michel, F-35410 Châteaugiron (FR); GMOUH, Said, F-35200 RENNES (FR); HASSINE, Fatima, Casablanca (MA)
(74) Mandataire: Grosset-Fournier, Chantal Catherine
(86) Numéro de dépôt international: PCT/FR2004/001724
(87) Numéro de publication internationale: WO 2005/005345

(56) Documents cités:
- FRAGA-DUBREUIL J ET AL: "Grafted ionic liquid-phase-supported synthesis of small organic molecules" TETRAHEDRON LETTERS, ELSEVIER SCIENCE PUBLISHERS, AMSTERDAM, NL, vol. 42, no. 35, 27 août 2001 (2001-08-27), pages 6097-6100, XP004298190 ISSN: 0040-4039
- DUBREUIL J F ET AL: "Rate accelerations of 1,3-dipolar cycloaddition reactions in ionic liquids" TETRAHEDRON LETTERS, ELSEVIER SCIENCE PUBLISHERS, AMSTERDAM, NL, vol. 41, no. 38, 16 septembre 2000 (2000-09-16), pages 7351-7355, XP004211923 ISSN: 0040-4039
- WEISHI MIAO, TAK HANG CHAN: "Exploration of ionic liquids as soluble supports for organic synthesis. Demonstration with suzuki coupling reaction" ORGANIC LETTERS, vol. 5, no. 26, 22 novembre 2003 (2003-11-22), pages 5003-5, XP002272080
- ANJAIAH S ET AL: "Synthesis and preliminary use of novel acrylic ester-derived task-specific ionic liquids" TETRAHEDRON LETTERS, ELSEVIER SCIENCE PUBLISHERS, AMSTERDAM, NL, vol. 45, no. 3, 12 janvier 2004 (2004-01-12), pages 569-571, XP004480197 ISSN: 0040-4039
- FRAGA-DUBREUIL J ET AL: "Efficient combination of task-specific ionic liquid and microwave dielectric heating applied to one-pot three component synthesis of a small library of 4-thiazolidinones" TETRAHEDRON, ELSEVIER SCIENCE PUBLISHERS, AMSTERDAM, NL, vol. 59, no. 32, 4 août 2003 (2003-08-04), pages 6121-6130, XP004442559 ISSN: 0040-4020

## Description

La présente invention a pour objet l'utilisation de sels d'onium fonctionnalisés en tant que support soluble pour la synthèse organique.

Depuis l'introduction de la méthode de Merrifield pour la synthèse de peptides (Merrifield, 1963), les supports insolubles de type résines ont été introduits dans beaucoup de synthèses pour faciliter la purification des produits et plus particulièrement dans le domaine de la chimie combinatoire au cours de ces 10 dernières années (Thompson et al., 1996 ; Toy et al., 2000 ; P.; Seeberger et al., 2000 ; V., 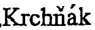 et M., W. Holladay, 2002 ; Mütter et al., 1979 ; Han et al., 1995 ; Harris et al., 1992 ; Nicolaou et al., 2002 ; Kates et al., 2000). Quoique très efficace, la synthèse sur support solide souffre toujours d'un certain nombre de problèmes liés à la nature hétérogène des conditions de réactions. En effet, les comportements cinétiques non linéaires, les distributions inégales, les sites non accessibles aux réactifs, les problèmes de solvatation, de synthèse pure posés par la phase solide ainsi que l'identification complexe des résines greffées restent des handicaps majeurs pour cette méthodologie.

Les inconvénients des supports solides ont conduit à l'exploration d'alternatives pour retrouver des conditions de réactions homogènes. De fait, l'utilisation des polymères solubles contourne les difficultés de la synthèse sur supports solides tout en conservant une grande partie de ses aspects positifs. La dénomination "Synthèse sur polymère soluble ou SPOS" est utilisée pour les réactions en phase liquide homogène réalisée sur un polymère fonctionnel soluble qui sert de groupement protecteur et dont les propriétés macromoléculaires facilite la purification des produits (Haag, 2001 ; Haag et al., 2002 ; Kim et al., 2000 ; Kim et al., 1996 ; Hovestad et al., 2000 ; Hodge, 1997 ; Frank et al., 1975 ; Han et al., 1997).

Ces méthodologies en phase-liquide évitent les difficultés de la synthèse en phase solide, par exemple le comportement cinétique non linéaire, la distribution (l'accès inégal aux sites d'interaction), les problèmes de solvatation liés à la nature du support, et les conditions opératoires qui ne sont pas transposables de manière triviale entre les réactions organiques standards en solution et la phase solide. Cependant, en remplaçant les résines réticulées insolubles par des supports polymères solubles, les avantages du support solide sont préservés : conditions de réactions classiques en milieu homogène mais aussi purification aisée des produits. De plus, les supports solubles présentent la possibilité d'analyse par les moyens classiques utilisés en chimie organique tels que les spectroscopies UV-visible, IRTF, et RMN aussi bien que la spectrométrie de masse à haute résolution. En outre, la chromatographie sur couches minces peut être employée pour le suivi des réactions sans exiger le clivage préliminaire du support (Mütter et al., 1979 ; Han et al., 1995 ; Harris, 1992), ce qui est un avantage de cette technologie. Une caractérisation rapide et efficace du support est un outil important, particulièrement pour les synthèses parallèle, combinatoire ou multiétape.

De la même façon qu'en phase solide, les supports solubles peuvent être séparés des molécules de faible poids moléculaire après chaque étape par ultrafiltration, dialyse; chromatographie préparative par exclusion (SEC), ou précipitation. Même si l'automatisation de ces techniques n'est pas aussi avancée qu'avec les résines, des progrès importants ont été faits ces dernières années. Les polymères utilisables comme supports solubles doivent être commercialement accessibles ou facilement préparés, être chimiquement stables, être correctement fonctionnalisés pour pouvoir accrocher une partie organique et être très solubles dans les solvants usuels. En général les polymères sont un mélange de molécules de différentes tailles qui ont des propriétés différentes. Les supports solubles devront avoir une polydispersité aussi proche de 1 que possible et une masse-moléculaire assez élevée pour être cristallisés à la température ambiante. La plupart des polymères solubles utilisés ont des squelettes hydrocarbonés (polymères de Janda) ou alkyle polyéthers et plus particulièrement les polyéthylènes glycols (PEGs).

Jusqu'à maintenant, le polymère le plus utilisé comme support soluble en synthèse organique est le polyéthylène glycol monométhylé (MPEG 5000) ne contenant qu'une fonction OH ou diol et de ce fait a une faible charge spécifique (0,2 mmol OH/g)(Mutter et al., 1974 ; Mutter et al., 1975 ; Gravert et al., 1997 ; Toy et al., 2000). Plus récemment, des PEGs de plus grande charge spécifique ont été préparés, pouvant être purifiés par précipitation. Ces PEGs en forme d'étoile (Chang et al., 1999 ; Knischka et al., 2000 ; Reed et al., 2000) ou ramifiés (Benaglia et al., 1998 ; Annunziata et al., 2000) sur les positions terminales peuvent atteindre des charges spécifiques de 1 mmol de OH/g de polymère mais sont longs à préparer.

Les polymères linéaires portant des groupes fonctionnels sur chaque unité monomère, comme l'alcool polyvinylique (Elias, 1997), les polyacrylamides (Wellings et al., 1987 ; Ranucci et al., 1994) et les polymères préparés par ROMP ("ring opening metathesis polymerisation")(Barrett et al., 1999 ; Barrett et al., 2000 ; Barrett et al., 2000) ont aussi été utilisés en SPOS. Ces polymères à forte charge spécifique sont d'utilisation problèmatique dans certains cas à cause de leur solubilité et stabilité limitées (Meier et al., 2001).

Aussi, des dendrimères parfaits (polyamidoamine, polysilane, polyester) ont-ils été utilisés comme supports en synthèse combinatoire. Ces supports solubles polymériques de grande charge spécifique théorique sont fragiles, de poids moléculaires relativement faibles et sont préparés par voie multiétape, ce qui limite leur utilisation en chimie combinatoire (Burgath et al., 2000).

Les polyéthers dendritiques aliphatiques (analogues ramifiés des PEGs) sont par contre chimiquement stables dans beaucoup de conditions de réaction et donc devraient être utiles comme supports polymères en synthèse organique. De plus, les propriétés chimiques de ces matériaux sont idéales pour la synthèse supportée en solution. Par ailleurs, la forme globulaire de ces polymères dendritiques peut favoriser leur purification par les techniques de membranes (dialyse et ultrafiltration) Les polyéthers aliphatiques dendrimériques contenant des unités 1-3 diol et 1-2 diol ont été préparés récemment en 6-7 étapes. Ils ont des charges spécifiques de 6-7 mmol de OH/g mais ils sont longs à préparer et de poids moléculaires relativement faibles (Jayaraman et al., 1998 ; Grayson et al., 1999 ; Haag et al., 2000).

Haag a rapporté récemment la synthèse contrôlée de polyglycérols dendritiques. Ces polyéthers polyols aliphatiques ont un squelette stable et sont facilement préparés en une étape à l'échelle de 1 kg (Haag et al., 2002). Ils ont des poids moléculaires pouvant atteindre 30 000 g/mole avec une polydispersité Mw/Mn-1,5. La structure dendrimérique contient statistiquement des unités glycérol incorporées linéairement (OH primaires et secondaires) et des 1,2 diols terminaux. La densité totale de groupes fonctionnels atteint 13,5 mmol de OH/g de polymère dont environ 30% (4,1 mmol/g) sont des 1,2 diols terminaux facilement accessibles et qui peuvent être utilisés directement pour greffage d'aldéhydes ou de cétones sur ces polymères sous forme d'acétals. Ces polyglycérols dendritiques ont été utilisés en SPOS pour l'aménagement fonctionnel de cétones ω-halogénées et pour le couplage de Suzuki. Un autre problème vient de leur structure : les fonctions OH à la périphérie utilisables pour la synthèse ne sont pas identiques, dans la mesure où il y a un mélange de OH secondaires et primaires qui n'auront pas la même réactivité et entraîneront donc des problèmes de sélectivité et probablement de réactivité secondaire.

Malgré les différents avantages présentés par les supports polymères solubles actuellement utilisés, les inconvénients liés à leur poids moléculaire élevé et à leur charge spécifique utilisable limitée sont de sévères handicaps à une utilisation généralisée. En effet, les PEGs les plus souvent utilisés pour la synthèse organique et la chimie combinatoire sur supports solubles ont des poids moléculaires compris entre 2000 et 10000 daltons et ne présentent qu'une charge spécifique comprise entre 0,1 et 1 mmol/g de polymère. L'analyse par spectroscopies RMN ¹H et ¹³C de ces polymères en solution est réalisable mais présente des difficultés car les signaux correspondant aux protons et aux carbones des PEGs ont une forte intensité par rapport aux signaux relatifs aux protons et carbones du substrat supporté. Des problèmes de viscosité des solutions sont aussi rencontrés à forte concentration. Les problèmes de purification de ces polymères sont aussi une sévère limitation à leur utilisation et à leur recyclage, en particulier dans une optique d'automatisation des synthèses.

Depuis quelques années, certains sels d'onium sous l'appellation "liquides ioniques" (Welton et al., 1999 ; Wasserscheid et al., 2000 ; Wasserscheid et Welton, 2003), moyennant un choix approprié des anions, sont de plus en plus utilisés en synthèse organique et en catalyse car ils présentent un certain nombre de propriétés physico-chimiques intéressantes et importantes telles que leur grande stabilité thermique, leur faible volatilité et leur tension de vapeur très faible, leur faible inflammabilité, leur fort pouvoir de solubilisation aussi bien des sels que des molécules organiques neutres et des polymères et enfin la possibilité d'un recyclage aisé.

Cependant, les sels d'onium décrits dans la littérature sont généralement synthétisés et utilisés en tant que tels pour leurs propriétés biologiques ou physiques (tensioactifs par exemple).

L'article de Fraga-Dubreuil et al. (Tetrahedron Letters 42 (2001) 6097-6100) concerne l'application des liquides ioniques dans des synthèses sur support en phase liquide, dans des conditions exemptes de solvants.

La présente invention a pour but de fournir une nouvelle utilisation des sels d'onium en tant que supports solubles pour la synthèse organique en phase homogène en présence d'au moins un solvant organique.

La présente invention a pour but de fournir de nouveaux supports solubles pour la synthèse organique en présence d'au moins un solvant, en remplacement des supports solubles de l'art antérieur tels que les PEG, lesdits nouveaux supports solubles étant plus faciles à préparer, à utiliser et à purifier, parfaitement définis et identifiés, moins coûteux et de fonctionnalisation aisée.

La présente invention a pour but de fournir de nouveaux supports solubles présentant une charge spécifique élevée et dont le recyclage est aisé.

La présente invention concerne l'utilisation d'un sel d'onium fonctionnalisé par au moins une fonction organique, en tant que support soluble, en présence d'au moins un solvant organique, pour la synthèse organique d'une molécule, en phase homogène, par au moins une transformation de ladite fonction organique, ledit sel d'onium permettant la libération de la molécule synthétisée,
ledit sel d'onium se présentant sous forme liquide ou solide à température ambiante, et répondant à la formule A₁⁺, X₁⁻,
dans laquelle :
- A₁⁺ représente un cation,
- X₁⁻ représente un anion,
A₁⁺ étant un cation fonctionnel ou polyfonctionnel, et/ou
X₁⁻ étant un anion fonctionnel ou polyfonctionnel,
le sel d'onium étant tel que sous sa forme initiale, c'est-à-dire avant la première transformation de ladite fonction organique, A₁⁺ et X₁⁻ ne sont pas liés entre eux par une liaison covalente,
et lorsque l'anion et le cation portent respectivement une fonction organique, celles-ci ne peuvent pas réagir entre elles avant la première transformation de ladite fonction organique.

L'utilisation en tant que support soluble, donc en solution dans un solvant ou un mélange de solvants, de sels d'onium est démontrée dans le cadre de l'invention. Ceci n'était pas a priori évident compte tenu des connaissances sur ces sels. Sous forme d'halogénures, ils sont généralement peu solubles dans les solvants usuels utilisés en chimie organique. Par ailleurs, leur fonctionnalisation peut poser, a priori, des problèmes de chimiosélectivité à cause de la présence d'un groupement onium chargé positivement qui est sujet à la β-élimination ou à la déprotonation en α en milieu basique.

L'expression "sel d'onium fonctionnalisé" désigne les sels d'ammonium, de phosphonium, de sulfonium, ainsi que tous les sels résultant de la quaternarisation d'une amine, d'une phosphine, d'une arsine, d'un thioéther ou d'un hétérocycle contenant l'un ou plusieurs de ces hétéroatomes, et portant au moins une fonction organique Fᵢ ou F'ᵢ. Cette expression désigne aussi un sel d'onium dont le cation tel que défini ci-dessus n'est pas fonctionnalisé mais dont l'anion porte une fonction F'ᵢ. Cette expression peut également désigner un sel dont l'anion et le cation portent au moins une fonction organique.

L'expression "support soluble" désigne une molécule fonctionnelle polymère ou un sel servant d'"ancre" pour effectuer, en solution, des transformations successives d'une molécule accrochée par la fonction. Cette ancre confère des propriétés à la molécule accrochée (donc finalement à l'ensemble formé par l'ancre et la molécule accrochée) qui permettent de purifier aisément par lavage, évaporation ou tout autre technique. Ceci ne pourrait être fait facilement avec des molécules volatiles et/ou solubles dans les solvants usuels par exemple. En utilisant cette technique, on peut utiliser des excès de réactifs, par exemple, comme dans le cas des résines de Merrifield insolubles. Un support soluble doit par définition être soluble dans un solvant. Ceci confère l'avantage d'effectuer les réactions en solution et de pouvoir en suivre l'avancement à l'aide de techniques d'analyse classiquement utilisées dans le domaine de la chimie organique. Un support soluble doit également être récupérable à la fin des transformations. En d'autres termes, les molécules synthétisées sur ce support doivent pouvoir être facilement décrochées. Par ailleurs, le squelette du support soluble ne doit pas réagir avec les réactifs utilisés, les réactions ayant lieu sélectivement sur les fonctions accrochées sur le squelette de base.

L'expression "synthèse organique d'une molécule en phase homogène" désigne la ou les transformation(s) de la ou des fonctions chimiques portée(s) par ledit sel d'onium, suivie d'une réaction de clivage libérant la molécule recherchée en solution dans un solvant donné ou dans un mélange de solvants et le sel de départ ou un sel recyclable en le support de départ.

L'expression "cation fonctionnel" désigne un groupe moléculaire qui possède au moins une fonction chimique, ainsi qu'une tête portant une charge positive.

L'expression "anion fonctionnel" désigne un groupe moléculaire qui possède au moins une fonction chimique, ainsi qu'une tête portant une charge négative.

Les sels d'onium susmentionnés sont solubilisés dans un solvant organique ou dans un mélange de solvants organiques puis mis en présence d'un excès ou non de réactif. Ils sont alors utilisés comme supports solubles. Une autre propriété de ces sels est qu'ils ne sont pas solubles dans certains solvants usuels tels que l'éther, les alcanes ou les hydrocarbures par exemple. De plus, ils ont des tensions de vapeur extrêmement faibles et peuvent donc être placés sous un vide poussé sans pertes. Ces deux propriétés permettent l'utilisation d'un excès de réactif qui sera ensuite facilement éliminé par lavage, par extraction ou sous vide poussé comme dans le cas des réactions sur résines ou sur PEG. De très nombreuses transformations chimiques des sels d'onium fonctionnels sont possibles. On peut utiliser ces sels comme des résines ou des polymères solubles.

La charge spécifique d'un support est définie par la quantité de réactif qui peut être supportée par gramme de support et s'exprime en mmol/g. Cela correspond en fait à ce que l'on pourrait appeler la fonctionnalité spécifique d'un support notée ***f*** que l'on pourra exprimer en millifonction par gramme (mf/g). Si le sel est monofonctionnel, la fonctionnalité spécifique (***f*** exprimée en mf/g) sera égale à la charge spécifique exprimée en mmol/g. Si le sel porte n fois la même fonction, la fonctionnalité spécifique *f* sera égale à n fois la charge spécifique x du sel. Lorsque les sels d'onium sont en solution, ce qui est pratiquement toujours le cas, il faut adapter les notions définies ci-dessus. La molarité d'une solution sera exprimée en mol/l ou en mmol/ml. Connaissant la densité des solutions, il est alors facile de convertir en mmol/g de solution donnant ainsi des éléments précis sur la charge spécifique des solutions pour comparaison avec les résines de Merrifield ou les solutions de supports solubles de type polymères (PEG ou autres).

Si le sel porte n fois la même fonction, une solution contenant par exemple une millimole de ce sel par gramme aura une fonctionnalité spécifique ***f*** de n mf/g.

Les sels d'onium monofonctionnalisés tels qu'utilisés dans le cadre de la présente invention présentent une charge spécifique supérieure à 1 mmol.g⁻¹ et pouvant atteindre jusqu'à 7 mmol.g⁻¹, à comparer avec celle du PEG 5000 qui est de 0,2 mmol.g⁻¹.

L'expression "transformation de la fonction organique" désigne la modification d'une fonction Fᵢ par un ou plusieurs réactifs et/ou catalyseurs et/ou par activation physique (chauffage, micro-ondes, ultrasons, radiations hv, pression, électrochimie...).

L'expression "sel d'onium sous sa forme initiale" désigne le sel dans lequel la fonction organique initiale n'a pas encore subi de transformation, c'est-à-dire n'a pas encore été impliquée dans une réaction, cette fonction étant dans ce qui suit désignée par F₀.

L'expression "première transformation de la fonction organique" désigne la modification de la fonction organique initiale portée par le sel d'onium sous sa forme initiale et qui sera symbolisée dans ce qui suit par la modification de F₀ en F₁.

Un sel dans lequel le cation et l'anion portent respectivement une fonction organique initiale nommée F₀ et F'₀, et dans lequel l'anion et le cation ne réagissent pas ensemble avant la première transformation desdites fonctions organiques, est un sel dans lequel les fonctions F₀ et F'₀ sont chimiquement compatibles ou encore chimiquement inertes l'une par rapport à l'autre. Ceci signifie donc que le sel en question est stable. F₀ et F'₀ pourront par contre réagir l'une sur l'autre sous l'effet d'une activation quelconque qui pourra être physique (radiations hv, micro-ondes, pression, chauffage...) ou chimique (catalyseur, autre réactif...)

Cette nouvelle façon de faire de la synthèse supportée est également nommée SOSSO pour Synthèse Organique Supportée sur Sel d'Onium (OSSOS en anglais pour Onium Salt Supported Organic Synthesis).

Une utilisation avantageuse selon l'invention est caractérisée en ce que le sel d'onium est purifié et/ou recyclé sous sa forme initiale après la libération de la molécule synthétisée.

Les procédés préférés de purification et/ou de recyclage utilisés sont notamment un procédé simple de lavage ou de recristallisation dans un solvant approprié.

Une utilisation avantageuse de la présente invention est caractérisée en ce que les cations et anions fonctionnels correspondent à une entité ionique, respectivement cationique Y⁺- et anionique Z⁻-, liée, éventuellement par l'intermédiaire d'un bras, respectivement L et M, notamment un groupe alkyle ou aralkyle ou alkaryle comprenant de 1 à 30 atomes de carbone, à au moins respectivement une fonction Fᵢ et F'ᵢ, Fᵢ variant de F₀ à Fₙ, F'ᵢ variant de F'₀ à F'ₙ, n étant un nombre entier variant de 1 à 20,
le cation fonctionnel A₁⁺ pouvant être représenté sous la forme Y⁺-L-Fᵢ, et
l'anion fonctionnel X₁⁻ sous la forme Z⁻-(M)ₖ-F'ᵢ, k étant égal à 0 ou 1.

L'expression "entité ionique" désigne la partie du cation ou de l'anion, qui porte la charge, respectivement positive ou négative.

Les fonctions Fᵢ et F'ᵢ sont notamment choisies parmi les fonctions suivantes :
hydroxyle, acide carboxylique, amide, sulfone, amine primaire, amine secondaire, aldéhyde, cétone, éthényle, éthynyle, diényle, éther, époxyde, phosphine (primaire, secondaire ou tertiaire), azoture, imine, cétène, cumulène, hétérocumulène, thiol, thioéther, sulfoxyde, groupements phosphorés, hétérocycles, acide sulfonique, silane, stannane ou aryle fonctionnel, et toute fonction résultant d'une transformation des fonctions précédentes par voie chimique ou induite par activation thermique, électrochimique, photochimique ou par toute autre technique physique telle que les irradiations micro-ondes, les ultrasons ou par pression.

Dans le terme "Y⁺-", le tiret "-" représente la liaison éventuelle entre l'entité cationique et le bras L.

Dans le terme "Z⁻-", le tiret "-" représente la liaison éventuelle entre l'entité anionique et le bras L.

Le terme "bras L" désigne une chaîne alkyle ou aralkyle ou alkaryle pouvant contenir un ou plusieurs hétéroatomes tels que l'azote, le phosphore, le soufre, l'oxygène, le silicium, l'étain, contenant entre 1 et 30 atomes de carbone, et ledit bras est notamment choisi parmi une chaîne alkyle contenant de 2 à 20 atomes de carbone et de 1 à 6 atomes d'oxygène ou d'azote.

Selon un mode de réalisation avantageux, le sel d'onium fonctionnalisé A₁⁺, X₁⁻ utilisé dans le cadre de la présente invention est soluble dans un solvant organique.

Selon un mode de réalisation avantageux, le sel d'onium fonctionnalisé A₁⁺, X₁⁻ utilisé dans le cadre de la présente invention est liquide à température ambiante.

Selon un mode de réalisation avantageux, le sel d'onium A₁⁺, X₁⁻ utilisé dans le cadre de la présente invention est solide à température ambiante et est liquéfiable dans une gamme de températures allant d'environ 25°C à environ 450°C, notamment d'environ 30°C à environ 150°C.

Une utilisation avantageuse de l'invention est caractérisée en ce que les fonctions organiques Fᵢ et F'ᵢ sont choisies parmi les fonctions classiques de la chimie organique, telles que les fonctions hydroxyle, acide carboxylique, amide, sulfone, amine primaire, amine secondaire, aldéhyde, cétone, éthényle, éthynyle, diényle, éther, époxyde, phosphine (primaire, secondaire ou tertiaire), azoture, imine, cétène, cumulène, hétérocumulène, thiol, thioéther, sulfoxyde, groupements phosphorés, hétérocycles, acide sulfonique, silane, stannane ou aryle fonctionnel.

Une utilisation avantageuse de la présente invention est caractérisée en ce que le poids moléculaire du sel d'onium fonctionnalisé est inférieur à 1500 g.mol⁻¹, notamment inférieur à 750 g.mol⁻¹, et est de préférence compris de 130 à 500 g.mol⁻¹.

Pour assurer une bonne productivité du support, il est nécessaire que la masse moléculaire du sel soit la plus faible possible (en raison de la charge spécifique telle que définie précédemment). Ainsi, on utilise de préférence des anions de masse la plus faible possible comme les chlorures afin de pouvoir utiliser éventuellement des cations de masse plus élevée (voir tableau 17 plus loin représentant les variations de charge spécifique en fonction de la masse).

Une utilisation avantageuse de la présente invention est caractérisée en ce que A₁⁺ est un cation fonctionnel et en ce que X₁⁻ est un anion non fonctionnel.

L'expression "anion non fonctionnel" désigne un groupe moléculaire qui ne possède pas de fonction chimique, une partie de ce groupe portant une charge négative.

Ce mode de réalisation permet d'effectuer des réactions spécifiques sur la partie cationique du sel d'onium. On peut donc maîtriser la sélectivité et la réactivité qui pourrait être différente sur l'anion et le cation.

La présente invention concerne également une utilisation telle que définie ci-dessus, dans laquelle le sel d'onium A₁⁺, X₁⁻ a comme forme initiale Y⁺-L-F₀, X₁⁻, pour l'obtention d'une molécule G, par transformation de ladite fonction initiale F₀ selon le schéma 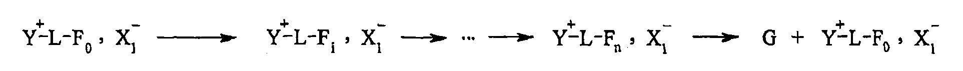 L étant tel que défini ci-dessus,
ladite molécule G étant obtenue par clivage au niveau de la fonction Fₙ,
et le sel d'onium fonctionnalisé pouvant être récupéré ou recyclé sous sa forme initiale Y⁺-L-F₀, X₁⁻, après la libération de G.

Les réactions utilisées pour la libération de G par clivage sont notamment les suivantes : transestérification, transamidation, réduction, lactonisation, lactamisation, cyclisation décrochante et couplage décrochant.

Une utilisation avantageuse de l'invention est caractérisée en ce que le cation fonctionnel A₁⁺ est choisi parmi les cations pyridinium imidazolium, ammonium, phosphonium ou sulfonium, cycliques ou non, substitués ou non, et de préférence ammonium ou phosphonium.

Une utilisation avantageuse selon l'invention est caractérisée en ce que le cation fonctionnel A₁⁺ est choisi parmi les cations ammonium quaternaire, cycliques ou non.

Une utilisation avantageuse selon l'invention est caractérisée en ce que X₁⁻ est un anion fonctionnel et A₁⁺ est un cation non fonctionnel.

L'expression "cation non fonctionnel" désigne un groupe moléculaire qui ne possède pas de fonction chimique, une partie de ce groupe portant une charge positive.

Ce mode de réalisation permet d'effectuer des modifications fonctionnelles uniquement sur la partie anionique.

La présente invention concerne également une utilisation telle que définie ci-dessus, dans laquelle le sel d'onium A₁⁺, X₁⁻ a comme forme initiale A₁⁺, Z⁻-(M)ₖ-F'₀, pour l'obtention d'une molécule G, par transformation de ladite fonction initiale F'₀ selon le schéma 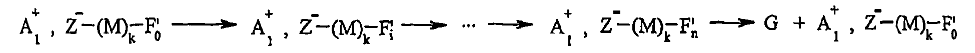 k et M étant tels que définis ci-dessus,
ladite molécule G étant obtenue par clivage de la fonction F'ₙ,
et le sel d'onium fonctionnalisé pouvant être récupéré ou recyclé sous sa forme initiale A₁⁺, Z⁻-(M)ₖ-F'₀, après la libération de G.

Les réactions utilisées pour la libération de G par clivage sont notamment les suivantes : transestérification, transamidation, féduction, lactonisation, lactamisation, cyclisation décrochante et couplage décrochant.

Une utilisation avantageuse de l'invention est caractérisée en ce que X₁⁻ est choisi parmi :
- la famille des phosphates : R₁PO₄²⁻, R₁R₂PO₄⁻,
- la famille des sulfates : R₁SO₄⁻,
- la famille des sulfonates : R₁SO₃⁻,
- la famille des carboxylates : R₁CO₂⁻,
ou parmi les anions suivants :

R₁N̅SO₂R₂

R₂SO₂N̅SO₂R₂

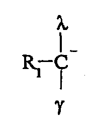

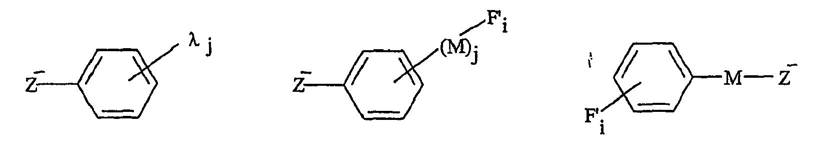

Z⁻, M et F'ᵢ étant tels que définis ci-dessus, Z⁻ représentant notamment O⁻, SO₃⁻, CO₂⁻, R₁PO₃⁻ ou R₁PO₂⁻,
j représentant un nombre entier compris de 1 à 5,
R₁ et R₂ pouvant représenter indépendamment l'un de l'autre un groupe alkyle fonctionnel, un groupe vinyle ou alcynyle, éventuellement fonctionnel, comprenant de 1 à 20 atomes de carbone, ou pouvant représenter un groupement aryle fonctionnel comprenant de 6 à 30 atomes de carbone,
γ et λ représentant un groupe électroattracteur, notamment choisi parmi les groupes : CO₂R'₁, SO₂R'₁, CN, NO₂, P(O)(OR'₁)_{2,} C(O)R'₁ et SO₃R'₁,
R'₁ représentant un groupe alkyle, éventuellement fonctionnel, comprenant de 1 à 20 atomes de carbone, ou un groupe aryle, éventuellement fonctionnel, comprenant de 6 à 30 atomes de carbone.

Une utilisation avantageuse selon l'invention est caractérisée en ce que A₁⁺ est un cation fonctionnel et X₁⁻ est un anion fonctionnel.

Ce mode de réalisation permet d'effectuer des transformations en parallèle ainsi que la réaction intra- ou intermoléculaire d'une fonction de A₁⁺ avec une fonction de X₁⁻.

La présente invention concerne également une utilisation telle que définie ci-dessus, dans laquelle le sel d'onium A₁⁺, X₁⁻ a comme forme initiale Y⁺-L-F₀, Z⁻-(M)ₖ-F'₀, pour l'obtention d'une molécule G, par transformation desdites fonctions initiales F₀ et F'₀ selon le schéma 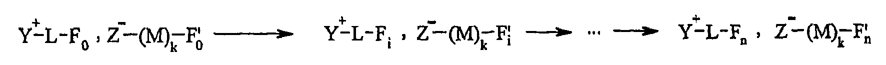 L, k et M étant tels que définis ci-dessus,
et par réaction de Fₙ sur F'ₙ dans le sel d'onium fonctionnalisé Y⁺-L-Fₙ, Z⁻-(M)ₖ-F'ₙ conduisant à la formation d'un sel interne de formule : 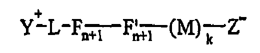 ladite molécule G étant obtenue par clivage du sel interne susmentionné et correspondant à la formule Fₙ₊₂-F'_{n+2,}
et le sel d' onium fonctionnalisé pouvant être récupéré ou recyclé sous sa forme initiale Y⁺-L-F₀, Z⁻-(M)ₖ-F'₀, après la libération de G.

Ce mode de réalisation permet d'effectuer des réactions intramoléculaires.

L'expression "sel interne" désigne une entité portant simultanément au moins un groupe chargé positivement et un groupe chargé négativement, distants d'au moins 2 atomes reliés par des liaisons covalentes.

Une utilisation selon la présente invention est caractérisée en ce que le sel d'onium est choisi parmi les sels suivants :

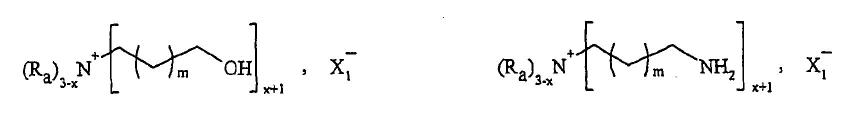

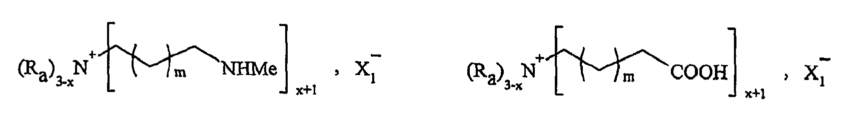

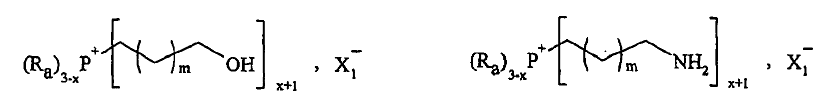

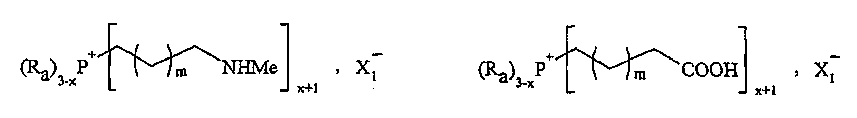

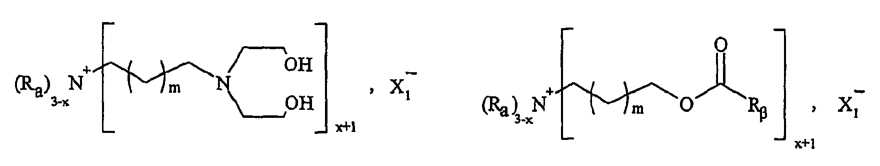

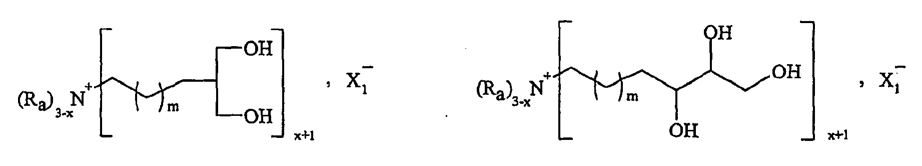

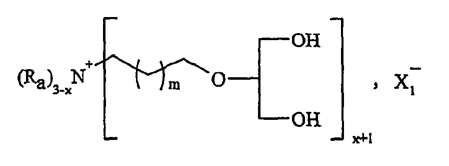

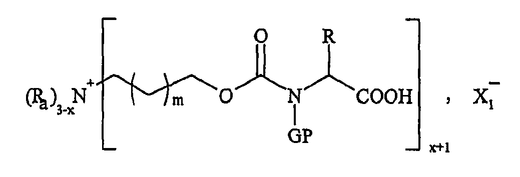

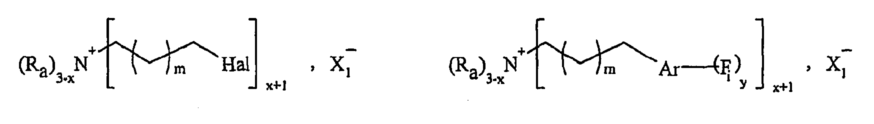

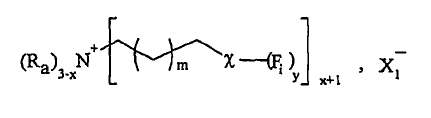

R représentant un atome d'hydrogène, un groupe alkyle, alkaryle ou aralkyle, fonctionnel ou non, comprenant de 1 à 20 atomes de carbone, ou un groupe aryle, fonctionnel ou non, comprenant de 6 à 30 atomes de carbone,
x représentant un nombre entier compris de 0 à 3,
y représentant un nombre entier compris de 1 à 5,
Ar représentant un noyau aromatique fonctionnel ou polyfonctionnel,
Fᵢ étant tel que défini précédemment,
Hal représentant un atome d'halogène, notamment choisi parmi le chlore, le brome et l'iode,
χ représentant un carbocycle ou un hétérocycle fonctionnel,
X₁⁻ étant choisi parmi : NTf₂⁻, PF₆⁻, BF₄⁻, Cl⁻, Br⁻, I⁻, CF₃SO₃⁻, MeSO₄⁻, EtSO₄⁻, MeSO₃⁻, C₆H₅SO₃⁻, pMeC₆H₄SO₃⁻,
m étant un nombre entier compris de 0 à 20,
R_{β} représentant un groupe diényle, vinyle, substitué ou non, alkyle fonctionnel comprenant de 1 à 20 atomes de carbone, ou aryle fonctionnel comprenant de 6 à 30 atomes de carbone, alcynyle substitué ou non, et étant notamment un groupe alkylvinyle, alkylalcynyle, alkylaryle, alkyldiényle, alkylmalonyle, acyle,
et Rₐ représentant un groupe alkyle ramifié ou non comprenant de 1 à 20 atomes de carbone, notamment un groupe éthyle, propyle, butyle, pentyle, hexyle, heptyle ou octyle.

La présente invention concerne également une utilisation telle que définie ci-dessus, caractérisée en ce que le ou les solvant(s) utilisé(s) est un solvant aprotique, choisi parmi :
- les solvants dont la constante diélectrique ε est inférieure ou égale à 2, tels que les alcanes, les carbures aromatiques comme le benzène, le toluène ou le xylène,
- les solvants dont la constante diélectrique ε est comprise entre environ 2 et 15, tels que les éthers, les halogénobenzènes ou le dichlorométhane, et
- les solvants dont la constante diélectrique ε est supérieure à 15, tels que l'acétonitrile, le nitrométhane, le DMF ou la diméthylacétamide.

On utilise souvent la constante diélectrique ε et/ou le moment dipolaire pour caractériser la polarité d'un solvant. Plus récemment, le paramètre de Dimroth-Reichardt E_{T}^{N} a été proposé pour mieux décrire la polarité des solvants (Reichardt, 1988).

Les solvants avantageusement utilisés dans l'invention sont le toluène, le dichlorométhane, le THF, l'acétonitrile et le DMF.

La présente invention concerne également une utilisation telle que définie ci-dessus, pour la synthèse organique, en continu, en discontinu, combinatoire, ou parallèle, et/ou pour la préparation de banques de produits.

Les avantages de l'utilisation des sels d'onium susmentionnés sont les suivants :
- de très nombreux sels d'onium fonctionnels sont connus, facilement accessibles et certains sont commerciaux ;
- les modifications fonctionnelles des sels d'onium sont généralement simples et facilement réalisables selon des méthodes décrites dans la littérature ; si elles n'existent pas, elles pourront être mises au point à partir des connaissances en chimie organique ;
- les réactions ont lieu en phase homogène, ce qui implique que toutes les connaissances de la réactivité en chimie organique, organométallique et catalytique sont applicables ; de plus, toutes les techniques d'analyse, incluant les RMN ¹H, ¹³C, ¹⁹F, ³¹P, ¹¹B, ¹⁵N etc..., la CLHP, l'IRTF, l'UV-visible, la fluorescence, les techniques électrochimiques, l'électrophorèse, la spectrométrie de masse etc..., peuvent être utilisées dans les conditions normales sans complications particulières ;
- les réactions sont réalisées aux concentrations habituelles de 0,5 à 1 mole par litre (voir beaucoup plus) ce qui représente un énorme avantage en terme de charge spécifique ;
- la purification des intermédiaires est généralement facile ;
- le recyclage de ces supports est aisé ;
- les solutions de sels dans les solvants habituels sont facilement transférables à l'aide des techniques de seringue et/ou de pompage ;
- les solutions de sels d'onium dans les solvants organiques se prêtent aisément aux techniques de partition et donc aux techniques de synthèse en parallèle ou combinatoire ; des bibliothèques de produits peuvent donc être facilement synthétisées ;
- les réactivités et les sélectivités dépendent de la nature de l'anion ou du cation ;
- la montée en échelle ("scale up") ne pose pas de problèmes ce qui présente un avantage majeur par rapport aux résines et aux polymères solubles ;
- une analogie est facilement établie entre cette nouvelle technologie et les techniques de synthèse sur résines de type Merrifield ou sur polymères solubles de type PEG, PG ou JANDA ; ces sels d'onium peuvent être fonctionnalisés comme les résines de Wang, Rink, silylalkyle, carbonate, carboxylique, formyle, hydroxyle, amino, oxime etc... ou les polymères fonctionnalisés mais sont d'une utilisation beaucoup plus aisée et avantageuse ;
- ils sont beaucoup moins coûteux ; cet avantage économique est très important car de nature à ouvrir un marché de substitution de grande ampleur.

La présente invention concerne également une utilisation telle que définie ci-dessus, pour la mise en oeuvre de réactions de cycloaddition, de préférence pour la mise en oeuvre de la réaction de Diels-Alder, selon l'un des schémas réactionnels suivants :
a) 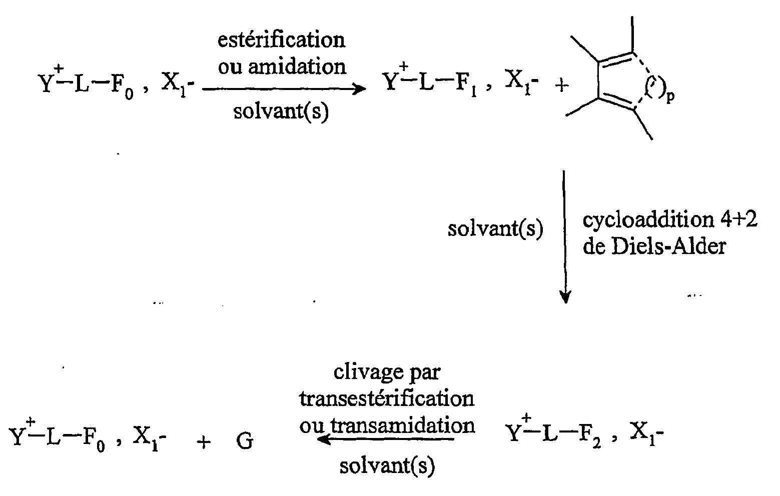 p étant un nombre entier variant de 0 à 2,
Y⁺- représentant un cation onium tel que défini précédemment, et étant de préférence un cation triméthylalkylammonium, triéthylalkylammonium, tributylalkylphosphonium, N-méthylimidazolium ou pyridinium,
L représentant un bras, notamment un groupe alkyle linéaire ou ramifié comprenant de 1 à 20 atomes de carbone, ou un groupe aralkyle ou alkaryle éventuellement fonctionnel, comprenant de 6 à 30 atomes de carbone, et étant de préférence un groupe alkyle linéaire de préférence un groupe alkyle linéaire de type (CH₂)ᵣ, r variant de 1 à 20, et de préférence de 2 à 10,
X₁⁻ étant tel que défini précédemment, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁ et F₂ étant telles que définies ci-dessous :
- F₀ correspond à un groupe -χ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante :

| | |
|---|---|
| 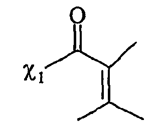 | χ₁ étant tel que défini ci-dessus, |

- F₂ répond à la formule suivante :

| |
|---|
| 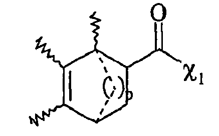 χ ₁ étant tel que défini ci-dessus, |

G répondant à la formule suivante : 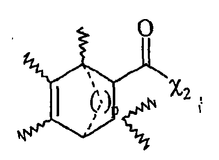 dans laquelle χ₂ représente soit un groupe ORg, Rg représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone,
la réaction d'estérification ou d'amidation dans ce schéma réactionnel étant effectuée par addition de l'acide carboxylique de formule suivante : 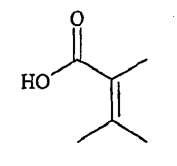
b) 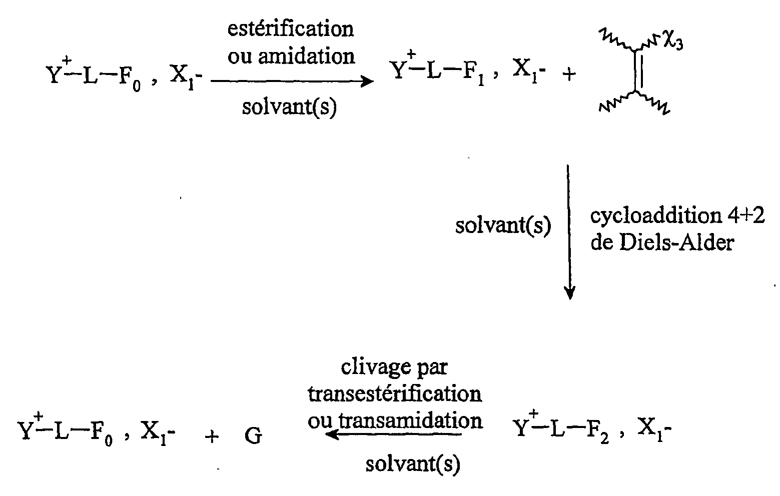 Y⁺-, L et X₁⁻ étant tels que définis précédemment,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, ou un mélange de ces solvants,
les fonctions F₀, F₁ et F₂ étant telles que définies ci-dessous :
- F₀ représente toute fonction permettant d'agrafer un diène-1,3, et est notamment choisie parmi les fonctions carbonyles, amines, alcoxy, silanes, stannanes et boranes, comprenant de 1 à 20 atomes de carbone,
- F₁ répond à la formule suivante :

| | |
|---|---|
| 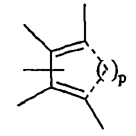 | p étant un nombre entier variant de 0 à 2, |

- F₂ répond à la formule suivante :

| | |
|---|---|
| 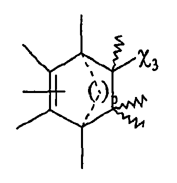 | χ₃ représentant un groupement électroattracteur, notamment choisi parmi les groupes cyano, alkoxycarbonyle, comprenant de 1 à 20 atomes de carbone, acyle comprenant de 2 à 20 atomes de carbone, benzoyle, sulfonyle, dialkoxyphosphonyle comprenant de 1 à 10 atomes de carbone, |

G répondant à la formule suivante :

| | |
|---|---|
| 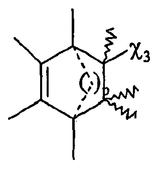 | χ₃ étant tel que défini ci-dessus. |

Le passage de F₀ à F₁ s'effectue de la façon suivante :
- par estérification entre le composé de formule Y⁺-L-OH, X₁⁻ et le composé de 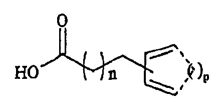
ou l'un de ses dérivés tels qu'un chlorure d'acide pour obtenir le composé suivant : 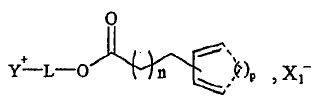
- par acylation entre le composé de formule 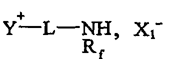 et le composé de formule 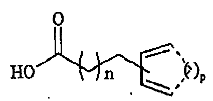 ou l'un de ses dérivés tels qu'un chlorure d'acide pour obtenir le composé suivant :

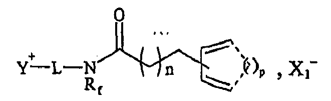

Le produit de formule 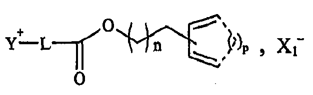 s'obtient par estérification des alcools de formule 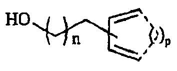 avec 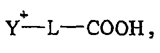 X₁⁻ ou 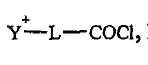
Le produit de formule 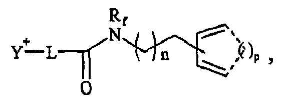 X₁⁻ s'obtient par acylation des amines de formule 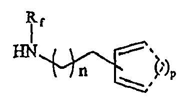 avec 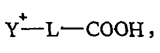 X₁⁻ ou 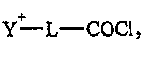 n, R_{f}, p, X₁⁻, Y et L étant tels que définis précédemment,
c) 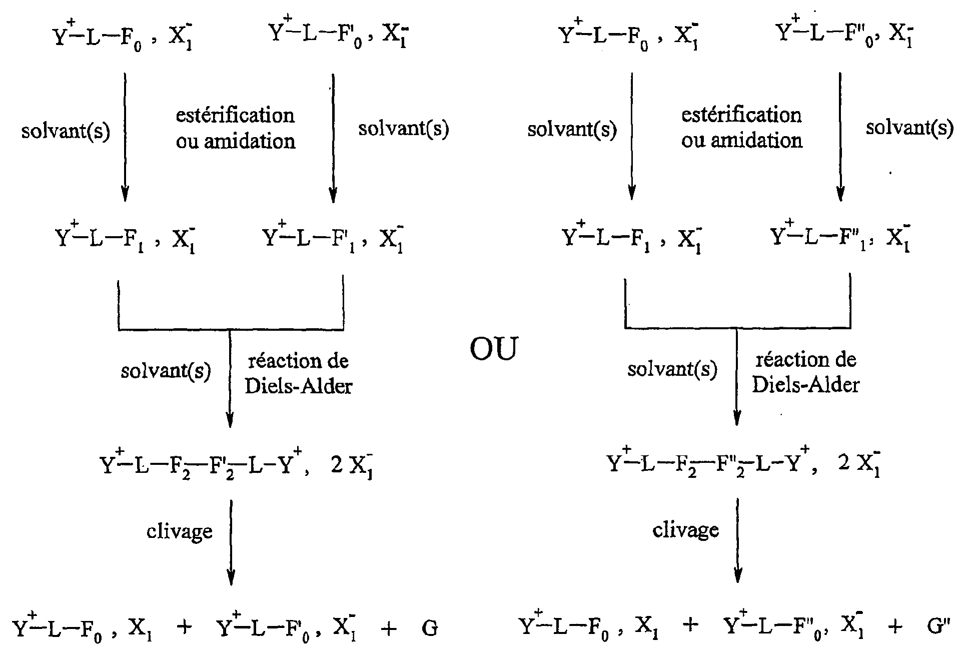 Y⁺-, L et X₁⁻ étant tels que définis précédemment,
le ou les solvants étant choisis parmi : le dichlorométhane, le tétrahydrofurane, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F'₀, F"o, F₁, F'₁, F"₁, F₂, F'₂ et F"₂ étant telles que définies ci-dessous :
- F₀ et F'₀ correspondent respectivement à un groupe -χ₁H et -χ'₁H, dans lequel χ₁ et χ'₁, identiques ou différents, représentent un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F"₀ correspond à une fonction -COOH ;
- F₁ répond à la formule suivante :

| | |
|---|---|
| 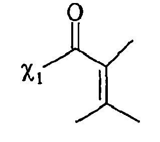 | χ₁ étant tel que défini ci-dessus, |

- F'₁ répond à la formule suivante :

| | |
|---|---|
| 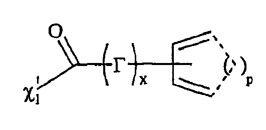 | p étant un nombre entier variant de 0 à 2, |
| | χ'₁ étant tel que défini ci-dessus, |
| | x étant égal à 0 ou 1, |
| | Γ représentant une chaîne alkyle comprenant de 1 à 30 atomes de carbone, alkaryle, aralkyle, aryle comprenant de 6 à 30 atomes de carbone, |

- F"₁ répond à la formule suivante :

| | |
|---|---|
| 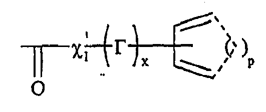 | p, x et r étant tels que définis ci-dessus, χ'₁ étant tel que défini ci-dessus, |

- F₂-F'₂ répond à la formule suivante :

| | |
|---|---|
| 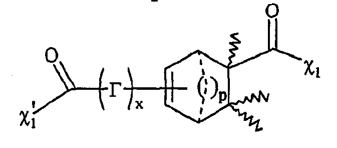 | p, χ₁, χ'₁, x et r étant tels que définis ci-dessus, |

- F₂-F"₂ répond à la formule suivante:

| | |
|---|---|
| 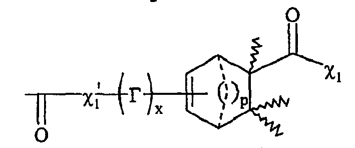 | p, χ₁, χ'₁, x et r étant tels que définis ci-dessus, |

- G répond à la formule suivante : 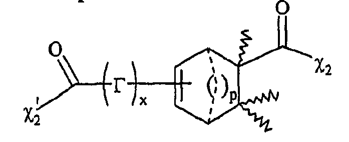
- G" répond à la formule suivante : 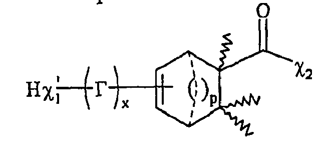 χ ₂ et χ'₂, identiques ou différents, représentent soit un groupe ORg, Rg représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone.

Dans le dernier schéma réactionnel (cas c) :
- le passage de F₀ à F₁ s'effectue par estérification ou amidation de l'acide carboxylique de formule 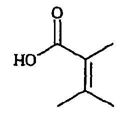
- le passage de F'₀ à F'₁ s'effectue par estérification ou amidation de l'acide carboxylique de formule 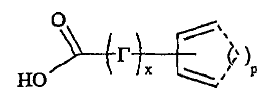 Γ , x et p étant tels que définis ci-dessus,
- le passage de F"₀ à F"₁ s'effectue par l'addition du composé de formule 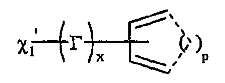 Γ , x, p et χ'₁ étant tels que définis ci-dessus.

La présente invention concerne l'utilisation telle que définie ci-dessus, pour la mise en oeuvre de réactions de couplage comme les réactions de Heck, de Suzuki, de Sonogashira ou d'Ullmann.

La présente invention concerne également l'utilisation telle que définie ci-dessus pour la mise en oeuvre de la réaction de Heck, selon l'un des schémas réactionnels suivants :

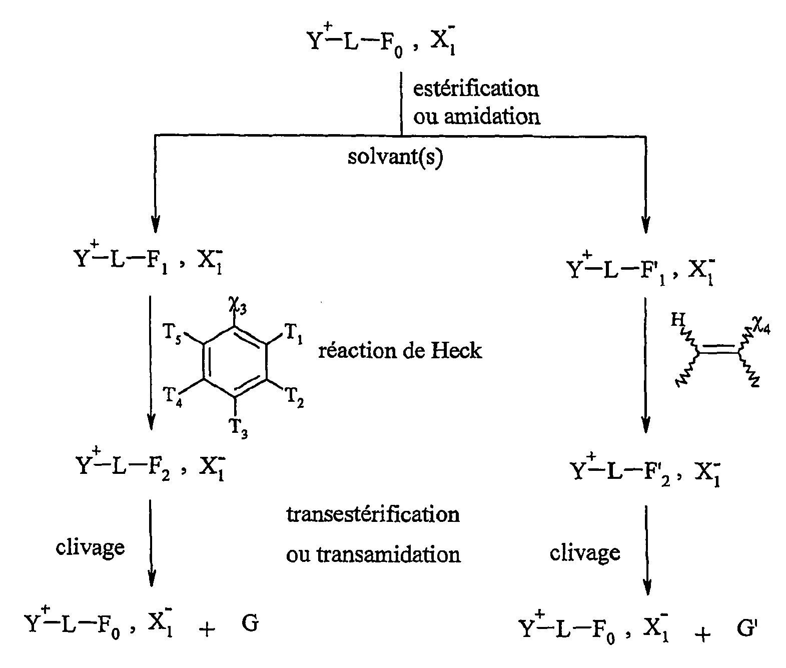

Y⁺- représentant un cation onium tel que défini ci-dessus, et étant de préférence un cation triméthylalkylammonium, triéthylalkylammonium, tributylalkylphosphonium, tricyclohexylalkylphosphonium, N-méthyl-N'-alkylimidazolium, N-alkylpyridinium, diméthylalkylsulfonium, diéthyl-alkylsulfonium,

L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 2 à 10,

X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁, F'₁, F₂ et F'₂ étant telles que définies ci-dessous :
- F₀ correspond à un groupe -χ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à l'une des formules suivantes : 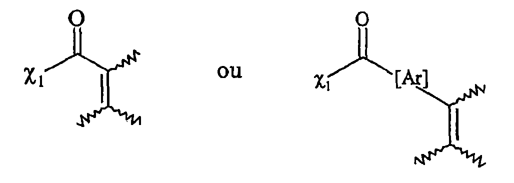 χ ₁ étant tel que défini ci-dessus,
[Ar] représentant un noyau aromatique, éventuellement substitué par un groupe alkyle, linéaire ou ramifié, comprenant de 1 à 20 atomes de carbone ou un groupe aryle comprenant de 6 à 30 atomes de carbone, ou un groupe fonctionnel notamment choisi parmi N02, CN, COOR, OR, COR, NHCOR, NRR', SO₂R, I, Br, R et R' représentant indépendamment l'un de l'autre un groupe alkyle comprenant de 1 à 20 atomes de carbone ou un groupe aryle comprenant de 6 à 30 atomes de carbone, [Ar] répondant de préférence à la formule suivante :

| | |
|---|---|
| 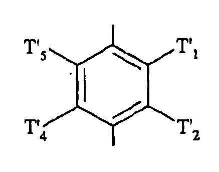 | dans laquelle T'₁, T'₂, T'₄ et T'₅ représentent indépendamment les uns des autres un atome d'hydrogène, un groupe alkyle, linéaire ou ramifié, comprenant de 1 à 20 atomes de carbone ou un groupe aryle comprenant de 6 à 30 atomes de carbone, ou un groupe fonctionnel notamment choisi parmi NO₂, CN, COOR, OR, COR, NHCOR, NRR', SO₂R, I, Br, R et R' représentant indépendamment l'un de l'autre un groupe alkyle comprenant de 1 à 20 atomes de carbone ou un groupe aryle comprenant de 6 à 30 atomes de carbone, |

- F₂ répond à l'une des formules suivantes : 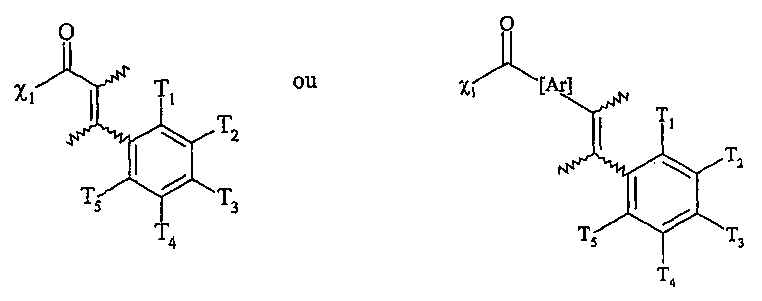 χ ₁ et Ar étant tels que définis ci-dessus,
T₁, T₂, T₃, T₄ et T₅ répondant à la définition donnée ci-dessus pour T'₁, T'₂, T'₄ et T'₅
G répondant à l'une des formules suivantes : 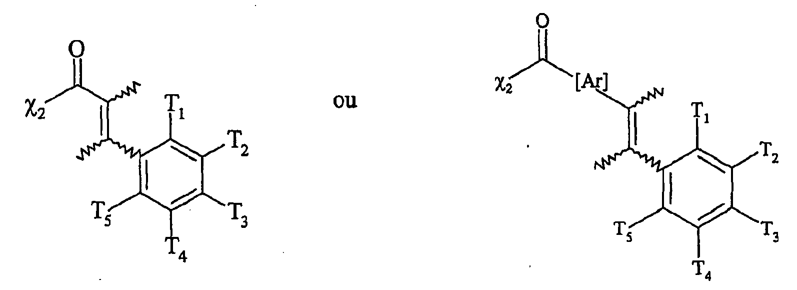 dans laquelle χ₂ représente soit un groupe -OR_{g}, Rg représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone,
χ₃ représentant un groupement partant, notamment choisi parmi les halogénures I, Cl et Br, les groupes mésylate, tosylate, triflate, sulfonate, sulfate ou phosphate,
l'entité 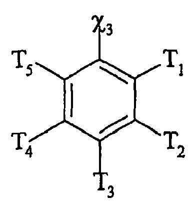 représentant notamment les groupes suivants : 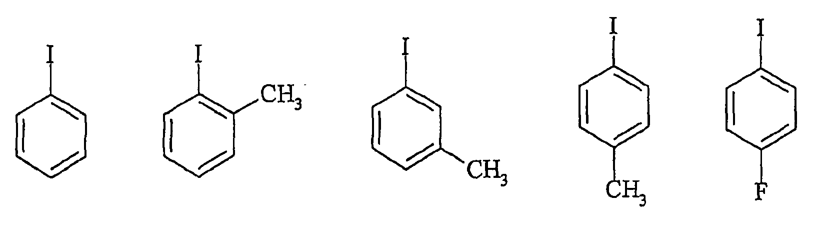 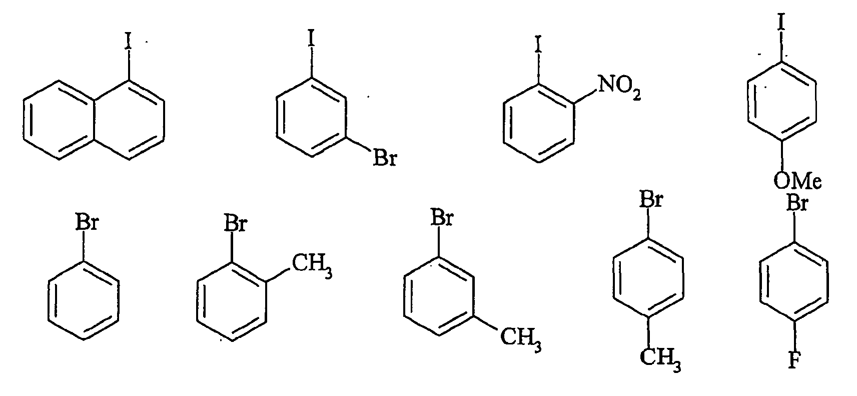 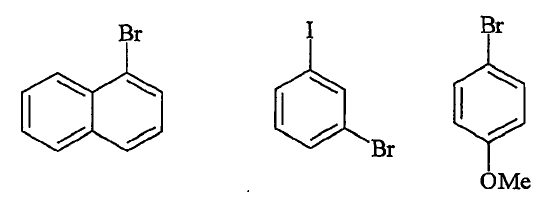
- F'₁ répond à la formule suivante :

| | |
|---|---|
| 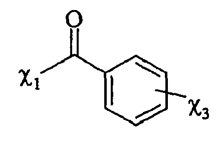 | χ₁ et χ₃ étant tels que définis ci-dessus, |

- F'₂ répond à la formule suivante :

| | |
|---|---|
| 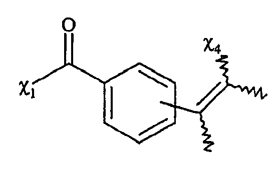 | χ₁ étant tel que défini ci-dessus, |
| | χ₄ représentant un groupe fonctionnel de type ester, amide, sulfone, phosphonate, silane, borane, ou un groupe alkyle, fonctionnel ou non, comprenant de 1 à 20 atomes de carbone, ou un groupe aryle, fonctionnel ou non, comprenant de 6 à 30 atomes de carbone, |

G' répondant à la formule suivante :

| | |
|---|---|
| 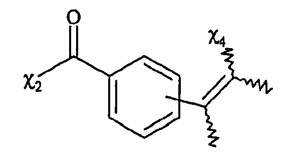 | χ₂ et χ₄ étant tels que définis ci-dessus. |

La partie gauche du schéma réactionnel ci-dessus correspond à la fixation du reste acrylique sur le support et la partie droite du schéma ci-dessus correspond à la fixation du reste arylique sur le support.

Le passage de F₀ à F₁ s'effectue par l'estérification de l'acide carboxylique de formule

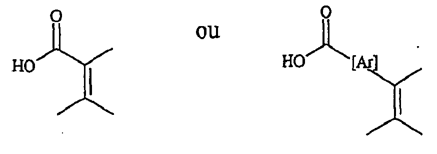

Le passage de F'₀ à F'₁ s'effectue par l'estérification de l'acide carboxylique de formule 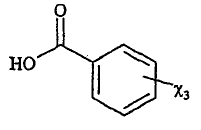 χ ₃ étant tel que défini ci-dessus.

La réaction de Heck peut être effectuée de trois façons différentes :
- en supportant la partie acrylique de la façon suivante : 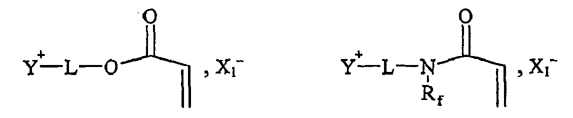 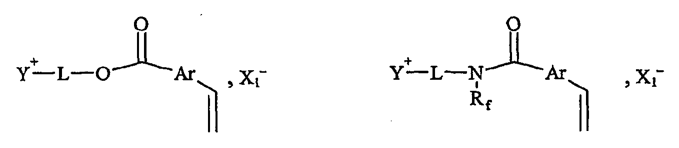 Y⁺-, X₁⁻, L, Ar et R_{f} étant tels que définis ci-dessus,
- en supportant la partie arylique de la façon suivante : 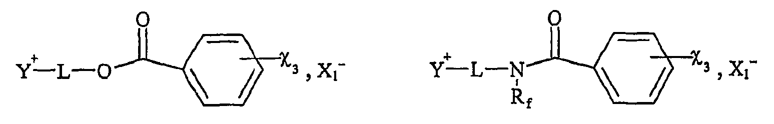 Y⁺-, X₁⁻, L, R_{f} et χ₃ étant tels que définis ci-dessus,
- en supportant les parties acrylique et arylique notamment de la façon suivante : 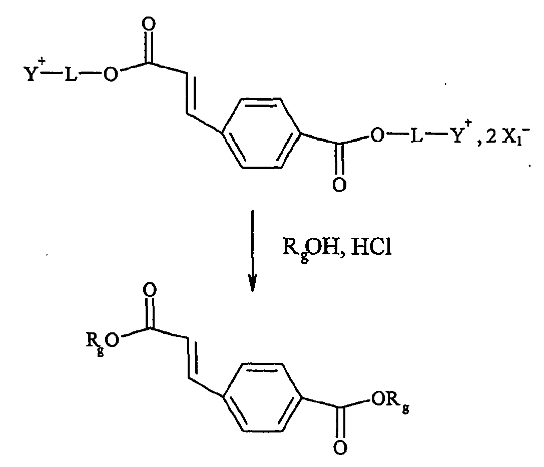 Y⁺-, X₁⁻, L et R_{g} étant tels que définis ci-dessus.

La présente invention concerne également l'utilisation telle que définie ci-dessus pour la mise en oeuvre du couplage de Suzuki, selon l'un des schémas réactionnels suivantes:
a) 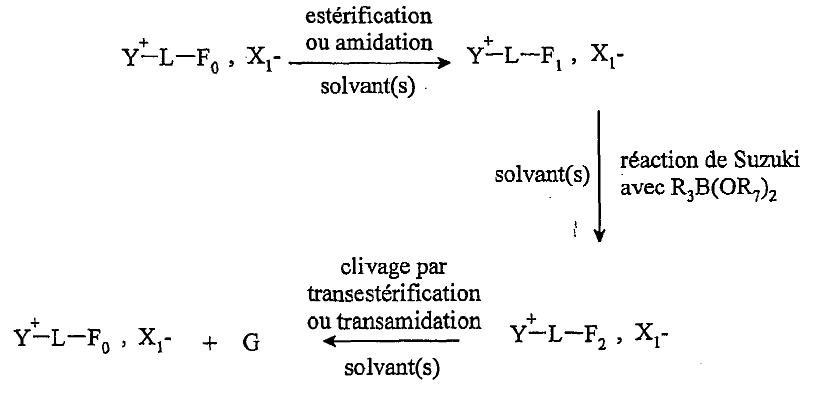 R₃ étant choisi parmi les groupes aryle, hétéroaryle, éthényle, diényle, allyle, éthynyle, substitués ou non, comprenant de 2 à 30 atomes de carbone,
   R₇ représentant un atome d'hydrogène ou un groupe alkyle, ramifié ou linéaire ou un groupe cycloalkyle comprenant de 1 à 12 atomes de carbone,
   Y⁺- représentant un cation onium tel que défini ci-dessus, et étant de préférence un cation triméthylalkylammonium, triéthylalkylammonium, tributylalkylphosphonium, N-méthylimidazolium ou pyridinium,
   L représentant un bras, notamment un groupe alkyle linéaire ou ramifié comprenant de 1 à 20 atomes de carbone, ou un groupe aralkyle éventuellement fonctionnel comprenant de 6 à 30 atomes de carbone, et étant de préférence un groupe alkyle linéaire de préférence un groupe alkyle linéaire de type (CH₂)ᵣ, r variant de 1 à 20, et de préférence de 1 à 10, X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
   le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
   les fonctions F₀, F₁ et F₂ étant telles que définies ci-dessous :
   - F₀ est de la forme -χ₁H, χ₁ représentant un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
   - F₁ est de la forme -Rₑ-χ, Rₑ représentant un groupe aromatique ou hétéroaromatique comprenant de 6 à 30 atomes de carbone, χ représentant un groupe partant choisi de préférence parmi Cl, Br, I, OTf, O-CO₂R⁵ ou OSO₃-R⁵, R⁵ représentant un groupe alkyle comprenant de 1 à 10 atomes de carbone ou un groupe aralkyle comprenant de 6 à 30 atomes de carbone, F₁ répondant de préférence à la formule suivante : 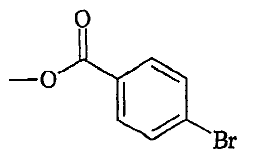
   - F₂ est de la forme -Rₑ-R₂, Rₑ étant tel que défini ci-dessus et R₂ étant choisi parmi les groupes aryle, hétéroaryle, éthényle, diényle, allyle, éthynyle, substitués ou non, comprenant de 2 à 30 atomes de carbone, F₂ répondant de préférence à la formule suivante : 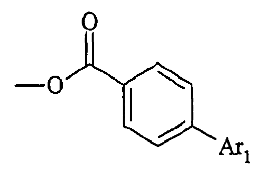
   Ar₁ représentant un groupe aromatique choisi de préférence parmi : 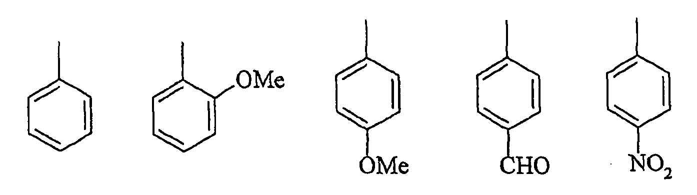 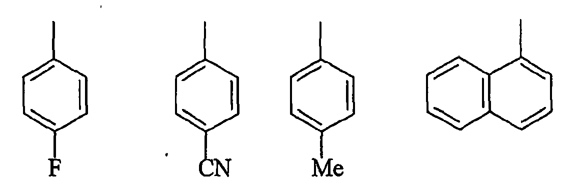 la molécule G étant de la forme R₂-R₃, R₂ et R₃ étant tels que définis ci-dessus, et répond notamment à la formule suivante : 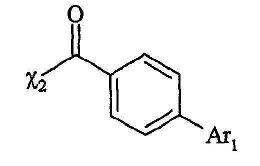 dans laquelle χ₂ représente soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone,
   Ar₁ est tel que défini ci-dessus.
   Lorsque F₀ représente un groupe -OH, on obtient la fonction F₁ par estérification notamment avec l'acide carboxylique de formule 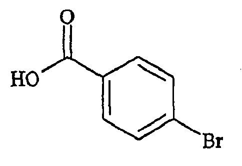
   Lorsque F₀ représente un groupe -NR_{f}H, on obtient la fonction F₁ par amidation notamment avec l'acide carboxylique de formule 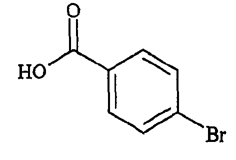
b) 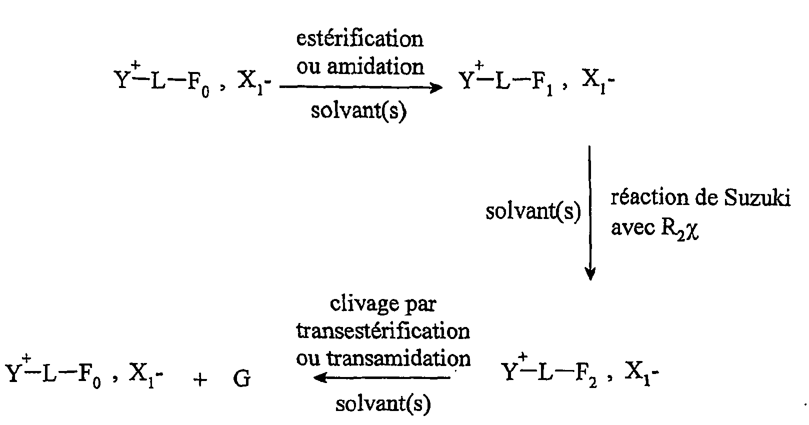 Y⁺- représentant un cation onium tel que défini ci-dessus, et étant de préférence un cation triméthylalkylammonium, triéthylalkylammonium, tributylalkylphosphonium, N-méthylimidazolium ou pyridinium,
   L représentant un bras, notamment un groupe alkyle linéaire ou ramifié comprenant de 1 à 20 atomes de carbone, ou un groupe aralkyle éventuellement fonctionnel comprenant de 6 à 30 atomes de carbone, et étant de préférence un groupe alkyle linéaire de préférence un groupe alkyle linéaire de type (CH₂)ᵣ, r variant de 1 à 20, et de préférence de 1 à 10,
   X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
   le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
   R₂ étant choisi parmi les groupes aryle, hétéroaryle, éthényle, diényle, allyle, éthynyle, substitués ou non, comprenant de 2 à 30 atomes de carbone,
   les fonctions F₀, F₁ et F₂ étant telles que définies ci-dessous :
   - F₀ est de la forme -χ₁H, χ₁ étant tel que défini ci-dessus,
   - F₁ est de la forme -R_{q}-B(OR₇)₂, R₇ étant tel que défini ci-dessus, et Rq correspondant à un groupe aryle comprenant de 6 à 30 atomes de carbone, hétéroaryle comprenant de 4 à 20 atomes de carbone, éthényle comprenant de 2 à 20 atomes de carbone, diényle comprenant de 3 à 20 atomes de carbone, allyle comprenant de 3 à 20 atomes de carbone, éthynyle comprenant de 2 à 20 atomes de carbone, substitués ou non, F₁ répondant de préférence à la formule suivante : 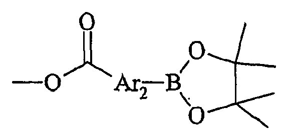
      Ar₂ correspondant à un groupe aryle substitué ou non comprenant de 6 à 30 atomes de carbone,
   - F₂ est de la forme -R_{q}-Rₑ, R_{q} et Rₑ étant tels que définis ci-dessus, F₂ répondant de préférence à la formule suivante : 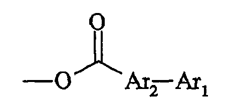 Ar₁ représentant un groupe aromatique choisi de préférence parmi : 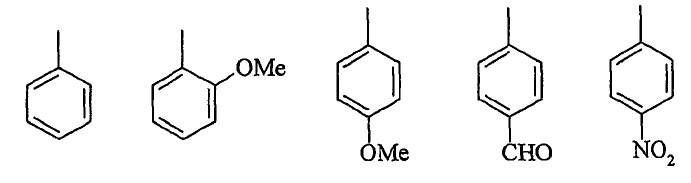 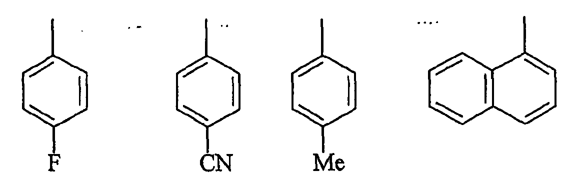
   la molécule G étant de la forme R₂-R₃, R₂ et R₃ étant tels que définis ci-dessus, et répondant notamment à la formule suivante : 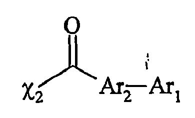 dans laquelle χ₂, Ar₁ et Ar₂ sont tels que définis ci-dessus,
c) 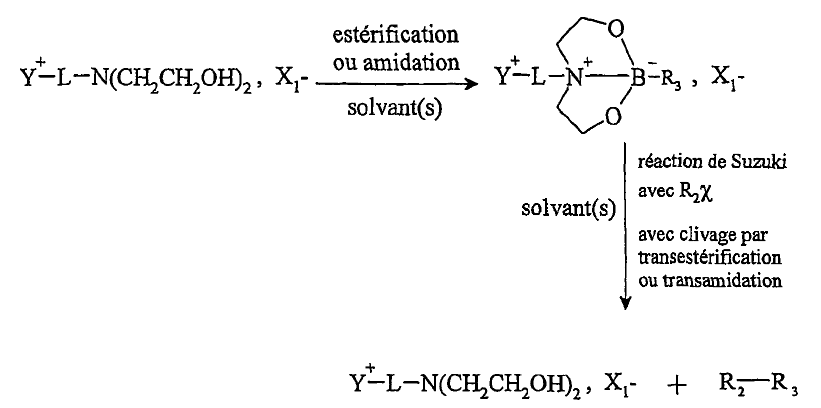 Y⁺-, L, X₁⁻, R₂ et R₃ étant tels que définis ci-dessus,
   R₃ étant de préférence un groupe phényle,
   le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants.

Le mode de réalisation a) correspond au cas où l'halogénure d'aryle est supporté et où l'acide boronique est libre.

Le mode de réalisation b) correspond au cas où l'acide boronique est supporté et où l'halogénure d'aryle est libre.

Les modes de réalisation a), b) et c) permettent de purifier facilement les produits de couplage sous forme de sels. En particulier, il est facile d'éliminer les produits d'homocouplage qui ne sont pas des sels par simple lavage avant transestérification.

Les avantages de ces différents modes de réalisation sont les suivants :
- les réactions sur support soluble se prêtent aux techniques de la chimie combinatoire et de la synthèse parallèle ;
- ces supports sont faciles à purifier car ce sont des sels insolubles dans un certain nombre de solvants ; ils peuvent donc être lavés et/ou recristallisés ; en particulier, dans le cas de la réaction de Suzuki, le produit d'homocouplage peut être facilement éliminé par simple lavage du sel avant la transestérification.

La présente invention concerne également l'utilisation telle que définie ci-dessus, pour la mise en oeuvre du couplage de Sonogashira, selon l'un des schémas réactionnels suivants:
a) 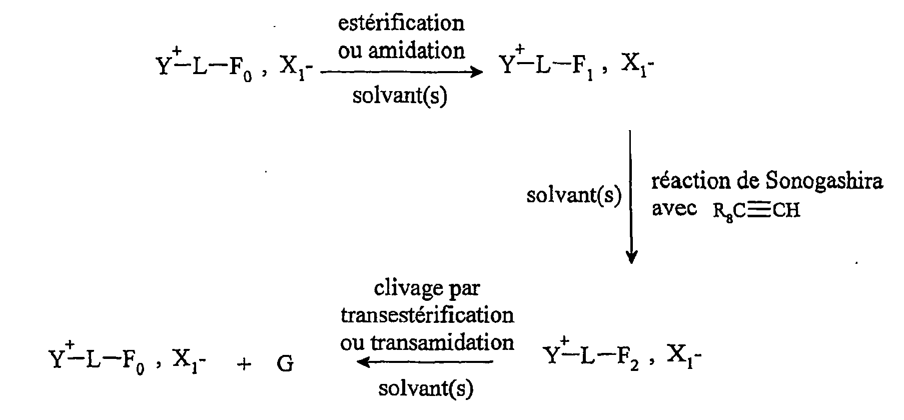 Y⁺- représentant un cation onium tel que défini ci-dessus, et étant de préférence un cation triméthylalkylammonium, triéthylalkylammonium, tributylalkylphosphonium, N-méthylimidazolium ou pyridinium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
R₈ représentant un groupe ORₕ, NRₕRᵤ, CORₕ, CN, SO₂Rₕ, SRₕ, un groupe alcényle, éthynyle, diényle, Rₕ et Rᵤ représentant, indépendamment l'un de l'autre, un atome d'hydrogène, un groupe alkyle comprenant de 1 à 20 atomes de carbone ou un groupe aryle comprenant de 6 à 30 atomes de carbone,
ou R₈ représentant un groupe alkyle, ramifié ou linéaire, éventuellement fonctionnel, comprenant de 1 à 20 atomes de carbone, ou un groupe aryle, ou un groupe alkaryle ou aralkyle, comprenant de 6 à 30 atomes de carbone, substitué ou non, lesdits groupes alkyle ou aryle pouvant être substitués par l'un des groupes fonctionnels suivants : un atome d'halogène, notamment Cl, un groupe ORₕ, NRₕRᵤ, CORₕ, CN, SO₂Rₕ, SRₕ, un groupe alcényle, éthynyle, diényle, vinyle, alcynyle, Rₕ et Rᵤ étant tels que définis précédemment,
R₈ étant notamment l'un des groupes suivants :

-(CH₂)ₛ-CH₃, -(CH₂)ₛ-CH₂OH, -(CH₂)ₛ-CH₂OMe,

s représentant un nombre entier compris entre 0 et 10, 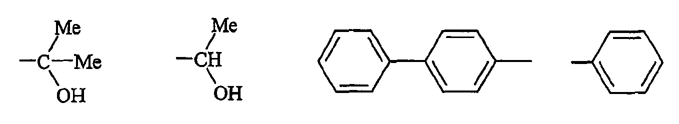 Me₃Si-
les fonctions F₀, F₁ et F₂ étant telles que définies ci-dessous :
- F₀ correspond à un groupe -χ₁H, dans lequel χ₁ représente un atome d'oxygène
ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante :

| | |
|---|---|
| 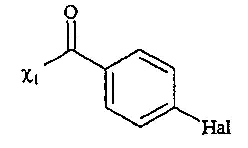 | χ₁ étant tel que défini ci-dessus, et Hal représentant un halogène, et étant de préférence l'iode, |

- F₂ répond à la formule suivante :

| | |
|---|---|
| 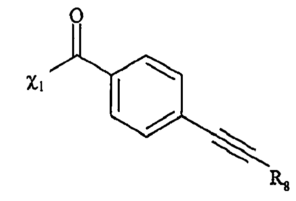 | χ₁ et R₈ étant tels que définis ci-dessus, |

G répondant à la formule suivante : 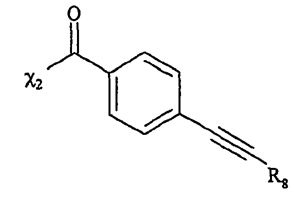 dans laquelle χ₂ représente soit un groupe -OR_{g}, Rg représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone, χ₂ représentant notamment un groupe OMe, OEt, OPr ou OBu.
La transformation de la fonction F₀ en F₁ s'effectue par une réaction d'estérification ou d'amidation avec l'acide carboxylique de formule : 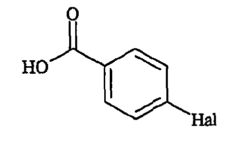
b) 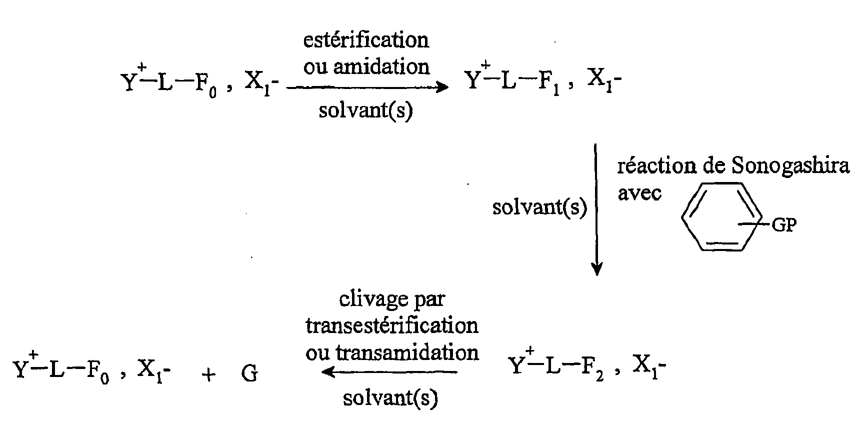 Y⁺- représentant un cation onium tel que défini ci-dessus, et étant de préférence un cation triméthylalkylammonium, triéthylalkylammonium, tributylalkylphosphonium, N-méthylimidazolium, alkylpyridinium, diméthylalkylsulfonium ou diéthylalkylsulfonium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
GP représentant un groupe partant, et étant notamment Cl, Br, I ou OTf,
les fonctions F₀, F₁ et F₂ étant telles que définies ci-dessous :
- F₀ correspond à un groupe -COOH,
- F₁ répond à la formule suivante : 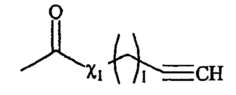 dans laquelle 1 représente un nombre entier variant de 1 à 20, et χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₂ répond à la formule suivante :

| | |
|---|---|
| 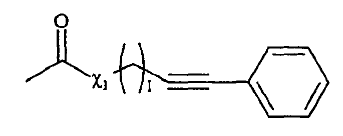 | χ₁ et 1 étant tels que définis ci-dessus, |

G répondant à la formule suivante : 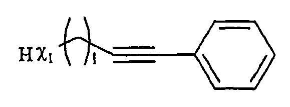 dans laquelle χ₁ et 1 sont tels que définis ci-dessus.

Le mode de réalisation a) correspond au cas où l'aromatique est supporté et où l'acétylénique est libre.

Le mode de réalisation b) correspond au cas où l'acétylénique est supporté et où l'aromatique est libre.

La présente invention concerne également l'utilisation telle que définie ci-dessus, pour la mise en oeuvre de la réaction de Baylis-Hilman, selon l'un des schémas réactionnels suivants :
a) 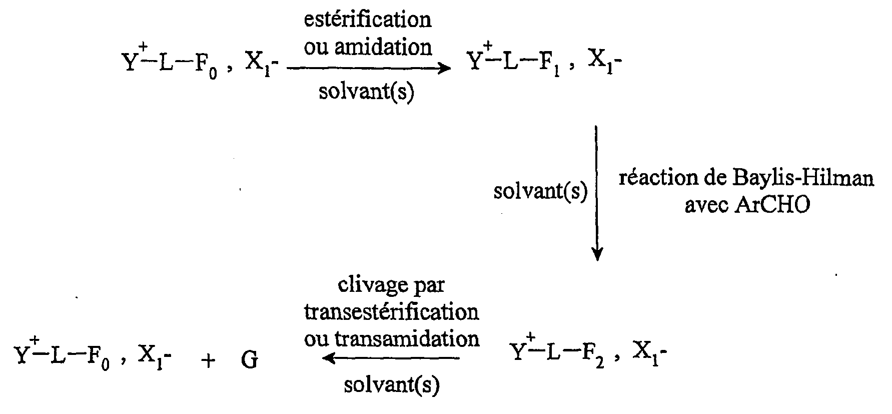 Y⁺- représentant un cation onium tel que défini ci-dessus, et étant de préférence un cation triméthylalkylammonium, triéthylalkylammonium, tributylalkylphosphonium, N-méthylimidazolium ou pyridinium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁ et F₂ étant telles que définies ci-dessous :
- F₀ représente un groupe -OH,
- F₁ répond à la formule suivante : 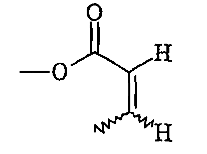
- F₂ répond à la formule suivante : 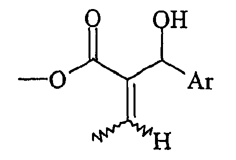
G répondant à la formule suivante :

| | |
|---|---|
| 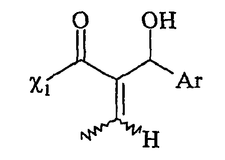 | χ₁ représentant un groupe -OH, ou un groupe -OR_{g}, R_{g} représentant un groupe alkyle, linéaire ou ramifié, comprenant de 1 à 20 atomes de carbone, |

Ar représentant un groupe aromatique ou hétéroaromatique, substitué ou non,
ArCHO étant notamment choisi parmi : 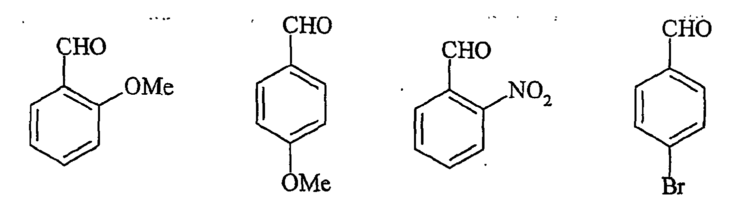 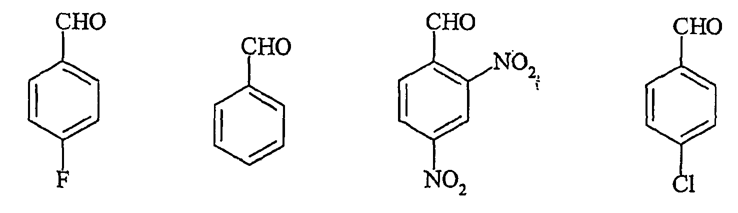
La transformation de la fonction F₀ en F₁ s'effectue par une réaction d'estérification ou d'amidation avec l'acide carboxylique de formule : 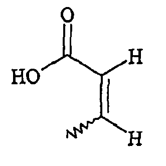
b) 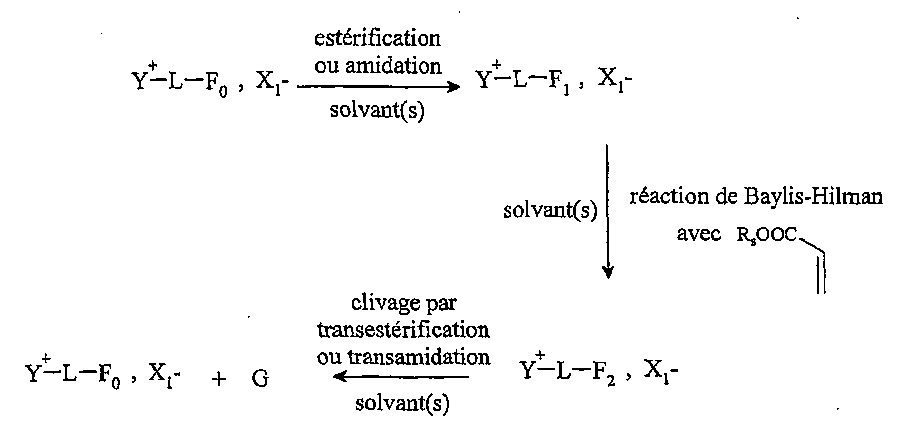 Y⁺- représentant un cation onium tel que défini ci-dessus, et étant de préférence un cation triméthylalkylammonium, triéthylalkylammonium, tributylalkylphosphonium, N-méthylimidazolium, alkylpyridinium, diméthylalkylsulfonium ou diéthylalkylsulfonium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
Rₛ représentant un atome d'hydrogène ou un groupe alkyle comprenant de 1 à 20 atomes de carbone ou aralkyle ou alkaryle comprenant de 7 à 30 atomes de carbone,
les fonctions F₀, F₁ et F₂ étant telles que définies ci-dessous :
- F₀ correspond à un groupe -χ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante :

| | |
|---|---|
| 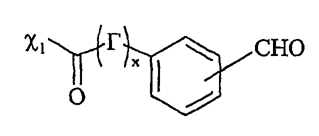 | χ₁ étant tel que défini ci-dessus, |
| | x étant égal à 0 ou 1, |
| | Γ représentant une chaîne alkyle comprenant de 1 à 20 atomes de carbone, alkaryle, aralkyle comprenant de 6 à 30 atomes de carbone, |

- F₂ répond à la formule suivante :

| | |
|---|---|
| 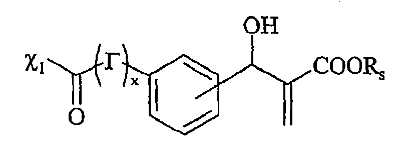 | χ₁, x, Rₛ et Γ étant tels que définis ci-dessus |

- G répondant à la formule suivante :

| | |
|---|---|
| 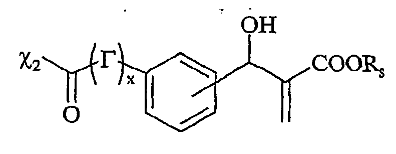 | χ₂, x, Rₛ et et Γ étant tels que définis ci- dessus |

dans laquelle χ₂ représente soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone, χ₂ représentant notamment un groupe OMe, OEt, OPr ou OBu.
c) 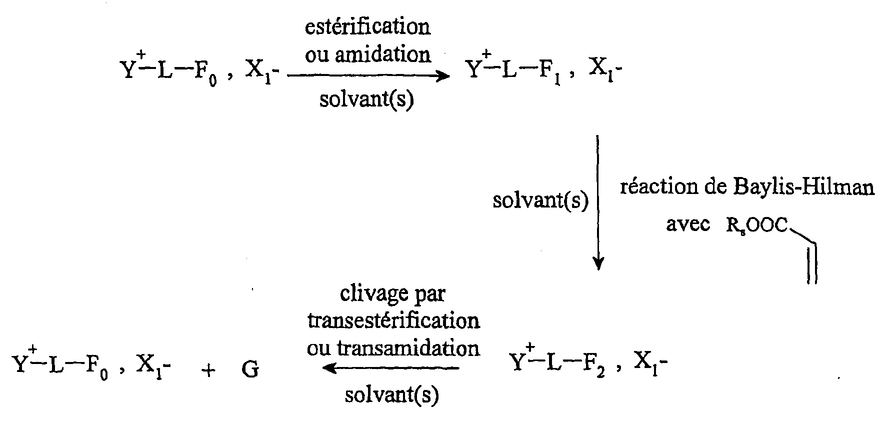 Y⁺- représentant un cation onium tel que défini ci-dessus, et étant de préférence un cation triméthylalkylammonium, triéthylalkylammonium, tributylalkylphosphonium, N-méthylimidazolium ou pyridinium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
Rₛ représentant un atome d'hydrogène ou un groupe alkyle comprenant de 1 à 20 atomes de carbone ou aralkyle ou alkaryle comprenant de 7 à 30 atomes de carbone,
les fonctions F₀, F₁ et F₂ étant telles que définies ci-dessous :
- F₀ correspond à un groupe -COχ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante : 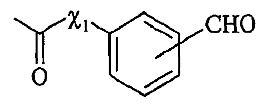
- F₂ répond à la formule suivante : 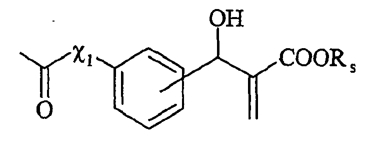
- G répondant à la formule suivante :

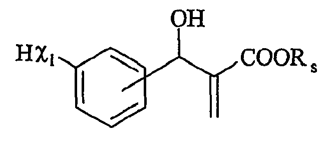

La présente invention concerne également l'utilisation telle que définie ci-dessus, pour la synthèse, éventuellement asymétrique, d'α-aminoacides, selon le schéma réactionnel suivant : 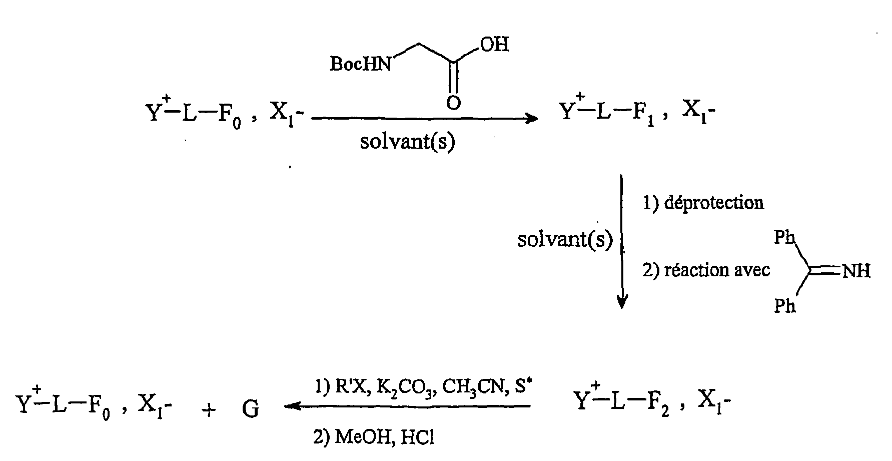 Y⁺- représentant un cation onium tel que défini ci-dessus, et étant de préférence un cation triméthylalkylammonium, triéthylalkylammonium ou tributylalkylphosphonium,
L représentant un bras, notamment un groupe alkyle linéaire ou ramifié comprenant de 1 à 20 atomes de carbone, ou un groupe aralkyle éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 3 à 6,
X₁⁻ étant tel que défini ci-dessus, et étant notamment Cl⁻, Br⁻, I⁻, ⁻N(SO₂CF₃)₂, BF₄⁻, PF₆⁻,
le ou les solvants étant choisis parmi : l'acétonitrile, le dichlorométhane, le tétrahydrofuranne, le dioxane, le toluène, le chlorobenzène ou un mélange de ces solvants,
R' représentant un groupe alkyle, linéaire ou ramifié, comprenant de 1 à 30 atomes de carbone, éventuellement fonctionnel,
S* représentant un agent chiral de transfert de phase tel que le bromure de O(9)-allyl-N-9-anthracényl-méthylcinchonidinium (voir Corey et al., 1998),
les fonctions F₀, F₁ et F₂ étant telles que définies ci-dessous :
- F₀ correspond à -OH,
- F₁ répond à la formule suivante : 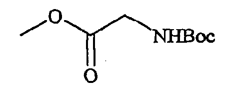
- F₂ répond à la formule suivante : 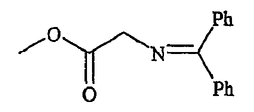
G répondant à la formule suivante :

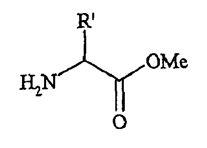

La présente invention concerne également l'utilisation telle que définie ci-dessus, pour la mise en oeuvre de réactions multi-composants.

Les réactions multi-composants (RMC) mettent en présence simultanément au moins trois partenaires dans des conditions expérimentales qui ne varient pas au cours du temps et permettent la création de plusieurs liaisons covalentes en cascade dans un seul réacteur, à la différence des réactions classiques ou deux réactifs conduisent à un produit par création d'une nouvelle liaison. Ainsi il est possible d'accéder en une seule étape à une molécule hautement fonctionnalisée à partir d'entités relativement simples. De plus les RMC allient convergence et économie d'atomes, deux principes essentiels en synthèse organique mais aussi en chimie combinatoire. Enfin, ces réactions ont généralement lieu avec un rendement élevé, puisqu'elles évitent la succession d'étapes des synthèses linéaires ou multiétapes qui, font, à chaque pas, chuter le rendement.

Les RMC les plus connues et les plus développées sont celles de Passérini et de Ugi (Ugi et al., 1999 ; Ugi et al., 2001 ; Domling et al., 2000 ; Ugi, 2001 ; Bienayme et al., 2000 ; Vanden Eynde et al., 2000 ; Domling, 2002).

La présente invention concerne également l'utilisation telle que définie ci-dessus, pour la mise en oeuvre de réactions multi-composants de type UGI, notamment pour la réaction de type Grieco selon l'un des schémas réactionnels suivants :
a) 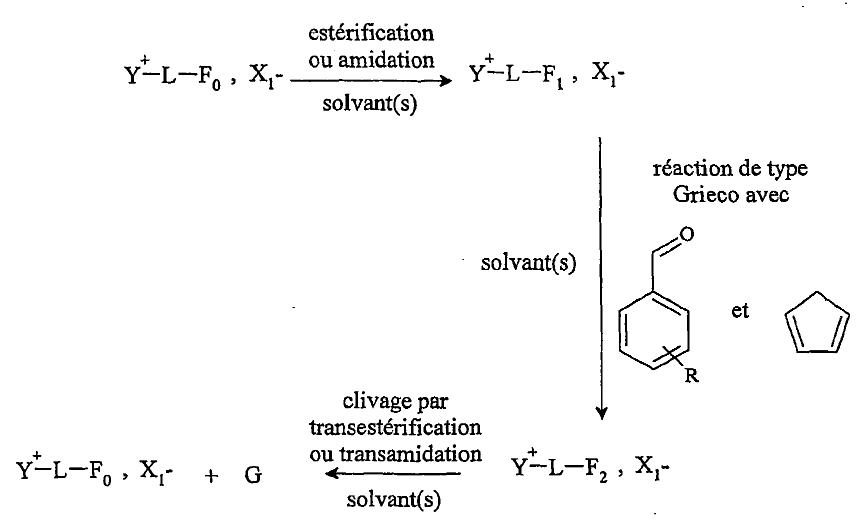 Y⁺- représentant un cation onium tel que défini ci-dessus, et étant de préférence un cation triméthylalkylammonium, triéthylalkylammonium, tributylalkylphosphonium, N-méthyl-N'-alkylimidazolium, N-alkylpyridinium, diméthylalkylsulfonium ou diéthylalkylsulfonium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
R représentant un atome d'hydrogène, un groupe nitro, de préférence en position para, un atome de chlore, de préférence en position para ou un groupe méthoxy, de préférence en position para,
les fonctions F₀, F₁ et F₂ étant telles que définies ci-dessous :
- F₀ représente un groupe -OH,
- F₁ répond à la formule suivante : 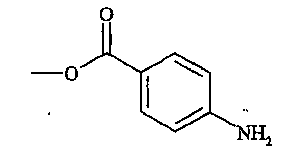
- F₂ répond à la formule suivante : 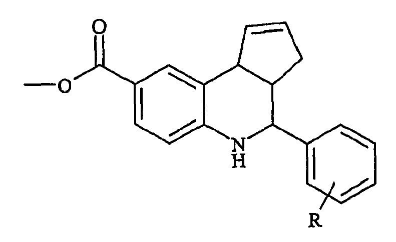
G répondant à la formule suivante :

| | |
|---|---|
| 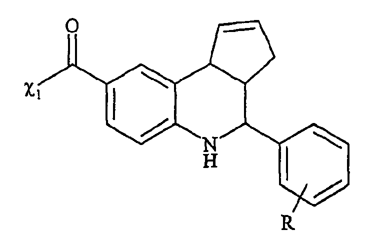 | χ₁ représentant un groupe -OH, ou un groupe -OR_{g}, R_{g} représentant un groupe alkyle, linéaire ou ramifié, comprenant de 1 à 20 atomes de carbone, |

b) 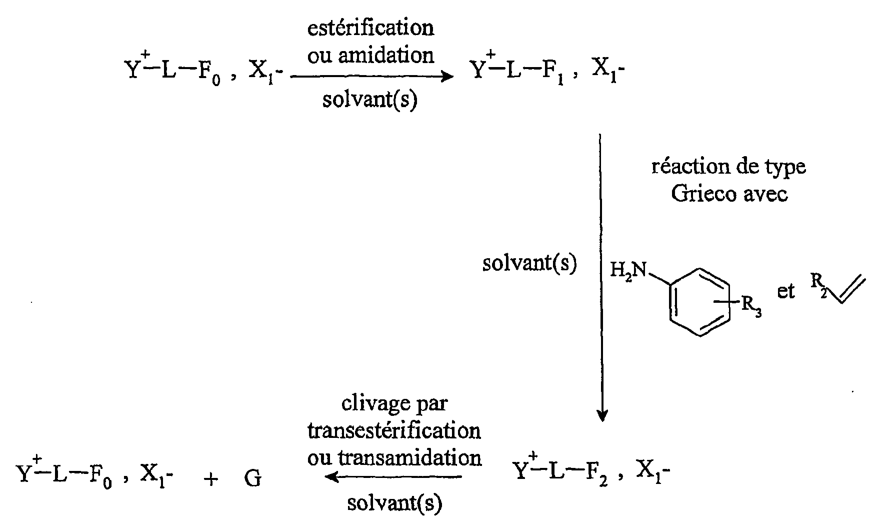 Y⁺- représentant un cation onium tel que défini ci-dessus, et étant de préférence un cation triméthylalkylammonium, triéthylalkylammonium, tributylalkylphosphonium, N-méthyl-N'-alkylimidazolium, N-alkylpyridinium, diméthylalkylsulfonium ou diéthylalkylsulfonium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
R₂ représentant un groupe alkyle, fonctionnel ou non, comprenant de 1 à 20 atomes de carbone, ou un groupe aryle, fonctionnel ou non, comprenant de 6 à 30 atomes de carbone, ou un groupe aralkyle ou alkaryle, fonctionnel ou non, comprenant de 7 à 50 atomes de carbone,
R₃ représentant un atome d'hydrogène, un groupe alkyle, linéaire ou ramifié, comprenant de 1 à 20 atomes de carbone ou un groupe aryle comprenant de 6 à 30 atomes de carbone, ou un groupe aralkyle ou alkaryle, fonctionnel ou non, comprenant de 7 à 50 atomes de carbone, ou un groupe fonctionnel notamment choisi parmi NO₂, CN, COOR, OR, COR, NHCOR, NRR', SO₂R, I, Br, R et R' représentant indépendamment l'un de l'autre un groupe alkyle comprenant de 1 à 20 atomes de carbone ou un groupe aryle comprenant de 6 à 30 atomes de carbone,
les fonctions F₀, F₁ et F₂ étant telles que définies ci-dessous :
- F₀ représente un groupe -OH,
- F₁ répond à la formule suivante : 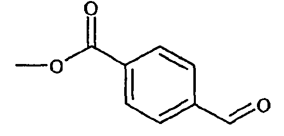
- F₂ répond à la formule suivante : 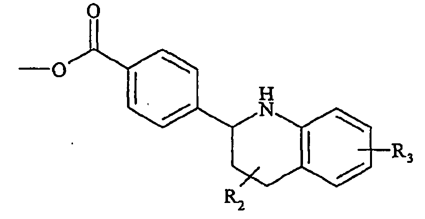
G répondant à la formule suivante :

| | |
|---|---|
| 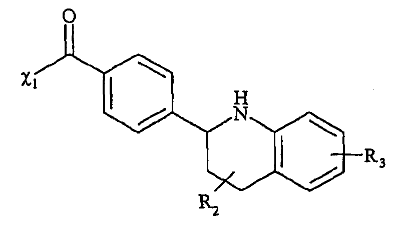 | χ₁ représentant un groupe -OH, ou un groupe -OR_{g}, R_{g} représentant un groupe alkyle, linéaire ou ramifié, comprenant de 1 à 20 atomes de carbone. |

c) 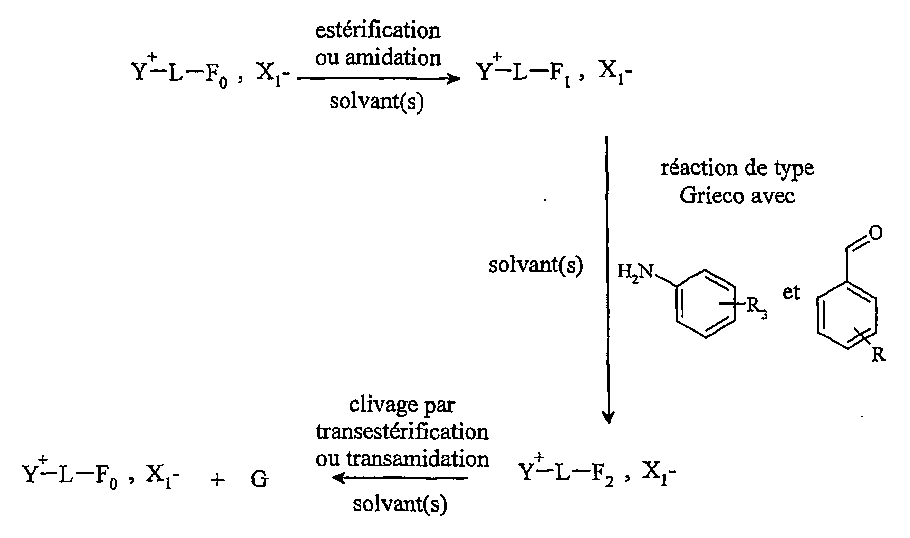 Y⁺- représentant un cation onium tel que défini ci-dessus, et étant de préférence un cation triméthylalkylammonium, triéthylalkylammonium, tributylalkylphosphonium, N-méthyl-N'-alkylimidazolium, N-alkylpyridinium, diméthylalkylsulfonium ou diéthylalkylsulfonium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, -N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
R représentant un atome d'hydrogène ou un groupe fonctionnel tel qu'un groupe nitro en position para, un atome de chlore en para ou un groupe méthoxy en position ortho, ou un groupe alkyle, fonctionnel ou non, comprenant de 1 à 20 atomes de carbone, ou un groupe aryle, fonctionnel ou non, comprenant de 6 à 30 atomes de carbone, ou un groupe aralkyle ou alkaryle, fonctionnel ou non, comprenant de 7 à 50 atomes de carbone,
R₃ représentant un atome d'hydrogène, un groupe alkyle, linéaire ou ramifié, comprenant de 1 à 20 atomes de carbone ou un groupe aryle comprenant de 6 à 30 atomes de carbone, ou un groupe aralkyle ou alkaryle, fonctionnel ou non, comprenant de 7 à 50 atomes de carbone, ou un groupe fonctionnel notamment choisi parmi NO₂, CN, COOR, OR, COR, NHCOR, NRR', SO₂R, I, Br, R et R' représentant indépendamment l'un de l'autre un groupe alkyle comprenant de 1 à 20 atomes de carbone ou un groupe aryle comprenant de 6 à 30 atomes de carbone,
les fonctions F₀, F₁ et F₂ étant telles que définies ci-dessous :
- F₀ représente toute fonction permettant d'accrocher et de décrocher un reste portant une oléfine, de préférence un ester, ou un amide.
- F₁ répond à l'une des formules générales suivantes :

| | |
|---|---|
| 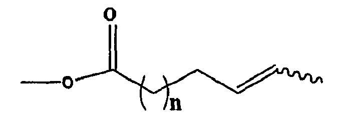 | n représentant un nombre entier variant de 1 à 10 |
| 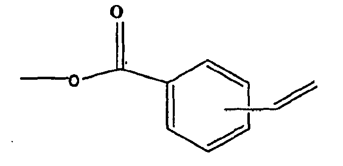 | |

- F₂ répond à l'une des formules générales suivantes : 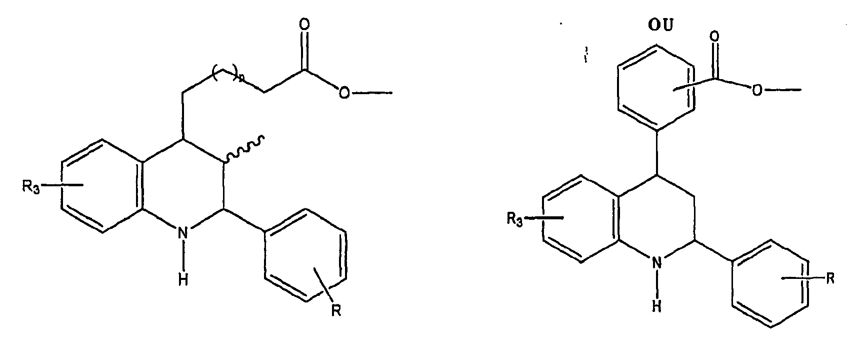 G répondant à l'une des formules générales suivantes : 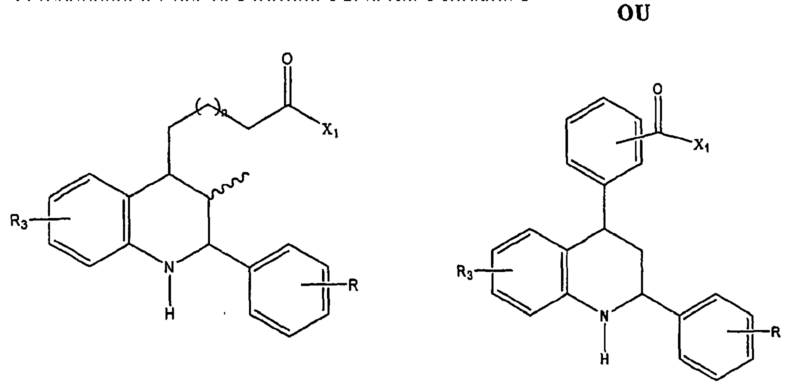 n, R et R₃ étant tels que définis ci-dessus, et
χ₁ représentant un groupe -OH, ou un groupe -OR_{g}, R_{g} représentant un groupe alkyle, linéaire ou ramifié, comprenant de 1 à 20 atomes de carbone.

La transformation de la fonction F₀ en F₁, dans le premier cas de figure, s'effectue par une réaction d'estérification avec l'acide carboxylique de formule :

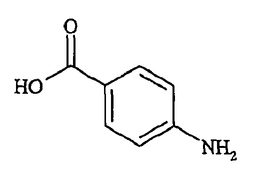

La présente invention concerne également l'utilisation telle que définie ci-dessus, pour la mise en oeuvre de réactions multi-composants, notamment pour la synthèse d'oléfines tétrasubstituées selon R.C. Larock et al. (2003), selon le schéma réactionnel suivant : 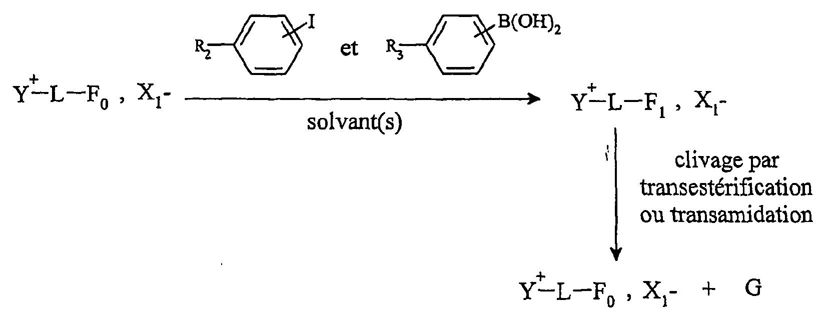 Y⁺- représentant un cation onium tel que défini ci-dessus, et étant de préférence un cation triméthylalkylammonium, triéthylalkylammonium, tributylalkylphosphonium, N-méthyl-N'-alkylimidazolium, N-alkylpyridinium, diméthylalkylsulfonium ou diéthylalkylsulfonium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
R₂ et R₃, de préférence en para, représentant un atome d'hydrogène, un groupe alkyle, linéaire ou ramifié, éventuellement fonctionnel, comprenant de 1 à 30 atomes de carbone, un groupe aryle, éventuellement substitué et/ou fonctionnel, comprenant de 6 à 30 atomes de carbone, un groupe fonctionnel, de préférence un groupe méthoxy, mono-alkylamino, dialkylamino, arylamino, cyano, ester, nitro, cétone, sulfonyle, alkylthio, sulfoxyde,
les fonctions F₀ et F₁ étant telles que définies ci-dessous :
- F₀ répond à la formule suivante :

| | |
|---|---|
| 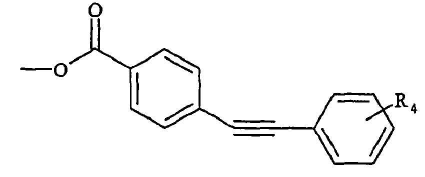 | R₄ représentant un groupe tel que défini pour R₂ et R₃ ci-dessus, |

- F₁ répond à l'une des formules suivantes : 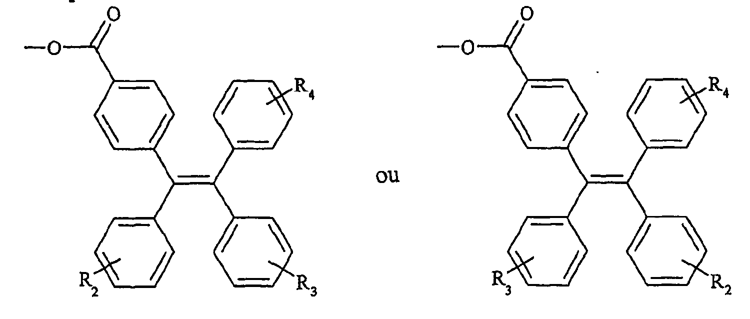 R₂, R₃ et R₄ étant tels que définis ci-dessus,
G répond à l'une des formules suivantes : 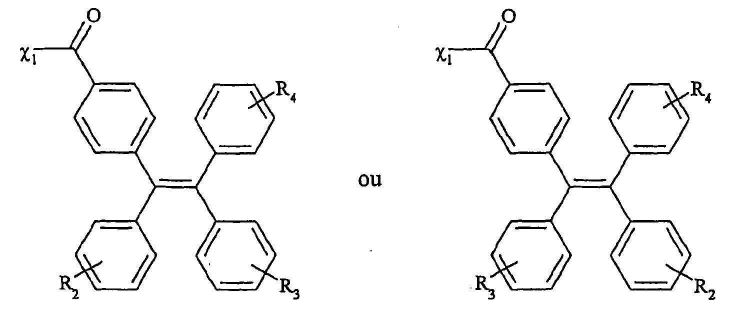 χ ₁ représentant un groupe -OH, ou un groupe -OR_{g}, R_{g} représentant un groupe alkyle, linéaire ou ramifié, comprenant de 1 à 20 atomes de carbone.

La transformation de F₀ en F₁ s'effectue par une cis-addition du groupement aryle provenant de l'halogénure d'aryle du côté le moins encombré ou à l'extrémité la plus riche en électrons de l'alcyne de départ, tandis que le groupement acyle provenant de l'acide arylboronique s'additionne à l'autre extrémité.

Dans le cadre de la présente invention, il est également possible d'utiliser des cations polyfonctionnels, que l'on définit comme étant des cations portant plusieurs fonctions, lesdites fonctions étant identiques ou différentes.

La présente invention concerne également l'utilisation telle que définie ci-dessus, pour la mise en oeuvre de réactions de cycloaddition, de préférence pour la mise en oeuvre de la réaction de Diels-Alder, selon le schéma réactionnel suivant : 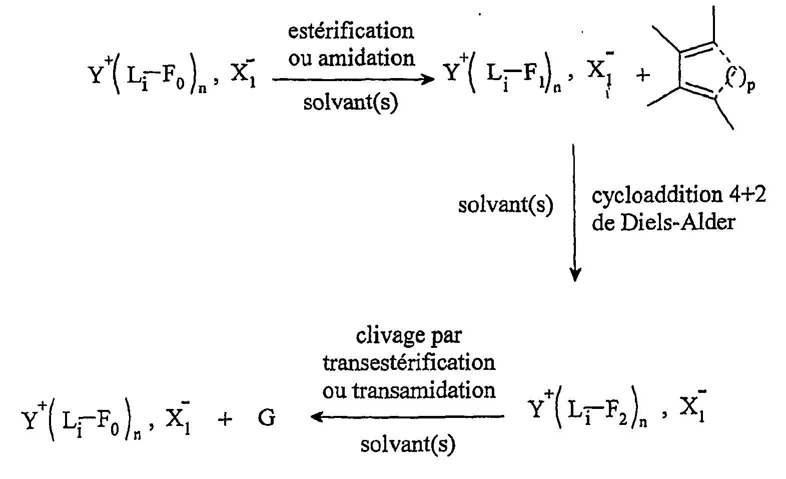 n étant un nombre entier variant de 2 à 4, tel que défini ci-dessous,
i étant un nombre entier variant de 1 à n,
p étant un nombre entier variant de 0 à 2,
Y⁺ représentant un cation onium tel que défini ci-dessus, de formule (R_{b})ₓ₋ₙΛ⁺ dans laquelle x représente un nombre entier égal à 3 ou 4, n étant égal à 2, 3 ou 4 lorsque x est égal à 4 et n étant égal à 2 ou 3 lorsque x est égal à 3, R_{b} représente un groupe alkyle comprenant de 1 à 20 atomes de carbone, un groupe aryle comprenant de 6 à 30 atomes de carbone ou un groupe aralkyle ou alkaryle comprenant de 6 à 30 atomes de carbone, lesdits groupes alkyle, aryle, aralkyle ou alkaryle susmentionnés étant non fonctionnels, et dans laquelle Λ⁺ représente un cation ammonium, imidazolium, phosphonium ou sulfonium, Y⁺ représentant notamment un cation alkylammonium, alkylphosphonium ou alkylsulfonium, et étant de préférence un cation tétraalkylammonium, tétraalkylphosphonium, dialkylimidazolium, trialkylsulfonium,
Lᵢ représentant un bras, notamment un groupe alkyle linéaire ou ramifié comprenant de 1 à 20 atomes de carbone, ou un groupe aralkyle ou alkaryle éventuellement fonctionnel, comprenant de 6 à 30 atomes de carbone, et étant de préférence un groupe alkyle linéaire de préférence un groupe alkyle linéaire de type (CH₂)ᵣ, r variant de 1 à 20, et de préférence de 2 à 10, les bras Lᵢ pouvant être identiques ou différents,
X₁⁻ étant tel que défini ci-dessus, et étant notamment Cl⁻, Br⁻, I⁻, CF₃CO₂⁻, CH₃CO₂⁻_{,} BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁ et F₂ étant telles que définies ci-dessous :
- F₀ correspond à un groupe -χ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante :

| | |
|---|---|
| 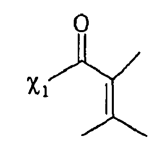 | χ₁ étant tel que défini ci-dessus, |

- F₂ répond à la formule suivante :

| | |
|---|---|
| 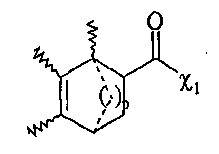 | χ₁ étant tel que défini ci-dessus, |

G répondant à la formule suivante : 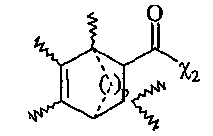 dans laquelle χ₂ représente soit un groupe OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone.

La notation Y⁺(Lᵢ-F₀)ₙ signifie que l'entité Y⁺ est substituée par n entités Lᵢ-F₀ reliées par liaison covalente.

L'utilisation d'un sel plurifonctionnel dans le cadre de la réaction de Diels-Alder permet d'augmenter la productivité des solutions utilisées. Par ailleurs, les bras peuvent être fonctionnalisés différemment et permettre soit des réactions intramoléculaires, soit des réactions en cascades, soit des réactions multicomposants.

La présente invention concerne l'utilisation telle que définie ci-dessus, pour la mise en oeuvre de la réaction de Heck, selon le schéma réactionnel suivant : 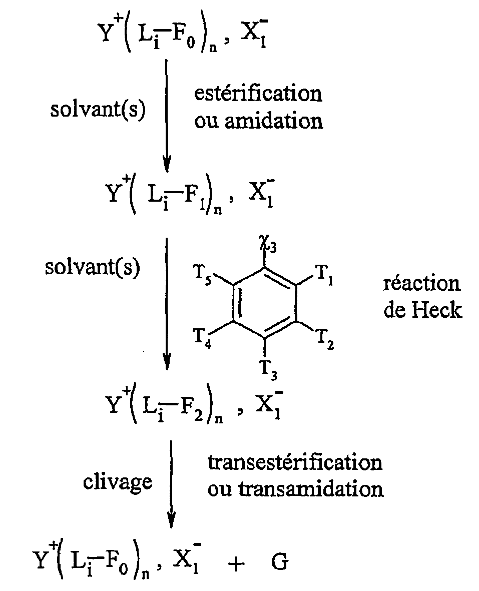 n étant un nombre entier variant de 2 à 4, tel que défini ci-dessous,
i étant un nombre entier variant de 1 à n,
Y⁺ représentant un cation onium tel que défini ci-dessus, de formule (R_{b})ₓ₋ₙΛ⁺ dans laquelle x représente un nombre entier égal à 3 ou 4, n étant égal à 2, 3 ou 4 lorsque x est égal à 4 et n étant égal à 2 ou 3 lorsque x est égal à 3, R_{b} représente un groupe alkyle comprenant de 1 à 20 atomes de carbone, un groupe aryle comprenant de 6 à 30 atomes de carbone ou un groupe aralkyle ou alkaryle comprenant de 6 à 30 atomes de carbone, lesdits groupes alkyle, aryle, aralkyle ou alkaryle susmentionnés étant non fonctionnels, et dans laquelle Λ⁺ représente un cation ammonium, imidazolium, phosphonium ou sulfonium, Y⁺ représentant notamment un cation alkylammonium, alkylphosphonium ou alkylsulfonium, et étant de préférence un cation tétraalkylammonium, tétraalkylphosphonium, dialkylimidazolium, trialkylsulfonium,
Lᵢ représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 2 à 10, les bras Lᵢ pouvant être identiques ou différents,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁ et F₂ étant telles que définies ci-dessous :
- F₀ correspond à un groupe -χ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante :

| | |
|---|---|
| 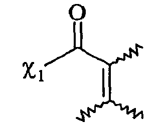 | χ₁ étant tel que défini ci-dessus, |

- F₂ répond à la formule suivante :

| | |
|---|---|
| 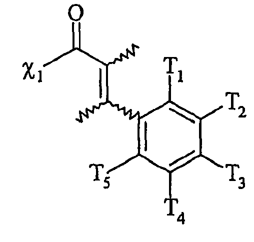 | χ₁ étant tel que défini ci-dessus, |

G répondant à la formule suivante : 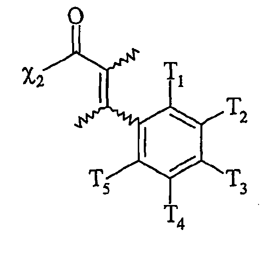 dans laquelle χ₂ représente soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone,
χ₃ représentant un groupement partant, notamment choisi parmi les halogénures I, Cl et Br, les groupes mésylate, tosylate, triflate, sulfonate, sulfate ou phosphate,
T₁, T₂, T₃, T₄ et T₅ représentant indépendamment les uns des autres un atome d'hydrogène, un groupe alkyle, linéaire ou ramifié, comprenant de 1 à 20 atomes de carbone ou un groupe aryle comprenant de 6 à 30 atomes de carbone, ou un groupe fonctionnel notamment choisi parmi NO₂, CN, COOR, OR, COR, NHCOR, NRR", SO₂R, I, Br, R et R" représentant indépendamment l'un de l'autre un groupe alkyle comprenant de 1 à 20 atomes de carbone ou un groupe aryle comprenant de 6 à 30 atomes de carbone,
l'entité 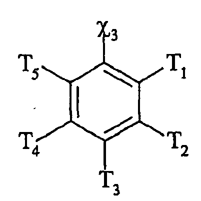 représentant notamment les groupes suivants :

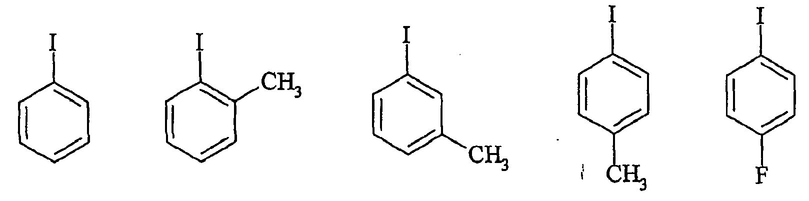

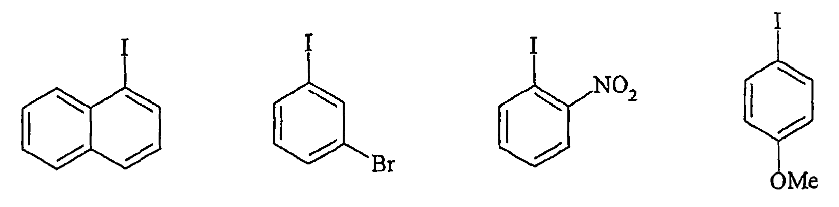

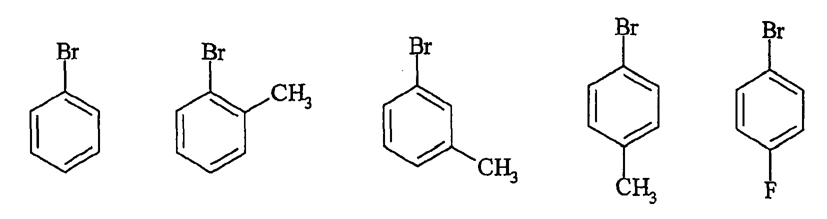

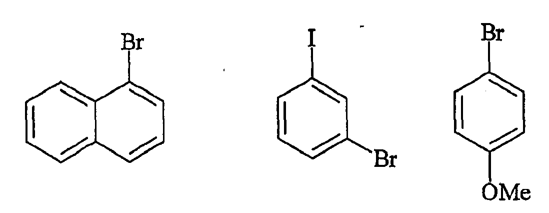

L'utilisation d'un sel plurifonctionnel dans le cadre de la réaction de Heck permet d'augmenter la productivité des solutions utilisées. Par ailleurs, les bras peuvent être fonctionnalisés différemment et permettre soit des réactions intramoléculaires, soit des réactions en cascades, soit des réactions multicomposants.

La présente invention concerne également l'utilisation telle que définie ci-dessus, pour la mise en oeuvre du couplage de Suzuki, selon le schéma réactionnel suivant : 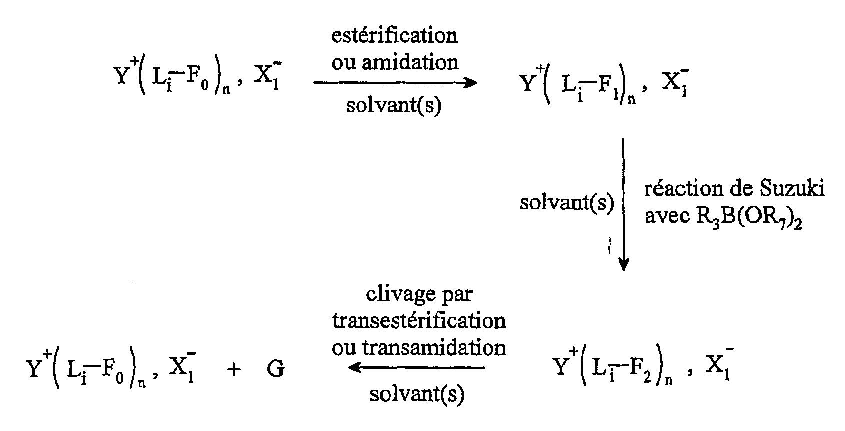 R₃ étant choisi parmi les groupes aryle, hétéroaryle, éthényle, diényle, allyle, éthynyle, substitués ou non, comprenant de 2 à 30 atomes de carbone,
R₇ représentant un atome d'hydrogène ou un groupe alkyle, ramifié ou linéaire ou un groupe cycloalkyle comprenant de 1 à 12 atomes de carbone,
n étant un nombre entier variant de 2 à 4, tel que défini ci-dessous,
i étant un nombre entier variant de 1 à n,
Y⁺ représentant un cation onium tel que défini ci-dessus, de formule (R_{b})ₓ₋ₙΛ⁺ dans laquelle x représente un nombre entier égal à 3 ou 4, n étant égal à 2, 3 ou 4 lorsque x est égal à 4 et n étant égal à 2 ou 3 lorsque x est égal à 3, R_{b} représente un groupe alkyle comprenant de 1 à 20 atomes de carbone, un groupe aryle comprenant de 6 à 30 atomes de carbone ou un groupe aralkyle ou alkaryle comprenant de 6 à 30 atomes de carbone, lesdits groupes alkyle, aryle, aralkyle ou alkaryle susmentionnés étant non fonctionnels, et dans laquelle Λ⁺ représente un cation ammonium, imidazolium, phosphonium ou sulfonium, Y⁺ représentant notamment un cation alkylammonium, alkylphosphonium ou alkylsulfonium, et étant de préférence un cation tétraalkylammonium, tétraalkylphosphonium, dialkylimidazolium, trialkylsulfonium,
Lᵢ représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 2 à 10, les bras Lᵢ pouvant être identiques ou différents,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, -N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁ et F₂ étant telles que définies ci-dessous :
- F₀ est de la forme -χ₁H, χ₁ représentant un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ est de la forme -Rₑ-χ, Rₑ représentant un groupe aromatique ou hétéroaromatique comprenant de 6 à 30 atomes de carbone, χ représentant un groupe partant choisi de préférence parmi Cl, Br, I, OTf, O-CO₂R⁵ ou OSO₃-R⁵, R⁵ représentant un groupe alkyle comprenant de 1 à 10 atomes de carbone ou un groupe aralkyle comprenant de 6 à 30 atomes de carbone, F₁ répondant de préférence à la formule suivante : 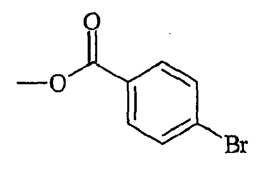
- F₂ est de la forme -Rₑ-R₂, Rₑ étant tel que défini ci-dessus et R₂ étant choisi parmi les groupes aryle, hétéroaryle, éthényle, diényle, allyle, éthynyle, substitués ou non, comprenant de 2 à 30 atomes de carbone, F₂ répondant de préférence à la formule suivante : 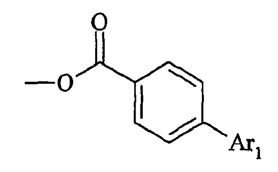 Ar₁ représentant un groupe aromatique choisi de préférence parmi : 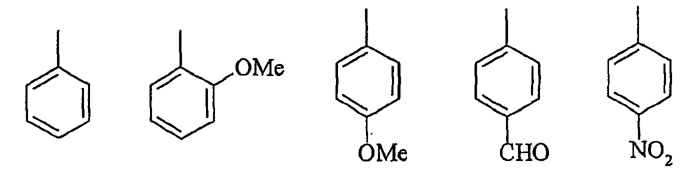 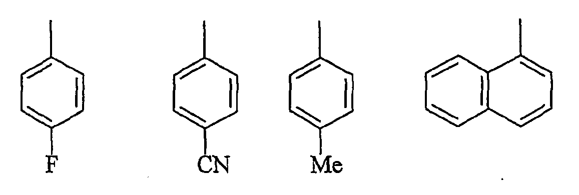
la molécule G étant de la forme R₂-R₃, R₂ et R₃ étant tels que définis ci-dessus, et répond notamment à la formule suivante : 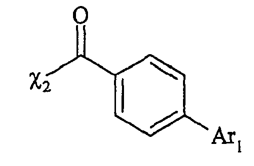 dans laquelle χ₂ représente soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone,
Ar₁ est tel que défini ci-dessus.

La présente invention concerne également l'utilisation telle que définie ci-dessus, pour la mise en oeuvre du couplage de Suzuki, selon le schéma réactionnel suivant : 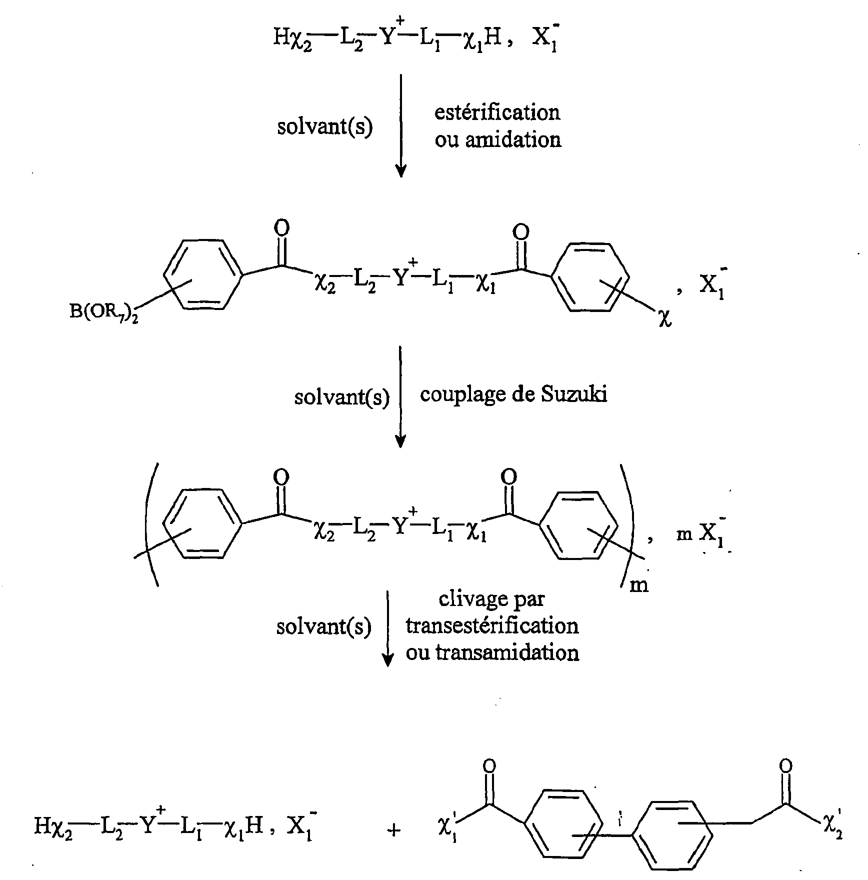 m représentant un nombre entier compris entre 1 et 50,
Y⁺ représentant un cation onium tel que défini ci-dessus, de formule (R_{b})ₓ₋₂Λ⁺ dans laquelle x représente un nombre entier égal à 3 ou 4 selon la nature de Λ⁺, à savoir un cation ammonium, phosphonium ou sulfonium respectivement, R_{b} représente un groupe alkyle comprenant de 1 à 20 atomes de carbone, un groupe aryle comprenant de 6 à 30 atomes de carbone ou un groupe aralkyle ou alkaryle comprenant de 6 à 30 atomes de carbone, lesdits groupes alkyle, aryle, aralkyle ou alkaryle susmentionnés étant non fonctionnels, et dans laquelle Λ⁺ représente un cation ammonium, imidazolium, phosphonium ou sulfonium, Y⁺ représentant notamment un cation alkylammonium, alkylphosphonium ou alkylsulfonium, et étant de préférence un cation tétraalkylammonium, tétraalkylphosphonium, dialkylimidazolium, trialkylsulfonium,
L₁ et L₂ représentant un bras, identiques ou différents, notamment un groupe alkyle linéaire ou ramifié comprenant de 1 à 20 atomes de carbone, ou un groupe aralkyle éventuellement fonctionnel comprenant de 6 à 30 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, -N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
χ₁ et χ₂, identiques ou différents, représentant un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
χ représentant un groupe partant choisi de préférence parmi Cl, Br, I, OTf, O-CO₂R⁵ ou OSO₃-R⁵, R⁵ représentant un groupe alkyle comprenant de 1 à 10 atomes de carbone ou un groupe aralkyle comprenant de 6 à 30 atomes de carbone,
R₇ représentant un atome d'hydrogène, un groupe alkyle, ramifié ou non, ou cycloalkyle, comprenant de 1 à 12 atomes de carbone, ou un groupe aryle, comprenant de 6 à 30 atomes de carbone,
χ'₁ et χ'₂, identiques ou différents, représentant soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone.

L'utilisation d'un sel plurifonctionnel dans le cadre de la réaction de Suzuki permet d'augmenter la productivité des solutions utilisées. Par ailleurs, les bras peuvent être fonctionnalisés différemment et permettre soit des réactions intramoléculaires, soit des réactions en cascades, soit des réactions multicomposants.

La présente invention concerne l'utilisation telle que définie ci-dessus, pour la mise en oeuvre de la réaction de Heck, selon le schéma réactionnel suivant : 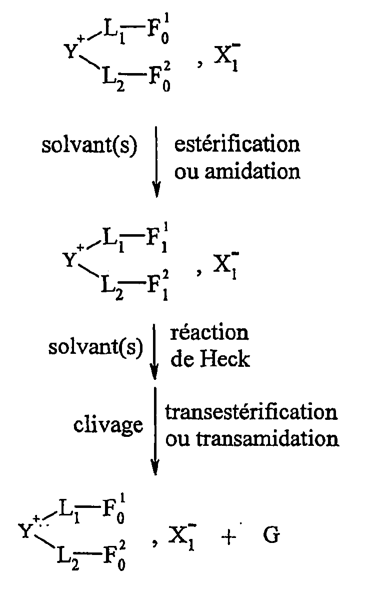 Y⁺ représentant un cation onium tel que défini ci-dessus, de formule (R_{b})ₓ₋₂Λ⁺ dans laquelle x représente un nombre entier égal à 3 ou 4, R_{b} représente un groupe alkyle comprenant de 1 à 20 atomes de carbone, un groupe aryle comprenant de 6 à 30 atomes de carbone ou un groupe aralkyle ou alkaryle comprenant de 6 à 30 atomes de carbone, lesdits groupes alkyle, aryle, aralkyle ou alkaryle susmentionnés étant non fonctionnels, et dans laquelle Λ⁺ représente un cation ammonium, imidazolium, phosphonium ou sulfonium, Y⁺ représentant notamment un cation alkylammonium, alkylphosphonium ou alkylsulfonium, et étant de préférence un cation tétraalkylammonium, tétraalkylphosphonium, dialkylimidazolium, trialkylsulfonium, Λ⁺ représentant un cation ammonium ou phosphonium lorsque x = 4 et un cation sulfonium lorsque x = 3,
L₁ et L₂, identiques ou différents, représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 2 à 10,
X₁⁻ étant tel que défini ci-dessus, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀¹, F₁¹, F₀² et F₁² étant telles que définies ci-dessous :
- F₀¹ correspond à un groupe -χ¹₁H, dans lequel χ¹₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₀² correspond à un groupe -χ²₁H, dans lequel χ²₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁¹ répond à la formule suivante :

| | |
|---|---|
| 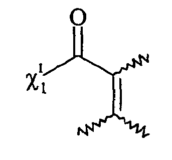 | χ¹₁ étant tel que défini ci-dessus, |

- F₁² répond à la formule suivante :

| | |
|---|---|
| 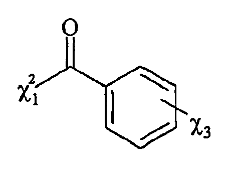 | χ²₁ représentant un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone, et |
| | χ₃ représentant un groupement partant, notamment choisi parmi les halogénures I, Cl et Br, les groupes mésylate, tosylate, triflate, sulfonate, sulfate ou phosphate, |

G répondant à la formule suivante : 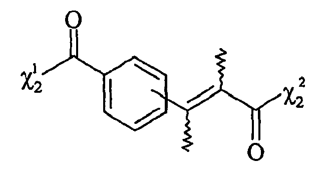 dans laquelle χ₂¹ et χ₂², identiques ou différents, représentent soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone.

L'utilisation d'un sel plurifonctionnel dans le cadre de la réaction de Heck permet d'augmenter la productivité des solutions utilisées. Par ailleurs, les bras peuvent être fonctionnalisés différemment et permettre soit des réactions intramoléculaires, soit des réactions en cascades, soit des réactions multicomposants.

### DESCRIPTION DES FIGURES

La Figure 1 représente des spectres de RMN du proton enregistrés à 200 MHz dans l'acétone D6, correspondant au suivi de la réaction de couplage de Heck entre le sel **5** (Y = (Me)₃N, n = 0) et l'iodobenzène. Le spectre du bas correspond au produit de départ **5d** dans l'acétone D6 et le spectre du haut au produit brut d'arrivée **24d** dans l'acétone D6.

La Figure 2 représente un chromatogramme correspondant au mélange des trois esters méthyliques **27a** à **27c** dont les spectres de masse sont décrits dans le tableau 7.

La Figure 3 représente un chromatogramme correspondant au mélange des sept esters méthyliques biaryliques **29a** à **29g** dont les spectres de masse sont décrits dans le tableau 8.

La Figure 4 représente un chromatogramme correspondant au mélange des neuf esters méthyliques biaryliques **31a** à **31i** dont les spectres de masse sont décrits dans le tableau 10.

La Figure 5 représente un chromatogramme correspondant au mélange des neuf esters éthyliques biaryliques **32a** à **32i** dont les spectres de masse sont décrits dans le tableau 11.

La Figure 6 représente un chromatogramme correspondant au mélange des neuf esters propyliques biaryliques **33a** à **33i** dont les spectres de masse sont décrits dans le tableau 12.

La Figure 7 représente des spectres de RMN du proton enregistrés à 200 MHz dans l'acétone D6, correspondant au suivi de la réaction de Sonogashira à partir du sel **6b.** Le spectre du bas correspond au produit de couplage de **6b** avec l'heptynol dans l'acétone D6 et le spectre du haut au produit de départ **6b** dans l'acétone D6.

La Figure 8 représente un chromatogramme correspondant au mélange des cinq esters méthyliques acétyléniques **35a** à **35e** dont les spectres de masse sont décrits dans le tableau 17.

La Figure 9 représente un chromatogramme correspondant au mélange des cinq esters éthyliques acétyléniques **36a** à **36e** dont les spectres de masse sont décrits dans le tableau 18.

La Figure 10 représente un chromatogramme correspondant au mélange des cinq esters propyliques acétyléniques **37a** à **37e** dont les spectres de masse sont décrits dans le tableau 19.

La Figure 11 représente un chromatogramme correspondant au mélange des cinq esters butyliques acétyléniques **38a** à **38e** dont les spectres de masse sont décrits dans le tableau 20.

La Figure 12 représente un chromatogramme correspondant au mélange des six alcools **40a** à **40f** dont les spectres de masse sont décrits dans le tableau 22.

La Figure 13 représente des spectres de RMN du proton enregistrés à 200 MHz dans l'acétone D6, correspondant au suivi de la réaction de Grieco réalisée sur un sel d'onium. Le spectre du haut correspond au produit de départ **7g** et le spectre du bas correspond au produit d'arrivée **42.**

La Figure 14 représente le chromatogramme HPLC des produits **47a** à **47e** (produits de couplage de Sonogashira).

La Figure 15 représente le chromatogramme GC/MS du mélange d'oléfines tétrasubstituées **49a** à **49e** (tableau 26).

La Figure 16 représente un chromatogramme du produit de couplage de Suzuki de **14a** (X = Cl) avec l'acide phénylboronique après transestérification par du méthanol.

La Figure 17 représente des spectres de RMN du proton enregistrés à 200 MHz dans l'acétone D6, correspondant au suivi de la réaction de cycloaddition entre le bis-ester d'acryloyle **13** et le cyclopentadiène. Le spectre du haut correspond au produit de départ **13** et le spectre du bas correspond au produit de cycloaddition obtenu après réaction avec le cyclopentadiène.

### PARTIE EXPÉRIMENTALE - PRÉPARATION DES COMPOSÉS

### I) SYNTHÈSE DES SELS FONCTIONNALISÉS :

### 1/ Sels d'ammonium 1 :

| **1** | 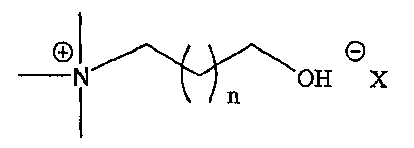 |
|---|---|
| **1a** | **n = 0 ; X = NTf₂** |
| **1b** | **n = 1 ; X = Cl** |
| **1c** | **n = 1 ; X = PF₆** |
| **1d** | **n = 1 ; X = BF₄** |
| **1e** | **n = 1 ; X = NTf₂** |
| **1f** | **n = 2 ; X = Cl** |
| **1g** | **n = 2 ; X = NTf₂** |
| **1h** | **n = 2 ; X = OTf** |
| **1i** | **n = 3 ; X = Cl** |
| **1j** | **n = 3 ; X = NTf₂** |

### * 1a :

Une solution de 2 g (0,01 mmol) du triméthyl-2-hydroxyéthylammonium dans 1ml d'eau est ajoutée à une solution de 1 g (0,018 mmol) de bis-trifluorométhanesulfonamidure de lithium. Le mélange est agité pendant 2 heures. Les deux phases obtenues sont séparées, et la phase aqueuse est extraite 2 fois par 15 ml de chlorure de méthylène. Le solvant est ensuite évaporé et le produit est séché sous vide.

| | |
|---|---|
| Huile visqueuse incolore | **Rdt** = 90 % |

***RMN¹H (200 MHz, Acétone D₆)* :** 3,35 (s, 9H) ; 4,14-4,40 (m, 2H) ; 4,05-4,63(m, 2H).
***RMN ¹³C (50 MHz, Acétone D₆)* :** 55,02 (t ; J_{C-N}=4,03 Hz) ; 57,23 ; 68,91 ; 121,05 (q, J=321,2 Hz).

### * 1b :

25 g (0,1 mol) de 3-chloropropanol, 30 ml d'une solution de triméthylamine à 45% dans l'eau (0,2 mol) et 100 ml d'acétonitrile sont portés à reflux pendant 36 heures. Le solvant est ensuite évaporé sous vide et le solide blanc obtenu est lavé par 2 fois 30 ml d'éther.

| | | |
|---|---|---|
| Solide blanc | **Rdt** = 82% | **Pf** = 158-160°C |

***RMN¹H (200 MHz, D₂O)*** : 1,80-2,05 (m, 2H) ; 3,00 (s, 9H) ; 3,20-3,41 (m, 2H); 3,60 (t, 2H, J = 7,1 Hz)
***RMN¹³C (50 MHz, D2O) :*** 25,68 ; 53,31 (t, J_{C-N}= 4,1 Hz); 58,52 ; 64,52.

| **Spectrométrie de masse (FAB) pour C₁₂H₃₂N₂O₂Cl** | |
|---|---|
| **Masse Théorique calculée pour (2C⁺, Cl⁻)⁺** | 271,2152 |
| **Masse Trouvée** | 271,2149 |

### * 1c :

Un mélange d'une solution de 10 g (65,3 mol) de chlorure de N,N',N"-triméthyle-3-hydroxypropylammonium (**1b**) dans 15ml d'eau et 13,23 ml (0,15 mol) de l'acide hexafluorophosphorique en solution à 60% dans l'eau est agité à température ambiante pendant 2 heures. Le milieu devient hétérogène immédiatement et le précipité formé est filtré et lavé à l'éther. Le solide blanc obtenu est séché sous vide.

| | | |
|---|---|---|
| Solide blanc | **Rdt** = 67% | **Pf** = 124-126°C |

***RMN¹H (200 MHz, CD₃CN)* :** 1,70 (m, 2H) ; 2,82 (s, 9H) ; 3,15 (m, 2H) ; 3,40 (t, 2H, J = 6,1 Hz).
***RMN¹³C (50 MHz, CD₃CN)* :** 25,44 ; 52,59 (t ; J = 4,2 Hz) ; 57,67 ; 64,26 (t, J = 3,8 Hz).

| **Spectrométrie de masse (FAB) pour C₁₂H₃₂N₂O₂F₆P** | |
|---|---|
| **Masse Théorique calculée pour (2C⁺, PF₆⁻)⁺** | 381,2106 |
| **Masse Trouvée** | 381,2098 |

### * 1d :

Un mélange d'une solution de 10 g (65 mmol) de chlorure de N,N',N"-triméthyle-3-hydroxypropylammoinum (**1b**) dans 15 ml d'eau et 9,1 ml (0,15 mol) de l'acide tétrafluoroborique à 50% dans l'eau est agité à température ambiante. Le milieu reste homogène. Après 12 heures, l'eau est évaporée à sec, le solide blanc obtenu est lavé par 2 fois 15 ml d'éther anhydre.

| | | |
|---|---|---|
| Solide blanc | **Rdt** = 82% | **Pf** = 110-112°C |

***RMN¹H (200 MHz, Acétone D₆)* :** 2,10-2,241 (m, 2H) ; 3,05 (s, 9H) ; 3,24-3,45 (m, 2H) ; 3,61 (t, J = 7,1 Hz, 2H).
***RMN¹³C (50 MHz, Acétone D₆)* :** 27,52 ; 53,35 (t ; J_{C-N} = 4,1 Hz) ; 58,25 ; 64,58.

### * 1e :

10 g du sel d'ammonium (**1b**) (65,3 mmol) sont dissous dans 10 ml d'eau. Cette solution est ajoutée à 20 g de bis-trifluorométhanesulfonamidure de lithium (71,9 mmol) dans 10 ml d'eau. Le mélange est agité pendant 2 heures à température ambiante. Les deux phases obtenues sont séparées, et la phase aqueuse est extraite 2 fois par 15 ml de chlorure de méthylène. Enfin le solvant est évaporé et le produit est séché sous vide.

| | |
|---|---|
| Huile visqueuse incolore | **Rdt** = 86% |

***RMN ¹H (200 MHz, Acétone D₆)* :** 2,00-2,21 (m, 2H) ; 3,25 (s, 9H) ; 3,50-3,80 (m, 4H).
***RMN ¹³C (50 MHz, Acétone D₆)* :** 29,14 ; 54,27 (t ; J_{C-N} = 4,1 Hz) ; 60,05 ; 66,09 ; 121,05 (q, J = 321,2 Hz).

### * 1f :

Dans un ballon de 250 ml on met 2 ml (23,90 mmol) de 3-chloropropanol, 4 ml d'une solution de triméthylamine à 45% dans l'eau (40 mmol). Le mélange est ensuite porté à reflux pendant 24 heures. Les solvants sont ensuite évaporés sous vide. Le solide blanc obtenu est lavé par 2 fois 30 ml d'éther.

| | | |
|---|---|---|
| Solide blanc | **Rdt** = 94% | **Pf** = 118-120°C |

***RMN ¹H (200 MHz, D₂O)* :** 1,6-1,78 (m, 2H) ; 1,85-2,05 (m, 2H) ; 3,25 (s, 9H) ; 3,4-3,5 (m, 2H) ; 3,60 (t, 2H, J = 7,2 Hz)
***RMN ¹³C (50 MHz, D2O)** :* 19,50 ; 28,43 ; 53,18 (t, J_{C-N} = 4,1 Hz) ; 61,11 ; 66,66.

### * 1g :

Dans un bécher, on prépare une solution de 1 g (0,01mole) du sel d'ammonium (**1f**) dans 1ml d'eau. Dans un autre bécher, on dissout de la même manière 2 g (0,018 mmol) de bis-trifluorométhanesulfonamidure de lithium. On mélange les deux solutions et on agite pendant 2 heures à température ambiante pour que l'échange soit total. Les deux phases obtenues sont séparées dans une ampoule à décanter, et la phase aqueuse est extraite 2 fois par 15 ml de chlorure de méthylène. Enfin le solvant est évaporé et le produit est séché sous vide.

| | |
|---|---|
| Huile visqueuse incolore | **Rdt** = 86% |

***RMN ¹H (200 MHz, Acétone D₆)*** : 1,5-1,65 (m, 2H); 1,9-2,2 (m, 2H); 3,3 (s, 9H) ; 3,50-3,65 (m, 5H).
***RMN ¹³C (50 MHz, Acétone D₆)*** : 20,91 ; 30,47 ; 53,99 (t ; J_{C-N} = 4,03 Hz) ; 61,88 ; 67,90 ; 121,05 (q, J = 321,2 Hz)

### * 1h :

A une solution de 4 g (34 mmol) de N,N'-diméthylamino-1-butanol dans 10 ml d'acétonitrile, on ajoute 4,71 g (37,4 mmol) de méthyltriflate à 0°C. On laisse remonter à température ambiante sous agitation et on agite ensuite pendant 2 heures. Après quoi le solvant est évaporé à sec et l'huile incolore obtenue est lavée par 3 × 10 ml d'éther et séchée sous vide.

| | |
|---|---|
| Huile incolore | **Rdt** = 97% |

***RMN ¹H (200 MHz, Acétone D₆)*** : 1,65-1,80 (m, 2H) ; 1,90-2,00 (m, 2H) ; 3,12 (s, 9H) ; 3,3-3,5 (m, 2H) ; 3,68 (t, 2H, J = 6,13 Hz)
***RMN ¹³C (50 MHz, Acétone D₆)*** : 28,36 ; 28.99 ; 51,93 (t ; J_{C-N} = 4,03 Hz) ; 60,47 ; 68,75 (t ; J_{C-N} = 4,03 Hz) ; 121,05 (q, J = 321,2 Hz)

### * 1i :

Dans un ballon de 250 ml on met 5 g (36 mmol) de 6-chlorohexanol, 10 ml d'une solution de triméthylamine à 45% dans l'eau (0,1 mol) et 100 ml d'acétonitrile pour homogénéiser le milieu. On porte ensuite le mélange à reflux pendant 36 heures. Le mélange eau/acétonitrile est évaporé sous vide et le solide blanc obtenu est lavé par 2 fois 30 ml d'éther.

| | | |
|---|---|---|
| Solide blanc | **Rdt** = 62% | **Pf** = 178-180°C |

***RMN 1H (200 MHz, MeOH)* :** 1,30-1,65 (m, 6H) ; 1,80-1,95 (m, 2H) ; 3,18 (s, 9H) ; 3,4-3,6 (m, 2H) ; 3,55 (t, 2H, J = 6,1 Hz).
***RMN ¹³C (50 MHz, MeDH)*** : 22,93 ; 25,48 ; 26,15 ; 32,35 ; 52,60 (t ; J = 4,1 Hz) ; 61,67 ; 66,76.

### * 1j :

Un mélange d'une solution de 10 g (51,2 mmol) de chlorure de N,N',N"-triméthyle-6-hydroxybutylammomum (1i)dans 15ml d'eau et 18,7g (6,66 mmol) de bis-trifluorométhanesulfonamidure de lithium est agité à température ambiante. Le milieu devient hétérogène immédiatement, et les deux phases sont séparées dans une ampoule à décanter. L'huile incolore obtenue est ensuite lavée deux fois par 3 ml d'eau et séchée à 50°C sous vide poussé.

| | |
|---|---|
| Huile incolore | **Rdt** = 93% |

***RMN ¹H (200 MHz, Acétone, D6)* :** 1,41-1,60 (m, 6H) ; 1,88-2,01 (m, 2H) ; 3,30 (s, 9H) ; 3,50-3,65 (m, 4H) ; 3,55(t, 2H, J = 6,1 Hz).
***RMN ¹³C (50 MHz, Acétone, D6)*** : 23,02 ; 25,60 ; 26,22 ; 53,01 (t; J = 4,1 Hz) ; 61,73 ; 66,99 ; 121,05 (q, J = 324,2 Hz).

### 2/ Sels de phosphonium 2 :

| | |
|---|---|
| **2** | 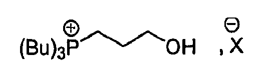 |

| | |
|---|---|
| **2a** | **X = Cl** |
| **2b** | **X = NTf₂** |

### * 2a :

2 ml (23,90 mmol) de 3-chloropropanol et 7,2 ml (28,68 mmol) de tributylphosphine sont portés à reflux pendant 14 h. L'excès de la tributylphosphine est ensuite éliminé par lavage à l'éther (3 × 10 ml).

| | |
|---|---|
| Huile incolore | **Rdt** = 72% |

***RMN ¹H (200 MHz, D₂O)*** : 0,90 (t; 9H ; J = 5,57 Hz) ; 1,30-1,60 (m, 12H ); 1,65-1,85 (m, 2H) ; 2,05-2,30 (m, 8H) ; 3,55-3,70(m, 2H).
***RMN ¹³C (50 MHz, D₂O)*** : 13,15 ; 14,91 ; 15,90 ; 17,57 ; 18,53 ; 23,10, 23,59 ; 23,89 ; 34,62 ; 42,52 ; 58,77 ; 61,32 ; 61,64.

### * 2b :

2 g (6,97 mmol) de bis-trifluorométhanesulfonamidure de lithium et 1 g (3,36 mmol) de chlorure de tributyl-3-hydroxypropyl phosphonium **(2a)** sont dissous dans 3 ml d'eau. Les deux solutions sont ensuite mélangées et agitées pendant 2 h à température ambiante.

La phase aqueuse est extraite par 2 fois 15 ml de chlorure de méthylène et les phases organiques sont rassemblées et séchées sur MgSO₄. Le solvant est ensuite évaporé à sec, puis le produit obtenu est séché sous vide.

| | |
|---|---|
| huile incolore | **Rdt** = 90% |

***RMN ¹H (200 MHz, Acétone D₆)* :** 0,75 (t ; 9H ; J = 7,13) ; 1,22-1,60 (m, 12H); 1,61-1,82 (m, 2H) ; 2,12-2,34 (m, 8H) ; 3,45-3,60 (m, 2H) ; 3,8 (t, 1H, J = 5 Hz)
***RMN ¹³C (50 MHz, Acétone D**₆**)*** : 13,99 ; 16,06 ; 17,05 ; 18,90 ; 19,86 ; 24,29 ; 24,38 ; 24,81 ; 25,12 ; 25,61 ; 25,70 ; 62,05 ; 62,35 ; 121,05 (q, J_{CF} = 374,2 Hz )

### 3/ Sels de pyridinium 3 :

| | |
|---|---|
| **3** | 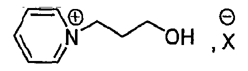 |

| | |
|---|---|
| **3a** | **X = Cl** |
| **3b** | **X = NTf₂** |

### * 3a :

Dans un ballon de 100 ml on introduit 2 ml (23,9 mmol) de 3-chloropropanol et 6 ml de pyridine. Le mélange réactionnel est chauffé à 80°C durant une nuit. L'excès de pyridine est éliminé à l'éther par lavage par 3 × 10 ml. Enfin, on cristallise le produit dans l'acétone.

| | | |
|---|---|---|
| Solide blanc | **Rdt** = 73 % | **Pf** = 68-70°C |

***RMN ¹H (200 MHz, D₂O)*** : 2,1-2,3 (m, 2H); 3,6 (t, J = 5,99 Hz, 2H) ; 3,75 (t, J = 7,19 Hz, 2H) ; 8,01 (t, J = 7,04 Hz, 2H) ; 8,55 (t, J = 9,97 Hz, 1H) ; 8,83 (d, J = 6,04Hz, 2H)
***RMN ¹³C (50 MHz, D₂O)*** : 33,14 ; 58,22 ; 59,51 ; 128,72 ; 144,89 ; 146,12

### * 3b :

On dissout dans un bécher 2 g (6,97 mmol) de bis-trifluorométhanesulfonamidure de lithium dans 3 ml d'eau. De la même manière, on dissout dans un autre bécher environ 1 g (5,76 mmol) de chlorure de 3-hydroxypropylpyridinium (**3a**) dans l'eau puis on mélange les deux solutions et on laisse agiter durant deux heures.

Le contenu du bécher est transvasé dans une ampoule à décanter. La phase aqueuse est extraite avec 2 fois 15 ml de chlorure de méthylène. Les phases organiques sont rassemblées et séchées sur MgSO₄. Le solvant est ensuite évaporé à sec puis le produit est séché sous vide.

| | |
|---|---|
| huile incolore | **Rdt** = 90% |

***RMN ¹H (200 MHz, Acétone)*** : 2,03-2,21 (m, 2H) ; 3,5 (t, J = 6,1H 2H) ; 3,8 (t, J = 6,9 Hz, 1H) ; 4,73 (t, J = 7Hz, 2H) ; 8,1 (t, J = 7 Hz, 2H) ; 8,52 (t, J = 9,8 Hz, 1H) ; 8,98(d, J = 6,1 Hz, 2H)
***RMN ¹³C (50 MHz, Acétone)*** : 34,57 ; 58,95 ; 60,95 ; 121,37 (q, J_{C-F} = 320,9 Hz) ; 129,67 ; 146,46 ; 147,11

### 4/ Sels d'imidazolium 4 :

| | |
|---|---|
| **4** | 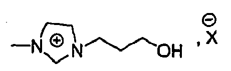 |

| | |
|---|---|
| **4a** | **X = Cl** |
| **4b** | **X = NTf₂** |

### * 4a :

Dans un ballon de 100 ml on introduit 2 ml (23,90 mmol) de 3-chloropropanol et 2,87 g (35 mmol) de la N-méthylimidazole. Le mélange réactionnel est chauffé à 80°C durant une nuit. L'excès de la N-méthylimidazole est éliminé par lavage à l'éther (3 × 10 ml) et le solide blanc obtenu est séché sous vide.

| | |
|---|---|
| Solide blanc | **Rdt** = 80 % |

***RMN ¹H (200 MHz, D₂O)*** : 1,96-2,25 (m, 2H); 3,55 (t, 2H, J = 6,11 Hz) ; 3,85 (s, 3H) ; 4,15 (t, 2H, J = 7,12 Hz) ; 7,43 (t apparent, 1H, J = 1,73 Hz) ; 7,48 (t apparent, 1H, J = 1,74 Hz) ; 8,75 (s, 1H)
***RMN ¹³C (50 MHz, D₂O)*** : 32,87 ; 35,40 ; 47,23 ; 58,29 ; 123,03 ; 124,26 ; 137,06

### * 4b :

On dissout dans un bécher 2 g (6,97 mmol) de LiNTf₂ dans 3 ml d'eau. De la même manière, on dissout dans un autre bécher environ 1 g de chlorure d'imidazolium **4a** dans l'eau. Les deux solutions sont mélangées et laissées agiter durant deux heures.

Le contenu du bécher est transvasé dans une ampoule à décanter. La phase aqueuse est extraite avec 2 fois 15 ml de chlorure de méthylène. Les phases organiques sont rassemblées et séchées sur MgSO₄. Le solvant est ensuite évaporé à sec puis le produit est séché sous vide.

| | |
|---|---|
| huile incolore | **Rdt** = 90% |

***RMN ¹H (200 MHz, Acétone)*** : 2,15-2,21 (m, 2H ) ; 3,65 (t, 2H, J = 6,11 Hz) ; 4,05 (s, 3H) ; 4,15 (t, 2H, J = 7,12 Hz) ; 7,68 (t apparent, 1H, J = 1,73 Hz) ; 7,73 (t apparent, 1H, J = 1,74 Hz) ; 8,95 (s, 1H)
***RMN ¹³C (50 MHz, Acétone)*** : 32,32 ; 35,92 ; 47,01 ; 58,35 ; 121,37 (q, J_{C-F} = 320,9 Hz) ; 123,25 ; 124,01 ; 136,50

### II) FONCTIONNALISATION DES SELS PRÉCÉDENTS :

### 1/ Ester acrylique 5 :

### Procédure générale de l'estérification par l'acide acrylique :

| | |
|---|---|
| **5** | 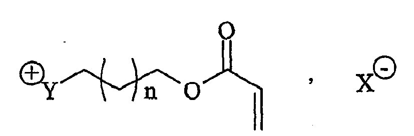 |

| **5** | **Y** | **n** | **X** |
|---|---|---|---|
| **5a** | **N(Me)₃** | **0** | **NTf₂** |
| **5b** | **N(Me)₃** | **1** | **Cl** |
| **5c** | **N(Me)₃** | **1** | **BF₄** |
| **5d** | **N(Me)₃** | **1** | **NTf₂** |
| **5e** | **N(Me)₃** | **2** | **NTf₂** |
| **5f** | **P(Bu)₃** | **1** | **NTf₂** |
| **5g** | **Py** | **1** | **NTf₂** |
| **5h** | **Im** | **1** | **NTf₂** |

Une solution du sel d'onium et de 3 équivalents de chlorure d'acryloyle dans l'acétonitrile est chauffée à 80°C en présence de 5 équivalents de K₂CO₃ solide pendant 2 heures. Le mélange réactionnel est ensuite placé sous vide à 40°C pour éliminer le solvant et l'excès du réactif. L'acrylate d'onium est ensuite extrait par le chlorure de méthylène.

### * 5a :

| | |
|---|---|
| huile rose | **Rdt** = 90% |

***RMN ¹H (200 MHz, Acétone D₆)*** : 3,5 (s, 9H) ; 4,03-4,40 (m, 2H) ; 4,73-4,85 (m, 2H) ; 6,05 (dd, 1H, J₁ = 1,92 Hz, J₂ = 10,5 Hz) ; 6,25 (dd, 1H, J₁ = 10,5 Hz, J₂ = 17,3 Hz) ; 6,45 (dd, 1H, J₁ = 1,9 Hz, J₂ = 17,3 Hz)
***RMN ¹³C (50 MHz, Acétone D₆)*** : 54,01 (t, J = 4,2 Hz) ; 58,25 ; 65,23 ; 121,05 (q, J_{CF} = 374,2 Hz ) ; 128,80 ; 132,24 ; 165,46

### * 5b :

| | | |
|---|---|---|
| Solide blanc | **Rdt** =100% | **Pf** =175-177°C |

***RMN ¹H (200 MHz, Acétone D₆)*** : 2,15-2,20 (m, 2H) ; 3,15 (s, 9H) ; 3,48-3,52 (m, 2H) ; 4,18 (t, 2H, J = 6,0 Hz) ; 5,75 (dd, 1H, J₁ = 1,92 Hz, J₂ = 10,5 Hz) ; 6,15 (dd, 1H, J₁ = 10,5 Hz, J₂ = 17,3 Hz) ; 6,15 (dd, 1H, J₁ = 1,9 Hz, J₂ = 17,3 Hz)
***RMN ¹³C (50 MHz, Acétone D₆)*** : 21,74 ; 52,23 (t, J = 4,2 Hz) ; 60,44 (t, J = 3,02) ; 62,6 ; 127,41 ; 130,65 ; 165,04

### * 5c :

| | |
|---|---|
| Huile incolore | **Rdt** = 93% |

***RMN 1H (200 MHz, Acétone D₆)*** : 2,28-3,31 (m, 2H) ; 3,32 (s, 9H) ; 3,06-3,15 (m, 2H) ; 4,52 (t, 2H, J = 6,6 Hz ) ; 5,80 (dd, 1H, J₁ = 1,9 Hz, J₂ = 10,0 Hz) ; 6,05 (dd, 1H, J₁ = 18,3 Hz, J₂ = 10,0 Hz) ; 6,15 (dd, 1H, J₁ = 1,9 Hz, J₂ = 18,3 Hz)
***RMN ¹³C (50 MHz, Acétone D₆)*** : 22,81 ; 53,28 ; 61,46 ; 63,83 ; 128,51 ; 131,72 ; 167,31

### * 5d :

| | |
|---|---|
| Huile incolore | **Rdt** =100%. |

***RMN ¹H (200 MHz, Acétone D₆)*** : 2,22-2,25 (m, 2H) ; 3,25 (s, 9H) ; 3,60-3,75 (m, 2H) ; 4,15 (t, 2H, J = 6,0 Hz) ; 5,80 (dd, 1H, J₁ = 1,92 Hz ; J₂ = 10,68 Hz) ; 6,05 (dd, 1H, J₁ = 17,2 ; J₂ = 10,7) ; 6,15 (dd, 1H, J₁ = 1,9 Hz ; J₂ = 17,2 Hz)
***RMN ¹³C (50 MHz, Acétone D₆)*** : 29,17 ; 54,16 (t, J = 4,0) ; 65,16 ; 65,23 ; 121,05 (q, J_{CF} = 374,2 Hz) ; 129,40 ; 132,15 ; 165,61
***RMN ¹⁹F (282 MHz, Acétone D₆)*** : *-* 79,8

| **Spectrométrie de masse (FAB) pour C₉H₁₈NO₂** | |
|---|---|
| **Masse Théorique calculée pour (C⁺)** | 172,1338 |
| **Masse Trouvée** | 172,1346 |

### * 5e :

| | |
|---|---|
| Huile incolore | **Rdt** = 77% |

***RMN ¹H (200 MHz, Acétone D₆)*** : 1,7-1,9 (m, 2H) ; 2,00-2,20 (m, 2H) ; 3,40 (s, 9H) ; 3,58-3,72 (m, 2H) ; 4,25 (t, 2H, J = 6,0 Hz) ; 5,80 (dd, 1H, J₁ = 1,92 Hz ; J₂ = 10,68 Hz) ; 6,05 (dd, 1H, J₁ = 17,2 ; J₂ = 10,7) ; 6,15 (dd, 1H, J₁ = 1,9 Hz ; J₂ = 17,2 Hz)
***RMN ¹³C (50 MHz, Acétone D₆)*** : 20,86 ; 26,44 ; 53,99 ; 64,52 ; 67,37 ; 122,19 (q, J_{CF} = 374,2 Hz) ; 129,69 ; 131,83 ; 166,99

### * 5f :

| | |
|---|---|
| Huile incolore | **Rdt** = 77% |

***RMN ¹H (200 MHz, Acétone D₆)*** : 1 (t, 9H, J = 7,2 Hz) ; 1,45-1,85 (m, 12H) ; 2,10-2,28 (m, 2H) ; 2,48-2,7 (m, 8H) ; 4,3 (t, 2H, J = 6,27 Hz) ; 5,58 (dd, 1H, J₁ = 0,3 Hz ; J₂ = 1,96 Hz) ; 6,18 (dd, 1H, J₁ = 17,16 Hz ; J₂ = 0,3 Hz) ; 6,4 (dd, 1H, J₁ = 17,16 Hz ; J₂ = 1,97 Hz)
***RMN ¹³C (50 MHz, Acétone D₆)*** : 13,09 ; 15,10 ; 16,09 ; 17,84 ; 18,80 ; 21,15 ; 21,22; 23,38; 23,47; 23,86 ; 24,18 ; 63,77; 64,11 ; 121,05 (q, J_{CF} = 374,2 Hz) ; 128,63 ; 131,12 ; 165,74

### * 5g :

| | |
|---|---|
| Huile incolore | **Rdt** = 80% |

***RMN ¹H (200 MHz, Acétone D₆)*** : 2,15-2,27 (m, 2H) ; 3,8 (t, J = 6,1 Hz, 2H) ; 4,96 (t, J= 6,8 Hz, 2H) ; 5,54 (dd, 1H, J₁ = 0,5 Hz ; J₂ = 2 Hz) ; 6,18 (dd, 1H, J₁ = 17,2 Hz ; J₂ = 0,3 Hz) ; 6,4 (dd, 1H, J₁ = 17,2 Hz ; J₂ = 2 Hz) ; 8,1 (t, J = 7 Hz, 2H) ; 8,52 (t, J = 9,8 Hz, 1H) ; 8,98 (d, J = 6,2 Hz, 2H)
***RMN ¹³C (50 MHz, Acétone D₆)*** : 30,39 ; 59,98 ; 61,42 ; 121,05 (q, J_{CF} = 374,2 Hz) ; 128,75 ; 128,98 ; 131,38 ; 145,48 ; 146,50 ; 165,78

### * 5h :

| | |
|---|---|
| Huile incolore | **Rdt** = 85% |

***RMN ¹H (200 MHz, Acétone D₆)*** : 2,3-2,5 (m, 2H ) ; 4,15 (s, 3H) ; 4,28 (t, 2H, J = 6,03 Hz) ; 4,63 (t, 2H, J = 6,99 Hz) ; 5,95 (dd, 1H, J₁ = 1,92 ; J₂ = 10,68 ) ; 6,18 (dd, 1H, J₁ = 17,2 Hz ; J₂ = 10,7 Hz) ; 6,4 (dd, 1H, J₁ = 1,9 Hz ; J₂ = 17,2 Hz) ; 7,8 (t apparent, 1H, J = 1,70 Hz) ; 7,95 (t apparent, 1H, J = 1,82 Hz) ; 9,2 (s, 1H)
***RMN ¹³C (50 MHz, Acétone D₆)* :** 29,32 ; 36,17 ; 47,38 ; 61,36 ; 121,05 (q, J_{CF} = 374,2 Hz) ; 122,97 ; 124,30 ; 128,44 ; 131,31 ; 136,948 ; 166,07

### 2/ Esters 4-iodobenzoïques 6 :

| | |
|---|---|
| **6** | 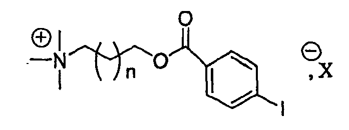 |

| **6** | **n** | **X** |
|---|---|---|
| **6a** | **2** | **I** |
| **6b** | **2** | **NTf₂** |
| **6c** | **2** | **PF₆** |
| **6d** | **2** | **BF₄** |
| **6e** | **2** | **OTf** |
| **6f** | **0** | **BF₄** |
| **6g** | **1** | **NTf₂** |

### * 6a :

A une solution de 4g (10,96 mmole) de para-iodobenzoate de 3-diméthylaminopropyle dans 10 ml d'acétonitrile, on ajoute goutte à goutte à 0°C 1,1 ml (13,15 mmole) d'iodure de méthyle. Le solide blanc ainsi formé est filtré puis lavé par 3 × 10 ml d'éther.

| | | |
|---|---|---|
| Solide blanc | **Rdt** = 99% | **Pf** = 248-250°C |

***RMN ¹H (200 MHz, Acétone D₆)*** : 1,9-2,05 (m, 4H); 3,20 (s, 9H); 3,42-3,55 (m, 2H) ; 4,45 (t, 2H, J = 6,05 Hz) ; 7,8 (d, 2H, J = 8,73 Hz) ; 7,95 (d, 2H, J₁ = 8,76 Hz)
***RMN ¹³C (50 MHz, Acétone D₆)*** : 19,89 ; 25,45 ; 53,37 (t, J_{C-N} = 4,1 Hz) ; 65,13 ; 66,42 ; 101,57 ; 131,19 ; 131,45 ; 138,38 ; 166,78

| **Spectrométrie de masse (FAB) pour C₂₈H₄₂N₂O₄I₃** | |
|---|---|
| **Masse Théorique calculée pour (2C⁺, I⁻)⁺** | 851,03 |
| **Masse Trouvée** | 851,10 |

### * 6b :

A une solution de 1 g (2,04 mmol)de **6a** dans 30 ml du mélange eau/acétone, on ajoute 2,65 mmol de LiNTf₂ en solution dans l'eau. Après 2 heures d'agitation à température ambiante, on évapore le solvant et on extrait le sel par 3 × 10 ml de chlorure de méthylène. Après évaporation de ce dernier, on obtient un solide blanc.

| | | |
|---|---|---|
| Solide blanc | **Rdt** = 90% | **Tf** = 48-50°C |

***RMN ¹H (200 MHz, Acétone D₆)*** : 1,82-2 (m, 2H) ; 2,1-2,22 (m, 2H) ; 3,38 (s, 9H) ; 3,6-3,72 (m, 2H) ; 4,4 (t, 2H, J = 6,24 Hz) ; 7,8 (d, 2H, J = 8,73 Hz) ; 7,95 (d, 2H, J₁ = 8,36 Hz)
***RMN ¹³C (50 MHz, Acétone D₆)*** : 26,66 ; 29,12 ; 54,12 (t, J_{C-N} = 4,1 Hz) ; 65,28 ; 67,40 ; 101,59 ; 121,17 (q, J_{C-F} = 320,9 Hz) ; 131,17 ; 132,28 ; 139,23 ; 166,74

| **Spectrométrie de masse (FAB) pour C₃₀H₄₂N₃O₈F₆I₂S₂** | |
|---|---|
| **Masse Théorique calculée pour (2C⁺, NTf₂⁻)⁺** | 1004,0407 |
| **Masse Trouvée** | 1004,0427 |

### * 6c :

A une solution de 1 g (2,04 mmol) de l'ammonium **6a** dans 30 ml du mélange eau/acétone on ajoute 2,65 mmol de HPf₆ en solution (60%) dans l'eau. Après 2h d'agitation à température ambiante le solvant est évaporé et le solide obtenu est lavé à l'éther (3 × 10ml).

| | | |
|---|---|---|
| Solide blanc | **Rdt** = 96% | **Tf** = 204-206°C |

***RMN ¹H (200 MHz, Acétone D₆)*** : 2,05-2,2 (m, 2H) ; 2,28-2,47 (m, 2H) ; 3,6 (s, 9H) ; 3,8-3,97 (m, 2H) ; 4,6 (t, 2H, J = 6,3 Hz) ; 8 (d, 2H, J = 8,58 Hz) ; 8,15 (d, 2H, J₁ = 8,58 Hz )
***RMN ¹³C (50 MHz, Acétone D₆)*** : 20,97 ; 26,65 ; 54,06 (t ; J = 4,03 Hz) ; 65,31 ; 67,4 (t ; J = 3,17 Hz) ; 101,57 ; 131,19 ; 132,32 ; 139,23 ; 166,75

**Spectrométrie de masse (FAB) pour C₂₈H₄₂N₂O₄F₆I₂P**

| | |
|---|---|
| **Masse Théorique calculée pour (2C⁺, PF₆⁻)⁺** | 869,0876 |
| **Masse Trouvée** | 869,0879 |

### * 6d :

A une solution de 1 g (2,04 mmol) de **6a** dans 3 ml d'eau on ajoute 2,65 mmol de HBF₄ en solution dans l'eau à 40 %. Après l'ajout de ce dernier, on observe la formation d'un solide blanc. Le mélange réactionnel est laissé agité deux heures à température ambiante. Le solide blanc obtenu après filtration est lavé par l'eau (pour éliminer l'excès de HBF₄) puis deux fois par 30 ml d'éther et enfin séché sous vide.

| | | |
|---|---|---|
| Solide blanc | **Rdt** = 86% | **Tf** = 184-186°C |

***RMN ¹H (200 MHz, Acétone D₆)*** : 1,90-2,00 (m, 2H) ; 2,15-2,28 (m, 2H) ; 3,4 (s, 9H) ; 3,65-3,80 (m, 2H) ; 4,42 (t, 2H, J = 6,3 Hz) ; 7,85 (d, 2H, J = 8,58 Hz) ; 8,15 (d, 2H, J₁ = 8,58 Hz)
***RMN ¹³C (50 MHz, Acétone D₆)*** : 20,96 ; 26,62 ; 53,97 (t ; J = 4,03 Hz) ; 65,34 ; 101,53 ; 131,22 ; 132,34 ; 139,23 ; 166,73

| **Spectrométrie de masse (FAB) pour C₂₈H₄₂N₂O₄F₄I₂B** | |
|---|---|
| **Masse Théorique calculée pour (2C⁺, BF₄⁻)⁺** | 811,1263 |
| **Masse Trouvée** | 811,1259 |

### * 6e :

A une solution de 1 g (10,96 mmol) de para-iodobenzoate de 3-diméthylaminopropyle dans 10 ml d'acétonitrile, on ajoute goutte à goutte à 0°C 1,1ml (13,15 mmol) de triflate de méthyle. Le solide blanc ainsi formé est filtré puis lavé par 3 × 10 ml d'éther.

| | | |
|---|---|---|
| Solide blanc | **Rdt**= 96% | **Pf** = 176-178°C |

***RMN ¹H (200 MHz, CD₃CN)*** : 1,8-2,1 (m, 4H); 3,06 (s, 9H) ; 3,35-3,45 (m, 2H) ; 4,4 (t, 2H, J = 6,05 Hz) ; 7,85 (d, 2H, J = 8,58 Hz) ; 8 (d, 2H, J₁ = 8,58 Hz)
***RMN ¹³C (50 MHz, CD₃CN)*** : 19,16 ; 24,66 ; 52,50 (t ; J = 4,03 Hz) ; 63,66 ; 65,62 (t ; J = 3,17 Hz) ; 99,89 ; 121,17 (q, J_{C-F} = 320,9 Hz) ; 129,58 ; 130,59 ; 137,57 ; 165,39

| **Spectrométrie de masse (FAB) pour C₂₉H₄₂N₂O₇F₃I₂S** | |
|---|---|
| **Masse Théorique calculée pour (2C⁺, OTf⁻)⁺** | 873,1 |
| **Masse Trouvée** | 873,1 |

### * 6f :

A un mélange du chlorure de (2-hydroxy-éthyl)-triméthylammonium commercial dans 100 ml de CH₃CN on ajoute : 1,5 éq de DCC, 1,5 éq de l'acide 4-iodobenzoïque et 0,2 éq de DMAP. Le mélange réactionnel est agité 4 heures à température ambiante puis évaporé à sec. L'ester formé est extrait par 3 × 30 ml d'eau. A cette solution on ajoute 1,5 eq de HBF₄ en solution à 50% dans l'eau. Le mélange réactionnel est agité deux heures à température ambiante. Le solide blanc obtenu après filtration est lavé par l'eau (pour éliminer l'excès de HBF₄) puis deux fois par 30 ml d'éther et enfin séché sous vide.

| | | |
|---|---|---|
| Aspect du produit : Solide blanc | **Rdt** = 90% | **Pf** = 200-202°C |

***RMN ¹H (CD**3**CN, 300Mhz)** :* 3,18 (s, 9H) ; 3,68-3,80 (m, 2H) ; 4,50-4,73 (m, 2H) ; 7,78 (dd, J₁=1,9 Hz, J₂=6,7 Hz ; 2H) ; 7,90 (dd, J₁=1,8Hz, J₂=6,6 Hz, 2H).
***RMN ¹³C (CD3CN, 75Mhz)* :** 53.40 (t, J=3,8Hz) ; 58,27 ; 64,38 (t, J=3,8Hz) ; 100,57 ; 128,65 ; 130,68 ; 137,76 ; 164,73.

| **Spectrométrie de masse (APCI) pour [C₁₂ H₁₇INO₂][BF₄] :** | |
|---|---|
| **Masse Théorique calculée pour (C⁺)** | 334.2 |
| **Masse Trouvée** | 334.0 |

### * 6g :

A une solution de 12,6 moles de l'alcool 1e dans 60 ml de CH₃CN on ajoute; 1.5 eq de DCC, 1.1 eq de l'acide 4-iodobenzoique et 0.2eq de DMAP. Le mélange réactionnel est agité 4 heures à température ambiante ; filtré et ensuite évaporé à sec. Le solide blanc obtenu est lavé plusieurs fois à l'éther et enfin séché sous vide.

| | | |
|---|---|---|
| Aspect du produit : solide blanc | **Rdt** = 95% | **Pf** = 48-50°C |

***RMN ¹H (300 MHz, Acétone D₆)*** : 2,40-2,60 (m, 2H); 3,47 (S, 9H) ; 3,80-4,00 (m, 2H) ; 4,53 (t, 2H, J=5,9 Hz) ; 7,83 (d, 2H, J=8,3Hz) ; 7,97 (d, 2H, J=8,3Hz).
***RMN ¹³C (50 MHz, Acétone D₆)*** : 23,07 ; 53,37 (t, J=3,8Hz) ; 62,19 ; 64,46 ; 100,91 ; 120,50 (q, J_{CF}=321,2 Hz); 129,97 ; 131,51 ; 138,33 ; 165,75.

| **Spectrométrie de masse (APCI) pour [C₁₃H₁₉INO2][C₂NS₂O₄F₆] :** | |
|---|---|
| **Masse Théorique calculée pour (C⁺)** | 348.2 |
| **Masse Trouvée** | 348.3 |

### 3/ Esters benzoïques 4-substitués 7 :

| | |
|---|---|
| **7** | 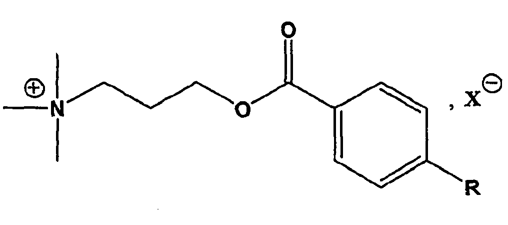 |

| **N°** | **X** | **R** |
|---|---|---|
| **7a** | **Cl** | **Br** |
| **7b** | **NTf₂** | **Br** |
| **7c** | **PF₆** | **Br** |
| **7d** | **BF₄** | **Br** |
| **7e** | **BF₄** | **CH₂=CH-** |
| **7f** | **Cl** | **NH₂** |
| **7g** | **NTf₂** | **NH₂** |
| **7h** | **BF₄** | **O=CH-** |

### * 7a :

Dans un ballon de 250 ml on introduit 2 g (13,1 mmol) de chlorure de N,N',N"-triméthyl-3-hydroxypropylammonium, 25 ml d'acétonitrile, 20 g de K₂CO₃ en poudre et 4 g (17,5 mmol)du chlorure d'acide du 4-bromobenzoïque. Après une nuit d'agitation à température ambiante, on filtre et on lave K₂CO₃ par 3 fois 15 ml de chlorure de méthylène et enfin on évapore à sec. On reprend à l'eau et on élimine l'excès de l'acide 4-bromobenzoïque qui cristallise par filtration. Le produit est ensuite cristallisé dans l'acétone après évaporation d'eau.

| | | |
|---|---|---|
| Solide blanc | **Rdt** = 60% | **Pf** =164-166°C |

***RMN ¹H (200 MHz, D₂O*)** : 2,21-2,34 (m, 2H) ; 3,12 (s, 9H) ; 3,30-3,58 (m, 2H) ; 4,35 (t, 2H, J = 6,8 Hz) ; 7,57 (d, 2H, J = 7,4 Hz) ; 7,80 (d, 2H, J₁ = 7,4 Hz)
***RMN ¹³C (50 MHz, D₂O)*** : 22,55 ; 30,61 ; 53,34 (t, J_{C-N} = 4,2 Hz) ; 62,69 ; 64,41 (t, J_{C-N} = 4,09 Hz) ; 128,34 ; 128,66 ; 131,37 ; 132,20 ; 167,84

| **Spectrométrie de masse (FAB) pour C₁₃H₁₉NO₂Br** | |
|---|---|
| **Masse Théorique calculée pour (C⁺)** | 300,0599 |
| **Masse Trouvée** | 302,0607 |

### * 7b :

Dans ce cas, la synthèse du substrat a été envisagée selon deux approches : par estérification directe du bis-trifluorométhanesulfonamidure de N,N,N-trimethyl-3-hydroxypropylammonium ou par métathèse à partir du chlorure correspondant.

### Estérification :

Dans un ballon de 250 ml, on introduit 4 g (10,5 mmol) de l'alcool, 20 ml d'acétonitrile, 2 ml d'une solution saturée de Na₂CO₃ dans l'eau et 4 g (17,5 mmol) de chlorure de l'acide 4-bromobenzoïque. Le mélange réactionnel est chauffé à 60°C durant une nuit. On évapore ensuite à sec, et on solubilise le résidu obtenu dans le chlorure de méthylène. On lave cette solution successivement par 2 × 20ml d'eau, 2 × 20 ml d'une solution de soude (1 N) et enfin par 2 × 20ml d'eau. On sèche la solution sur sulfate de magnésium et on évapore à sec le solvant. On reprend dans l'acétone et élimine les traces d'acides par précipitation à 4°C. Après séchage sous vide, on obtient un solide blanc pur.
Rdt = 90 %.

### Métathèse

Dans un ballon de 100 ml on solubilise 1 g (2,98 mmol) de **1b** dans 5 ml d'eau. A cette solution on ajoute 1,1 g (3,19 mmol) de bis-trifluorométhanesulfonamidure de lithium (LiNTf₂) en solution dans 3 ml d'eau. Le mélange réactionnel est agité 2 heures à température ambiante avant d'extraire notre produit par 20 ml de chlorure de méthylène. Après évaporation de ce dernier, on obtient un solide blanc qui est séché sous vide.

| | | |
|---|---|---|
| Solide blanc | **Rdt** = 90% | **Tf** = 86-88°C |

***RHN ¹H (200 MHz, Acétone D₆)*** : 2,64-2,83 (m, 2H) ; 3,59 (s, 9H) ; 3,96-4,06 (m, 2H) ; 4,71 (t, 2H, J = 6,76 Hz) ; 7,90 (d, 2H, J = 8,9 Hz) ; 8,19 (d, 2H, J = 8,9 Hz)
***RMN ¹³C (50 MHz, Acétone D₆)*** : 23,96 ; 54,24 (t, J_{C-N} = 4,2 Hz) ; 63,09 ; 65,35 (t, J_{C-N} = 4,0 Hz) ; 121,46 (q, J_{C-F} = 322,0 Hz) ; 128,95 ; 130,42 ; 132,58 ; 133,12 ; 166,34

| **Spectrométrie de masse (FAB) pour C₂₈H₃₈F₆N₃O₈S₂Br₂** | |
|---|---|
| **Masse Théorique calculée pour (2C⁺, NTf₂⁻)** | 880,0371 |
| **Masse Trouvée** | 880,0375 |

### * 7c :

A une solution de 1 g (2,98 mmol) de **1b** dans 3 ml d'eau on ajoute 0,5 ml (5,7 mmol) de HPF₆ à 60% dans l'eau. Le mélange réactionnel est agité deux heures à température ambiante. Le solide blanc obtenu après filtration est lavé à l'eau puis deux fois par 30 ml d'éther et enfin séché sous vide.

| | | |
|---|---|---|
| Solide blanc | **Rdt** = 96% | **Pf** = 154-156°C |

***RMN ¹H (200 MHz, Acétone D₆)*** : 2,45-2,59 (m, 21-1); 3,40 (s, 9H); 3,79-3,85 (m, 2H) ; 4,50 (t, 2H, j=6.0 Hz) ; 7,55 (dd, 2H, J₁=1,9 Hz ; J₂= 7,7 Hz ) ; 8,00 (dd, 2H, J₁=1,9 Hz ; J₂= 7,7 Hz)
***RMN ¹³C (50 MHz, Acétone D₆)*** : 22,96 ; 53,23 (t, J = 4,0 Hz) ; 62,31 ; 64,34 ; 128,06 ; 129,48 ; 131,79 ; 132,25 ; 165,63
***RMN ¹⁹F (282 MHz, Acétone D₆)*** : -71,6 (d ; J = 707,3Hz ; P-F)
***RMN ³¹P (Acétone, 129,5 MHz)** δ* : -142 (m, J = 0,7 Hz, P-F₆)

| **Spectrométrie de masse (FAB) pour C₂₆H₃₈F₆N₂Br₂O₄P** | |
|---|---|
| **Masse Théorique calculée pour (2C⁺, PF₆⁻)** | 745,0840 |
| **Masse Trouvée** | 745,0824 |

### * 7d :

A une solution de 1 g (2,98 mmol) de **1b** dans 3 ml d'eau on ajoute 1 ml de HBF₄ en solution dans l'eau à 40%. Après l'ajout de ce dernier, on observe la formation d'un solide blanc. Le mélange réactionnel est agité deux heures à température ambiante. Le solide blanc obtenu après filtration est lavé par l'eau (pour éliminer l'excès de HBF₄) puis deux fois par 30 ml d'éther et enfin séché sous vide.

| | | |
|---|---|---|
| Solide blanc | **Rdt** = 98% | **Tf** = 154-156°C |

***RMN ¹H (200 MHz, Acétone D₆)*** : 2,39-2,57 (m, 2H) ; 3,35 (s, 9H) ; 3,70-3,87 (m, 2H) ; 4,50 (t, 2H,J = 5,91 Hz), 7,73 (dd, 2H, J₁ = 1,97 Hz ; J₂ = 6,77 Hz) ; 8,02 (dd, 2H, J₁ = 1,77 Hz ; J₂ = 6,47 Hz)
***RMN ¹³C (50 MHz, Acétone D₆)*** : 22,96 ; 53,14 (4,1 Hz) ; 62,35 ; 64,29 ; 128,01 ; 129,52 ; 131,85 ; 132,24 ; 165,61
***RMN ¹⁹F (282 MHz, Acétone D₆)*** : -150.16(s, B-F)

| **Spectrométrie de masse (FAB) pour C₂₆H₃₈F₄N₂O₄Br₂B** | |
|---|---|
| **Masse Théorique calculée pour (2C⁺, BF4⁻)** | 689,1228 |
| **Masse Trouvée** | 689,1234 |

### * 7e :

A un mélange de l'alcool 1b dans 100 ml de CH₃CN on ajoute : 1,5 eq de DCC 1,5 eq de l'acide 4-vinylbenzoïque en présence de 0,02 eq de DMAP. Le mélange réactionnel est agité pendant 4 heures à température ambiante puis évaporé à sec. L'ester formé est extrait par 3 × 30 ml d'eau. A cette solution on ajoute 1,5 eq de NaBF₄ en solution dans l'eau. Le mélange réactionnel est agité deux heures à température ambiante. Le solide blanc obtenu après filtration est lavé à l'eau (pour éliminer l'excès de NaBF₄) puis deux fois par 30 ml d'éther et enfin séché sous vide.

| | | |
|---|---|---|
| Aspect du produit : solide blanc | **Rdt** = 75 % | **Pf** = 138-140°C |

***RMN ¹H (300 MHz, Acétone D₆)***: 2.30-2.48 (m, 2H) ; 3.38 (S, 9H) ; 3.69-3.82 (m, 2H) ; 4.36 (t, 2H, J=6.0 Hz), 6.58 (d, 1H, J=16.1Hz) ; 7.40-7.50 (m, 3H) ; 7.63-7.78 (m, 3H).
***RMN ¹³C (75MHz, Acétone D₆)*** : 22.68 ; 52.79 (t, J= 3.8 Hz) ; 60.88 ; 63.86 (t, J= 3.32Hz) ; 117.76 ; 128.20 ; 128.95 ; 130.43 ; 134.42 ; 144.86 ; 165.99.

| **Spectrométrie de masse (APCI) pour [C₁₅ H₂₂NO₂][BF₄]** | |
|---|---|
| **Masse Théorique calculée pour (C⁺)** | 248.3 |
| **Masse Trouvée** | 248.2 |

### * 7f :

### Méthode 1

A une solution de 5 g d'acide 4-aminobenzoïque (36,46 mmol) dans 30 ml de 3-chloropropanol, on ajoute, goutte à goutte et à 0°C, 5 ml de chlorure de thionyle (68,54 mmol). Le mélange réactionnel est ensuite porté à 100°C pendant 3 heures. 50 ml d'éther sont ensuite ajoutés et le solide blanc ainsi formé est filtré puis lavé par 3 × 10 ml d'éther. Le solide est ensuite dissous dans 30 ml d'eau et l'ester est ensuite extrait par 3 × 20 ml d'acétate d'éthyle. La phase organique est enfin lavée par 20 ml d'une solution saturé de K₂CO₃ et évaporée.

Le mélange constitué du solide blanc obtenu, de 5 ml d'une solution de triméthylamine à 45% dans l'eau et de 0.5g de NaI sont dissout dans 20 ml d'acétonitrile. Puis porté au reflux pendant 16 heures. Après quoi le solvant est évaporé et le produit est cristallisé dans l'acétone.

| | | |
|---|---|---|
| Solide blanc | Rdt (en deux étapes)= 58% | Pf= 120-122°C |

### Méthode 2

A un mélange de l'alcool 1b dans 100 ml de CH₃CN on ajoute : 1,5 eq de DCC 1,5 eq de l'acide 4-nitrobenzoïque en présence de 0,02 eq de DMAP. Le mélange réactionnel est laissé sous agitation 4 heures à température ambiante puis évaporé à sec et l'ester formé est extrait par 3 × 30 ml d'eau. La réduction du groupement nitro est réalisée en ajoutant à cette solution 0,01 eq de Pd/C 5% et sous agitation à température ambiante sous une pression de 5 bars d'hydrogène pendant 8 heures. Après filtration et évaporation de l'eau on recristallise le produit dans l'acétone.
Rdt= 90%
***RMN ¹H (200 MHz, D₂O)*** : 2,1-2,25 (m, 2H) ; 3,09 (s, 9H) ; 3,32-3,45 (m, 2H) ; 4,48 (t, 2H, J = 6,15 Hz) ; 6,75 (d, 2H, J = 7,78 Hz) ; 7,72 (d, 2H, J = 7,78 Hz).
***RMN ¹³C (50 MHz, D₂O)** :* 22,61 ; 30,63 ; 53,37 (t, J_{C-N} = 4,1 Hz) ; 61,88 ; 64,23 ; 114,91 ; 118,20 ; 132,02 ; 153,03 ; 168,80

### * 7g :

A une solution de 1 g (3,67 mmol) de 7f dans 10 ml d'eau on ajoute 4,77 mmol de LiNTf₂ en solution dans l'eau. Après 2 heures d'agitation à température ambiante, le solide blanc obtenu est filtré puis séché.

| | | |
|---|---|---|
| Solide blanc | Rdt = 93% | Pf = 109-110°C |

***RMN ¹H (200 MHz, Acétone D₆)*** : 2,35-2,51 (m, 2H) ; 3,41 (s, 9H) ; 3,78-3,92 (m, 2H) ; 4,5 (t, 2H, J = 6,1 Hz) ; 5,51 (signal large, 2H) ; 6,58 (d, 2H, J = 7,9 Hz) ; 7,84 (d, 2H, J₁ = 7,7 Hz)
***RMN ¹³C (50 MHz, Acétone D₆)*** : 24,17 ; 29.12 ; 54,12 (t, J_{C-N} = 4,1 Hz) ; 61,72 ; 65,60 ; 114,25 ; 118,33 ; 121,17 (q, J_{C-F} = 320,9 Hz) ; 132,72 ; 154,74 ; 167,08

### * 7h :

A un mélange de l'alcool 1b dans 100 ml de CH₃CN on ajoute : 1,5 eq de DCC, 1,5 eq de 4-carboxybenzaldéhyde et 0,2 eq de DMAP. Le mélange réactionnel est agité 4 heures à température ambiante puis évaporé à sec. L'ester formé est extrait par 3 × 30 ml d'eau. A cette solution de l'ester dans l'eau on ajoute 1,5 eq de HBF₄ en solution à 50% dans l'eau. Le mélange réactionnel est agité deux heures à température ambiante. Le solide jaune obtenu après filtration est lavé par l'eau (pour éliminer l'excès de HBF₄) puis deux fois par 30 ml d'éther et enfin séché sous vide.

| | | |
|---|---|---|
| Aspect du produit : solide jaune | **Rdt** = 90% | **Pf** = 146-148°C |

***RMN ¹H (200 MHz, CD₃CN)*** : 2,10-2,33 (m, 2H) ; 3,05 (s, 9H) ; 3,36-3,55 (m, 2H) ; 4,45 (t, 2H, J=5,8 Hz), 8,00 (dd, 2H, J₁=1,6Hz, J₂=6,6Hz) ; 8,13 (dd, 2H, J₁=1,4Hz, J₂=8,3 Hz) ; 10,10 (s, 1H).
***RMN ¹³C (50MHz, CD₃CN)* :** 22,08 ; 52,66 (t, J= 3,8Hz) ; 61,53 ; 63,52 (t, J= 3,02Hz) ; 129,10 ; 129,80 ; 134,39 ; 139,27 ; 164,89 ; 192,04.

| **Spectrométrie de masse (APCI) pour [C₁₄ H₂₀NO₃][BF₄]:** | | |
|---|---|---|
| | **Masse Théorique calculée pour (C⁺)** | 250,3 |
| | **Masse Trouvée** | 250,2 |

### 4/ Alcyne supporté :

| | |
|---|---|
| **8** | 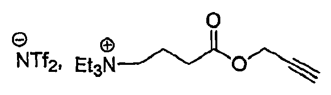 |

A une solution de 25 mmol d'alcool propargylique dans 25 ml de chlorure de méthylène anhydre et 5eq de Et₃N sont ajoutés 0,9eq du chlorure de 4-chlorobutyryle à 0°C au goutte à goutte. Le mélange réactionnel est agité 3 heures à température, évaporé à sec et l'ester formé est isolé après extraction par 3 × 10 ml d'éther suivie d'une distillation au four à boules (60°C, 0.2mmHg). Le sel obtenu après réaction de quaternarisation à 80°C en présence de la triéthylamine, de l'acétonitrile et de NaI est engagée dans la réaction de métathèse dans l'eau en présence de LiNTf₂ pour donner le produit 8.

| | |
|---|---|
| Aspect du produit : huile orange | Rdt = 85 % |

***RMN ¹H (200 MHz, Acétone D₆)*** : 1,36-1,55 (m, 9H); 2,05-2,30 (m, 2H) ; 2,65 (t, J=6,7 Hz, 2H) ; 3,05-3,15 (m, 1H) ; 3,39-3,68 (m, 8H) ; 4,76 (d, J=2,5Hz, 2H).
***RMN ¹³C (50 MHz, Acétone D₆)*** : 7,13 ; 17,20 ; 52,19 ; 53,19 (t, J=2,8Hz) ; 56,07 ; 120,46 (q, J_{CF}=321,4 Hz) ; 171,68.

| **Spectrométrie de masse (APCI) pour [C₁₃ H₂₄NO2][C₂NS₂O₄F₆]** | |
|---|---|
| **Masse Théorique calculée pou**r **(C⁺)** | 226.3 |
| **Masse Trouvée** | 226.4 |

### III) SELS D'ONIUM À ANION FONCTIONNALISÉ :

| | |
|---|---|
| **9** | 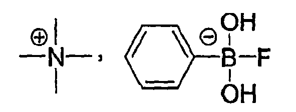 |

Dans un ballon monocol de 10 mL, on introduit 0,1 g (2,1 mmol) de fluorure de tétraméthylammonium anhydre. Puis on ajoute 1 mL de THF anhydre et on homogénéise la solution en chauffant si nécessaire. Enfin, on introduit 0,13 g d'acide phénylboronique (2,1 mmol). On laisse agiter durant environ 2 heures à température ambiante.

Après 2 heures d'agitation, on rajoute de l'éther anhydre puis on filtre le solide sur verre fritté. Le solide est lavé 2 à 3 fois par 20 ml d'éther et pour finir, il est placé sous vide pour le sécher.

| | | |
|---|---|---|
| Solide blanc | **Rdt** = 82% | **Pf** = 162-164°C |

***RMN ¹H (200 MHz, Acétone D₆)*** : 3,15 (s, 12H); 6,8-7,4 (m, 3H); 7,50-7,70 (m, 2H)
***RMC ¹³C (50 MHz, Acétone. D₆)*** : 56,19 (t, J_{C-N} = 3,97 Hz) ; 127,47 ; 128,36 ; 130,91 ; 132,98 ; 135,96
***Spectre RMN ¹⁹F (300 MHz, Acétone. D₆)*** : -136,40 (multiplet)
***Spectre RMN ¹¹B (300 MHz, Acétone. D₆)*** : 4,66 (D, J _{B-F} = 27,4 Hz) (56%) ; 28,5 (44%)

### IV) ESTERS DÉRIVÉS DE LA GLYCINE :

| | |
|---|---|
| **10** | 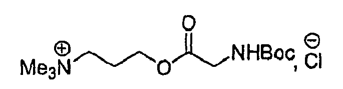 |

2,0 g (11,3 mmol) de N-Boc-glycine et 1,6 g de **1b** (11,2 mmol) sont dissous dans 15 ml de chlorure de méthylène, on ajoute 11,2 mmol de la DCC et 5% molaire de DMAP. Le mélange est agité à température ambiante pendant 18 heures. Après filtration du précipité formé au cours de la réaction, le filtrat est évaporé à sec et le produit obtenu est lavé par 5 × 20 ml d'éther puis séché sous vide.

| | |
|---|---|
| Huile incolore | **Rdt** = 92% |

***RMN ¹H (200 MHz, D₂O)*** : 1,39 (s, 9H) ; 2,4-2,5 (m, 2H) ; 3,08 (s, 9H) ; 3,35-3,45 (m, 2H) ; 3,75 (s, 2H) ; 3,32 (t, 2H, J = 6,9Hz)
***RMN ¹³C (50 MHz, D_{2O})*** : 23,82 ; 39,07 ; 47,31 ; 53,69 (t, J_{C-N} = 4.0 Hz) ; 61,62 ; 65,33 ; 166,92 ; 178,06

| | |
|---|---|
| **11** | 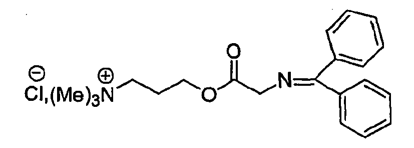 |

On fait barboter de l'acide chlorhydrique dans une solution de l'ester **10** dans 15 ml de chlorure de méthylène pendant 6 heures. Le précipité blanc qui se forme après 24 heures d'agitation à température ambiante est lavé deux fois à l'éther. Le produit ainsi obtenu est ajouté à une solution de diphénylméthylène imine (2,0 g ; 11 mmol) dans 15 ml de chlorure de méthylène. Le mélange réactionnel est ensuite agité à température ambiante pendant 24 heure. On filtre le précipité qui se forme; on évapore le solvant puis on lave par 3 × 10 ml d'éther.
**Rdt en 2 étapes : 62%**
***RMN ¹H (200 MHz,D₂O)*** : 2,49-2,52 (m, 2H) ; 3,18 (s, 9H) ; 3,43-3,49 (m, 2H) ; 4,19 (s, 2H) ; 4,31 (t, 2H, J = 6,9 Hz) ; 7,18-7,67 (m, 8H) ; 7,82 (m, 2H)
***RMN ¹³C (50 MHz, D₂O)*** : 23,89 ; 54,03 ; 55,14 (t, J_{C-N} = 4,0 Hz) ; 61,98 ; 65,74 ; 127,98 ; 128,33 ; 128,94 ; 129,19 ; 130,67 ; 130,93 ; 140,03 ; 167,68 ; 177,38

### V) SELS D'ONIUM BIFONCTIONNELS :

### 1/ Synthèse des sels 12 :

| | |
|---|---|
| **12** | 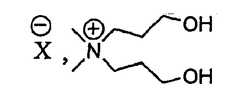 |

| | |
|---|---|
| **12a** | **X = Cl** |
| **12b** | **X = NTf₂** |

### * 12a :

A une solution de 4,5g de diméthylamine (0,1 mole) à 45 % dans l'eau est mélangée à 30 ml d'acétonitrile. On ajoute 17 g de K₂CO₃, 150 mg de NaI et 25 ml (0,3 mol) de chloropropanol. Le mélange réactionnel est chauffé à 70°C durant une nuit. Après évaporation de l'acétonitrile on extrait le sel par 3 × 10ml de méthanol et il est ensuite cristallisé dans l'acétone.

| | | |
|---|---|---|
| Solide blanc | **Rdt** = 87% | **Tf**=112-114°C |

***RMN ¹H (200 MHz, D₂O)*** : 1,7-1,85 (m, 4H) ; 2,85 (s, 6H) ; 3,05-3,2 (m, 4H) ; 3,4 (t, 4H, J = 6,1 Hz)
***RMN ¹³C (50 MHz, D₂O)*** : 25,25 ; 51,10 (t, J_{C-N} = 4,1 Hz) ; 58,94 ; 62,01 (t, J_{C-N} = 4,0 Hz)

| **Spectrométrie de masse (FAB) pour C₁₆H₄₀N₂O₄Cl** | |
|---|---|
| **Masse Théorique calculée pour (2C^{+,} Cl⁻)** | 359,2677 |
| **Masse Trouvée** | 359,2675 |

### * 12b :

Dans un ballon de 100ml on introduit une solution de 5,0 g (25,30 mmol) de **12a** dans 10 ml d'eau distillée. A cette solution on ajoute une solution de 10 g (32,89 mmol) de LiNTf₂ dans l'eau.

Après 2 heures d'agitation à température ambiante on évapore l'eau et on extrait le produit par 3 × 10 ml d'acétone.

| | |
|---|---|
| Huile incolore | Rdt = 90% |

***RMN ¹H (200 MHz, D₂O)* :** 1,82-2,05 (m, 4H) ; 3 (s, 6H) ; 3,25-3,40 (m, 4H) ; 3,46(t, 4H, J = 6,9 Hz)
***RMN ¹³C (50 MHz, D₂O)* :** 25,34 ; 51,09 ; 58,53 ; 62,13 ; 121,05 (q, J = 321,2 Hz)

### 2/ Synthèse des diesters 13 :

| | |
|---|---|
| **13** | 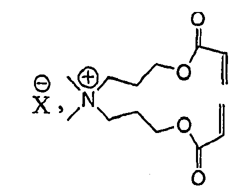 |

### * X = NTf₂

Une solution de 2,7 g (6,02 mmol) de **12b** et de 6 équivalents de chlorure d'acryloyle dans l'acétonitrile est chauffée à 80°C en présence de 10 équivalents de K₂CO₃ solide pendant 2 heures. Le mélange est ensuite placé sous vide à 40°C pour éliminer le solvant et l'excès du réactif. L'acrylate d'ammonium ainsi obtenu par extraction par le dichlorométhane est stable à 4°C et peut être stocké pendant plusieurs mois.

| | |
|---|---|
| Huile jaune pâle | **Rdt** = 87 % |

***RMN ¹H (200 MHz, Acétone D₆)*** : 2,3-2,5 (m, 4H) ; 3,42 (s, 6H) ; 3,72-3,82 (m, 4H) ; 4,32 (t, 2H, J= 5,8 Hz) ; 5,95 (dd, 2H, J₁ = 1,8 ; J₂ = 10,1) ; 6,18 (dd, 2H, J₁ = 10,1 Hz ; J₂ = 17,1 Hz) ; 6,4 (dd, 2H, J₁ = 2,1 Hz ; J₂ = 17,1 Hz)
***RMN ¹³C (50 MHz, Acétone D₆)*** : 22.52 ; 51,14 ; 61,89 ; 61,95 ; 121,05 (q, J=321,2Hz) ; 128,78 ; 131,02 ; 165,81

| **Spectrométrie de masse (FAB) pour C₁₄H₂₄O₄N** | |
|---|---|
| **Masse Théorique calculée pour (C⁺)** | 270,1750 |
| **Masse Trouvée** | 270,1703 |

### 2/ Synthèse des diesters 14 :

| | |
|---|---|
| **14** | 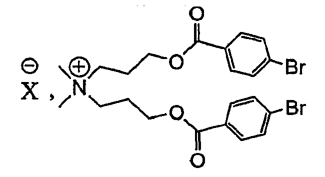 |

| | |
|---|---|
| **14a** | **X = Cl** |
| **14b** | **X = BF₄** |

### * 14a :

Dans un ballon de 100 ml on introduit 1 g (5,06 mmol) de chlorure de **12a,** 10 ml d'acétonitrile, 4,3 g de K₂CO₃ en poudre et 4,2 g (19,14 mmol) du chlorure d'acide du 4-bromobenzoïque. Après une nuit d'agitation à température ambiante, on filtre et on lave K₂CO₃ par 3 fois 15 ml de chlorure de méthylène et enfin on évapore à sec. On reprend à l'eau et on élimine l'excès de l'acide 4-bromobenzoïque qui cristallise par filtration. Le produit est ensuite cristallisé dans l'acétone après évaporation d'eau.

| | | |
|---|---|---|
| Solide blanc | **Rdt** = 60% | **Pf**= 112-114°C |

***RMN ¹H (200 MHz, MeOH-d₄)*** : 2,05-2,25 (m, 4H) ; 3,1 (s, 6H) ; 3,35-3,53 (m, 4H) ; 4,21 (t, 4H, J = 5,8 Hz) ; 7,1 (d, 4H, J = 8,6 Hz) ; 7,35 (d, 4H, J₁ = 8,6 Hz).
***RMN ¹³C (50 MHz, MeOH-d₄)*** : 23,87 ; 52,50 ; 62,50 ; 63,64 ; 130,08 ; 130,16 ; 132,78 ; 133,63 ; 168,22

| **Spectrométrie de masse (FAB) pour C₂₂H₂₆NO₄Br₂** | |
|---|---|
| **Masse Théorique calculée pour (C⁺)** | 526,0229 |
| **Masse Trouvée** | 526,0221 |

### * 14b :

A une solution de 100 mg (0,18 mmol) de **14a** dans 5ml d'eau on ajoute 0,2 ml de HBF₄ en solution dans l'eau à 40 %. Après l'ajout de ce dernier, on observe la formation d'un solide blanc. Le mélange réactionnel est agité deux heures à température ambiante. Le solide blanc obtenu après filtration est lavé à l'eau (pour éliminer l'excès de HBF₄) puis deux fois par 30 ml d'éther et enfin séché sous vide.

| | | |
|---|---|---|
| Solide blanc | **Rdt** = 80% | **Pf**= 172-174°C |

***RMN ¹H (200 MHz, Acétone D₆)*** : 2,4-2,57 (m, 4H) ; 3,4 (s, 6H) ; 3,8-3,94 (m, 4H) ; 4,45 (t, 4H, J = 5,8 Hz) ; 7,67 (d, 4H, J = 8,6 Hz) ; 7,95 (d, 4H, J₁ = 8,6 Hz)
***RMN ¹³C (50 MHz, Acétone D₆)*** : 23,55 ; 51,99 (t, J = 4,1 Hz) ; 62,81 ; 63,12 ; 128,92 ; 130,41 ; 132,65 ; 133,14 ; 166,36

| **Spectrométrie de masse (FAB) pour C₂₂H₂₆NO₄Br₂** | |
|---|---|
| **Masse Théorique calculée pour (C⁺)** | 526,0229 |
| **Masse Trouvée** | 526,0216 |

### VI) SELS D'ONIUM TRI- ET TÉTRAFONCTIONNELS PORTANT DES FONCTIONS IDENTIQUES :

### 1/ Sels trifonctionnels 15 :

| | |
|---|---|
| **15** | 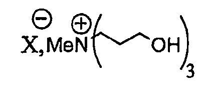 |

| | |
|---|---|
| **15a** | **X = Cl** |
| **15b** | **X = NTf₂** |

### * 15a :

Dans un tube de Schlenk, on introduit 3 ml (87,15 mmol) de méthylamine, 20 ml d'acétonitrile, 12 g de K₂CO₃, 53 mg de NaI et 12 ml de 3-chloropropanol. Le mélange réactionnel est chauffé à 80°C durant 48 heures. Après évaporation du solvant on extrait le sel par 3 × 10ml du méthanol. Il est ensuite cristallisé dans l'acétone après évaporation du solvant.

| | | |
|---|---|---|
| Solide blanc | **Rdt** = **75%** | **Tf**> 260°C |

***RMN ¹H (200 MHz, D₂O)*** : 1,8-2,03 (m, 6H) ; 3,1 (s, 3H) ; 3,25-3,4 (m, 6H) ; 3,4 (t, 6H, J = 5,8 Hz)
***RMN ¹³C (50 MHz, D₂O)*** : 24,89 ; 48,6 ; 58,47 ; 59,60

### * 15b :

Dans un ballon de 100 ml on introduit une solution de 0,4 g (1,65 mmol) de **15a** dans 1 ml d'eau distillée. A cette solution on ajoute une solution de 0,7 g (2,14 mmol) de LiNTf₂ dans l'eau. Après 2 heures d'agitation à température ambiante, on évapore l'eau et on extrait **14b** par 3 × 10 ml d'acétone. Après évaporation du solvant et séchage sous vide, on obtient 0,64 g d'une huile incolore soit un rendement de 80%.
***RMN ¹H (200 MHz, MeOH-d₄)*** : 1,92-2,01 (m, 6H) ; 3,1 (s, 3H) ; 3,38-3,52 (m, 6H) ; 3,7 (t, 6H, J = 5,5 Hz)
***RMN ¹³C (50 MHz, MeOH-d₄)*** : 25,25 ; 29,92 ; 58,35 ; 59,80 ; 121,05 (q, J = 321,2 Hz)

### 2/ Sels tétrafonctionnels 16 :

| | |
|---|---|
| **16** | 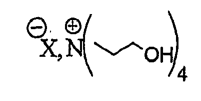 |

| | |
|---|---|
| **16a** | **X = Cl** |
| **16b** | **X = NTf₂** |

### * 16a :

Dans un ballon de 100 ml on introduit 1 g (5,23 mmol) de propanolamine et 2 ml (23,90 mmol) de 3-chloro-1-propanol. Après une nuit de chauffage du mélange réactionnel à 150°C, on élimine l'excès de chloropropanol par lavage à l'éther (3 × 10ml). On isole ainsi le produit par filtration après cristallisation dans l'acétone.

| | |
|---|---|
| Solide blanc | **Rdt** = 58% |

***RMN ¹H (200 MHz, D₂O)*** : 2-2,2 (m, 8H) ; 3,4-3,6 (m, 8H) ; 3,8(t, 8H, J = 5,7 Hz)
***RMN ¹³C (50 MHz, D₂O)*** : 24,49 ; 56,56 ; 58,40

### * 16b :

Dans un ballon de 100ml on introduit une solution de 1 g (3,5 mmol) de **16a** dans 5 ml d'eau distillée. A cette solution on ajoute une solution de 2 g (4,55 mmol) de LiNTf₂ dans l'eau. Après 2 heures d'agitation à température ambiante on évapore l'eau et on extrait **15b** par 3 × 5 ml de méthanol. Après évaporation du solvant et séchage sous vide, on obtient une huile incolore.

| | |
|---|---|
| Huile incolore | **Rdt**= 85%, |

***RMN ¹H (200 MHz, D₂O)*** : 1,7-1,93 (m, 8H) ; 3,15-3,30 (m, 8H) ; 3,58 (t, 8H, J = 5,8 Hz)
***RMN ¹³C (50 MHz, D₂O)*** : 24,56 ; 56,67 ; 58,38 ; 121,05 (q, J = 321,2 Hz)

### VII) SYNTHÈSE DES SELS D'ONIUM PLURIFONCTIONNELS PORTANT DES FONCTIONS DIFFÉRENTES :

### 1/ Préparation des amines tertiaires précurseurs 17 :

| | |
|---|---|
| **17** | 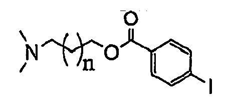 |

| | |
|---|---|
| **17a** | n = 1 |
| **17b** | n = 2 |

### * 17a :

Dans un ballon de 100 ml, on introduit 1 g (9,7 mmol) de 1-diméthylanaino-3-propanol, 10 ml de chlorure de méthylène anhydre, 3 g de K₂CO₃ et 2,84 (10,66 mmol) de chlorure dérivé de l'acide 4-iodobenzoïque. Après 3 heures d'agitation à température ambiante la réaction est totale. Le mélange réactionnel est ensuite filtré et évaporé à sec. On obtient un solide blanc avec un rendement de 90% (Pf = 48-50°C).
***RMN ¹H (200 MHz, CDCl₃)*** : 1,87-2,03 (m, 2H) ; 2,3(s, 6H) ; 2,45 (t, 2H, J = 7,0 Hz) ; 4 (t, 2H, J = 6,5 Hz) ; 7,3-7,5 (m, 4H)
***RMN ¹³C (50 MHz CDCl₃)*** : 27,36 ; 45,85 ; 56,58 ; 63,96 ; 101,15 ; 130,17 ; 131,37 ; 138,04 ; 166,34

### * 17b :

Dans un ballon de 100 ml, on introduit 2,8 g (23,93 mmol) de 1-N,N'-diméthylamino-4-butanol, 10 ml de chlorure de méthylène anhydre, 6,6g de K₂CO₃ et 7,0 g (26,27 mmol) de chlorure d'acide. La réaction est exothermique. Après 3 heures d'agitation à température ambiante la réaction est totale. Le mélange réactionnel est ensuite filtré et évaporé à sec pour donner un solide blanc, avec un rendement de 88%.
***RMN ¹H (200 MHz, CDCl₃)*** : 1,78-1,9 (m, 4H) ; 2,5(s, 6H) ; 2,65 (t, 2H, J = 5,3 Hz) ; 4,35 (t, 2H, J = 6,0 Hz) ; 7,7-7,85 (m, 4H)
***RMN ¹³C (50 MHz CDCl₃)*** : 20,25 ; 24,86 ; 43,51 ; 57,34 ; 63,47 ; 98,97 ; 128,39 ; 129,39 ; 136,16 ; 164,26

### 2/ Synthèse des sels 18 :

| | |
|---|---|
| **18** | 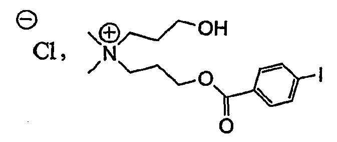 |

| | |
|---|---|
| **18a** | **X = Cl** |
| **18b** | **X = NTf₂** |

### * 18a :

Dans un ballon de 50 ml on introduit 1,0g (3 mmol) de **17a** et 0,5 ml de 3-chloro-1-propanol. Après 30 minutes de chauffage du mélange réactionnel à 110°C, on observe la formation d'un solide blanc. Ce dernier est isolé par filtration après cristallisation dans l'acétone et lavage par 3 × 10ml d'éther.

| | | |
|---|---|---|
| Solide blanc | **Rdt** = 94% | **Tf** = 180-182°C. |

***RMN ¹H (200 MHz, D₂O)*** : 1,8-2 (m, 2H) ; 2,10-2,30(m, 2H) ; 3,05 (s, 6H) ; 3,25-3,45(m, 4H) ; 3,6 (t, 1H, J = 0,7 Hz) ; 4,35(t, 2H, J = 0,7 Hz) ; 7,85 (d, 2H, J = 8,1 Hz) ; 8 (d, 2H, J= 8,1 Hz)
***RMN ¹³C (50 MHz, D₂O)*** : 22,18 ; 25,29 ; 51,27 (t, J = 4,3 Hz) ; 58,42 ; 62,00 ; 101,85 ; 129,94 ; 131,13 ; 138,26 ; 165,49

| **Spectrométrie de masse (FAB) pour C₁₅H₂₃NO₃I** | |
|---|---|
| **Masse Théorique calculée pour (C⁺)** | 392,0723 |
| **Masse Trouvée** | 392,0720 |

### * 18b :

Dans un ballon de 100ml on introduit une solution de 1,0 g (2,34 mmol) de **18a** dans 5 ml d'eau distillée. A cette solution on ajoute une solution de 3,04 mmol de LiNTf₂ dans l'eau. Après 2 heures d'agitation à température ambiante on extrait **18b** par 3 × 5 ml de chlorure de méthylène qui est ensuite chassé sous vide pour donner un solide blanc.

| | | |
|---|---|---|
| Solide blanc | **Rdt** = 91% | **Tf** = 90-92°C |

***RMN ¹H (200 MHz, Acétone D₆)*** : 2,12-2,63 (m, 2H) ;2,45-2,63(m, 2H) 3,4 (s, 6H) ; 3,7-3,93 (m, 6H) ; 4,1 (t, 1H, J = 0,3 Hz), 4,55 (t, 2H,J = 0,7 Hz) ; 7,85 (d, 2H, J = 8,1 Hz) ; 8 (d, 2H, J = 8,1 Hz)
***RMN ¹³C (50 MHz, Acétone D₆)*** : 22,62 ; 26,03 ; 51,16 (t, J = 4,3 Hz) ; 58,56 ; 62,21 ; 100,96 ; 121,01 (q ; J_{CF3} = 321,0 Hz) ; 129,94 ; 131,51 ; 138,33 ; 165,79.

| **Spectrométrie de masse (FAB) pour C₃₂H₄₆N₃O₁₀F₆I₂S₂** | |
|---|---|
| **Masse Théorique calculée pour (2C⁺, NTf2⁻)** | 1064,0618 |
| **Masse Trouvée** | 1064,0607 |

### 3/ Synthèse des sels à fonctionnalités différentes 19 :

| | |
|---|---|
| **19** | 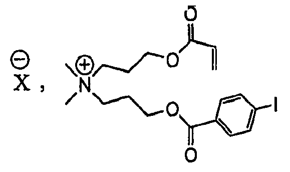 |

| | |
|---|---|
| **19a** | **X = Cl** |
| **19b** | **X = NTf₂** |

Une solution de 1 mmol de **18a** ou **18b** et de 3 équivalents de chlorure d'acryloyle dans l'acétonitrile est chauffée à 80°C en présence de 5 équivalents de K₂CO₃ solide pendant 2 heures. Le mélange est ensuite filtré puis évaporé sous vide à 40°C. L'ester d'ammonium est ensuite extrait par le chlorure de méthylène et stocké après évaporation du solvant à 4°C.

| | |
|---|---|
| **19a :** Solide blanc | **Rdt** =75 %. |

***RMN ¹H (200 MHz, D₂O)*** : 2,05- 2,32 (m, 4H) ; 3,1 (s, 6H) ; 3,35-3,55 (m, 2H) ; 4,18 (t, 2H, J = 5,5 Hz) ; 4,35 (t, 2H, J = 5,8 Hz) 5,90 (dd, 1H, J₁ = 1,9 Hz ; J₂ = 10,7) ; 6,05 (dd, 1H, J₁ = 17,2 Hz ; J₂ = 10,7 Hz) ; 6,3 (dd, 1H, J₁ = 1,9 Hz ; J₂ = 17,2 Hz) ; 7,62 (d, 2H, J = 8,6 Hz) ; 7,82 (d, 2H, J = 7,8 Hz)
***RMN ¹³C (50 MHz, D₂O)*** : 22,09 ; 22,86 ; 51,39 (t, J = 4,2 Hz) ; 62,13 ; 62,65 ; 63,03 ; 63,35 ; 101,78 ; 127,53 ; 128,92 ; 131,13 ; 133,05 ; 139,63 ; 168,07 ; 168,45

| **Spectrométrie de masse (FAB) pour C₁₈H₂₅NO₄I** | |
|---|---|
| **Masse Théorique calculée pour (C⁺)** | 426,0828 |
| **Masse Trouvée** | 446,0821 |

### 19b :

Dans un ballon de 100ml on introduit une solution de 1,0g (1,80 mmol) de **19a** dans 5 ml d'eau distillée. A cette solution on ajoute une solution de 2 mmol de LiNTf₂ en solution dans l'eau. Après 2 heures d'agitation à température ambiante on extrait **19b** par 3 × 5 ml de chlorure de méthylène. Après élimination du solvant sous vide, on obtient une huile incolore. **Rdt**= 78%
***RMN ¹H (200 MHz, Acétone D₆)*** : 2,31-2,65 (m, 4H) ; 3,5 (s, 6H) ; 3,75-3,93 (m, 4H) ; 4,32 (t, 2H, J = 6,0 Hz), 4,55 (t, 2H, J = 6,1 Hz) ; 5,95 (dd, 1H, J₁ = 1,9 Hz, J₂ = 10,3 Hz) ; 6,2 (dd ; 1H ; J₁ = 17,2 Hz ; J₂ =10,3 Hz) ; 6,43 (dd ; 1H ; J₁ =1,9 Hz ; J₂ = 17,2 Hz) ; 7,82 (d, 2H, J = 8,1 Hz) ; 7,78(d, 2H, J = 8,1 Hz)
***RMN ¹³C (50 MHz, Acétone D₆)*** : 23,49 ; 23,54 ; 52,12 (t, J = 4,31 Hz) ; 62,16 ; 62,89 ; 100,84 ; 121,09 (q, J_{CF3} = 321 Hz) ; 124,56 ; 129,38 ; 130,82; 132,22 ; 132,38 ; 139,23 ; 166,66.

### 34i :

A une solution de 1g (1,55 mmol) de 6b dans 10 ml du mélange CH₃CN/NEt₃ (2/1), on additionne 4 eq du phénylacétylène et 5% molaire de CuI. Le mélange réactionnel est laissé agité 5mn à température ambiante avant d'ajouter 2,5% de PdCl₂ (PPh₃)₂.

Après 15 minutes d'agitation à température ambiante, le mélange réactionnel est évaporé à sec et lavé à l'éther pour éliminer l'excès des réactifs. L'huile ainsi obtenue est solubilisée dans le chlorure de méthylène et ensuite lavée par un solution diluée de K₂CO₃ dans l'eau pour libérer Et₃N de son chlorhydrate formé au cours de la réaction. Après traitement par Na₂SO₄ et évaporation du chlorure de méthylène, le produit est isolé par filtration après cristallisation dans l'éther et séchage sous vide.

| | | |
|---|---|---|
| **Aspect du produit :** solide blanc | Rdt = 87% | Pf = 228-230°C |

***RMN ¹H (CD₃CN, 300Mhz)*** : 1,72-1,93 (m, 4H) ; 3,05 (s, 9H) ; 3,23-3,40 (m, 2H) ; 4,38 (t, J=6,1 Hz, 2H) ; 7,38-7,45 (m, 3H) ; 7,55-7,64 (m, 2H) ; 7,70 (dd, J=8,5 Hz, 2H) ; 8,10 (dd, J=8,5 Hz, 2H).
***RMN ¹³C (CD3CN, 75Mhz)*** : 19,17 ; 24,70 ; 52,61 (t, J=3,8 Hz); 63,86 ; 65,65 (t, J=3,0 Hz) ; 87,93 ; 91,93 ; 124,63 (q, J_{CF}=412 Hz) ; 128,42 ; 128,85 ; 129,24 ; 129,44 ; 131,20 ; 131,24 ; 165,63.

| **Spectrométrie de masse (APCI) pour [C₂₂ H₂₆NO₂][C₂NS₂O₄F₆]:** | |
|---|---|
| **Masse Théorique calculée pour (C⁺)** | 336,4 |
| **Masse Trouvée** | 336,0 |

### Applications à la synthèse - Protocole général des différentes réactions :

### 1/ réaction de Diels-Alder :

### a- Pour le cyclopentadiène :

Une solution de l'ester acrylique supporté (du mono au tétra-fonctionnalisé) et de 10 équivalents de cyclopentadiène dans 2 ml de chlorure de méthylène est agitée pendant deux heures à température ambiante, L'excès du réactif et le solvant sont ensuite éliminés sous vide.Le produit de réaction ainsi obtenu est mis en solution dans du méthanol en présence de trois gouttes d'acide chlorhydrique 12 N. Après douze heures au reflux, la transestérification est totale et le produit est alors extrait avec du pentane après évaporation de l'alcool sous vide. Le pentane est ensuite éliminé sous vide pour donner les esters méthyliques purs.

### b- Pour les divers autres diènes :

A une solution de 5a dans l'acétonitrile, on ajoute 0,01eq d'hydroquinone et 5eq de diènes. Le mélange réactionnel est chauffé à 120°C dans des tubes scellés, évaporé à sec puis lavé à l'éther. Le produit de réaction ainsi obtenu est mis en solution dans du méthanol en présence d'une quantité catalytique d'acide chlorhydrique 12 N. Après douze heures au reflux, le produit est alors extrait avec de l'éther après évaporation de l'alcool sous vide. L'éther est ensuite éliminé sous vide pour donner les esters méthyliques purs.

### 2/ réaction de Heck :

### a- Avec l'ester acrylique supporté 5 :

1,5 mmol de substrat supporté sont dissous dans 2 ml de solvant. A cette solution, on ajoute 5 équivalents d'halogénure d'aryle, 1 équivalent de K₂CO₃ comme base et 1 % molaire d'acétate de palladium. A la fin de la réaction on élimine le solvant et l'excès du réactif par lavage à l'éther puis on ajoute le méthanol (2ml), l'acide chlorhydrique 12 N (3 gouttes) et on porte à reflux.

Après 12 heures, le produit de couplage est extrait à l'éther après évaporation de l'alcool. La solution éthérée est ensuite évaporée à sec conduisant au produit attendu.

### b- Avec l'ester iodoaryle supporté 6g :

0,5 mmol de substrat supporté 6g est dissout dans 1 ml de DMF. A cette solution, on ajoute 5 éq d'oléfine (acrylate de tertiobutyle, diméthylacrylamide ou styrène), 1.5éq de K₂CO₃ comme base et 1 à 5% molaire d'acétate de palladium. A la fin de la réaction, on élimine le solvant et l'excès du réactif par lavage à l'éther puis on ajoute le méthanol (2 ml), l'acide chlorhydrique 12N (3 gouttes) et on porte à reflux.

Après 12 heures, le produit de couplage est extrait à l'éther après évaporation de l'alcool et neutralisation du milieu par l'ajout d'une solution diluée de K₂CO₃ dans l'eau. La solution éthérée est ensuite évaporée à sec.

### c- Avec le styrène supporté 7e :

A une solution de 800 mg (2.38mmole) de 7e dans 3 ml de DMF, nous ajoutons 1 ml de NEt₃ (3eq), 27mg de Pd(OAc)₂ (0,05eq) et 0,125eq de chaque iodure utilisé. Après deux heures de chauffage à 110°C, l'huile ainsi obtenue après l'ajout de l'éther est transestérifiée en présence du méthanol et de quelques gouttes de l'acide chlorhydrique.

### 3/ Réaction de couplage de Suzuki :

### Procédure générale pour les sels mono et bifonctionnalisés :

A une solution de 100 mg de l'halogénure d'aryle supporté dans 1ml de DMF on additionne 0,95 équivalents de l'acide boronique (par fonction), 2 équivalents de K₂CO₃ et 1% molaire d'acétate de palladium. Le mélange réactionnel est chauffé 5 heures à 80°C. Après quoi, on ajoute un alcool et on porte le mélange à reflux pendant 12 heures, en présence de 0,1 ml d'acide chlorhydrique concentré (12 N). Après transestérification et évaporation de l'alcool, l'ester formé est extrait du milieu par lavage à l'éther (3 × 10 ml).

### 4/ Réaction de couplage de Sonogashira :

### a- Pour l'ester iodoaryle supporté :

A une solution de 100 mg de l'halogénure d'aryle supporté dans 1 ml de solvant on additionne 4 équivalents de l'alcyne, 1 équivalent de K₂CO₃ et un mélange (1/2 catalyseur/CuI). Le mélange réactionnel est chauffé 1 heure à 40°C. Après quoi, on ajoute un alcool et on porte le mélange à reflux pendant 12 heures, en présence de 0,1 ml d'acide chlorhydrique concentré (12 N). Après transestérification et évaporation de l'alcool, l'ester formé est extrait du milieu par lavage à l'éther (3 × 10 ml) et isolé après évaporation de l'éther.

### b- Pour l'alcyne supporté :

A une solution de 100 mg de l'alcyne supporté 8 dans 1 ml du mélange CH₃CN/NEt₃ (2/1) on additionne 4 eq de l'iodure d'aryle et 0,2 eq de CuI. Le mélange réactionnel est agité 5 mn avant d'ajouter 0,1eq de PdCl₂(PPh₃)₂. Après réaction, les alcools formés sont extraits à l'éther après évaporation du solvant et élimination de l'excès des réactifs suivie d'une réaction de saponification en présence de 5ml de NaOH(2N).

### 5/ Alkylation de la Base de Schiff supportée :

A un mélange de la base de Schiff supportée **11** (1g ; 2,6mmol), et de 2 équivalents de K₂CO₃ dans l'acétonitrile (2 ml) on ajoute le dérivé halogéné RX (4 mmol) à température ambiante. Le mélange est ensuite porté à reflux sous une agitation vigoureuse. Après 12 heures, le milieu réactionnel est filtré puis évaporé à sec.

La transestérification et l'hydrolyse de l'imine sont réalisées au reflux du méthanol en présence d'acide chlorhydrique concentré pendant 12 heures. Après évaporation du solvant le mélange est dissous dans 1 ml d'eau. L'aminoacide libre est extrait par le dichlorométhane après neutralisation du milieu.

### 6/ Réaction multicomposant de type Grieco :

Sous courant d'argon on mélange 100 mg de l'amine **34** supporté, 500 µl d'une solution 1 M de l'aldéhyde dans l'acétonitrile, 500 µl d'une solution 1 M de cyclopentadiène dans l'acétonitrile et 50 µl d'une solution de 1% de TFA dans l'acétonitrile. Le mélange réactionnel est agité une nuit à température ambiante. Après évaporation à sec et lavage à l'éther, on obtient un solide.

### 7/ Synthèse des oléfines tétrasubstituées :

A une solution de 100 mg (0,16 mmole) de 34i dans 0,5 ml du mélange DMF/H₂O, on ajoute 2eq de l'iodure, 3eq de l'acide boronique et 1,2 mg de PdCl₂(PPh₃)₂ (0,01eq). Après trois heures de chauffage à 100°C, l'huile orange obtenue après ajout de l'éther est solubilisée dans 10ml de chlorure de méthylène et lavée par2 × 3ml d'H₂O. Après traitement de la phase aqueuse par MgSO₄ et évaporation à sec, les oléfines tétrasubstituées sont isolées par filtration après cristallisation dans l'éther.

### EXEMPLES

Pour montrer l'intérêt des sels d'ammonium comme nouveaux supports solubles, les réactifs **5, 6, 7** et **11** permettant de mettre en oeuvre plusieurs types de réactions fondamentales en chimie organique ont été choisis:
- les esters acryliques **5** ont été engagés dans des réactions de cycloadditions et de couplages 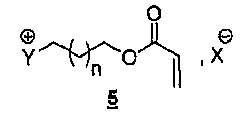
- un ester arylique substitué par un R (Br, I, ou CH2=CH) sur le noyau aromatique qui a été testé dans trois exemples de réactions de couplage. 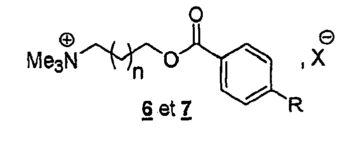
- un ester arylique substitué par un R = NH₂ sur le noyau aromatique qui a été testé dans la réaction de Griéco. 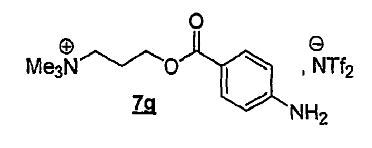
- la base de Schiff dérivée de la glycine qui, après alkylation, conduira à des aminoacides supérieurs 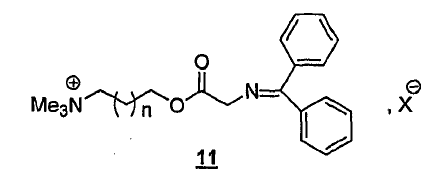
- un ester arylique substitué par un R = PhC≡CH sur le noyau aromatique qui a été testé dans la synthèse des oléfines tétrasubstituées.

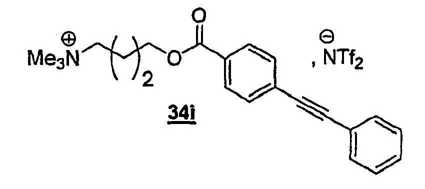

Afin d'augmenter la charge spécifique des sels d'onium, les présents Inventeurs ont également développé de nouveaux sels d'onium portant plus d'un bras fonctionnalisé.

La synthèse de supports et de réactifs supportés ainsi que leurs applications dans quelques exemples est décrite en détail dans la partie expérimentale qui suit.

### A - Exemple 1 : Réaction de Diels-Alder :

### 1) Réaction avec le cyclopentadiène.

La richesse et le potentiel synthétique de la réaction de Diels-Alder ont incité les chimistes à rechercher des méthodes permettant d'en augmenter d'une part la vitesse et le rendement, d'autre part la régio et la stéréosélectivité. Cette réaction est le premier exemple choisi pour montrer l'efficacité de la synthèse supportée sur sels d'onium.

La réaction de Diels-Alder entre un diénophile supporté sur un sel d'onium **5** et le cyclopentadiène dans le chlorure de méthylène comme solvant a donc été étudiée selon le schéma suivant :

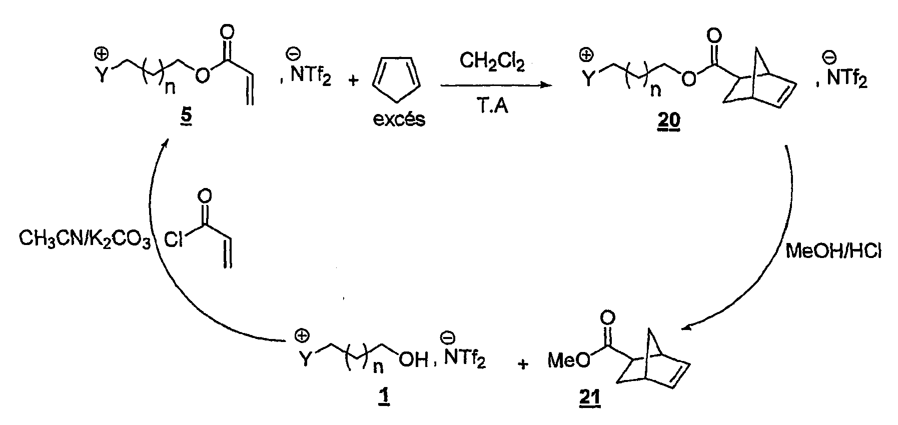

Dans cette partie du travail les Inventeurs ont étudié de façon précise l'influence de :
1) la longueur de chaîne carbonée qui sépare la fonction acryloyle de la fonction ammonium
2) la nature du cation et de l'anion sur la réactivité
3) le recyclage du support

### Procédure pour la réaction de Diels-Alder :

L'acrylate supporté **5** et 10 équivalents du cyclopentadiène sont dissous dans 2 ml de chlorure de méthylène. La solution est ensuite agitée pendant deux heures à température ambiante. Le solvant et l'excès de réactif sont ensuite éliminés sous vide puis le produit de réaction est solubilisé dans du méthanol en présence de quelques gouttes d'acide chlorhydrique 12 N. Après douze heures à reflux, la transestérification est totale et le produit **21** est alors extrait avec du pentane.

### Les résultats obtenus sont regroupés dans le tableau 1 :

**Tableau 1 : Influence de la longueur du bras (n) et de la nature du cation onium sur la réactivité de l'ester acrylique**

| **essai** | **Y** | **n** | **conv (%)** |
|---|---|---|---|
| 1 | Me₃N | 0 | 85 |
| 2 | Me₃N | 1 | 78 |
| 3 | Me₃N | 4 | 41 |
| 4 | 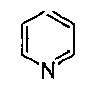 | 1 | 62 |
| 5 | P(Bu)₃ | 1 | 48 |

Les résultats du tableau montrent que la longueur de la chaîne alkyle du bras de greffage influence la réactivité de l'ester acrylique. En effet, l'augmentation de la longueur de la chaîne alkyle séparant les deux fonctions ammonium et ester réduit la vitesse de réaction. Ceci suggère qu'une activation de l'acryloyle due à l'effet électroattracteur de la fonction triméthylammonium appauvrit en électrons la double liaison acrylique et la rend plus réactive (comparer les essais 1, 2 et 3). De même, la nature du cation influence la réactivité (comparer les essais 2, 4 et 5). Il faut en outre noter que la sélectivité endo/exo est la même quelque soit la nature et la composition du support.

Enfin, la possibilité de recyclage du support a été testée dans le cas des sels d'ammonium. Les résultats obtenus sont regroupés dans le tableau 2.

**Tableau 2 : Recyclage du support 5**

| **essai** | **Rendement en ester 21** |
|---|---|
| | **(2 étapes)** |
| 1^{ère} réaction | 78 |
| 1^{er} recyclage | 75 |
| 2^{éme} recyclage | 77 |
| 3^{éme} recyclage | 81 |

Au cours des opérations de recyclage, une stabilité au niveau de la réactivité, de la sélectivité et du rendement des réactions a été constatée.

### 2) Réaction de 5a avec divers autres diènes :

On a utilisé l'acide acrylique **5a** comme diénophile dans la réaction de Diels-Alder avec différents diènes. Pour ceci, une solution de 0,85 mol/l de **5a** dans l'acétonitrile est chauffée à 120°C dans des tubes scellés en présence de 0,01% d'hydroquinone selon le schéma ci-dessus. Après réaction, le mélange réactionnel est évaporé à sec puis lavé à l'éther. Ceci nous a permis d'isoler les cycloadduits **22aa-ad** avec des bons rendements (Tableau 3).

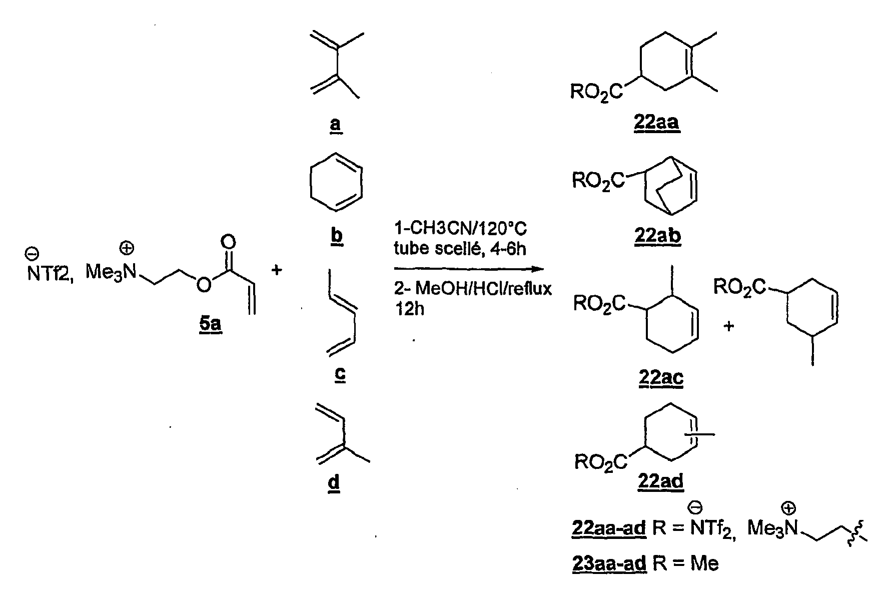

**Tableau 3 : Rendement en cycloadduits 22aa-ad.**

| **Essai** | **Diènes** | **Temps (h)** | **Rdt (%)** |
|---|---|---|---|
| 1 | 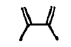 | 4 | 90 |
| 2 | 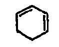 | 6 | 85 |
| 3 | 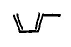 | 4 | 80 |
| 4 | 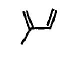 | 4 | 88 |

Les esters méthyliques **23aa-ad** sont obtenus par réaction de transestérification des cycloadduits **22aa-ad** après 12 heures au reflux du méthanol en présence d'une quantité catalytique d'acide chlorhydrique. Le tableau 4 regroupe les rendements en **23aa-ad** isolés.

**Tableau 4 : Rendement en cycloadduits 23aa-ad.**

| **23a** | **Diènes** | **Rdt (%)** |
|---|---|---|
| **23aa** | 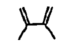 | 85 |
| **23ab** | 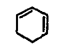 | 83 |
| **23ac** | 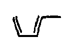 | 73 |
| **23ad** | 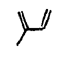 | 79 |

### B - Exemple 2 : Réactions de Couplage

La formation de liaisons carbone-carbone est une opération fondamentale en chimie organique. Parmi le grand nombre de réactions possibles, les méthodes catalytiques utilisant des catalyseurs organométalliques sont extrêmement importantes. Pour notre part, nous avons testé ces nouveaux supports dans les réactions de couplage qui ont déjà fait l'objet de nombreux travaux sur résines et polymères solubles (Franzen, 2000 ; Bertineina et al.,1998 ; Wendebom et al.,1998).

Les différentes réactions de couplage étudiées sont les suivantes :
1) le couplage de Heck
2) le couplage de Suzuki
3) le couplage de Sonogashira

Dans les différents exemples qui suivent, nous avons supporté des esters acryliques, ou des esters aryliques ou encore les deux réactifs :

### 1) Couplage de Heck :

### → Avec un ester acrylique supporté

Dans cet exemple un ester acrylique supporté est engagé dans la réaction de Heck en présence d'acétate de palladium comme catalyseur, de bicarbonate de potassium comme base et d'un iodure d'aryle en large excès comme réactif selon le schéma qui suit :

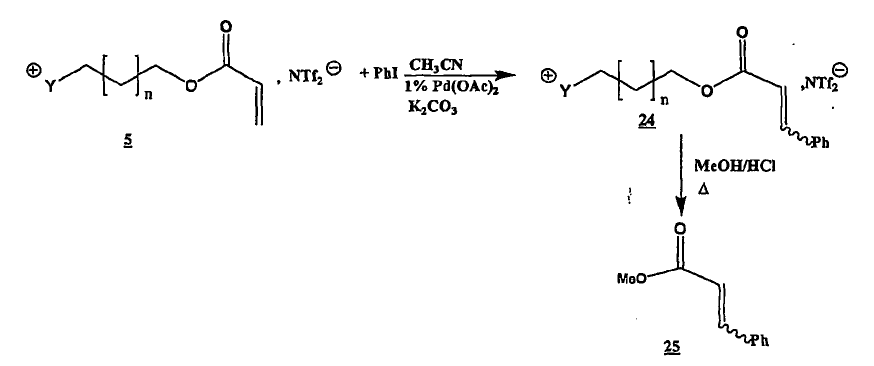

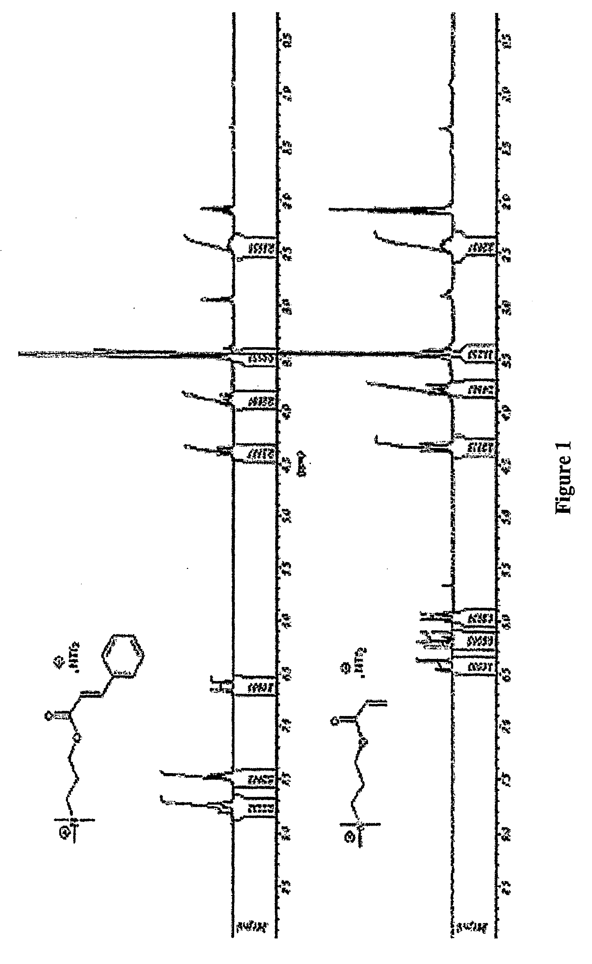

Toutes les réactions de couplage ont été réalisées à 100°C. Le suivi des réactions a été effectué par RMN du proton (**Figure 1**)(essai 3 du tableau **5**).

La Figure 1 illustre la possibilité du suivi par RMN ¹H des réactions et sa simplicité. Il est facile de constater la disparition complète des signaux entre 5,9 et 6,5 ppm correspondant aux trois protons de la double liaison du substrat **5d,** et l'apparition des signaux de la double liaison du produit de couplage **24.**

Les différents paramètres qui influencent cette réaction ont ensuite été étudiés pour mettre au point les conditions optimales. Les résultats obtenus sont regroupés dans le tableau 5 :

**Tableau 5 : Influence de la nature du cation, du support et du bras espaceur sur la réaction de couplage de Heck**

| **essai** | **A** | **n** | **Temps** | **Concentration** | **Conversion** | **Rdt en 25 isolé** | **Rapport E/Z^{H}** |
|---|---|---|---|---|---|---|---|
| | | | **(h)** | **(mol/l)** | **(%)¹** | | |
| 1 | $( Me ⁢ {)}_{3} ⁢ {N}^{⊕}$ | 1 | 2 | 0,05 | 70 | - | 88/12 |
| 2 | $( Me ⁢ {)}_{3} ⁢ {N}^{⊕}$ | 0 | 1 | 0,1 | 100 | 83 | > 99/1 |
| 3 | $( Me ⁢ {)}_{3} ⁢ {N}^{⊕}$ | 1 | 1 | 0,1 | 100 | 86 | > 99/1 |
| 4 | $( Me ⁢ {)}_{3} ⁢ {N}^{⊕}$ | 4 | 1 | 0,1 | 100 | 80 | > 99/1 |
| 5 | 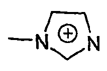 | 1 | 1 | 0,1 | 100 | 85 | > 99/1 |
| 6 | 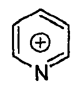 | 1 | 1 | 0,1 | 87 | 84 | > 99/1 |
| 7 | $( Bu ⁢ {)}_{3} ⁢ {P}^{⊕}$ | 1 | 1 | 0,1 | 100 | 79 | > 99/1 |

| | | | | | | | |
|---|---|---|---|---|---|---|---|
| **i : % de conversion de 5 en 24 déterminé par RMN.** **ii : déterminé par RMN et confirmé par GC après transestérification.** | | | | | | | |

Les essais 1 et 2 montrent que la concentration du milieu réactionnel, outre qu'elle réduit la quantité du solvant, influence aussi bien la réactivité que la sélectivité de la réaction. Ainsi en doublant la concentration, l'isomère trans est obtenu exclusivement.

On a remarqué une relation directe entre réactivité et nature du cation du substrat supporté. En effet, en présence du cation pyridinium, la vitesse de réaction est réduite et 87% seulement de produit de couplage sont formés dans les conditions standards. En revanche la longueur de la chaîne alkyle séparant l'ester acrylique et la fonction ammonium n'a aucune influence ni sur la réactivité, ni sur la sélectivité (comparer les essais 2, 3 et 4).

### → Avec l'ester d'iodoaryle supporté 6g

L'ester d'iodoaryle supporté sur sel d'ammonium **6g** a été utilisé dans la réaction de Heck.

Une solution (0.85 mol/l) du sel **6g** dans le DMF est chauffée à 100 °C, en présence d'acétate de palladium comme catalyseur, de bicarbonate de potassium comme base et d'un large excès de l'oléfine. Les rendements en produits isolés sont regroupés dans le tableau 6 :

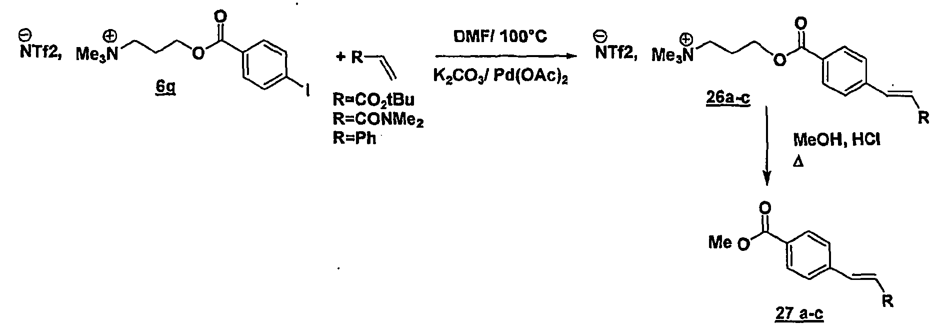

**Tableau 6 : Rendements des produits de couplage 26**

| **N°** | **R** | **Quantité de Pd(OAc)₂(%)** | **Rdt(%)(Temps h)** |
|---|---|---|---|
| 26a | CO₂tBu | 1 | 84(1) |
| 26b | CONMe₂ | 5 | 80(3) |
| 26c | Ph | 5 | 80(3) |

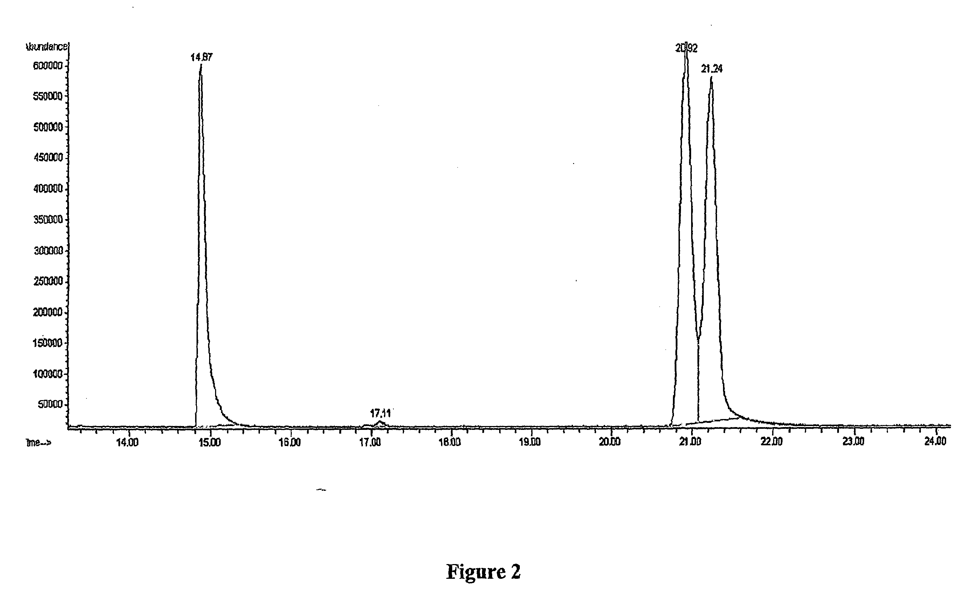

Le clivage des produits de couplage est réalisé par réaction de transestérification d'un mélange de ces trois sels en présence du méthanol et d'une quantité catalytique d'acide chlorhydrique. La réaction est quantitative et les esters méthyliques obtenus sont extraits à l'éther après neutralisation par une solution de K₂CO₃ et ensuite injectés en GC/MS. Le chromatogramme suivant montre les temps de retentions des différent esters (Figure 2 et tableau 7).

**Tableau 7 : Caractérisation par GC/MS de la librairie des esters 27**

| **N°** | **R** | **Temps de rétention en minutes** | **Masse moléculaire** |
|---|---|---|---|
| **27a** | CO₂tBu | 14.87 | 220 |
| **27b** | CONMe₂ | 20.92 | 233 |
| **27c** | Ph | 21.24 | 238 |

### → Avec le styrène supporté 7e

Dans cet exemple, le styrène 7e est mis en réaction avec un mélange équimoléculaire de 7 iodures d'aryle différents conduisant ainsi, après réaction, à un petite bibliothèque des 7 produits 28 ou 29. Ainsi, dans un même puits, on introduit une solution 0,85 mol/l de 7e dans le DMF à laquelle on ajoute 3 équivalents de triéthylamine, 5% d'acétate de palladium et un mélange des sept iodures d'aryle en quantité stoechiométrique (Schéma ci-dessous).

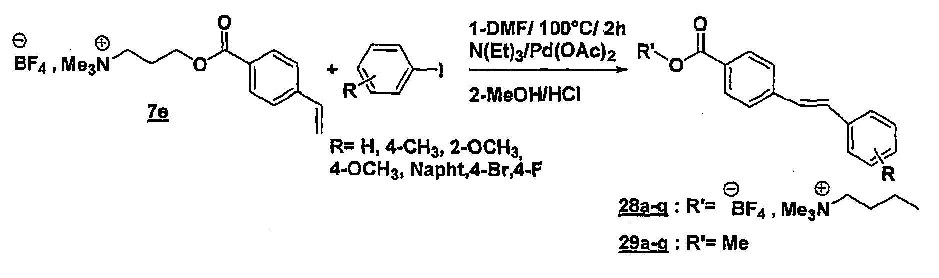

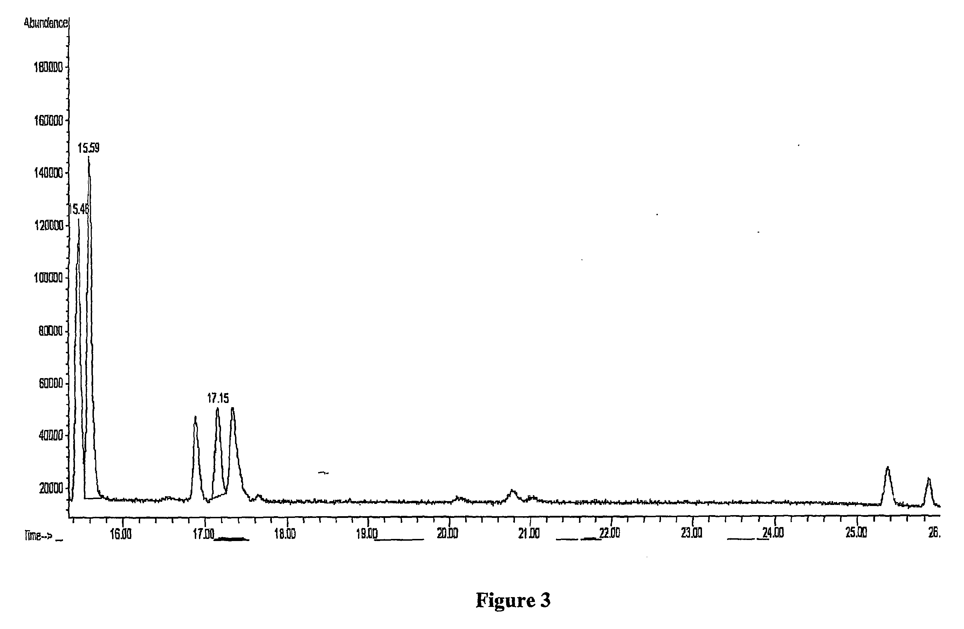

L'avancement de la réaction est suivi par HPLC en suivant la disparition des iodures d'aryle. Le mélange des sels 28a-g est ensuite transestérifié par ajout de méthanol et d'une quantité catalytique d'acide chlorhydrique. Après évaporation du solvant, les esters formés 29a-g sont extraits à l'éther et injecté en GC/MS. (Figure 3)

Le tableau 8 regroupe l'attribution des esters 29 obtenus.

**Tableau 8 : Caractérisation par GC/MS de la librairie des esters.**

| **29** | **R** | **Temps de rétention** | **Masse moléculaire** |
|---|---|---|---|
| **29a** | F | 15.46 | 256 |
| **29b** | H | 15.59 | 238 |
| **29c** | CH₃ | 16.89 et 17.15 | 252 |
| | | 17.33 | |
| **29d** et **29e** | 2 et 4-MeO | | 268 |
| | | 20.15 | |
| **29f** | Br | 20.80 | 317 |
| **29g** | Napht | 25.36 et 26.00 | 290 |

### 2) Couplage de Suzuki :

Le second exemple de réaction de couplage où les supports onium ont été utilisés est la réaction de Suzuki qui consiste en un couplage d'un halogénure d'aryle avec un acide aryl boronique. Cette étude a été réalisée selon deux approches différentes :
1) en supportant un halogénure d'aryle
2) en supportant simultanément un halogénure d'aryle et un acide boronique.

### * Halogénure d'aryle supporté.

Dans cette étude on a utilisé l'acide 3-iodobenzoïque et l'acide 4-bromobenzoïque supporté sur un sel d'onium, et on a choisi d'utiliser le DMF et le dioxane comme solvants très utilisés dans ce type de réaction sur résine ou polymère soluble. Nous avons donc étudié l'effet de la température et de l'anion du support.

Ces différentes études ont été réalisées en utilisant l'acide phénylboronique et l'acétate de palladium comme catalyseur selon le schéma réactionnel suivant :

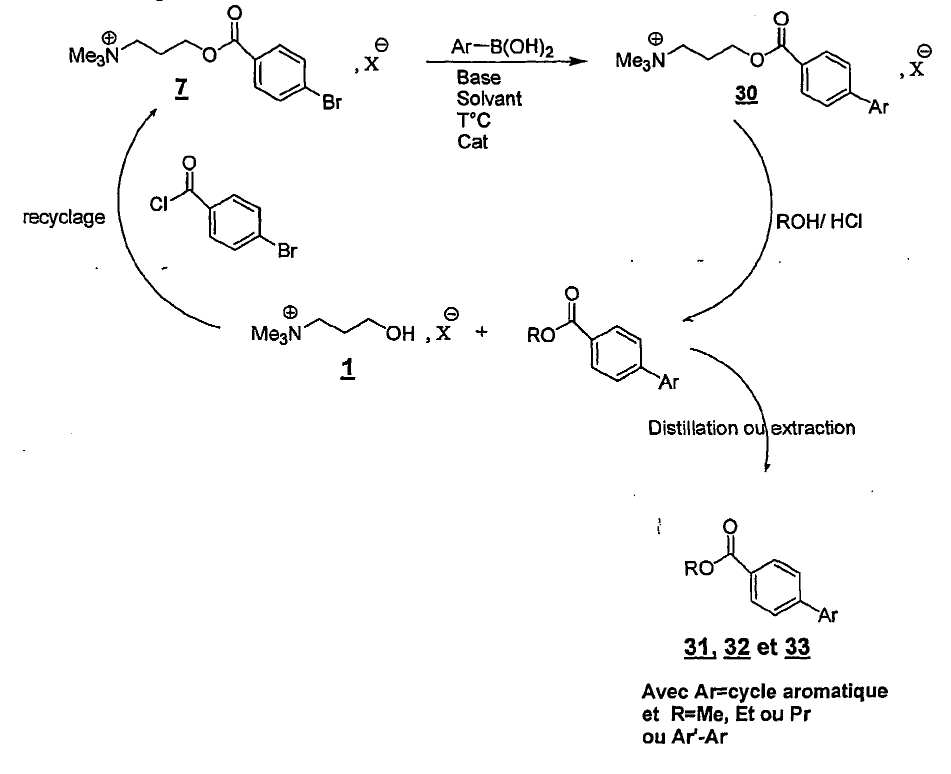

**Tableau 9: Influence de la température et du solvant sur la réaction de couplage de Suzuki en présence de K₂CO₃ (2 équivalents) et de Pd(OAc)₂ (1% molaire).**

| essai | Solvant | X | Température (°C) | Taux de conversion | Ar-Ar' | Ar-Ar |
|---|---|---|---|---|---|---|
| 1 | DMF | NTf₂ | 20 (18h) | 99,1 | 98,7 | 0,4 |
| 2 | DMF | NTf₂ | 80 (5h) | 100 | 96,4 | 3,2 |
| 3 | Dioxane | NTf₂ | 80 (5h) | 70 | 40 | 30 |

L'examen du tableau 9 montre que dans le DMF après cinq heures à 80°C, la réaction est totale avec une excellente sélectivité. Par contre, dans le cas du dioxane, dans les mêmes conditions, 70% de conversion ont été observés avec formation de 30% de produit d'homocouplage (Ar-Ar). Il faut noter que le produit désiré est isolé après transestérification avec un rendement de 95% et une pureté de 99,9% (essai 2).

Au vu des résultats obtenus lors de cette étude non exhaustive, il a été choisi de travailler dans les conditions suivantes pour la préparation d'une librairie d'esters biaryliques :
Solvant : DMF
Température : 80°C
Base : K₂CO₃ solide pour la simplicité du traitement de réaction
Pré-catalyseur : Pd(OAc)₂

Pour la préparation de cette librairie d'esters biaryliques, on a dans un premier temps réalisé une série de réactions de couplage en parallèle avec 9 acides arylboroniques et l'acide 4-bromobenzoique supporté. Ensuite, les 9 esters biaryliques supportés sont mélangés pour former une solution homogène, qui est alors divisée en trois portions égales. Après quoi, chacune des solutions est solubilisée dans un alcool différent après évaporation du DMF sous vide. Quelques gouttes d'acide chlorhydrique concentré (12 N) sont ensuite ajoutées puis le mélange est porté 18 heures à reflux. Après évaporation à sec, le mélange des biarylesters est extrait par l'éther. 3 séries de 9 esters sont donc obtenues et sont analysées en GC/MS. Les différents biarylesters attendus sont tous obtenus quantitativement (aucune trace d'esters aryliques correspondant au produit de départ n'a été détectée par GC/MS) et identifiés sans ambiguïté.

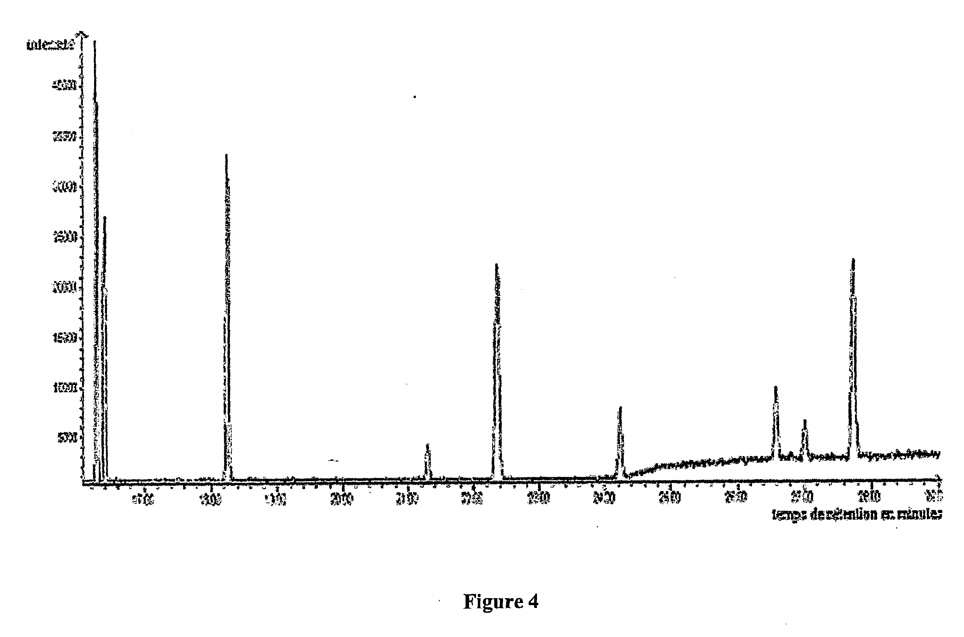

Tous les résultats sont rassemblés ci-dessous dans les tableaux 9 à 11 et les chromatogrammes correspondant aux mélanges des esters biaryliques représentés dans les figures 4 à 6.

### a/ Esters méthyliques biaryliques 31a-i :

Le tableau **10** ci-après correspond au chromatogramme de la figure 4.

**Tableau 10 : Caractéristiques GC/MS de la librairie des esters méthyliques 31a-i**

| **31** | Ar | Temps de rétention | Masse moléculaire |
|---|---|---|---|
| **31a** et **31b** | 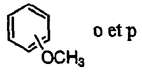 | 21,31 ; 22,35 | 242 |
| **31c** | 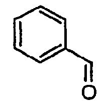 | 23,24 | 240 |
| **31d** | 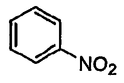 | 28,58 | 257 |
| **31e** | 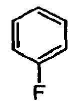 | 16,28 | 230 |
| **31f** | 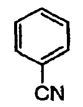 | 24,23 | 237 |
| **31g** | 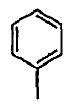 | 18,25 | 226 |
| **31h** | 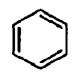 | 16,41 | 212 |
| **31i** | 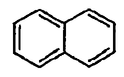 | 27,74 | 262 |

### bl Esters éthyliques biaryliques 32a-i :

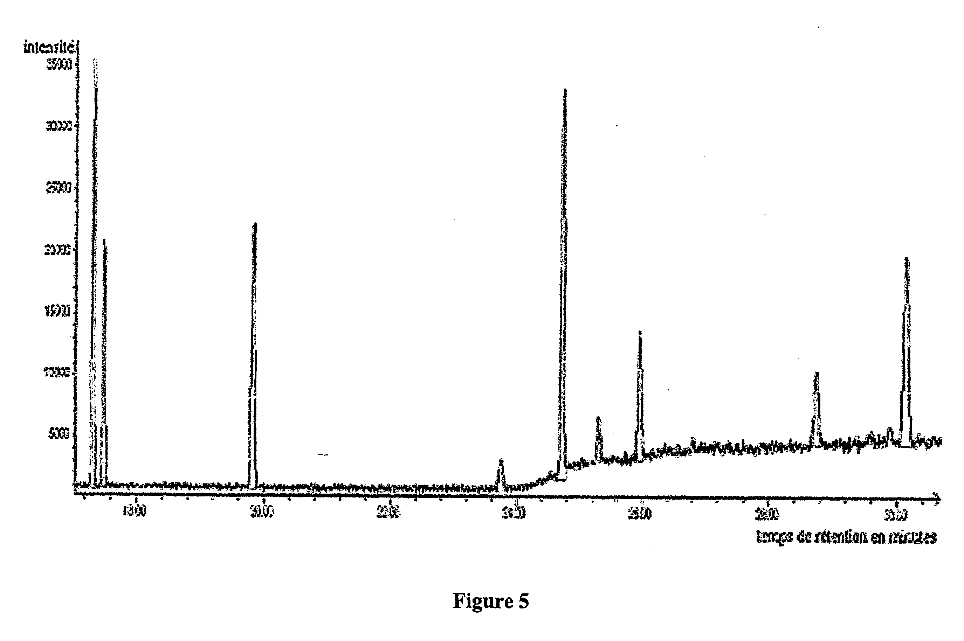

Le tableau **11** ci-après correspond au chromatogramme de la figure 5.

**Tableau 11 : Caractéristiques GC/MS de la librairie des esters éthyliques 32a-i**

| **32** | Ar | Temps de rétention(mn) | Masse moléculaire |
|---|---|---|---|
| **32a et 32b** | 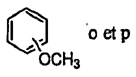 | 23,76 ;24,73 | 256 |
| **32c** | 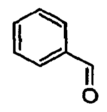 | 25,19 | 254 |
| **32d** | 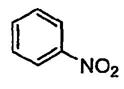 | 28,72 | 271 |
| **32e** | 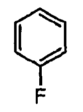 | 17,33 | 244 |
| **32f** | 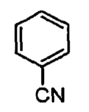 | 25,95 | 251 |
| **32g** | 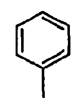 | 19,82 | 240 |
| **32h** | 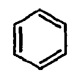 | 17,50 | 226 |
| **32i** | 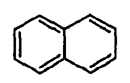 | 30,12 | 276 |

### cl Esters biaryliques propyliques 33a-i

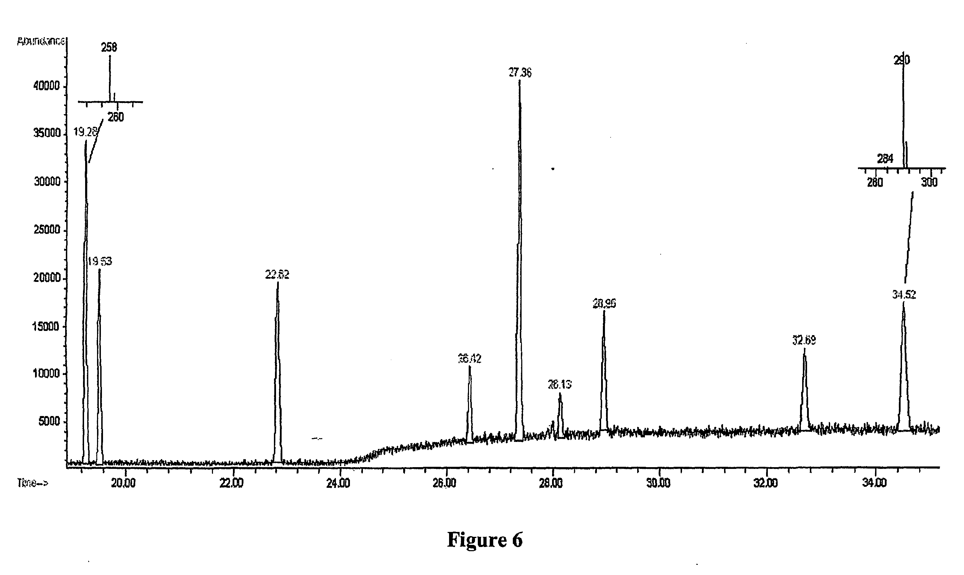

Le tableau 12 ci-après correspond au chromatogramme de la figure 6.

**Tableau 12 : Caractéristiques GC/MS de la librairie des esters propyliques 33a-i**

| **33** | **Ar** | **Temps de rétention en minutes** | **Masse moléculaire Déterminée par MS** |
|---|---|---|---|
| **33a et 33b** | 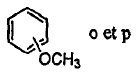 | 26,42, 27,36 | 270 |
| **33c** | 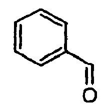 | 28,13 | 268 |
| **33d** | 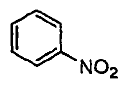 | 32,29 | 285 |
| **33e** | 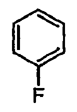 | 19,28 | 258 |
| **33f** | 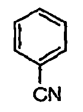 | 28,96 | 265 |
| **33g** | 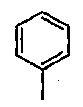 | 22,82 | 254 |
| **33h** | 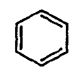 | 19,53 | 240 |
| **33i** | 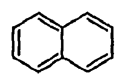 | 34,52 | 290 |

### * Halogénure d'aryle et acide boronique supportés simultanément :

On a greffé un acide arylboronique sur un anion afin de l'engager ensuite dans une réaction de couplage de Suzuki décrochant.

L'acide phénylboronique est greffé sur un support onium via l'anion. En effet, si l'anion X⁻ du sel support est assez nucléophile, il va réagir avec l'acide phénylboronique en quaternarisant l'atome de bore pour donner un borate. Le nucléophile de choix est le fluorure.

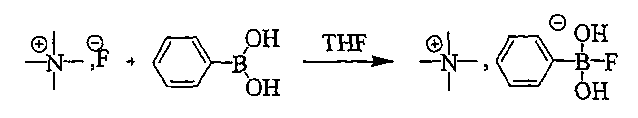

Cette réaction de quaternarisation a été réalisée en solubilisant, à température ambiante, le fluorure de tétraméthylammonium dans le THF (anhydre) puis ajout de l'acide phénylboronique. Après 18 heures d'agitation à température ambiante, le précipité qui se forme est filtré puis lavé à l'éther. Le rendement en produit isolé est de l'ordre de 80%. Le suivi de cette réaction a été réalisé à l'aide de la RMN du bore et du fluore.

Le couplage de Suzuki est réalisé dans les mêmes conditions que celles décrites dans la première partie, entre l'acide 4-bromobenzoique supporté et l'acide phénylboronique supporté selon le schéma réactionnel suivant :

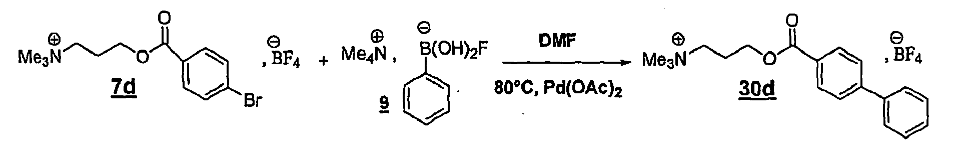

La réaction conduit au produit de couplage avec un excellent rendement (98% en produit isolé pur après transestérification) et une réactivité supérieure à celle observée dans le cas de l'acide phénylboronique. Le support **1d** est récupéré quantitativement et peut être réutilisé.

### 3) Couplage de Sonogashira :

Un autre exemple de couplage où cette famille de supports solubles a été testée est celui de Sonogashira qui consiste en un couplage d'un halogénure d'aryle et d'un alcyne vrai. Cette étude a été effectuée en supportant l'halogénure d'aryle ou l'alcyne sur un sel d'onium.

### • Halogénure d'aryle supporté :

Dans un premier temps et afin de mettre au point les conditions optimales, on a étudié l'influence des différents paramètres sur la réaction de couplage. On a donc examiné l'effet de la température, de la nature du solvant, du catalyseur, de la base et du contre-ion du support ionique.

Ces différentes études ont été réalisées en utilisant l'acide 4-iodobenzoique selon le schéma réactionnel suivant :

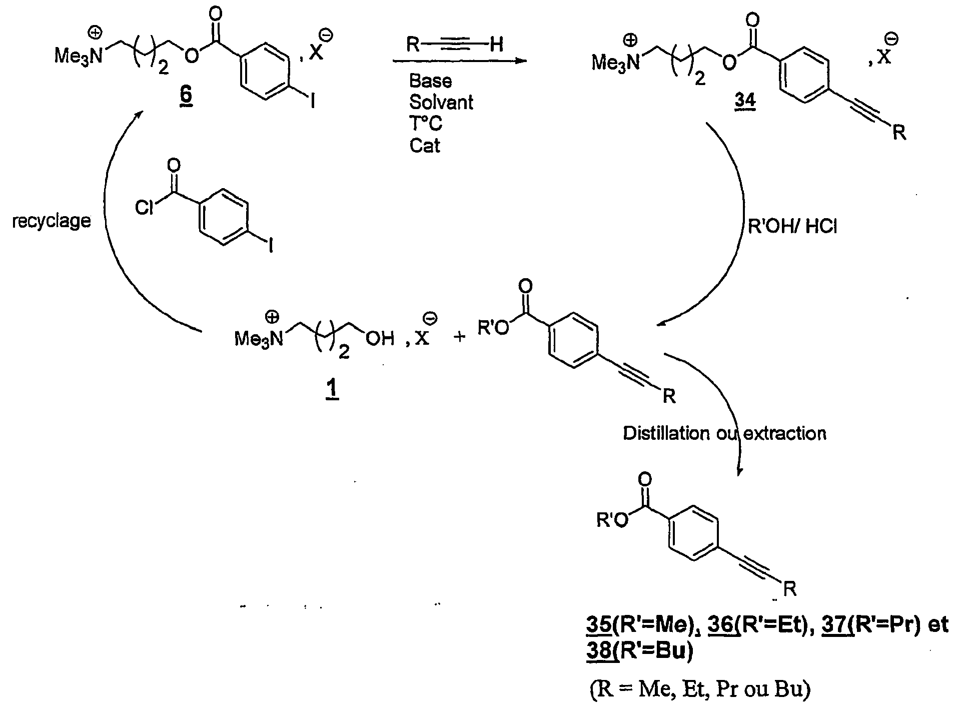

### 1 - Effet de la nature du solvant et de la base :

Cette étude a été effectuée en opérant avec 2,5 % de catalyseur, 5 % de CuI et une température de 40°C afin de déterminer l'influence de la nature de la base et du solvant sur la réaction de couplage. On a donc fait varier la nature de la base ou le solvant :

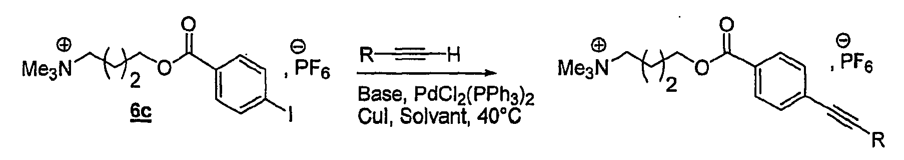

Les différents essais réalisés à 40°C après 1 heure de réaction sont regroupés dans le tableau 13 :

**Tableau 13 : couplage de Sonogashira à 40°C pendant 1 heure (influence de la base et du solvant)**

| **essai** | **base** | **Solvant** | **Taux Conv.(%)** |
|---|---|---|---|
| 1 | K₂CO₃(s) | CH₃CN | 57ⁱ |
| 2 | N(Et)₃ | CH₃CN | 100ⁱ |
| 3 | N(Et)₃ | DMF | 100ⁱⁱ |
| 4 | N(Et)₃ | THF | 75ⁱⁱ |
| 5 | N(Et)₃ | Toluène | 65ⁱⁱ |
| 6 | N(Et)₃ | CH₂Cl₂ | 100ⁱⁱ |
| 7 | N(Et)₃ | Acétone | 100" |
| 8 | N(Et)₃ | Dioxane | 100ⁱⁱ |
| 9 | N(Et)₃ | CH₃CN | 100ⁱⁱ |

| | | | |
|---|---|---|---|
| i : X = PF₆ ; R = butyle ii: X = NTf₂; R = pentyle | | | |

L'examen du tableau 13 montre que l'emploi de la triéthylamine permet d'observer une meilleure réactivité comparée à l'utilisation du bicarbonate de potassium solide comme base (comparer les essais 1 et 2).

Les essais 2 à 8 montrent que différents solvants organiques usuels peuvent être utilisés. En revanche, une diminution de réactivité a été observée dans les cas du toluène et du tétrahydrofurane où le milieu réactionnel est hétérogène.

### 2 - Effet de la nature de l'anion du sel d'onium :

L'étude comparative a été effectuée sur la réaction de couplage du 1-heptyne sur le 4-iodobenzoate de NN',N"-triméthylbutylammonium, en utilisant l'acétonitrile comme solvant, la triéthylamine comme base et le PdCl₂(PPh₃)₂ comme catalyseur, selon le schéma réactionnel suivant :

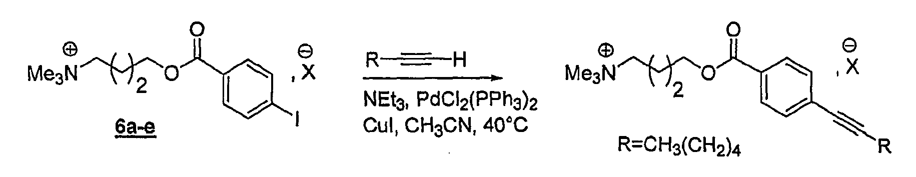

Les résultats obtenus après 15 min d'agitation à 40°C sont regroupés dans le tableau 14 suivant :

**Tableau 14 : Couplage de Sonogashira (15' à 40°C) : influence de l'anion.**

| **essai** | **X** | **Taux de conversion (%)** |
|---|---|---|
| 1 | I | 50 |
| 2 | NTf₂ | 100 |
| 3 | BF₄ | 70 |
| 4 | PF₆ | 97 |
| 5 | CH₃SO₄ | 95 |
| 6 | CF₃SO₃ | 91 |

L'examen du tableau 14 montre que la réaction de couplage a lieu quelle que soit la nature de l'anion. En revanche, une meilleure réactivité est observée dans le cas du triflate, du méthylsulfate, de l'hexafluorophosphate et du bis-trifluorométhane sulfonamidure (triflimide).

### 3 - Effet de la nature de l'alcyne :

Afin de généraliser cette méthodologie on a réalisé le couplage de l'halogénure d'aryle supporté **6b** avec divers alcynes fonctionnalisés ou non.

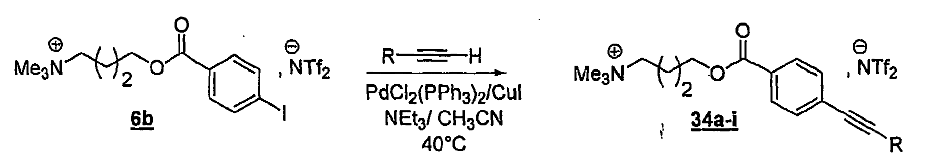

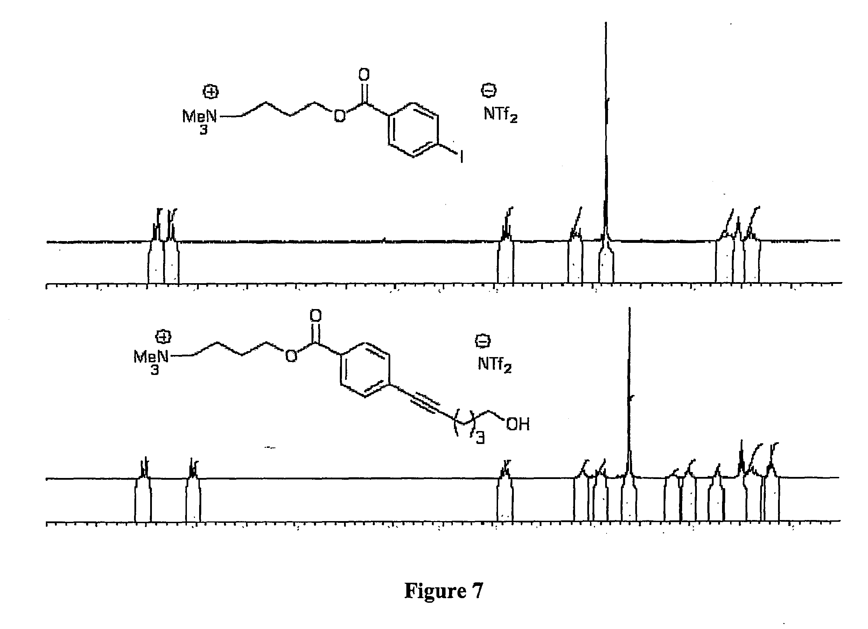

Ces différentes réactions ont été suivies par RMN du proton. La figure 7 montre la simplicité et la facilité de l'interprétation de tels spectres.

Les résultats obtenus en faisant varier les alcynes sont regroupés dans le tableau 15.

**Tableau 15 : couplage de Sonogashira de 6b avec différents alcynes.**

| **essai** | **Alcyne** | **Temps de réactions (h)** | **Taux Conv.(%)** | **Rdtⁱ (%)** |
|---|---|---|---|---|
| 1 | 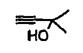 | 1 | 100 | 85 |
| 2 | 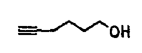 | 1 | 100 | 75 |
| 3 | 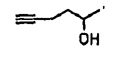 | 1 | 100 | 92 |
| 4 | 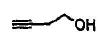 | 1 | 100 | 83" |
| 5 | 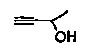 | 1 | 100 | 88 |
| 6 | 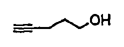 | 1 | 100 | 77 |
| 7 | 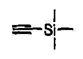 | 1 | 100 | 95 |
| 8 | 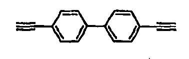 | 1 | 100 | 63ⁱⁱ |
| 9 | 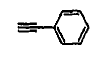 | 1 | 100 | 94 |

| | | | | |
|---|---|---|---|---|
| i : Rdt en produit isolé pur. ii : Rdt en produit isolé après transestérification par le méthanol. | | | | |

### 4 - Effet de la nature et de la quantité du catalyseur:

La réaction de couplage du 4-iodobenzoate de N,N,N-triméthylbutylammonium et de l'hexyne a été effectuée en présence de triéthylamine comme base et de l'acétonitrile comme solvant selon le schéma réactionnel suivant :

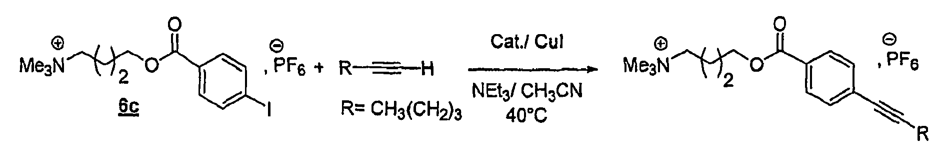

Les résultats obtenus sont regroupés dans le tableau 16 suivant :

**Tableau 16 : conditions du couplage de Sonogashira dans l'acétonitrile de l'iodoaryle 6c avec le 1-hexyne.**

| **essai** | **catalyseur** | **cata/CuI** | **Température** | **Temps de** | **Taux de conversion** |
|---|---|---|---|---|---|
| | | **(% / %)** | **(°C)** | **réaction (h)** | **(%)ⁱ** |
| 1 | PdCl₂(PPh₃)₂ | 10/20 | 20 | 0,2 | 100ⁱⁱ |
| 2 | PdCl₂(PPh₃)₂ | 10/20 | 40 | 0,2 | 100ⁱⁱ |
| 3 | PdCl₂(PPh₃)₂ | 5/10 | 20 | 0,2 | 100 |
| 4 | PdCl₂(PPh₃)₂ | 2,5/5 | 20 | 0,2 | 100 |
| 5 | PdCl₂(PPh₃)₂ | 1/2 | 20 | 1 | 70 |
| 6 | PdCl₂(PPh₃)₂ | 2,5/0 | 20 | 0,2 | <10% |
| 7 | PdCl₂(PPh₃)₂ | 2,5/5 | 40 | 0,2 | 100 |
| 8 | PdCl₂ | 2,5/5 | 40 | 0,2 | <10 |
| 9 | Pd(OAc)₂ | 2,5/5 | 40 | 0,2 | <10 |

| | | | | | |
|---|---|---|---|---|---|
| i : rendement déterminé par RMN. ii : rendement déterminé par GC/MS après transestérification. | | | | | |

On constate les points suivants :
- la réaction a lieu à température ambiante avec des temps records même en présence de 2,5% en catalyseur. Ces résultats sont d'un intérêt majeur sachant que, dans le cas du support solide la même réaction n'est totale qu'après 24h en présence de 10% de catalyseur ;
- la présence de ligand phosphoré accélère nettement la réaction, et le PdCl₂(PPh₃)₂ reste, de loin, le catalyseur de choix pour cette réaction (comparer les essais 7, 8 et 9) ;
- la réactivité reste identique en diminuant le pourcentage de catalyseur de 10 à 2,5%. En revanche, la réduction à 1% provoque une chute de la réactivité (essai 5). De même, l'essai 6 montre que la présence de CuI n'est pas indispensable mais elle accélère la réaction.

### 5 - Application en chimie combinatoire :

Après avoir analysé les résultats obtenus lors de cette étude, il a été décidé de travailler dans les conditions suivantes pour la préparation d'une librairie d'alcynes aromatiques :
Solvant : CH₃CN
Température : 40°C
Base : Triéthylamine

Pour la préparation de cette librairie d'esters on a opéré comme suit :

Dans un premier temps, une série de réactions de couplage en parallèle avec 5 alcynes et l'acide 4-iodobenzoique supporté a été réalisée. Ensuite, les 5 milieux réactionnels ont été mélangés pour former une solution homogène. Après évaporation de l'acétonitrile et lavage à l'éther, le résidu est alors divisé en quatre portions égales. Chaque partie est solubilisée dans un alcool en présence de 3 gouttes d'acide chlorhydrique concentré (12 N) puis le mélange est porté à reflux pendant 18 heures. Après évaporation à sec de l'alcool, le mélange des produits est extrait à l'éther. Les 4 séries de 5 alcynes obtenues sont alors analysées en GC/MS. Les différents alcynes attendus sont tous identifiés sans ambiguïté.

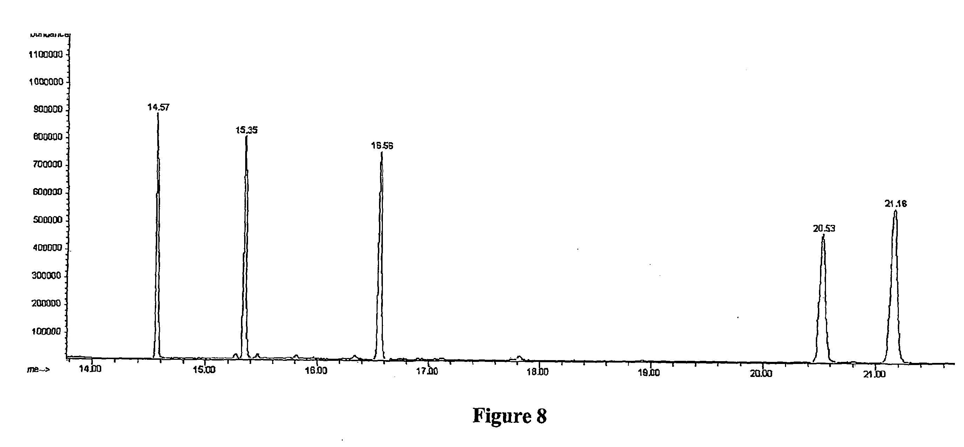

Les résultats obtenus sont répertoriés ci-dessous sous forme de tableaux. Les chromatogrammes des mélanges d'alcynes sont également reproduits (Figures 8 à 11).

### a/ Esters méthyliques acétyléniques 35a-e :

Le tableau 17 ci-après correspond au chromatogramme de la Figure 8.

**Tableau 17 : Caractérisation par GC/MS de la librairie des esters méthyliques**

| **35** | **R** | **Temps de rétention** | **Masse moléculaire** |
|---|---|---|---|
| | | **en minutes** | |
| **35a** | 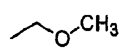 | 14,57 | 204 |
| **35b** | 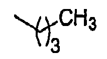 | 15,35 | 216 |
| **35c** | 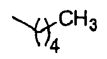 | 16,56 | 230 |
| **35d** | 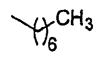 | 20,53 | 236 |
| **35e** | 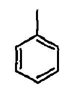 | 21,16 | 258 |

### bl Esters éthyliques 36a-e :

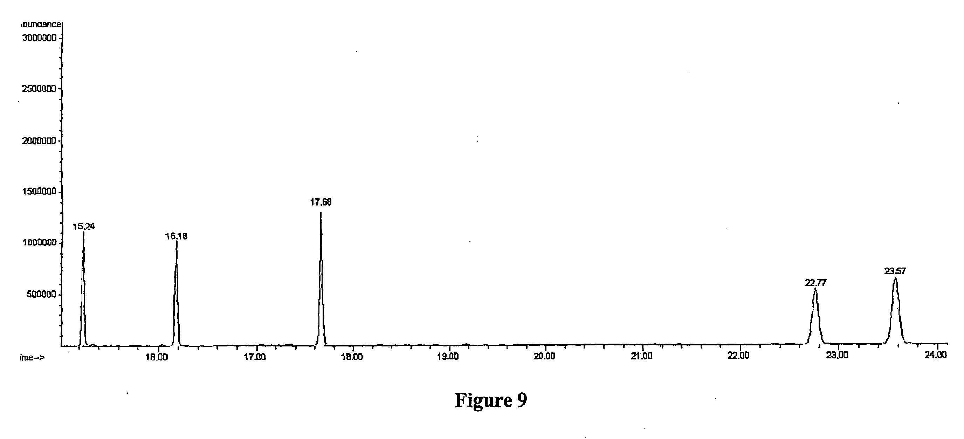

Le tableau 18 ci-après correspond au chromatogramme de la Figure 9.

**Tableau 18 : Caractérisation par GC/MS de la librairie des esters éthyliques**

| **36** | **R** | **Temps de rétention** | **Masse moléculaire** |
|---|---|---|---|
| **36a** | 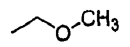 | 15,24 | 218 |
| **36b** | 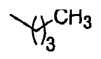 | 16,18 | 230 |
| **36c** | 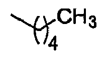 | 17,68 | 244 |
| **36d** | 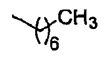 | 22,77 | 250 |
| **36e** | 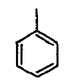 | 23,57 | 272 |

### c/ Esters propyliques 37a-e :

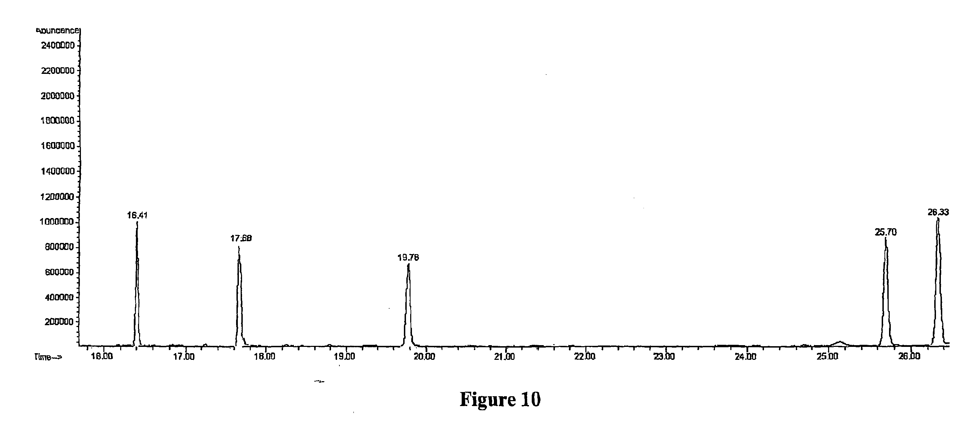

Le tableau 19 ci-après correspond au chromatogramme de la Figure 10.

**Tableau 19 : Caractérisation par GC/MS de la librairie des esters propyliques**

| **37** | **R** | **Temps de rétention** | **Masse moléculaire** |
|---|---|---|---|
| **37a** | 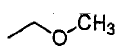 | 16,41 | 232 |
| **37b** | 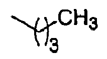 | 17,68 | 244 |
| **37c** | 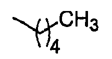 | 19,78 | 258 |
| **37d** | 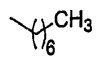 | 25,70 | 264 |
| **37e** | 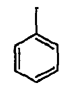 | 26,33 | 286 |

### dl Esters butyliques 38a-e :

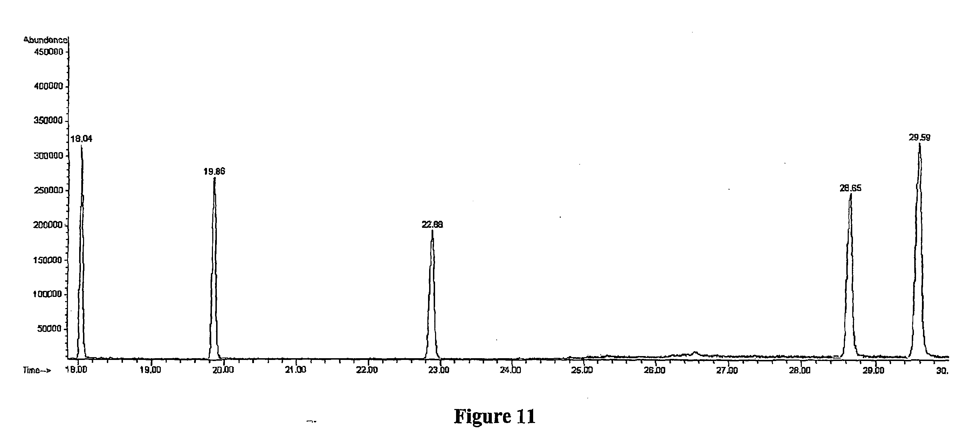

Le tableau 20 ci-après correspond au chromatogramme de la Figure 11.

**Tableau 20 : Caractérisation par GC/MS de la librairie des esters butyliques**

| **38** | **R** | **Temps de rétention** | **Masse moléculaire** |
|---|---|---|---|
| **38a** | 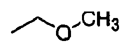 | 16.41 | 232 |
| **38b** | 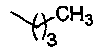 | 17.68 | 244 |
| **38c** | 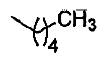 | 19.78 | 258 |
| **38d** | 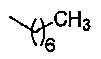 | 25.70 | 264 |
| **38e** | 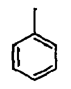 | 26.33 | 286 |

### • Alcyne supporté 8 :

Nous avons engagé l'alcyne supporté **8** dans la réaction de Sonogashira avec différents iodures. Ceci en présence de PdCl₂(PPh₃)₂ comme catalyseur et CuI comme co-catalyseur. Après 20mn d'agitation à température ambiante, la réaction est totale conduisant à **39a-f.**

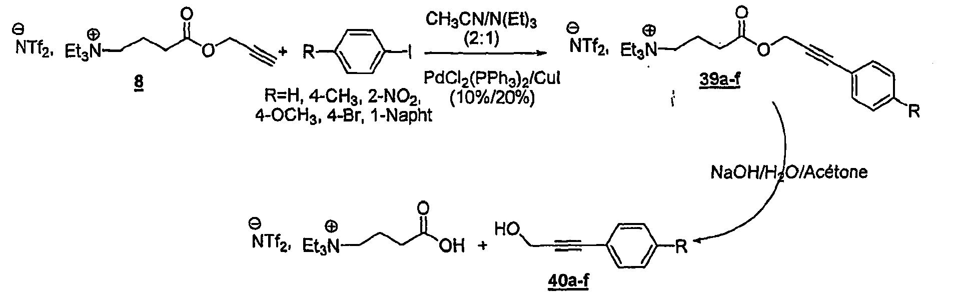

Après réaction, les mélanges réactionnels sont évaporés à sec et lavés à l'éther pour éliminer l'excès des réactifs. Les huiles ainsi obtenues sont solubilisées dans le chlorure de méthylène puis les solutions sont lavées par une solution aqueuse de K₂CO₃ pour libérer Et₃N de son chlorhydrate formé au cours de la réaction. Après traitement par Na₂SO₄ et évaporation du chlorure de méthylène, les sels sont isolés avec un bon rendement. Ceci est illustré dans le tableau 21.

**Tableau 21 : Rendement en produits 39a-f**

| **Entrée** | **R** | **Rdt(%)** |
|---|---|---|
| **39a** | H | 77 |
| **39b** | 4-CH₃ | 89 |
| **39c** | 2-NO₂ | 81 |
| **39d** | 4-OCH₃ | 92 |
| **39e** | 4-Br | 80 |
| **39f** | 1-Napht | 86 |

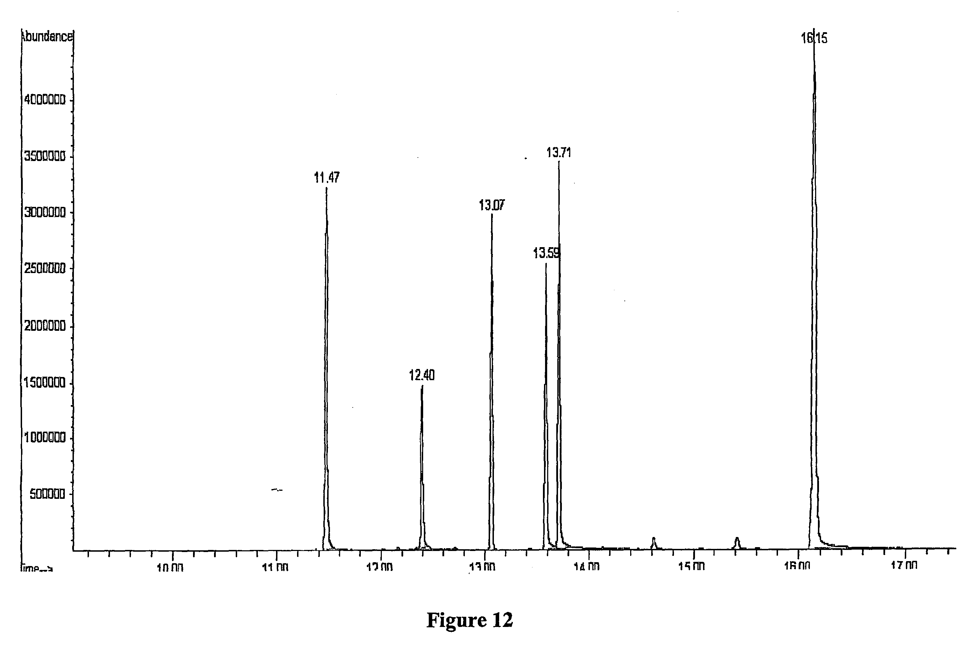

Les alcools formés sont extraits à l'éther après saponification en présence d'une solution aqueuse de NaOH à 5% dans l'eau des mélanges des sels isolés précédemment et ensuite injectés en GC-MS(Figure 12 et tableau 22).

**Tableau 22 : Caractérisation par GC/MS de la librairie des alcools 40a-f**

| **40** | **R** | **Temps de rétention** | **Masse moléculaire** |
|---|---|---|---|
| **40a** | H | 11,47 | 132 |
| **40b** | 4-CH₃ | 12,40 | 146 |
| **40c** | 2-NO₂ | 13,07 | 177 |
| **40d** | 4-OCH₃ | 13,59 | 162 |
| **40e** | 4-Br | 13,71 | 211 |
| **40f** | 1-Napht | 16,15 | 182 |

### C - Exemple 3 : Synthèse d' α-aminoacides :

On a choisi l'alkylation des imines dérivées de la glycine et de la benzophénone comme réaction modèle afin d'explorer le potentiel des sels d'onium comme supports dans ce type de réaction. L'intérêt vis-à-vis de ces substrats réside notamment dans la possibilité d'accéder à des α-aminoacides substitués et éventuellement de réaliser leur synthèse asymétrique.

La séquence utilisée met en oeuvre l'alkylation d'un iminoester dérivé de la glycine et de la benzophénone dans les conditions de transfert de phase selon la méthode décrite par O'Donnel et al. (1989) en solution et permet de synthétiser des aminoacides supérieurs selon le schéma suivant :

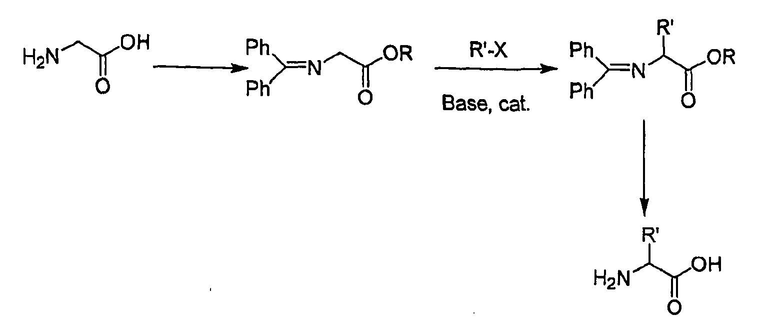

Hormis les vingt et un (L)-α-aminoacides naturels connus et isolés à partir d'hydrolysats de protéines, d'autres non naturels possèdent des propriétés biologiques intéressantes. Par exemple, l'incorporation au niveau d'un peptide d'un aminoacide ayant des substituants bien choisis peut provoquer des contraintes conformationnelles et augmenter la sélectivité vis-à-vis d'un récepteur. On peut citer par exemple l'inhibition des décarboxylases, hydrolases ou transférases par un α-alkyl aminoacide qui joue le rôle de substrat suicide (Williams, R.M. "Synthesis of Optically Active α-Amino Acids" Pergamon Press, Oxford, 1989).

La (D)-phénylglycine (A) et la (D)-p-hydroxyphénylglycine (B), commercialisées par la compagnie hollandaise DSM, constituent respectivement les matières premières pour la production des antibiotiques ampicilline (A) et amoxicilline (B). Les ventes de ceux-ci atteignent 1,5-1,7 milliards de dollars par an.

Malgré la demande accrue en α-aminoacides, la fermentation reste la principale méthode pour leur préparation. Depuis la fin des années 80, des méthodes de synthèse ont vu leur application se concrétiser dans le domaine industriel Les α-aminoacides non naturels sont obtenus par deux méthodes :
- la synthèse asymétrique.
- Le dédoublement qui reste la méthode de choix.

Par ailleurs, la synthèse sur support solide ou soluble de type PEG reste parmi les méthodes les plus efficaces et simples à mettre en oeuvre pour accéder à ces molécules (Lindström et al., 2002).

On a donc testé les sels d'onium comme supports solubles pour la préparation des α-aminoacides supérieurs selon le schéma réactionnel suivant :

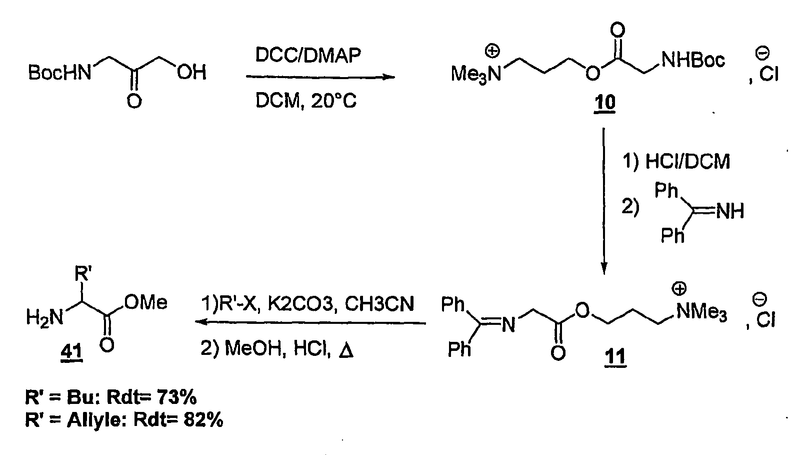

La réaction d'estérification est effectuée directement sur la N-Boc-glycine en présence d'un équivalent de DCC et de 5% de DMAP à température ambiante dans le dichlorométhane (DCM). Le groupement protecteur Boc est ensuite éliminé en présence d'acide chlorhydrique dans le DCM avec un bon rendement. De même, la condensation de l'aminoester avec la diphénylméthylène imine conduit à l'iminoester **11** avec un excellent rendement et peut-être conservé plusieurs jours à température ambiante.

Il faut noter que le support qui est un sel d'ammonium joue aussi le rôle du catalyseur de transfert de phase dans l'étape d'alkylation de la base de Schiff **11,** et nous avons constaté une réactivité plus importante que celle décrite dans la littérature pour l'alkylation du même substrat supporté sur PEG ou support solide.

### D - Exemple 4 : les Réactions Multi-Composants RMC

Les réactions multi-composants mettent en présence simultanément au moins trois partenaires dans des conditions expérimentales qui ne varient pas au cours du temps et permettent la création de plusieurs liaisons covalentes en cascade dans un seul réacteur, à la différence des réactions classiques ou deux réactifs conduisent à un produit par création d'une nouvelle liaison. Ainsi il est possible d'accéder en une seule étape à une molécule hautement fonctionnalisée à partir d'entités relativement simples. De plus les RCM allient convergence et économie d'atomes, deux principes essentiels en synthèse organique mais aussi en chimie combinatoire. Signalons enfin que ces réactions ont généralement lieu avec un rendement élevé, puisqu'elles évitent la succession d'étapes des synthèses linéaires ou multiétapes qui, font, à chaque pas, chuter le rendement.

Les RMC les plus connues et les plus développées sont celles de Passérini et de Ugi. Un des réactifs clef de ces réactions est un isonitrile de formule générale RN=C, dont la structure électronique du carbone terminal compte un doublet et une lacune électronique (structure de type carbénique) et permet le passage d'un atome de carbone formellement divalent à un atome de carbone tétravalent par addition d'un électrophile **et** d'un nucléophile. Le schéma qui suit représente un exemple de réaction de Passérini (réaction 3CC pour 3 component condensation).

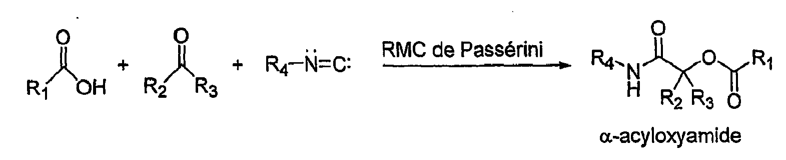

Bien sûr les RMC ont été transposées sur support solide. Par exemple une résine à terminaison amine a été engagée dans une réaction de type Ugi pour conduire après clivage à une série d'adduits d'une grande pureté avec des rendements s'échelonnant de moyens à excellents :

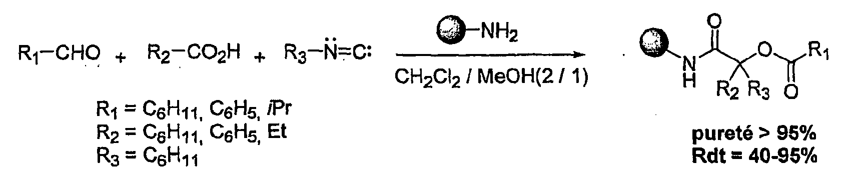

Bien que les réactions de Ugi et Passerini soient les plus connues et les plus développées, il existe d'autres RMC, qui répondent au critère essentiel que tous les réactifs sont présents dès le début de la réaction et les conditions ne varient pas au cours de celles-ci. A la différence des réactions de Ugi et Passerini, ces autres réactions ne reposent pas sur l'utilisation d'un isonitrile comme un des acteurs centraux de la création de nouvelles liaisons covalentes. Ces différents types de réactions permettent d'accéder à des structures hautement fonctionnalisées variées en une seule étape.

### • Synthèse de quinoléines substituées selon la réaction de Grieco:

Les quinoléines substituées sont des pharmacophores intéressants. Leur synthèse sur support solide a été réalisée par une RCM dite de Doebner, mettant en jeu une aniline, un aldéhyde et un composé α-dicarbonylé. Les quinoléines sont obtenues avec une grande pureté et de très bons rendements.

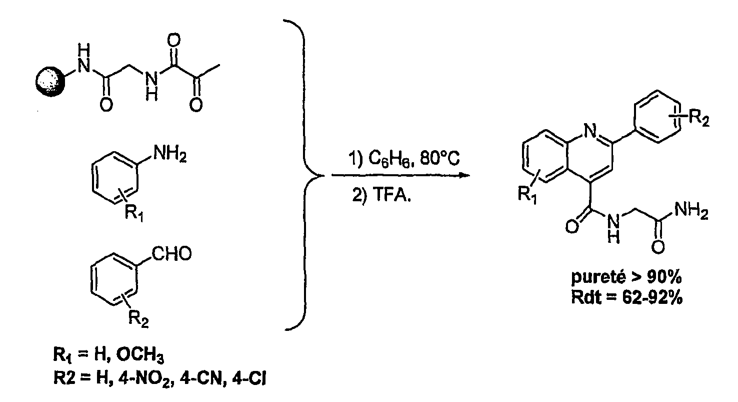

Il a été décidé ici de profiter des nombreux avantages offerts par les supports onium, comme cela a été mis en évidence dans les différents exemples décrits précédemment. Ainsi, on a choisi de les tester dans les RMC de type Grieco (Grieco et al., 1988). Cet exemple a fait l'objet de plusieurs travaux décrits par W. Armstrong et al. (1997 et 1998) sur support solide et il a permis la préparation d'une librairie de 80 produits avec des rendements allant de 50 à 93%.

### a- Utilisation de l'aniline supporté 7g dans la réaction de Grieco :

Pour ce faire, on a supporté l'aniline **7g** qui a été mise en présence d'un aldéhyde et du cyclopentadiène pour conduire à des tétrahydroquinoléines. Cet exemple à trois composants, consiste en une première condensation de l'aldéhyde et de l'aniline pour conduire à l'imine. Celle-ci réagit ensuite dans ce qui est formellement une réaction de Diels-Alder avec le cyclopentadiène en présence d'une quantité catalytique d'acide trifluoroacétique.

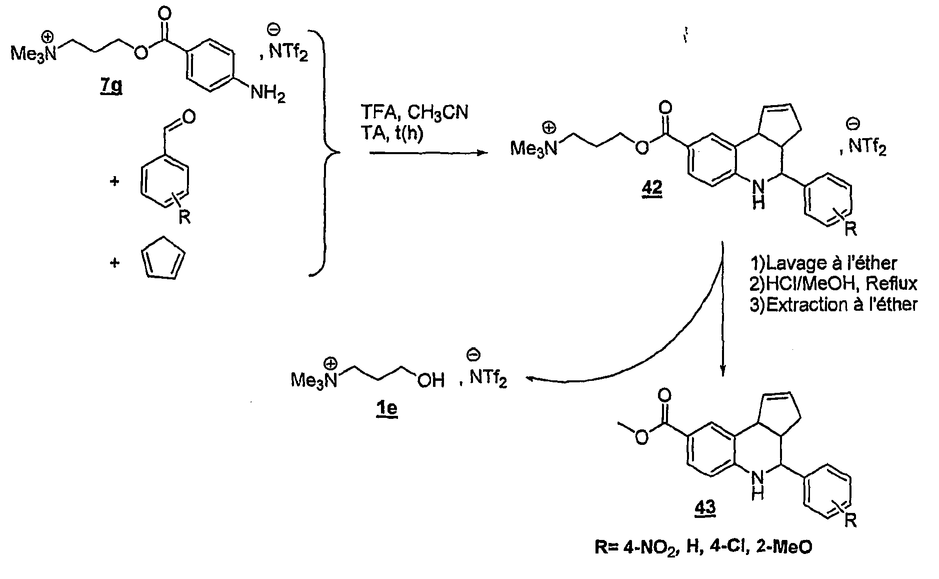

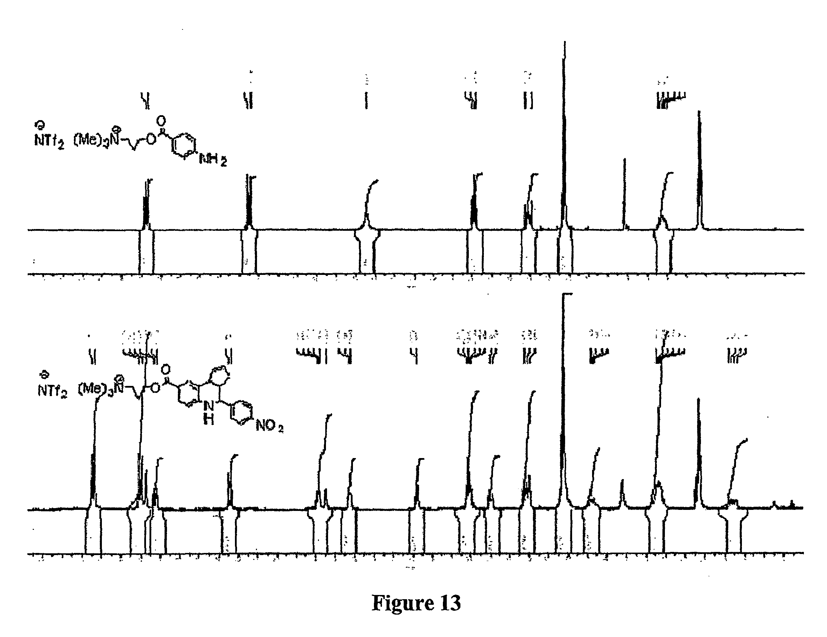

Le suivi des différentes réactions a été effectué par RMN ¹H **(****Figure 13****)** et on a observé une conversion allant de 80% à 100% selon la nature de l'aldéhyde. En effet, en présence d'aldéhyde riche en électrons la réaction est ralentie. Ainsi en présence du 4-nitrobenzaldéhyde la réaction est totale au bout de 12 heures alors que l'avancement n'est que de 70% dans le cas du benzaldéhyde. Le schéma réactionnel ci-dessus illustre le cas du 4-nitrobenzaldéhyde, après évaporation du solvant (CH₃CN) et lavage à l'éther pour éliminer l'excès des deux réactifs et de l'acide trifluoroacétique. Ce schéma montre aussi que le suivi d'une réaction qui conduit à des composés complexes est possible et d'une clarté remarquable. La transestérification par le méthanol conduit à des produits très propres qui sont extraits à l'éther et purifiés par filtration sur silice.

Les différents exemples que nous avons réalisés et les résultats obtenus sont regroupés dans le tableau 23 ci-dessous.

**Tableau 23 : Réaction de Grieco réalisée sur sels d'ammonium utilisés comme support soluble :**

| **R** | **Temps en (h)** | **Rdt de 42 en (%)** | **Rdt de 43 en (%)** |
|---|---|---|---|
| 4-NO₂ | 12 | 98 | 82 |
| H | 12 | 70 | 68 |
| 4-OMe | 12 | 60 | 54 |
| 4-Cl | 14 | 96 | 83 |

### Exemple : synthèse de la tétrahydroquinoléine 43 dérivée du benzaldéhyde :

Sous courant d'argon on mélange 100 mg de l'amine **7g** supporté, 500 µl d'une solution 1M du para-nitrobenzaldéhyde dans l'acétonitrile, 500 µl d'une solution 1M de cyclopentadiène dans l'acétonitrile et 50 µl d'une solution à 1% de TFA dans l'acétonitrile. Le mélange réactionnel est agité une nuit à température ambiante. Après évaporation à sec et lavage à l'éther, on obtient un solide jaune.
Rdt = 71%
***RMN ¹H (200 MHz, Acétone D₆) :*** 1,55-1,8 (m, 1H) ; 2,35-2,58 (m, 3H) ; 3,01-3,2 (m, 1H) ; 3,4 (s, 9H) ; 3,7-3,9 (m, 2H) ; 4,1-4,22 (m, 1H); 4,42 (t, 2H, J = 5,81 Hz) ; 4,9-5,0 (m, 1H) ; 5,58-5,75 (m, 1H) ; 5,7-5,8 (m, 1H) ; 5,93-6,05 (m, 1H) ; 6,9 (d, 1H, J = 8,4 Hz) ; 7,6- 7,95 (m, 4H) ; 8,28 (d, 2H, J = 8,6 Hz).
***RMN ¹³C (50 MHz, Acétone D₆) :*** 22,92 ; 31,25 ; 45,35 ; 45,40 ; 52,96 (t, J_{C-N} = 4,07 Hz) ; 56,46 ; 60,67 ; 64,28 ; 115,40 ; 119,45 ; 120,13 (q, J_{C-F} = 321,194 Hz), 123,40 ; 124,65 ; 127,68 ; 128,17 ; 129,70 ; 130,98, 134,26 ; 147,20 ; 150,12 ; 150,50 ; 165,77

### b- Utilisation de l'aldéhyde supporté 7h dans la réaction de Grieco:

Dans ce travail, l'aldéhyde supporté 7h a été engagé dans la réaction de Grieco avec différentes amines et oléfine, dans l'acétonitrile comme solvant et en présence de TFA comme catalyseur comme le montre le schéma suivant :

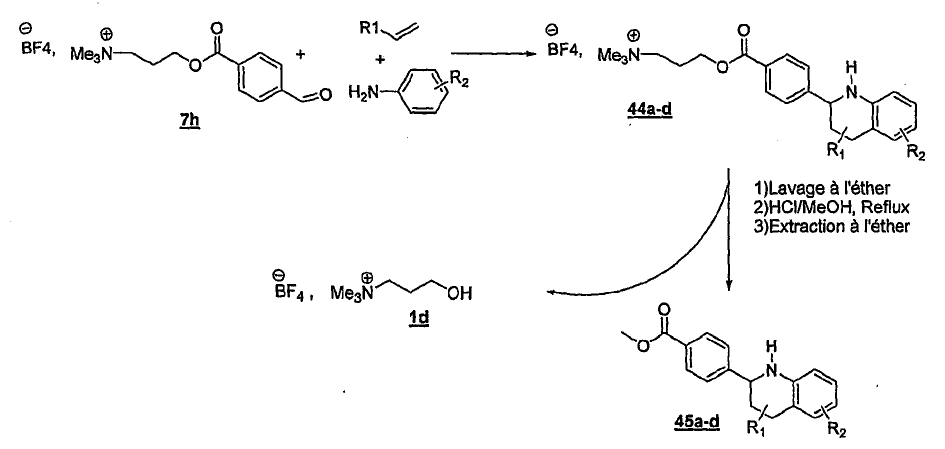

Après deux heures d'agitation à température ambiante d'une solution de 0,85 mol/l de 7h dans l'acétonitile en présence de 1,2 équivalents de TFA, 1 équivalent de l'amine et 2 équivalents de l'oléfine. Le suivi de la réaction réalisé par HPLC montre qu'elle est totale après 2 heures. Après l'ajout de l'éther les sels formés 44a-d sont isolés par filtration suivie d'une étape de lavage à l'éther. Les produits 45a-d sont isolés par extraction à l'éther après réaction de transestérification au reflux du méthanol en présence d'une quantité catalytique de l'acide chlorhydrique. Le milieu est ensuite neutralisé par ajout d'une solution diluée de de K₂CO₃ après évaporation du solvant. Le tableau 24 regroupe les rendements en 44 et 45 isolés.

**Tableau 24 : Rendements en produits 44 et 45 isolés.**

| **Produits** | **R₁** | **R₂** | **Rdt de 44 en %** | **Rdt de 45 en** % |
|---|---|---|---|---|
| **44a** | Cyclopentadiène | 3-NO₂ | 90 | 85 |
| **44b** | Cyclopentadiène | 4-Br | 88 | 77 |
| **44c** | Cyclopentadiène | H | 77 | 70 |
| **44d** | Indène | 4-Br | 92 | 67 |

### Exemple : Synthèse de 44c :

A une solution de 100 mg (0,3 mmole) de **7h** dans 0,4 ml d'acétonitrile, on a ajouté 1 eq de l'aniline, 2 eq de cyclopentadiène et 1,2 eq de TFA. Le mélange réactionnel est agité deux heures à température ambiante, évaporé à sec et ensuite lavé à l'éther. Le produit de la réaction est isolé par filtration après cristallisation dans l'éther.
***RMN ¹H (300 MHz, Acétone D₆) :*** 1,58-1,75 (m, 1H) ; 2,40-2,65 (m, 3H) ; 2,97-2,98 (m, 1H) ; 3,00-3,15(m, 1H) ; 3,40 (s, 9H) ; 3,77-3,92 (m, 2H) ; 4,03-4,15 (m, 1H) ; 4,46 (t, 2H, J = 5,94 Hz) ; 4,68-4,76 (m, 1H) ; 5,55-5,62 (m, 1H) ; 6,58-7,13 (m, 4H) ; 7,60 (d, 2H, J = 8,3 Hz) ; 8,05 (d, 2H, J = 8,4 Hz).
***RMN ¹³C (75 MHz, Acétone D₆) :*** 23,58 ; 32,19 ; 46,69 ; 47,06 ; 53,67 (t, J = 3,8 Hz); 58,16; 62,42; 64,81; 117,00; 119,49; 126,47; 126,88; 127,59; 129,52; 129,63 ; 130,18 ; 130,43 ; 135,44 ; 146,90 ; 149,82 ; 166,57.

| **Spectrométrie de masse (APCI) pour [C₂₅H₃₁N₂O₂][BF₄] :** | | |
|---|---|---|
| | **Masse Théorique calculée pour (C⁺)** | 391,5 |
| | **Masse Trouvée** | 391,4 |

### • Synthèse des oléfines tétrasubstituées :

### a- Synthèse individuelle d'oléfines tétrasubstituées :

Les oléfines tétrasubstituées peuvent être obtenues par réaction de Mc Murry ou par réaction d'oléfination de Wittig. Cependant, les régio- et stéréo- sélectivités sont les problèmes majeurs associés à ces deux procédés. D'autres approches, mettent en oeuvre la carbolithiation des alcynes, des réactions des oxiranes portant un groupement CF₃, les organosilanes, l'électrotelluration. Cependant, ces approches utilisent, généralement, des réactifs non facilement disponibles et en plus s'accompagnent parfois d'un défaut dé la régio- et la stéreosélectivité.

Les oléfines tétrasubstituées peuvent être préparées par réaction d'addition intermoléculaire d'un intermédiaire arylpalladium à un alcyne interne, suivie d'une réaction de couplage avec les organométalliques dérivés du bore, de l'étain ou du zinc.

En 2003, Zhou et al ont développé un procédé de synthèse d'oléfines tétrasubstituées utilisant le palladium comme catalyseur. Ce procédé met en oeuvre le couplage intermoléculaire d'un iodoaryle, d'un alcyne interne et d'un acide arylboronique⁷.

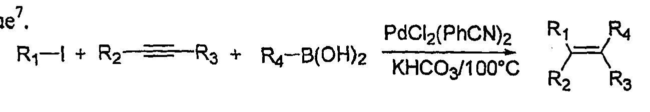

Ces oléfines ont été synthétisées sur support sel d'onium. Pour cela, nous avons utilisé comme alcyne interne le sel 34i obtenu par réaction de Sonogashira entre l'iodure supporté 6b et le phénylacétylène. La réaction est réalisée à 100°C dans un mélange DMF/H₂O (80/20) comme solvant, KHCO₃ comme base et PdCl₂ (PPh₃)₂ comme catalyseur.

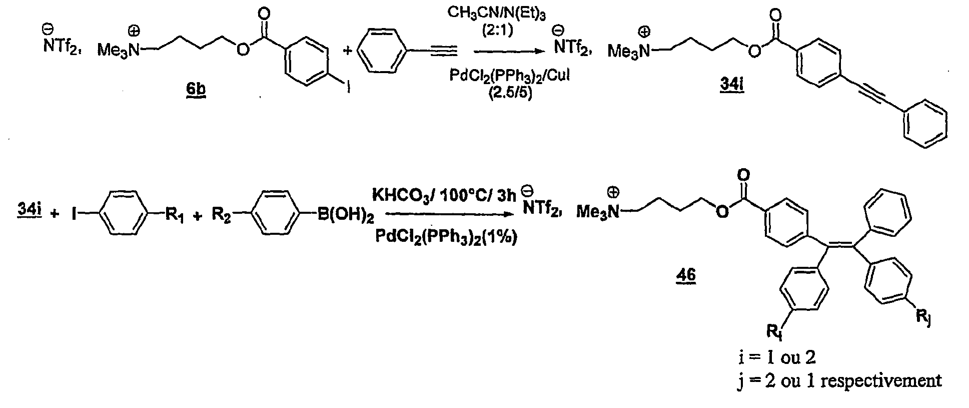

Le tableau 25 regroupe les rendements en sels isolés 46 après 3 heures de chauffage à 100°C.

**Tableau 25 : Synthèse des oléfines^{*} 46.**

| **Entrée** | **R₁** | **R₂** | **Rdt(%)** |
|---|---|---|---|
| 1 | H | H | 67 |
| 2 | CH₃ | H | 60 |
| 3 | CH₃O | H | 70 |
| 4 | H | 1-Napht | 68 |
| 5 | CH₃O | 1-Napht | 86 |
| 6 | CH₃ | 1-Napht | 83 |

| | | | |
|---|---|---|---|
| ^{*} Mélange de 2 oléfines régioisomères | | | |

### b- Synthèse d'oléfines tétrasubstituées 47 en mélange :

L'iodure supporté 6f est engagé dans le même puit et dans les mêmes conditions que précédemment dans la réaction de Sonogashira en l'absence de CuI avec cinq alcynes différents.

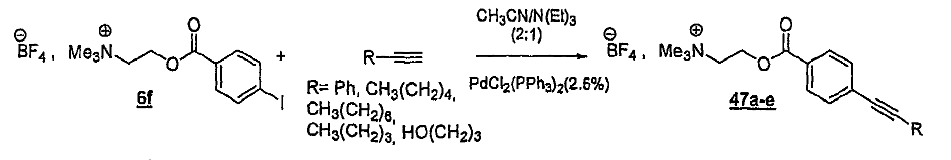

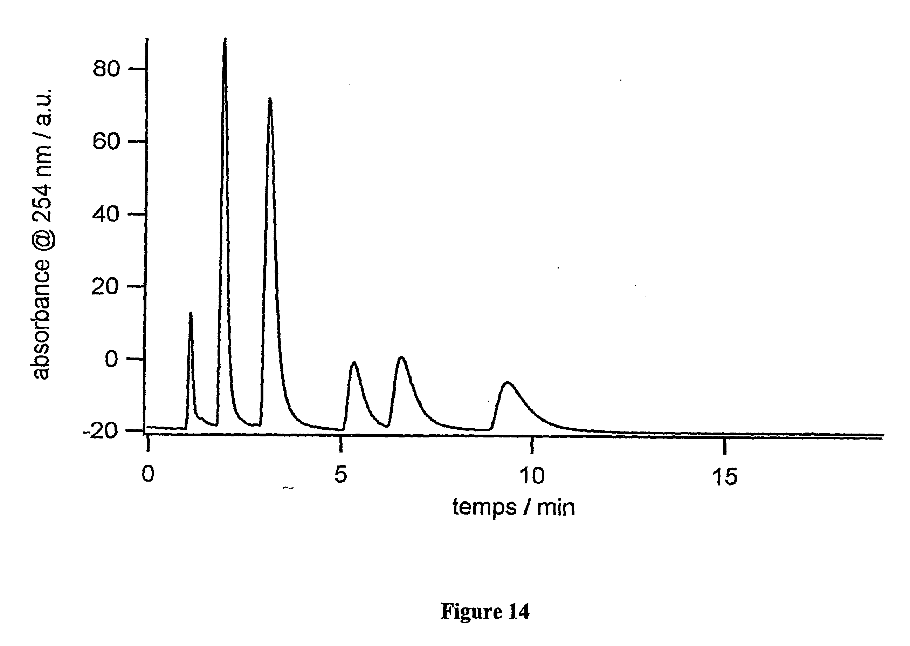

Le suivi de la réaction par HPLC montre l'apparition des produits de couplage de Sonogashira **47** dont les temps de rétention sont 1.92, 3.09, 5.33, 6.57 et 9.35 après une nuit d'agitation à température ambiante (Figure 14).

Après évaporation à sec du solvant et lavage à l'éther, le mélange obtenu est divisé en trois parties. Chaque partie est engagée pour former des oléfines tétrasubstituées sans l'ajout de catalyseur. Ceci, en présence de l'acide phénylboronique et trois iodures d'aryle. Après trois heures de chauffage à 100°C les mélanges réactionnels sont transestérifiés séparément en présence du méthanol et d'acide chlorhydrique.

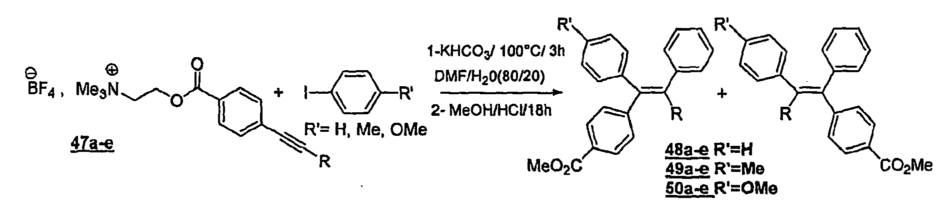

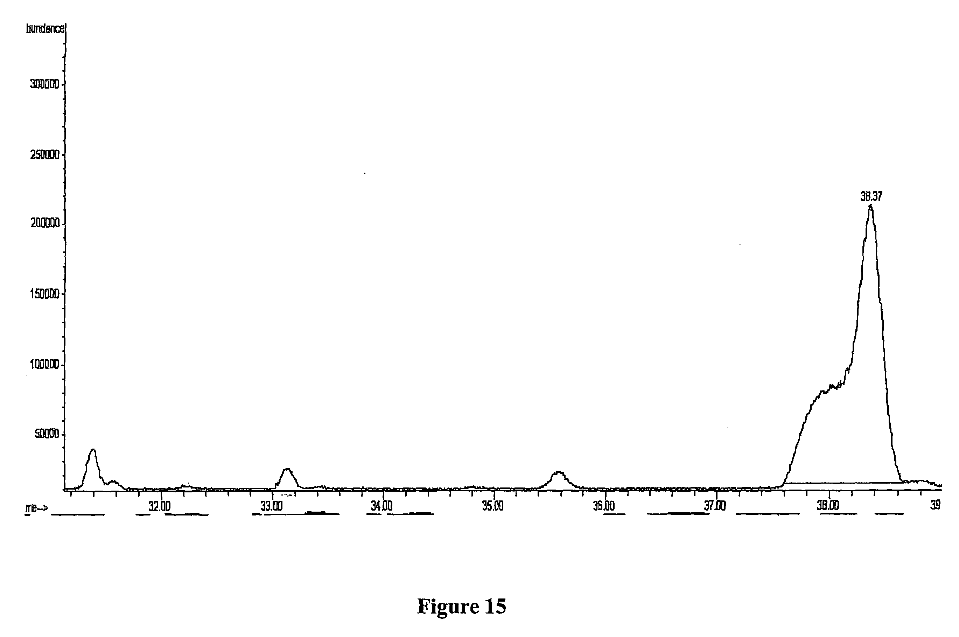

Les oléfines tétrasubstituées sont extraites à l'éther après évaporation du méthanol et injectées en GC/MS. Le chromatogramme présenté en figure 15 illustre le cas du 4-iodotoluène (**49a-e**).

**Tableau 26 : Caractérisation par GC/MS de la librairie d'oléfines tétrasubstituées 49a-e.**

| **49** | **R** | **Temps de rétention** | **Masse moléculaire** |
|---|---|---|---|
| **49a** | CH₃(CH₂)₃ | 31.37 et 31.59 | 384 |
| **49b** | CH₃(CH₂)₄ | 33.12 et 33.42 | 398 |
| **49c et49d** | HO(CH₂)₃ et Ph | 37.50-38.50 | 386 et 404 |
| **49e** | CH₃(CH₂)₆ | 38.80 | 426 |

### E - AUGMENTATION DE LA PRODUCTIVITE DES SOLUTIONS DE SELS D'ONIUM DANS LES SOLVANTS POLAIRES CONVENTIONNELS.

On rappelle que la charge spécifique d'un support est définie par la quantité de réactif qui peut être supportée par gramme de support et s'exprime en mmol/g. Cela correspond en fait à ce que l'on pourrait appeler une ***fonctionnalité spécifique*** d'un support notée ***f*** que l'on pourra exprimer en millifonction par gramme (mf/g.) Dans le cas des solutions de sels d'onium dans les solvants polaires, la molarité sera exprimée en mol/l ou en mmol/ml. Connaissant la densité des solutions, il est alors facile de convertir en mmol/g pour obtenir des éléments de comparaison avec les résines de Merrifield ou les solutions de polymères solubles de type PEGs ou autre. Si le sel est monofonctionnel, la fonctionnalité spécifique (***f*** exprimée en mf/g) sera égale à la charge spécifique exprimée en mmol/g. Si le sel porte n fois la même fonction, une solution contenant par exemple une millimole de ce sel par gramme aura une fonctionnalité spécifique ***f*** de n.mf/g.

Dans le cas des sels d'onium testés dans la partie précédente des exemples (cas des sels d'onium mono fonctionnalisés), la charge spécifique est supérieure à 1 mmol.g⁻¹ et peut atteindre jusqu'à 7 mmol.g⁻¹ (voir tableau 27 ci-dessous).

**Tableau 27**

| 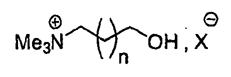 | Masse moléculaire | Charge spécifique du support en (mmol/g) | Charge en poids d'une solution molaire en mg/ml |
|---|---|---|---|
| **n=0 ; X=NTf₂** | 384 | 2,60 | 384 |
| **n=1 ; X=Cl** | 153,5 | 6,51 | 153,5 |
| **n=1 ; X=PF₆** | 263 | 3,80 | 263 |
| **n=1 ; X=BF₄** | 205 | 4,87 | 205 |
| **n=1 ; X=NTf₂** | 398 | 2,51 | 398 |
| **n=2 ; X=Cl** | 167,5 | 5,97 | 167,5 |
| **n=2 ; X=NTf₂** | 412 | 2,42 | 412 |
| **n=2 ; X=OTf** | 283 | 3,53 | 283 |
| **n=3 ; X=Cl** | 181,5 | 5,50 | 181.5 |
| **n=3 ; X=NTf₂** | 426 | 2,34 | 426 |
| **PEG 5000** | 5000 | 0,2 | 5000 (5g) |

A titre de comparaison, la charge spécifique des PEG, qui sont les supports solubles les plus utilisés, est habituellement comprise entre 0,1 et 1 mmol.g⁻¹. Des valeurs supérieures (de l'ordre de 10 mmol g⁻¹) sont atteintes dans le cas bien particulier d'un PEG possédant une structure de type dendrimère telle que celle décrite par Haag (Haag et al., 2002).

On a synthétisé des sels d'ammonium portant deux bras fonctionnels (ou plus). Les 4 substituants de l'atome d'azote peuvent être simultanément fonctionnalisés multipliant d'autant la densité de fonction de ces sels.

On peut aussi envisager de synthétiser des sels d'onium à structure dendrimérique, dont la charge spécifique sera ainsi naturellement multipliée.

Il faut également signaler que les solutions de sels d'onium dans les solvants couramment utilisés (CH₂Cl₂, CH₃CN, DMF, H₂O...) restent peu visqueuses même en opérant à des concentrations supérieures à 4 moles par litre. Ceci représente un avantage majeur par rapport aux solutions de PEG dont la concentration est généralement inférieure à 1 mol.l⁻¹

### 1 - Sels d'ammonium bifonctionnels :

La condensation de 3-chloropropanol et de la diméthylamine est réalisée à reflux dans l'eau pour conduire après 24 heures au sel bifonctionnalisé avec un bon rendement selon le schéma suivant :

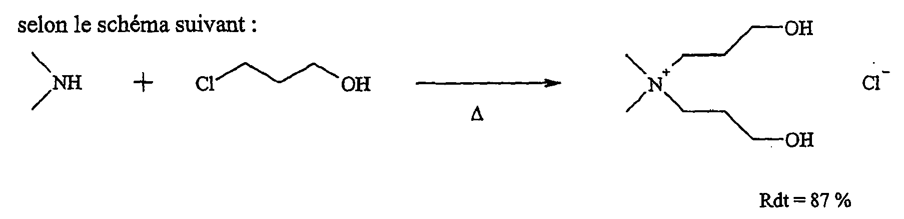

Ces nouveaux supports ont ensuite été testés dans les mêmes réactions que leurs analogues monofonctionnalisés. Pour cela on a supporté des esters d'acryloyle et aryliques halogénés, qui sont ensuite engagés dans des réactions de cycloaddition de Diels-Alder et de couplages..

Les séquences réactionnelles et les résultats obtenus sont présentés sur les schémas ci-dessous.

### a- Réaction de cycloaddition :

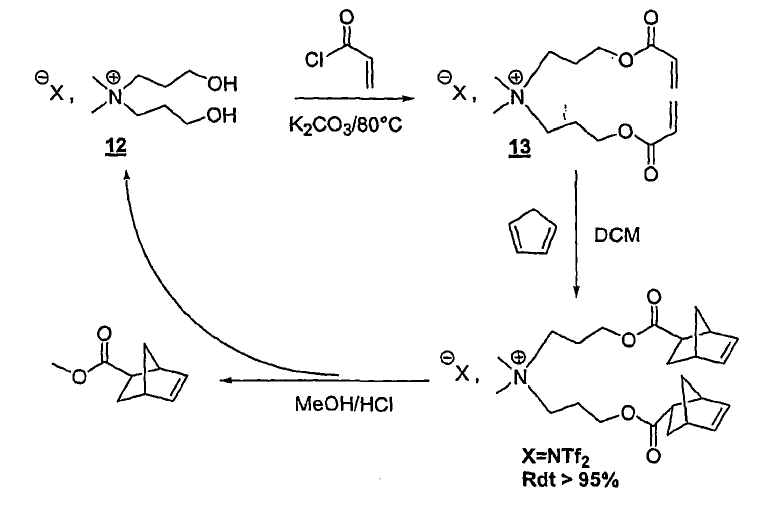

### b- Couplage de Heck :

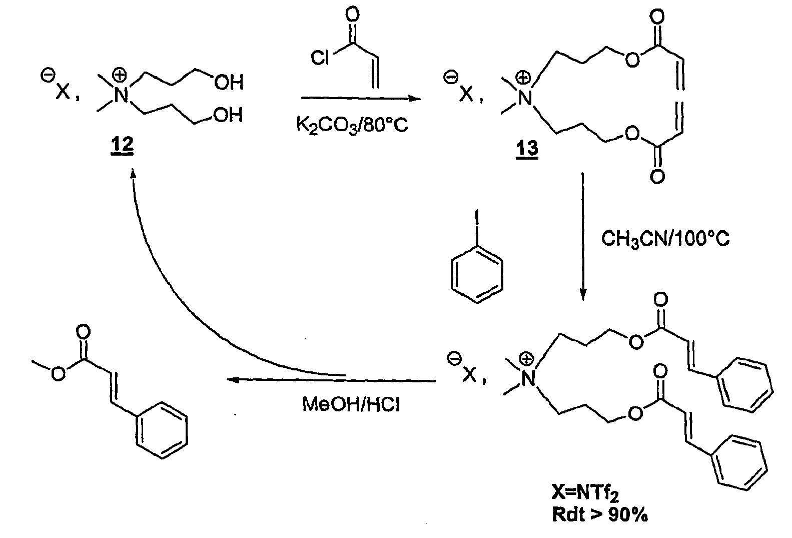

### c- Couplage de Suzuki :

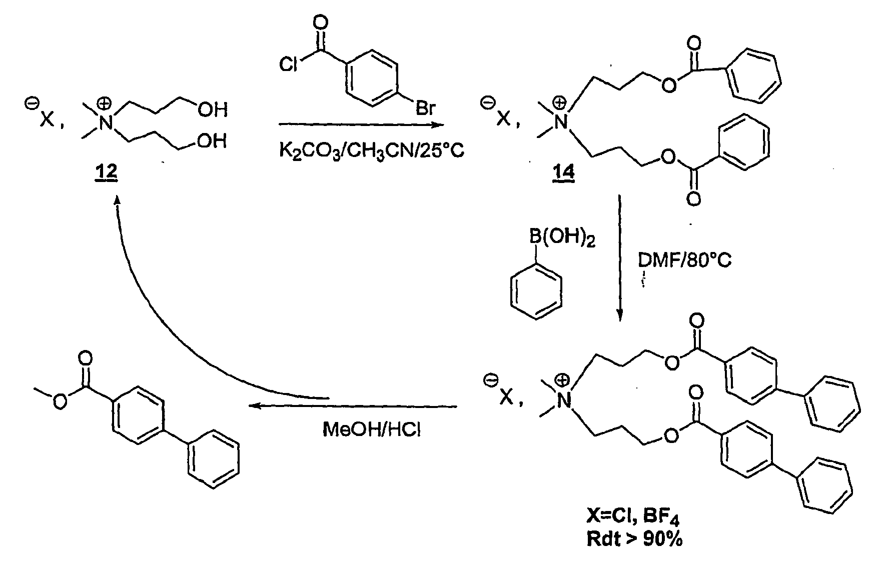

La transposition des réactions réalisées sur les supports monofonctionnalisés aux analogues bifonctionnalisés n'entraîne aucune différence de réactivité ou de sélectivité. De même, les rendements en produits isolés et leur pureté restent excellents.

Les métathèses d'anions ont été réalisées soit avant le greffage des substrats (cas de l'anion bis-trifluorométhanesulfonamidure) ou après (cas de l'anion tétrafluoroborate).

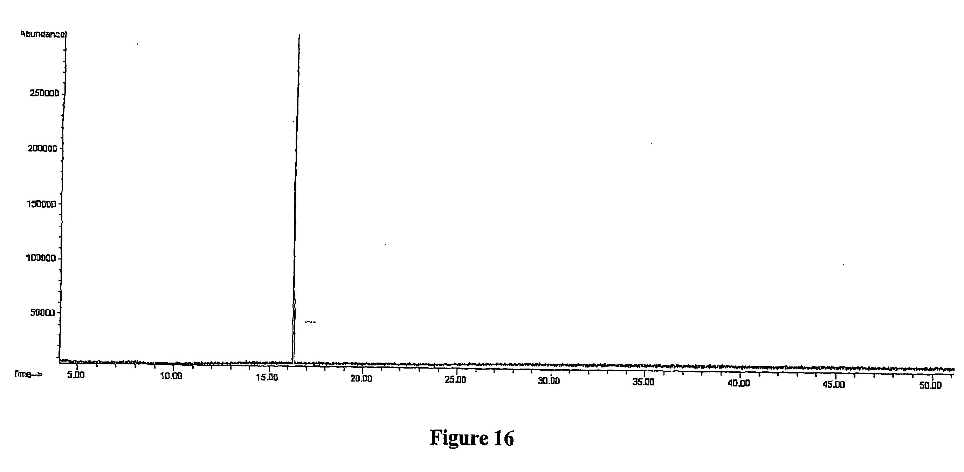

Le chromatogramme correspondant aux produits de couplage de Suzuki réalisé sur le chlorure d'ammonium bifonctionnel (14, X=Cl) est présenté dans la Figure 16. Ce chromatogramme met en évidence la pureté du produit brut isolé après transestérification par du méthanol et extraction à l'éther diéthylique avec un rendement de 92%.

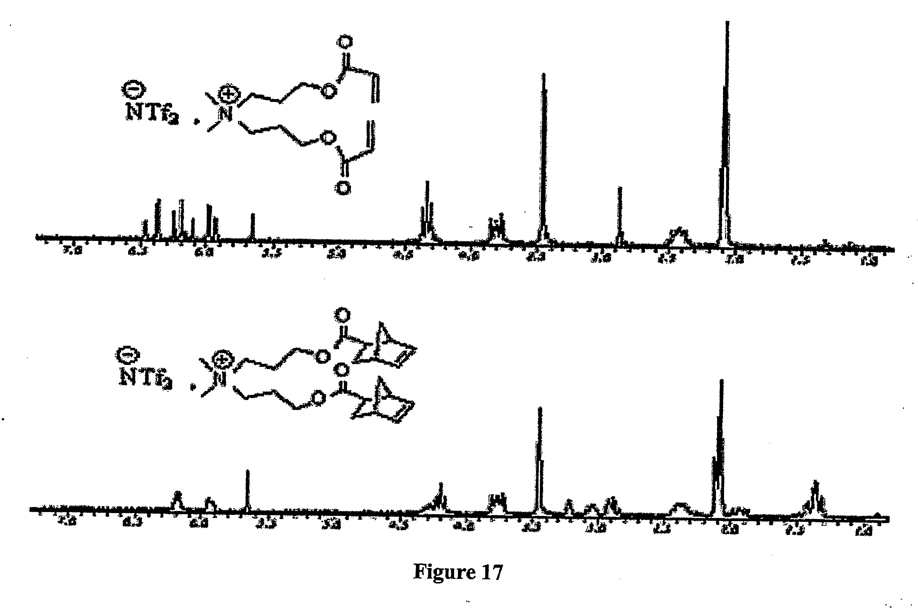

Le suivi de ces différentes réactions a été réalisé par RMN du proton comme dans le cas des supports monofonctionnalisés (voir Figure 17).

Dans un deuxième volet, nous nous sommes intéressés à augmenter davantage la densité de fonction des sels d'ammonium par tri- et tétrafonctionnalisation selon les séquences réactionnelles suivantes :

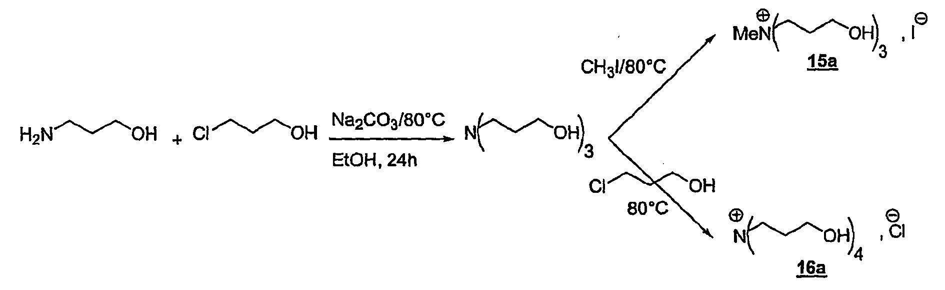

Les sels tri- et tétra-fonctionalisés ont été préparés à partir de la tripropanolamine par quaternarisation au moyen d'iodure de méthyle ou du 3-chloropropanol conduisant respectivement à **15a** ou **16a.**

On a ensuite utilisé les sels de bis-trifluorométhanesulfonamidure d'ammonium dérivés de **15a** et **16a** obtenus par métathèse dans l'eau pour supporter l'ester acrylique. Ces derniers sont ensuite engagés dans les réactions de cycloaddition comme diénophiles en présence du cyclopentadiène et dans la réaction de couplage de Heck. La réaction de cycloaddition est totale après 2 heures et permet d'isoler le produit avec des rendements supérieurs à 75% et une pureté de 97%. De même le couplage de Heck a été réalisé avec les mêmes rendements que ceux obtenus avec les sels mono et bifonctionnalisés (> 95%).

### a- Réactions de cycloaddition :

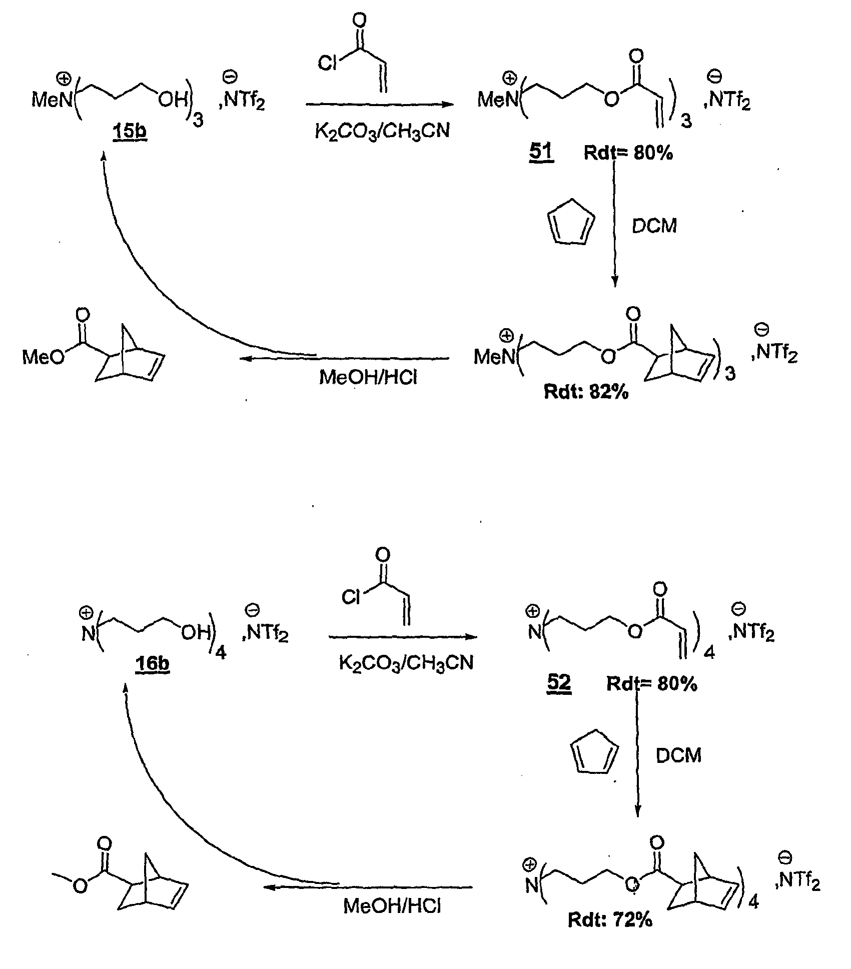

### b- Couplages de Heck :

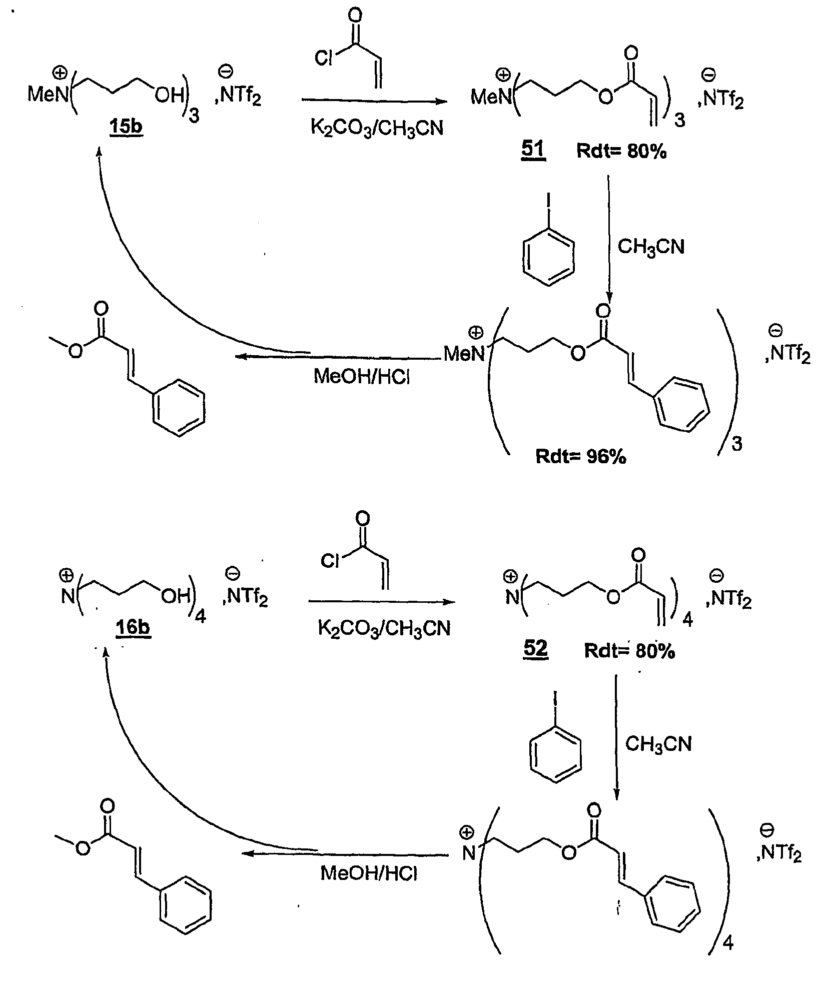

### 3 - Sels d'onium supportant des fonctions différentes :

Au cours de cette étude, on a montré que les sels d'onium présentent des potentialités et des propriétés très intéressantes comme supports solubles en synthèse organique. En effet, en plus des avantages que nous avons déjà exposés dans les premières parties de ce manuscrit, une des possibilités offertes par ces nouveaux supports solubles est de porter deux fonctions (ou plus) de nature différente sur un même cation à condition qu'elles n'interagissent pas entre elles.

A titre d'illustration, nous nous limiterons à la description de sels d'ammonium portant simultanément un ester acrylique et un halogénure d'aryle de formule suivante :

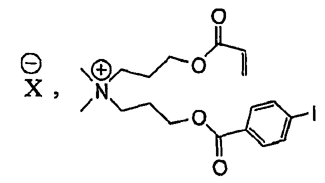

Le sel d'ammonium préparé selon le schéma réactionnel ci-dessous a été engagé dans la réaction de couplage de Heck. Après une heure à 100°C en présence d'acétate de palladium des produits ont été isolés, qui après transestérification par le méthanol en présence de quelques gouttes d'acide chlorhydrique concentré conduisent au produit de couplage de Heck avec un rendement de 66% et une pureté supérieure à 98%. Il faut noter que seul l'isomère trans a été observé dans les deux cas.

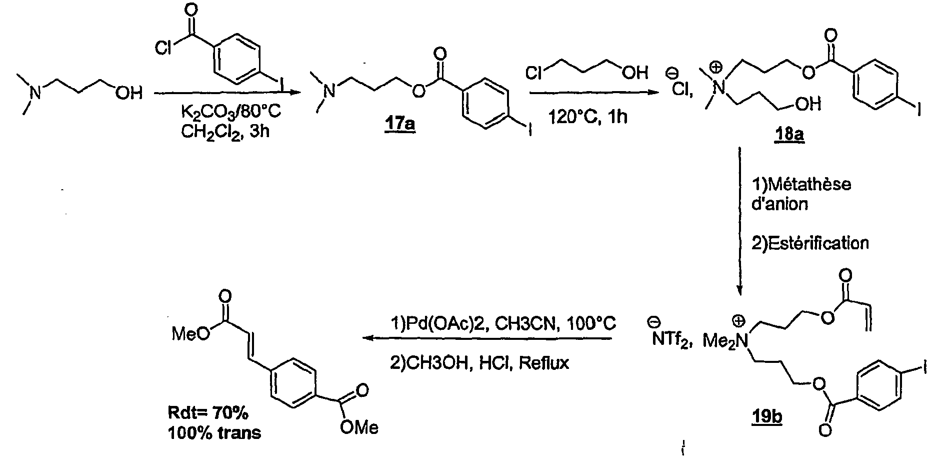

L'insolubilité du produit de réaction avant la transestérification dans la dernière étape dans différents solvants laisse penser qu'une structure de type polymère se forme :

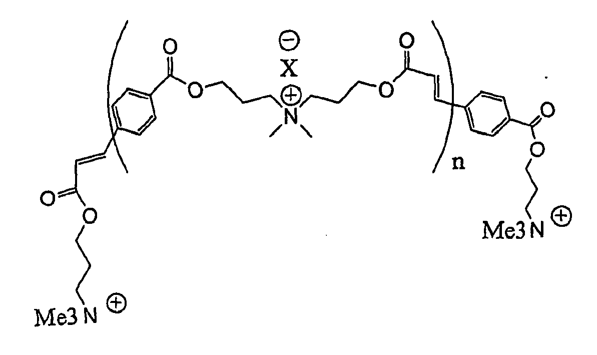

Si le nombre d'atomes est choisi de façon judicieuse pour conduire au produit de cyclisation (couplage intramoléculaire) la forme cis sera obtenue exclusivement En effet, une telle cyclisation a été déjà observée dans des conditions similaires.

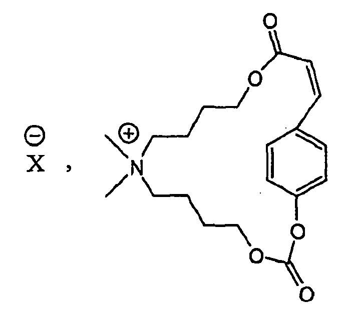

Les résultats préliminaires démontrent l'importance de cette famille de nouveaux supports. Les applications potentielles peuvent en outre encore être enrichies en supportant sur un des bras un catalyseur ou ligand, et sur ceux restants un ou plusieurs réactifs identiques ou non. Cette nouvelle technologie offre un choix énorme et illimité d'applications.

### RÉFÉRENCES

- Alexander, S.K. et Armstrong, R.W. (1997) Tetrahedron Lett., 38, 6163,
- Alexander, S.K.; Leonsmith, A.V.; Armstrong, R.W. (1998) Tetrahedron Lett., 54, 7987,
- Annunziata, R.; Benaglia, M.; Cinquini, M.; Cozzi, F. (2000) Chem.-Eur. J., 6, 133-138,
- Barrett, A. G. M.; Cramp, S. M.; Roberts, R. S.; Zecri, F. (1999) J. Org. Lett., 1, 579-582,
- Barrett, A. G. M.; Cramp, S. M.; Roberts, R. S.; Zécri, F. (2000) J. Org. Lett., 2, 261-264,
- Barrett, A. G. M.; Cramp, S. M.; Roberts, R. S.; Zécri, F. (2000) J. Comb. Chem. High Throughput Screening, 3, 131-133,
- Benaglia, M.; Annunziata, R.; Cinquini, M.; Cozzi, F.; Ressel, S. (1998) J. Org. Chem., 63, 8628-8629,
- Berteina, S.; Wendebom, S.; Brill, K.-D.; De Mesmaeker, A. (1998) Synlett, 6, 676,
- Bienayme, H.; Schmitt, P. (2000) Actualité Chimique, 9, 29-34,
- Bleicher, K.H.; Wareing, J.R. (1998) Tetrahedron Letters, 39 (26), 4587,
- Burgath, A.; Sunder, A.; Frey, H. (2000) Macromol. Chem. Phys., 201, 782-791,
- Chang, J.; Oyelaran, O.; Esser, C. K.; Kath, G. S.; King, G. W.; Uhrig, B. G.; et al. (1999) Tetrahedron Lett., 40, 4477-4480,
- Corey et al. (1998) Tetraedron Lett., 39, 5347-5350,
- Domling, A.; Ugi, I. (2000) Angew. Chem., Int. Ed., 39(18), 3168-3210,
- Domling, A. (2002) Current Opinion in Chemical Biology, 6(3), 306-313,
- Elias, H. G. (1997) An Introduction to Polymer Science; VCH: Weinheim; p 163,
- Frank, H.; Hagenmaier, H. (1975) Experientia, 31, 131-133,
- Franzen, R. (2000) Canadian Journal of Chemistry, 78(7), 957-962,
- Gravert, D. J.; Janda, K. D. (1997) Chem. Rev., 97, 489-509,
- Grayson, S. M.; Jayaraman, M.; Fréchet, J. M. (1999) J. Chem. Commun., 1329-1330,
- Grieco, P.; Bahsas, A. (1988) Tetrahedron Lett., 29, 5855,
- Haag, R. (2001) Chem.-Eur. J., 7, 327-335,
- Haag, R.; Hebel, A.; Stumbé, J. F. (2002) In Handbook of Combinatorial Chemistry; Nicolaou, K. C., Hanko, R., Hartwig, W., Eds.; Wiley-VCH: Weinheim; pp 24-58,
- Haag, R.; Sunder, A.; Hebel, A.; et Roller, S. (2002) J. Comb. Chem., 4, 112-119,
- Haag, R.; Sunder, A.; Stumbé, J.-F. (2000) J. Am. Chem. Soc., 122, 2954-2955,
- Han, H.; Janda, K. D. (1997) Angew. Chem., Int. Ed. Engl., 36, 1731-1733,
- Han, H.; Wolfe, M. M.; Brenner, S.; Janda, K. D. (1995) Proc. Natl. Acad. Sci. U.S.A., 92, 6419,
- Harris, J. M. (1992) In Poly(Ethylene Glycol) Chemistry: Biotechnical and Biomedical Applications; Harris, J. M., Ed.; Plenum Press: New York; p 2,
- Hodge, P. (1997) Chem. Soc. Rev., 26, 417-424,
- Hovestad, N. J.; Ford, A.; Jastrzebski, J. T. B. H.; van Koten, G. (2000) J. Org. Chem., 65, 6338-6344,
- Jayaraman, M.; Fréchet, J. M. J. (1998) J. Am. Chem. Soc., 120, 12996-12997,
- Kates, Steven A.; Albericio, Fernando (2000) Solid-Phase Synthesis,
- Kim, R. M.; Chang, J. (2000) In Combinatorial Chemistry; Fenniri, H., Ed.; Oxford University Press: Oxford; pp 353-371,
- Kim, R. M.; Manna, M.; Hutchins, S. M.; Griffin, P. R.; Yates, N. A.; Bernick, A. M.; Chapman, K. T. (1996) Proc. Natl. Acad. Sci. U.S.A., 93, 10012-10017,
- Knischka, R.; Lutz, P.; Sunder, A.; Mülhaupt, R.; Frey, H. (2000) Macromolecules, 33, 315-320,
- Lindström Ulf M. (2002) Chem. Rev. 102 (8), 2751-2772,
- Meier, S.; Reisinger, H.; Haag, R.; Mecking, S.; Mülhaupt, R.; Stelzer, F. (2001) Chem. Commun., 855-856,
- Merrifield, R. B. (1963) J. Am. Chem. Soc., 85, 2149,
- Mutter, M.; Baeyer, E. (1974) Angew. Chem., Int. Ed. Engl., 13, 88-89,
- Mütter, M.; Bayer, E. (1979) In The Peptides; Academic Press: New York, Vol. 2, p 285,
- Mutter, M.; Uhmann, R.; Baeyer, E. (1975) Liebigs Ann. Chem., 901-915,
- Nicolaou, K.C.; Hanko, R.; Hartwig, W. (2002) Handbook of Combinatorial Chemistry, Volume 2: Drugs, Catalysts, Materials,
- Nicolaou, K.C.; Hanko, R.; Hartwig, W. (2002) Handbook of Combinatorial Chemistry, Volume 1: Drugs, Catalysts, Materials,
- O'Donnel, M.J.; Wu, S.; Bennett, W.D. (1989) J. Am. Chem. Soc., 111, 2353,
- Ranucci, E.; Spagnoli, G.; Sartore, L.; Ferruti, P.; Caliceti, P.; Schiavon, O.; Veronese, F. (1994) Macromol. Chem. Phys., 195, 3469-3479,
- Haag, R.; Sunder, A.; Hebel, A. et Roller, S. (2002) J. Comb. Chem., 4, 112-119,
- Reed, N. N.; Janda, K. D. (2000) Org. Lett., 2, 1311-1313,
- Reichardt, Christian (1988) Solvents and solvent effects in organic chemistry, Weinheim, Bâle, Suisse ; Cambridge ; New York, NY : VCH,
- Seeberger, P. H.; Haase, W.-C. (2000) Chem. Rev., 100, 4349,
- Thompson, L. A.; Ellman, J. A. (1996) Chem. Rev., 96, 555,
- Toy, P.; Janda, K. D. (2000) Acc. Chem. Res., 33, 546-554,
- Ugi, I.; Domling, A.; Ebert, B. (1999) Combinatorial Chemistry, 125-165,
- Ugi, I. (2001) Pure and Applied Chemistry, 73(1), 187-191,
- Ugi, I.; Heck, S. (2001) J. Comb. Chem. High Throughput Screening, 4(1), 1-34,
- V., 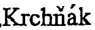 et M., W. Holladay (2002) Chem. Rev., 102, 61-91,
- Vanden Eynde, J.J.; Mayence, A. (2000) International Journal of Medicine, Biology and the Environment, 28(1), 89-95,
- Wasserscheid, P.; Keim, W. (2000) Ang. Chem. Int. Ed., 39, 3772-3789,
- Wellings, D. A.; Williams, A. (1987) React. Polym., 6, 143-157,
- Welton, T. (1999) Chem. Rev., 99, 2071-2083,
- Wendebom, S.; Berteina, S.; Brill, K.-D.; De Mesmaeker, A. (1998) Synlett, 6, 671,
- Zhou, C.; Emrich, D.E.; Larock, R.C. (2003) Org. Lett., 5, 1579-1582.

## Revendications

1. Utilisation d'un sel d'onium fonctionnalisé par au moins une fonction organique, en tant que support soluble, en présence d'au moins un solvant organique, pour la synthèse organique d'une molécule, en phase homogène, ladite synthèse organique comprenant au moins une transformation de ladite fonction organique, suivie d'une réaction de clivage libérant la molécule synthétisée en solution dans ledit solvant et le sel d'onium sous sa forme initiale, c'est-à-dire avant la transformation de ladite fonction organique,
ledit sel d'onium se présentant sous forme liquide ou solide à température ambiante, et répondant à la formule A₁⁺, X₁⁻,
dans laquelle :
- A₁⁺ représente un cation,
- X₁⁻ représente un anion,
A₁⁺ étant un cation fonctionnel ou polyfonctionnel, et/ou
X₁⁻ étant un anion fonctionnel ou polyfonctionnel,
le sel d'onium étant tel que sous sa forme initiale, c'est-à-dire avant la première transformation de ladite fonction organique, A₁⁺ et X₁⁻ ne sont pas liés entre eux par une liaison covalente,
et lorsque l'anion et le cation portent respectivement une fonction organique, celles-ci ne peuvent pas réagir entre elles avant la première transformation de ladite fonction organique.

2. Utilisation selon la revendication 1, **caractérisée en ce que** le sel d'onium est purifié et/ou recyclé sous sa forme initiale après la libération de la molécule synthétisée.

3. Utilisation selon la revendication 1 ou 2, **caractérisée en ce que** les cations et anions fonctionnels correspondent à une entité ionique, respectivement cationique Y⁺- et anionique Z⁻-, liée, éventuellement par l'intermédiaire d'un bras, respectivement L et M, notamment un groupe alkyle ou aralkyle ou alkaryle comprenant de 1 à 30 atomes de carbone, à au moins respectivement une fonction Fᵢ et F'ᵢ, Fᵢ variant de F₀ à Fₙ, F'ᵢ variant de F'₀ à F'ₙ, n étant un nombre entier variant de 1 à 10,
le cation fonctionnel A₁⁺ pouvant être représenté sous la forme Y⁺-L-Fᵢ, et
l'anion fonctionnel X₁⁻ sous la forme Z⁻-(M)ₖ-F'ᵢ, k étant égal à 0 ou 1.

4. Utilisation selon l'une quelconque des revendications 1 à 3, **caractérisée en ce que** les fonctions organiques Fᵢ et F'ᵢ sont choisies parmi les fonctions classiques de la chimie organique, telles que les fonctions hydroxyle, acide carboxylique, amide, sulfone, amine primaire, amine secondaire, aldéhyde, cétone, éthényle, éthynyle, diényle, éther, époxyde, phosphine (primaire, secondaire ou tertiaire), azoture, imine, cétène, cumulène, hétérocumulène, thiol, thioéther, sulfoxyde, groupements phosphorés, hétérocycles, acide sulfonique, silane, stannane ou aryle fonctionnel.

5. Utilisation selon l'une quelconque des revendications 1 à 4, **caractérisée en ce que** le poids moléculaire du sel d'onium fonctionnalisé est inférieur à 1500 g.mol⁻¹, notamment inférieur à 750 g.mol⁻¹, et est de préférence compris de 130 à 500 g.mol⁻¹.

6. Utilisation selon l'une quelconque des revendications 1 à 5, **caractérisée en ce que** A₁⁺ est un cation fonctionnel et **en ce que** X₁⁻ est un anion non fonctionnel.

7. Utilisation selon la revendication 6, dans laquelle le sel d'onium A₁⁺, X₁⁻ a comme forme initiale Y⁺-L-F₀, X₁⁻, pour l'obtention d'une molécule G, par transformation de ladite fonction initiale F₀ selon le schéma 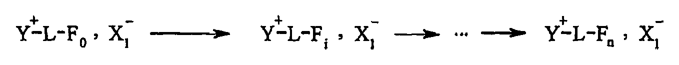 L étant tel que défini dans la revendication 3,
ladite molécule G étant obtenue par clivage de la fonction Fₙ,
et le sel d'onium fonctionnalisé pouvant être récupéré ou recyclé sous sa forme initiale Y⁺-L-F₀, X₁⁻, après la libération de G.

8. Utilisation selon l'une quelconque des revendications 1 à 7, **caractérisée en ce que** le cation fonctionnel A₁⁺ est choisi parmi les cations pyridinium, imidazolium, ammonium, phosphonium ou sulfonium, cycliques ou non, substitués ou non, et de préférence ammonium ou phosphonium.

9. Utilisation selon la revendication 8, **caractérisée en ce que** le cation fonctionnel A₁⁺ est choisi parmi les cations ammoniums quaternaires, cycliques ou non.

10. Utilisation selon l'une quelconque des revendications 1 à 5, **caractérisée en ce que** X₁⁻ est un anion fonctionnel et A₁⁺ est un cation non fonctionnel.

11. Utilisation selon la revendication 10, dans laquelle le sel d'onium A₁⁺, X₁⁻ a comme forme initiale A₁⁺, Z⁻-(M)ₖ-F'₀, pour l'obtention d'une molécule G, par transformation de ladite fonction initiale F'₀ selon le schéma 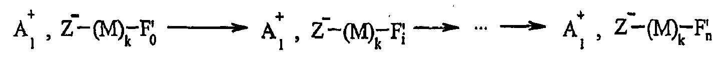 k et M étant tels que définis dans la revendication 3,
ladite molécule G étant obtenue par clivage de la fonction F'ₙ,
et le sel d'onium fonctionnalisé pouvant être récupéré ou recyclé sous sa forme initiale A₁⁺, Z⁻-(M)ₖ-F'₀, après la libération de G.

12. Utilisation selon la revendication 10 ou 11, **caractérisée en ce que** X₁⁻ est choisi parmi :
- la famille des phosphates : R₁PO₄²⁻, R₁R₂PO₄⁻,
- la famille des sulfates : R₁SO₄⁻,
- la famille des sulfonates : R₁SO₃⁻,
- la famille des carboxylates : R₁CO₂⁻,
ou parmi les anions suivants :
R₁N̅SO₂R₂
R₂SO₂N̅SO₂R₂ 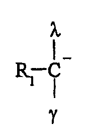 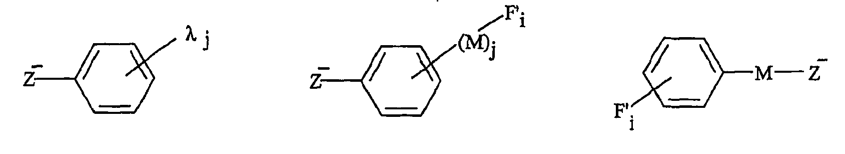
Z⁻, M et F'ᵢ étant tels que définis dans la revendication 3, Z⁻ représentant notamment O⁻, SO₃⁻, CO₂⁻, R₁PO₃⁻ ou R₁PO₂⁻,
j représentant un nombre entier compris de 1 à 5,
R₁ et R₂ pouvant représenter indépendamment l'un de l'autre un groupe alkyle fonctionnel, un groupe vinyle ou alcynyle, éventuellement fonctionnel, comprenant de 1 à 20 atomes de carbone, ou pouvant représenter un groupement aryle fonctionnel comprenant de 6 à 30 atomes de carbone,
γ et λ représentant un groupe électroattracteur, notamment choisi parmi les groupes : CO₂R', SO₂R', CN, NO₂, P(O)(OR')₂, C(O)R' et SO₃R',
R' représentant un groupe alkyle, éventuellement fonctionnel, comprenant de 1 à 20 atomes de carbone, ou un groupe aryle, éventuellement fonctionnel, comprenant de 6 à 30 atomes de carbone.

13. Utilisation selon l'une quelconque des revendications 1 à 5, **caractérisée en ce que** A₁⁺ est un cation fonctionnel et X₁⁻ est un anion fonctionnel.

14. Utilisation selon la revendication 13, dans laquelle le sel d'onium A₁⁺, X₁⁻ a comme forme initiale Y⁺-L-F₀, Z⁻-(M)ₖ-F'₀, pour l'obtention d'une molécule G, par transformation desdites fonctions initiales F₀ et F'₀ selon le schéma 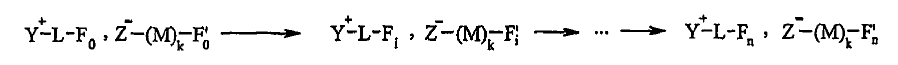 L, k et M étant tels que définis dans la revendication 3,
et par réaction de Fₙ sur F'ₙ dans le sel d'onium fonctionnalisé Y⁺-L-Fₙ, Z⁻-(M)ₖ-F'ₙ conduisant à la formation d'un sel interne de formule : 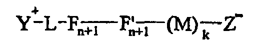 ladite molécule G étant obtenue par clivage du sel interne susmentionné et correspondant à la formule Fₙ₊₂-F'ₙ₊₂,
et le sel d'onium fonctionnalisé pouvant être récupéré ou recyclé sous sa forme initiale Y⁺-L-F₀, Z⁻-(M)ₖ-F'₀, après la libération de G.

15. Utilisation selon l'une quelconque des revendications 1 à 14, **caractérisée en ce que** le sel d'onium est choisi parmi les sels suivants : 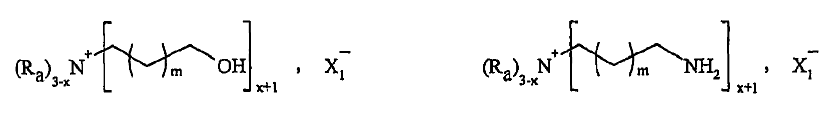 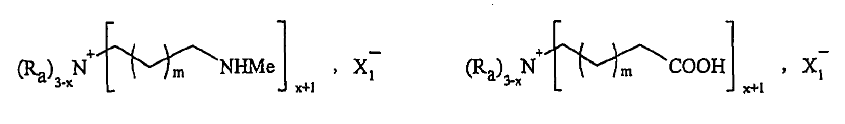 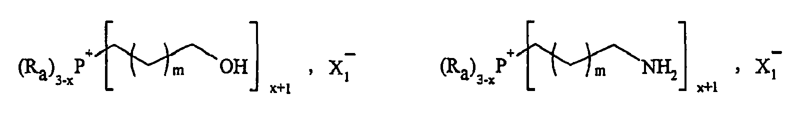 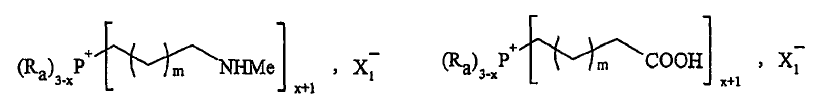 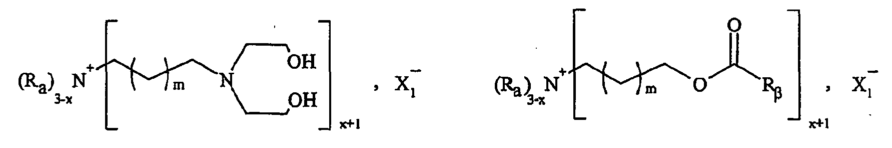 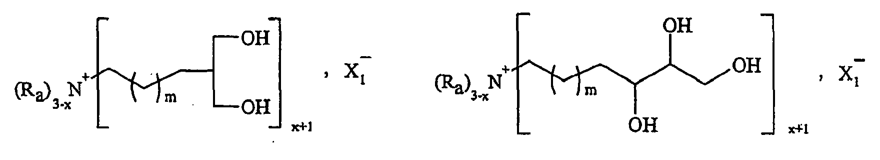 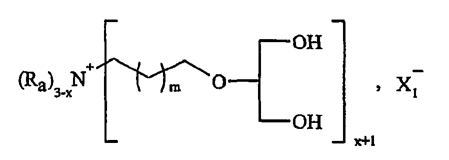 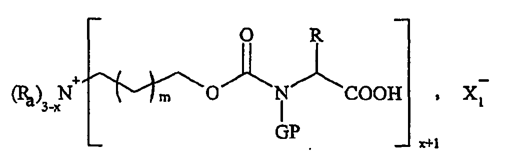 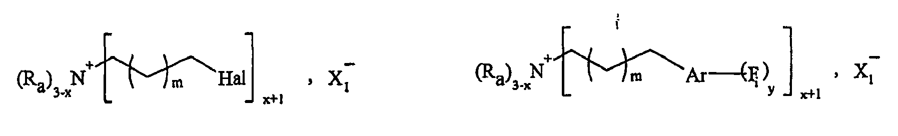 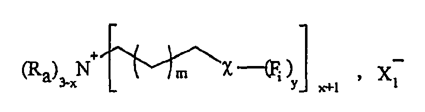 R représentant un atome d'hydrogène, un groupe alkyle, fonctionnel ou non, comprenant de 1 à 20 atomes de carbone, ou un groupe aryle, fonctionnel ou non, comprenant de 6 à 30 atomes de carbone,
x représentant un nombre entier compris de 0 à 3,
y représentant un nombre entier compris de 1 à 5,
Ar représentant un noyau aromatique fonctionnel ou polyfonctionnel,
Fᵢ étant tel que défini dans la revendication 4,
Hal représentant un atome d'halogène, notamment choisi parmi le chlore, le brome et l'iode,
χ représentant un carbocycle ou un hétérocycle fonctionnel,
X₁⁻ étant choisi parmi : NTf₂⁻, PF₆⁻, BF₄⁻, Cl⁻, Br⁻, I⁻, CF₃SO₃⁻, MeSO₄⁻, EtSO₄⁻, MeSO₃⁻, C₆H₅SO₃⁻, pMeC₆H₄SO₃⁻,
m étant un nombre entier compris de 0 à 20,
R_{β} représentant un groupe diényle, vinyle, substitué ou non, alkyle fonctionnel comprenant de 1 à 20 atomes de carbone, ou aryle fonctionnel comprenant de 6 à 30 atomes de carbone, alcynyle substitué ou non, et étant notamment un groupe alkylvinyle, alkylalcynyle, alkylaryle, akyldiényle, alkylmalonyle, acyle,
et Rₐ représentant un groupe alkyle ramifié ou non comprenant de 1 à 20 atomes de carbone, notamment un groupe éthyle, propyle, butyle, pentyle, hexyle, heptyle ou octyle.

16. Utilisation selon l'une quelconque des revendications 1 à 15, **caractérisée en ce que** le ou les solvant(s) utilisé(s) est un solvant aprotique, choisi parmi :
- les solvants dont la constante diélectrique ε est inférieure ou égale à 2, tels que les alcanes, les carbures aromatiques comme le benzène, le toluène ou le xylène,
- les solvants dont la constante diélectrique ε est comprise entre environ 2 et 15, tels que les éthers, les halogénobenzènes ou le dichlorométhane, et
- les solvants dont la constante diélectrique ε est supérieure à 15, tels que l'acétonitrile, le nitrométhane, le DMF ou la diméthylacétamide.

17. Utilisation selon l'une quelconque des revendications 1 à 16, pour la synthèse organique, en continu, en discontinu, combinatoire, ou parallèle, et/ou pour la préparation de banques de produits.

18. Utilisation selon l'une quelconque des revendications 1 à 17, pour la mise en oeuvre de réactions de cycloaddition, de préférence pour la mise en oeuvre de la réaction de Diels-Alder, selon l'un des schémas réactionnels suivants :
a) 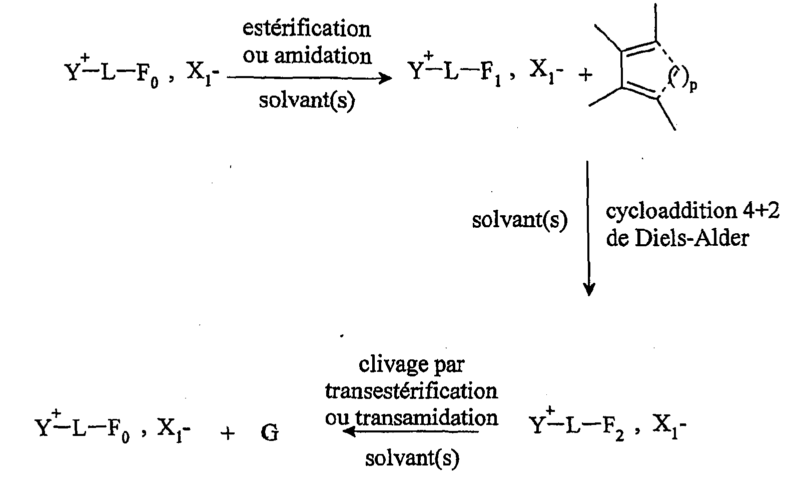 p étant un nombre entier variant de 0 à 2,
Y⁺- représentant un cation onium tel que défini dans l'une des revendications 3 à 17, et étant de préférence un cation triméthylalkylammonium, triéthylalkylammonium, tributylalkylphosphonium, N-méthylimidazolium ou pyridinium,
L représentant un bras, notamment un groupe alkyle linéaire ou ramifié comprenant de 1 à 20 atomes de carbone, ou un groupe aralkyle ou alkaryle éventuellement fonctionnel, comprenant de 6 à 30 atomes de carbone, et étant de préférence un groupe alkyle linéaire de préférence un groupe alkyle linéaire de type (CH₂)ᵣ, r variant de 1 à 20, et de préférence de 2 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁ et F₂ étant telles que définies ci-dessous :
- F₀ correspond à un groupe -χ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante :
| | |
|---|---|
| 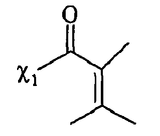 | χ₁ étant tel que défini ci-dessus, |
- F₂ répond à la formule suivante :
| | |
|---|---|
| 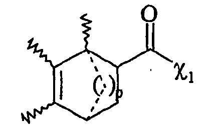 | χ₁ étant tel que défini ci-dessus, |
G répondant à la formule suivante : 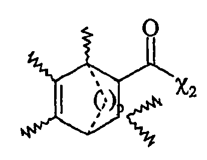 dans laquelle χ₂ représente soit un groupe ORg, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone,
b) 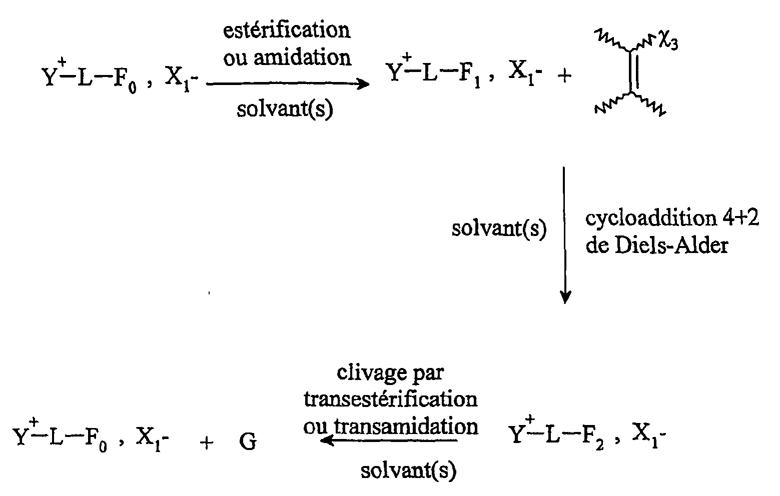 Y⁺-, L et X₁⁻ étant tels que définis précédemment,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, ou un mélange de ces solvants,
les fonctions F₀, F₁ et F₂ étant telles que définies ci-dessous :
- F₀ représente toute fonction permettant d'agrafer un diène-1,3, et est notamment choisi parmi les fonctions carbonyles, amines, alcoxy, silanes, stannanes et boranes, comprenant de 1 à 20 atomes de carbone,
- F₁ répond à la formule suivante :
| | |
|---|---|
| 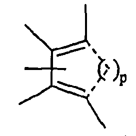 | p étant un nombre entier variant de 0 à 2, |
- F₂ répond à la formule suivante :
| | |
|---|---|
| 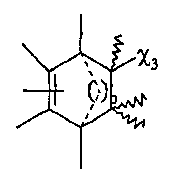 | χ₃ représentant un groupement électroattracteur, notamment choisi parmi les groupes cyano, alkoxycarbonyle, comprenant de 1 à 20 atomes de carbone, acyle comprenant de 2 à 20 atomes de carbone, benzoyle, sulfonyle, dialkoxyphosphonyle comprenant de 1 à 10 atomes de carbone, |
G répondant à la formule suivante :
| | |
|---|---|
| 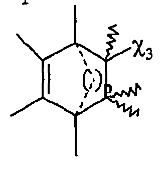 | χ₃ étant tel que défini ci-dessus. |
**c)** 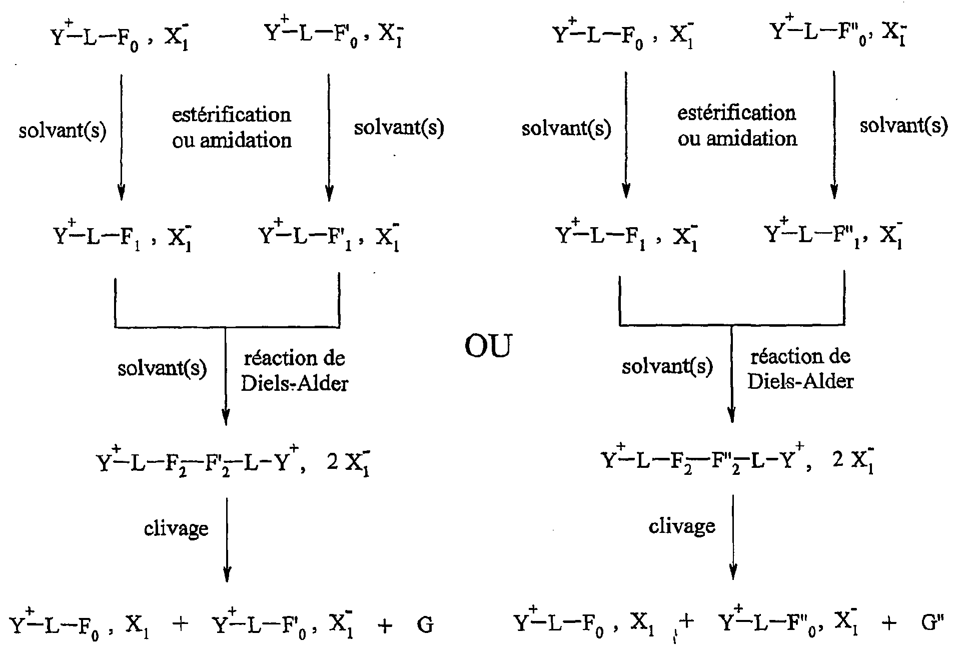 Y⁺-, L et X₁⁻ étant tels que définis précédemment,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F'₀, F"_{0,} F₁, F'₁, F"₁, F₂, F'₂ et F"₂ étant telles que définies ci-dessous :
- F₀ et F'₀ correspondent respectivement à un groupe -χ₁H et -χ'₁H, dans lequel χ₁ et χ'₁, identiques ou différents, représentent un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F"₀ correspond à une fonction -COOH ;
- F₁ répond à la formule suivante :
| | |
|---|---|
| 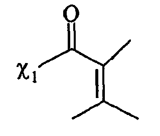 | χ₁ étant tel que défini ci-dessus, |
- F'₁ répond à la formule suivante :
| | |
|---|---|
| 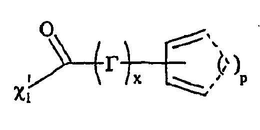 | p étant un nombre entier variant de 0 à 2, |
| | χ'₁ étant tel que défini ci-dessus, |
| | x étant égal à 0 ou 1, |
| | r représentant une chaîne alkyle comprenant de 1 à 30 atomes de carbone, alkaryle, aralkyle, aryle comprenant de 6 à 30 atomes de carbone, |
- F"₁ répond à la formule suivante :
| | |
|---|---|
| 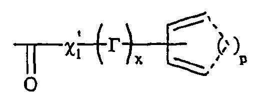 | p, x et r étant tels que définis ci-dessus, χ'₁ étant tel que défini ci-dessus, |
- F₂-F'₂ répond à la formule suivante :
| | |
|---|---|
| 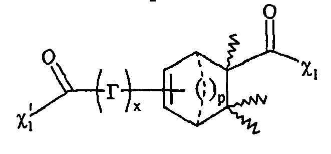 | p, χ₁, χ'₁, x et Γ étant tels que définis ci-dessus, |
- F₂-F"₂ répond à la formule suivante :
| | |
|---|---|
| 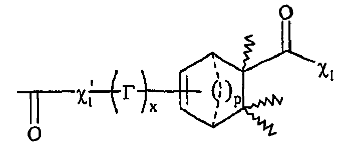 | p, χ₁, χ'₁, x et Γ étant tels que définis ci-dessus, |
- G répond à la formule suivante : 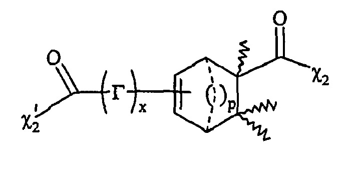
- G" répond à la formule suivante : 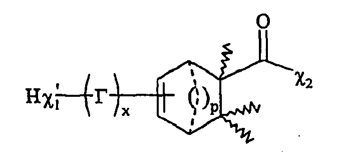
χ ₂ et χ'₂, identiques ou différents, représentent soit un groupe OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes-de carbone,

19. Utilisation selon l'une quelconque des revendications 1 à 17, pour la mise en oeuvre de réactions de couplage comme les réactions de Heck, de Suzuki, de Sonogashira ou d'Ullmann.

20. Utilisation selon la revendication 19 pour la mise en oeuvre de la réaction de Heck, selon l'un des schémas réactionnels suivants : 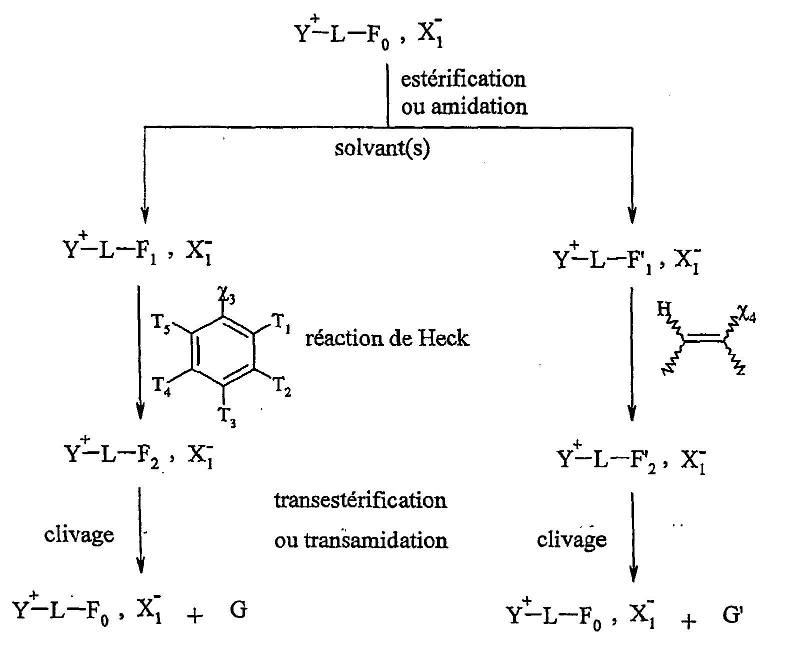 Y⁺- représentant un cation onium tel que défini dans l'une des revendications 3 à 17, et étant de préférence un cation triméthylalkylammonium, triéthylalkylammonium, tributylalkylphosphonium, N-méthylimidazolium ou pyridinium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 2 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁, F'₁, F₂ et F'₂ étant telles que définies ci-dessous :
- F₀ correspond à un groupe -χ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à l'une des formules suivantes : 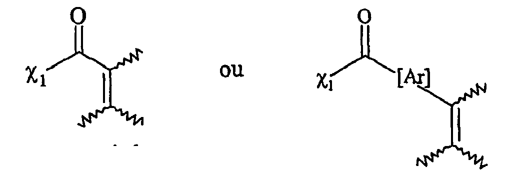 χ ₁ étant tel que défini ci-dessus,
[Ar] représentant un noyau aromatique, éventuellement substitué par un groupe alkyle, linéaire ou ramifié, comprenant de 1 à 20 atomes de carbone ou un groupe aryle comprenant de 6 à 30 atomes de carbone, ou un groupe fonctionnel notamment choisi parmi NO₂, CN, COOR, OR, COR, NHCOR, NRR', SO₂R, I, Br, R et **R'** représentant indépendamment l'un de l'autre un groupe alkyle comprenant de 1 à 20 atomes de carbone ou un groupe aryle comprenant de 6 à 30 atomes de carbone, [Ar] répondant de préférence à la formule suivante :
| | |
|---|---|
| 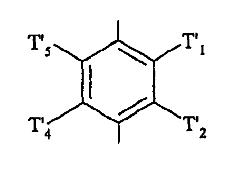 | dans laquelle T'₁, T'₂, T'₄ et T'₅ représentent indépendamment les uns des autres un atome d'hydrogène, un groupe alkyle, linéaire ou ramifié, comprenant de 1 à 20 atomes de carbone ou un groupe aryle comprenant de 6 à 30 atomes de carbone, ou un groupe fonctionnel notamment choisi parmi NO₂, CN, COOR, OR, COR, NHCOR, **NRR',** SO₂R, I, Br, R et **R'** représentant indépendamment l'un de l'autre un groupe alkyle comprenant de 1 à 20 atomes de carbone ou un groupe aryle comprenant de 6 à 30 atomes de carbone, |
- F₂ répond à l'une des formules suivantes : 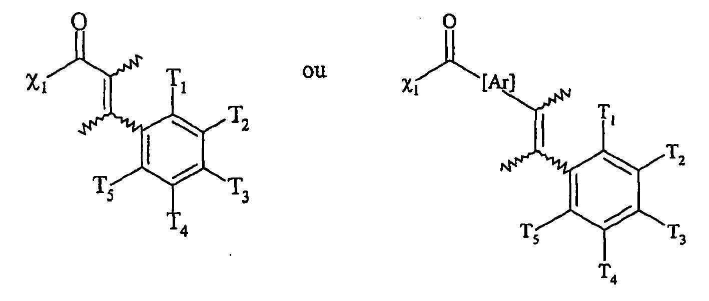 χ ₁ et Ar étant tels que définis ci-dessus,
T₁, T₂, T₃, T₄ et T₅ répondant à la définition donnée ci-dessus pour T'₁, T'₂, T'₄ et T'₅
G répondant à l'une des formules suivantes : 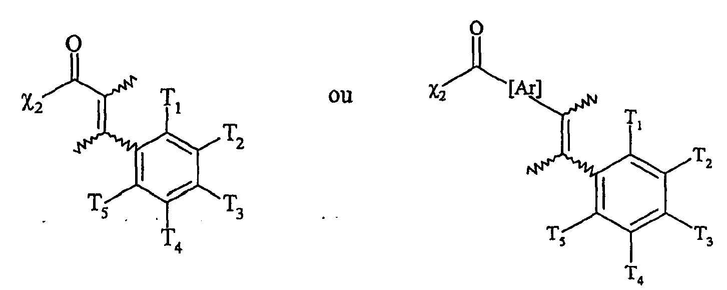 dans laquelle χ₂ représente soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone,
χ₃ représentant un groupement partant, notamment choisi parmi les halogénures I, Cl et Br, les groupes mésylate, tosylate, triflate, sulfonate, sulfate ou phosphate,
l'entité 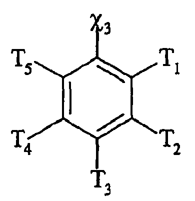 représentant notamment les groupes suivants : 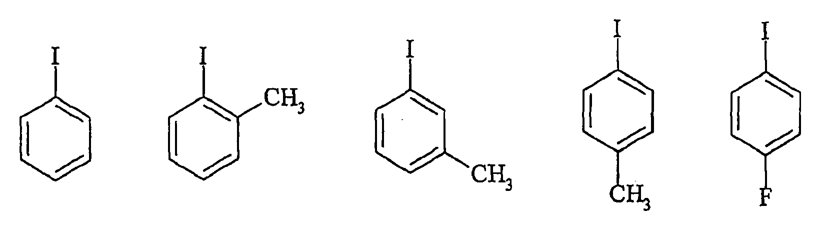 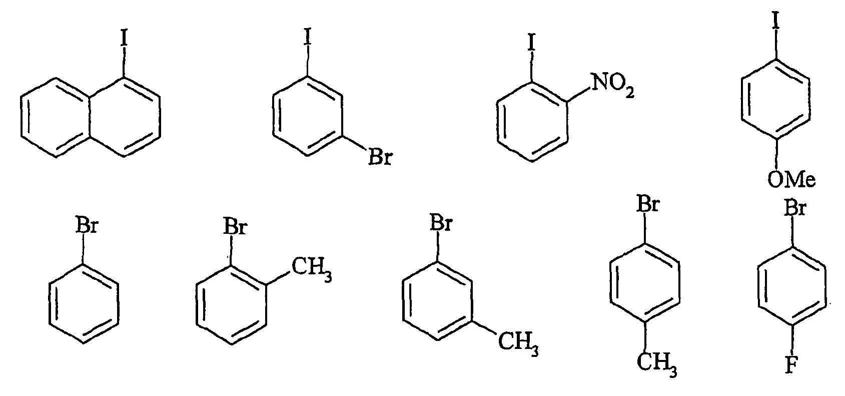 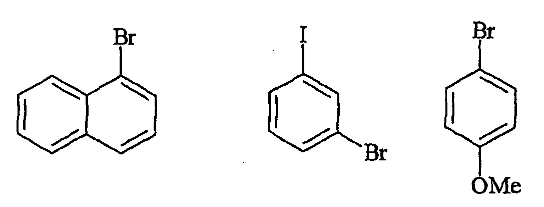
- F'₁ répond à la formule suivante :
| | |
|---|---|
| 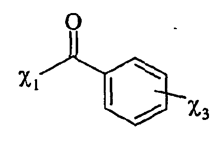 | χ₁ et χ₃ étant tels que définis ci-dessus, |
- F'₂ répond à la formule suivante :
| | |
|---|---|
| 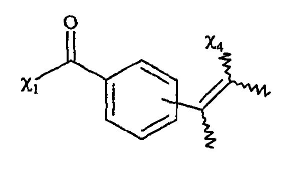 | χ₁ étant tel que défini ci-dessus, |
| | χ₄ représentant un groupe fonctionnel de type ester, amide, sulfone, phosphonate, silane, borane, ou un groupe alkyle, fonctionnel ou non, comprenant de 1 à 20 atomes de carbone, ou un groupe aryle, fonctionnel ou non, comprenant de 6 à 30 atomes de carbone, |
G' répondant à la formule suivante :
| | |
|---|---|
| 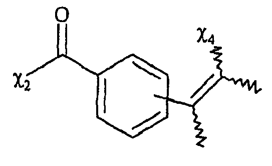 | χ₂ et χ₄ étant tels que définis ci-dessus. |

21. Utilisation selon la revendication 19 pour la mise en oeuvre du couplage de Suzuki, selon l'un des schémas réactionnels suivants :
a) 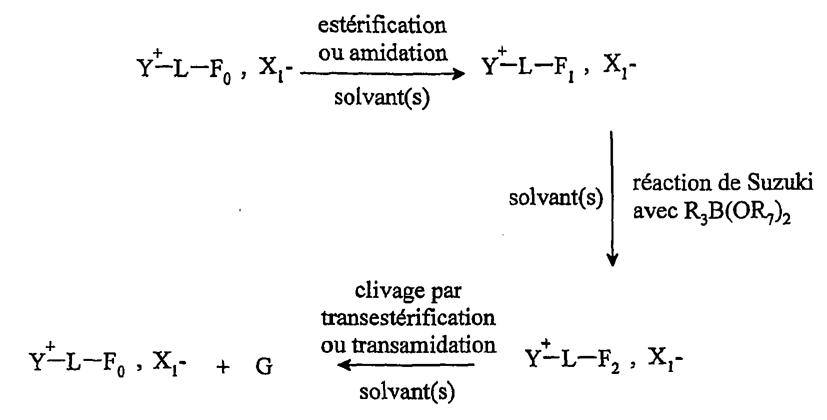 R₃ étant choisi parmi les groupes aryle, hétéroaryle, éthényle, diényle, allyle, éthynyle, substitués ou non, comprenant de 2 à 30 atomes de carbone,
R₇ représentant un atome d'hydrogène ou un groupe alkyle, ramifié ou linéaire ou un groupe cycloalkyle comprenant de 1 à 12 atomes de carbone,
Y⁺- représentant un cation onium tel que défini dans l'une des revendications 3 à 17, et étant de préférence un cation triméthylalkylammonium, triéthylalkylammonium, tributylalkylphosphonium, N-méthylimidazolium ou pyridinium,
L représentant un bras, notamment un groupe alkyle linéaire ou ramifié comprenant de 1 à 20 atomes de carbone, ou un groupe aralkyle éventuellement fonctionnel comprenant de 6 à 30 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, -N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁ et F₂ étant telles que définies ci-dessous :
- F₀ est de la forme -χ₁H, χ₁ représentant un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ est de la forme -Rₑ-χ, Rₑ représentant un groupe aromatique ou hétéroaromatique comprenant de 6 à 30 atomes de carbone, χ représentant un groupe partant choisi de préférence parmi Cl, Br, I, OTf, O-CO₂R⁵ ou OSO₃-R⁵, R⁵ représentant un groupe alkyle comprenant de 1 à 10 atomes de carbone ou un groupe aralkyle comprenant de 6 à 30 atomes de carbone, F₁ répondant de préférence à la formule suivante : 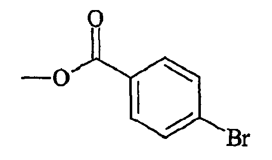
- F₂ est de la forme -Rₑ-R₂, Rₑ étant tel que défini ci-dessus et R₂ étant choisi parmi les groupes aryle, hétéroaryle, éthényle, diényle, allyle, éthynyle, substitués ou non, comprenant de 2 à 30 atomes de carbone, F₂ répondant de préférence à la formule suivante : 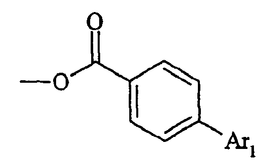 Ar₁ représentant un groupe aromatique choisi de préférence parmi : 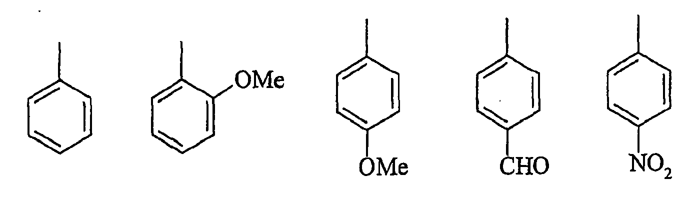 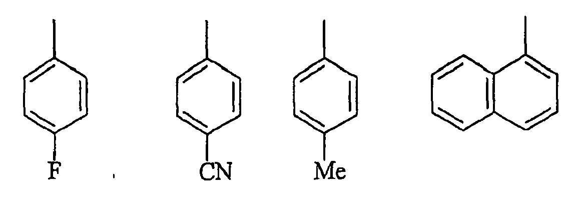 la molécule G étant de la forme R₂-R₃, R₂ et R₃ étant tels que définis ci-dessus, et répond notamment à la formule suivante : 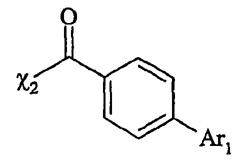
dans laquelle χ₂ représente soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone,
Ar₁ est tel que défini ci-dessus,
**b)** 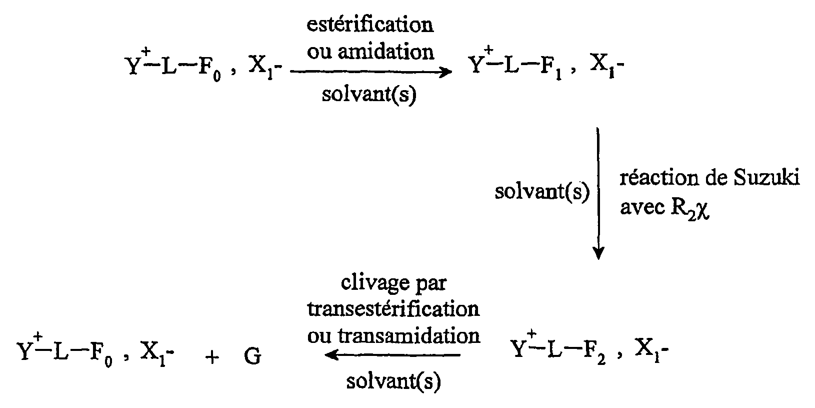 Y⁺- représentant un cation onium tel que défini dans l'une des revendications 3 à 17, et étant de préférence un cation triméthylalkylammonium, triéthylalkylammonium, tributylalkylphosphonium, N-méthylimidazolium ou pyridinium,
L représentant un bras, notamment un groupe alkyle linéaire ou ramifié comprenant de 1 à 20 atomes de carbone, ou un groupe aralkyle éventuellement fonctionnel comprenant de 6 à 30 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
R₂ étant choisi parmi les groupes aryle, hétéroaryle, éthényle, diényle, allyle, éthynyle, substitués ou non, comprenant de 2 à 30 atomes de carbone,
les fonctions F₀, F₁ et F₂ étant telles que définies ci-dessous :
- F₀ est de la forme -χ₁H, χ₁ étant tel que défini ci-dessus,
- F₁ est de la forme -R_{q}-B(OR₇)₂, R₇ étant tel que défini ci-dessus, et R_{q} correspondant à un groupe aryle comprenant de 6 à 30 atomes de carbone, hétéroaryle comprenant de 4 à 20 atomes de carbone, éthényle comprenant de 2 à 20 atomes de carbone, diényle comprenant de 3 à 20 atomes de carbone, allyle comprenant de 3 à 20 atomes de carbone, éthynyle comprenant de 2 à 20 atomes de carbone, substitués ou non, F₁ répondant de préférence à la formule suivante : 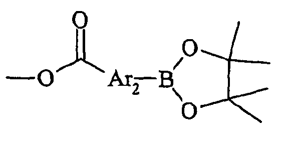 Ar₂ correspondant à un groupe aryle substitué ou non comprenant de 6 à 30 atomes de carbone,
- F₂ est de la forme -R_{q}-Rₑ, Rq et Rₑ étant tels que définis ci-dessus, F₂ répondant de préférence à la formule suivante : 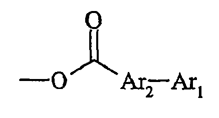 Ar₁ représentant un groupe aromatique choisi de préférence parmi : 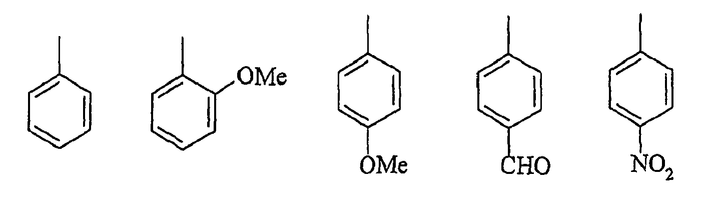 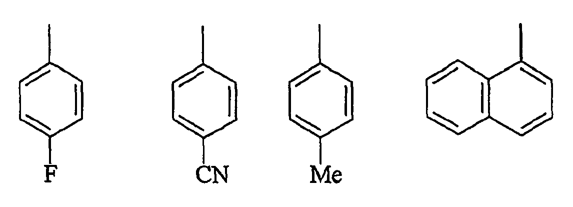 la molécule G étant de la forme R₂-R₃, R₂ et R₃ étant tels que définis ci-dessus, et répondant notamment à la formule suivante : 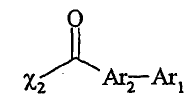
dans laquelle χ₂, Ar₁ et Ar₂ sont tels que définis ci-dessus,
**c)** 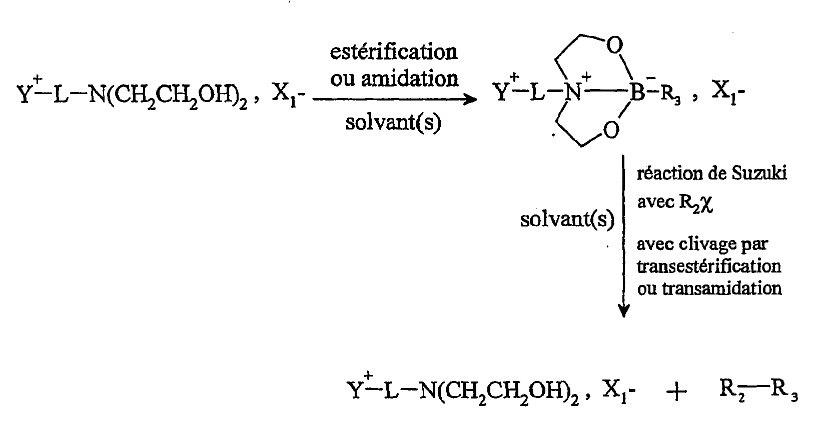 Y⁺-, L, X₁⁻, R₂ et R₃ étant tels que définis ci-dessus,
R₃ étant de préférence un groupe phényle,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
**d)** 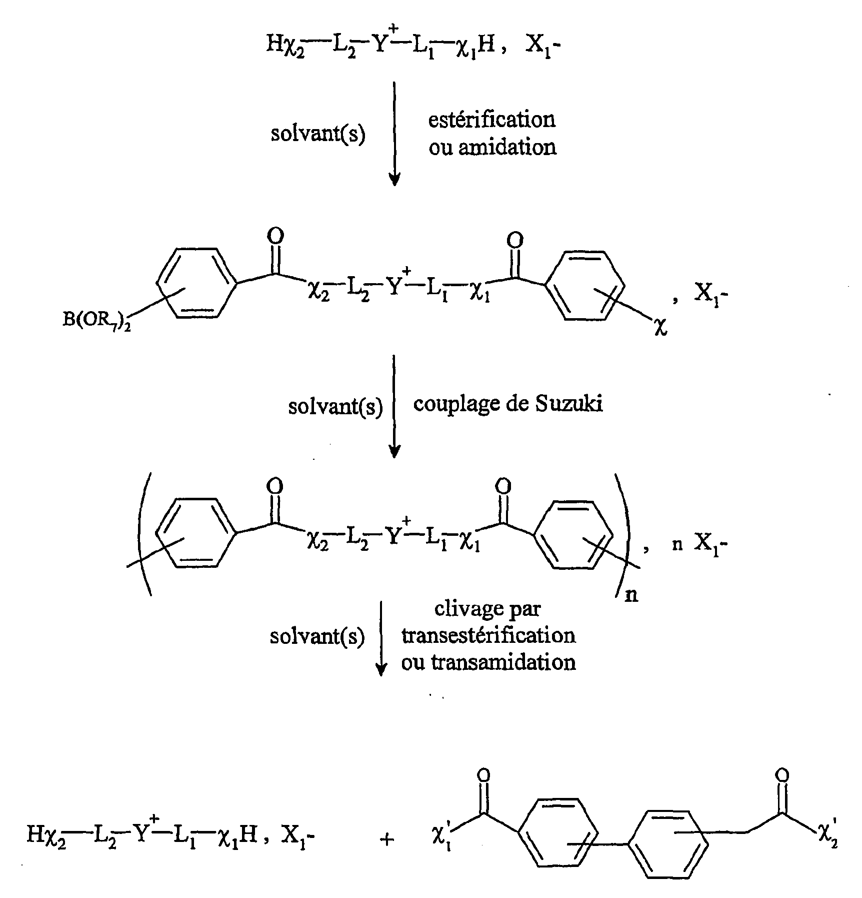 n représentant un nombre entier compris entre 1 et 50,
Y⁺- représentant un cation onium tel que défini dans l'une des revendications 3 à 17, et étant de préférence un cation triméthylalkylammonium, triéthylalkylammonium, tributylalkylphosphonium, N-méthylimidazolium ou pyridinium,
L₁ et L₂ représentant un bras, identiques ou différents, notamment un groupe alkyle linéaire ou ramifié comprenant de 1 à 20 atomes de carbone, ou un groupe aralkyl éventuellement fonctionnel comprenant de 6 à 30 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
χ₁ et χ₂, identiques ou différents, représentant un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
χ représentant un groupe partant choisi de préférence parmi Cl, Br, I, OTf, O-CO₂R⁵ ou OSO₃-R⁵, R⁵ représentant un groupe alkyle comprenant de 1 à 10 atomes de carbone ou un groupe aralkyle comprenant de 6 à 30 atomes de carbone,
R₇ représentant un atome d'hydrogène, un groupe alkyle, ramifié ou non, ou cycloalkyle, comprenant de 1 à 12 atomes de carbone, ou un groupe aryle, comprenant de 6 à 30 atomes de carbone,
χ'₁ et χ'₂, identiques ou différents, représentant soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone.

22. Utilisation selon la revendication 19 pour la mise en oeuvre du couplage de Sonogashira, selon l'un des schémas réactionnels suivants :
a) 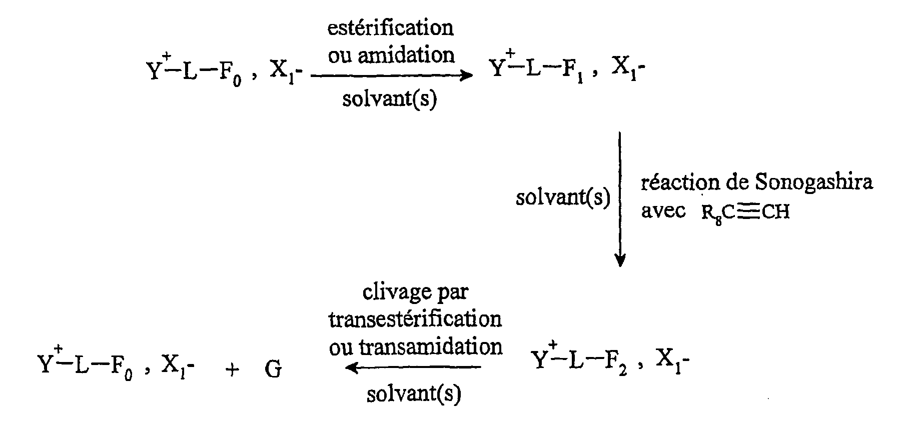 Y⁺- représentant un cation onium tel que défini dans l'une des revendications 3 à 17, et étant de préférence un cation triméthylalkylammonium, triéthylalkylammonium, tributylalkylphosphonium, N-méthylimidazolium ou pyridinium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
R₈ représentant un groupe ORₕ, NRₕRᵤ, CORₕ, CN, SO₂Rₕ, SRₕ, un groupe alcényle, éthynyle, diényle, Rₕ et Rᵤ représentant, indépendamment l'un de l'autre, un atome d'hydrogène, un groupe alkyle comprenant de 1 à 20 atomes de carbone ou un groupe aryle comprenant de 6 à 30 atomes de carbone,
ou R₈ représentant un groupe alkyle, ramifié ou linéaire, éventuellement fonctionnel, comprenant de 1 à 20 atomes de carbone, ou un groupe aryle, ou un groupe alkaryle ou aralkyle, comprenant de 6 à 30 atomes de carbone, substitué ou non, lesdits groupes alkyle ou aryle pouvant être substitués par l'un des groupes fonctionnels suivants : un atome d'halogène, notamment Cl, un groupe ORₕ, NRₕRᵤ, CORₕ, CN, SO₂Rₕ, SRₕ, un groupe alcényle, éthynyle, diényle, vinyle, alcynyle, Rₕ et Rᵤ étant tels que définis précédemment,
R₈ étant notamment l'un des groupes suivants :
-(CH₂)ₛ-CH₃, -(CH₂)ₛ-CH₂OH, -(CH₂)ₛ-CH₂OMe,
s représentant un nombre entier compris entre 0 et 10, 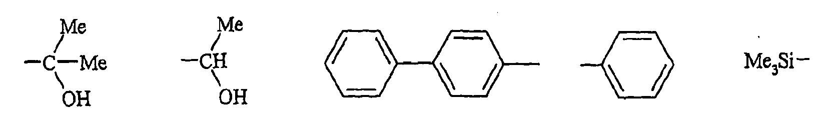 les fonctions F₀, F₁ et F₂ étant telles que définies ci-dessous :
- F₀ correspond à un groupe -χ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante :
| | |
|---|---|
| 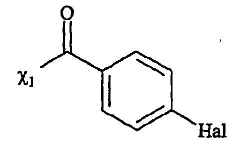 | χ₁ étant tel que défini ci-dessus, et Hal représentant un halogène, et étant de préférence l'iode, |
- F₂ répond à la formule suivante :
| | |
|---|---|
| 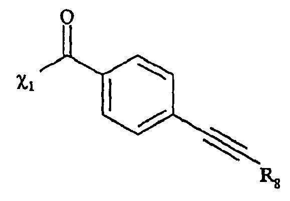 | χ₁ et R₈ étant tels que définis ci-dessus, |
G répondant à la formule suivante : 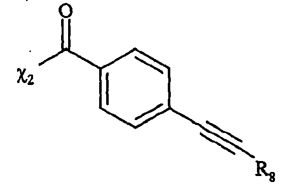 dans laquelle χ₂ représente soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone, χ₂ représentant notamment un groupe OMe, OEt, OPr ou OBu.
b) 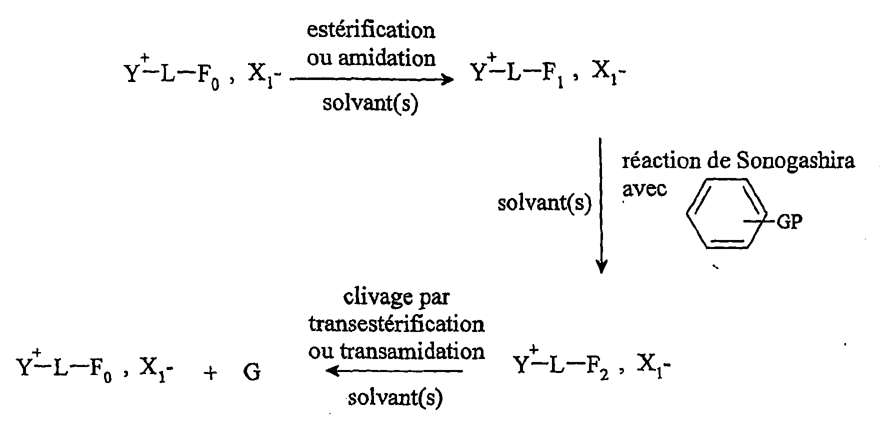 Y⁺- représentant un cation onium tel que défini dans l'une des revendications 3 à 17, et étant de préférence un cation triméthylalkylammonium, triéthylalkylammonium, tributylalkylphosphonium, N-méthylimidazolium, alkylpyridinium, diméthylalkylsulfonium ou diéthylalkyl-sulfonium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
GP représentant un groupe partant, et étant notamment Cl, Br, I ou OTf,
les fonctions F₀, F₁ et F₂ étant telles que définies ci-dessous :
- F₀ correspond à un groupe -COOH,
- F₁ répond à la formule suivante : 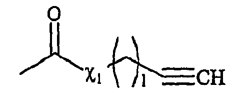 dans laquelle 1 représente un nombre entier variant de 1 à 20, et χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₂ répond à la formule suivante :
| | |
|---|---|
| 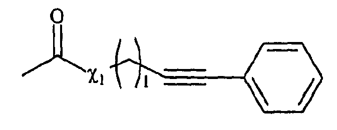 | χ₁ et 1 étant tels que définis ci-dessus, |
G répondant à la formule suivante : 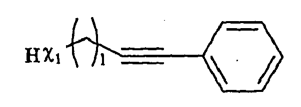 dans laquelle χ₁ et 1 sont tels que définis ci-dessus.

23. Utilisation selon l'une quelconque des revendications 1 à 17, pour la mise en oeuvre de la réaction de Baylis-Hilman, selon l'un des schémas réactionnels suivants :
**a)** 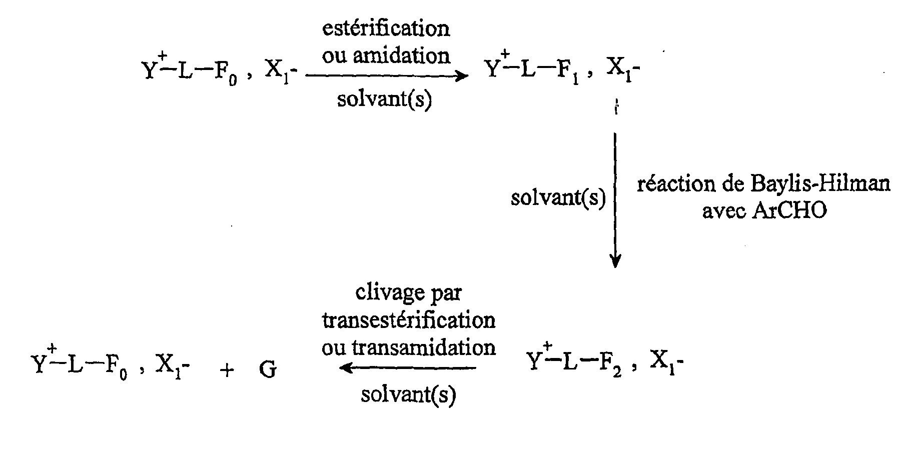 Y⁺- représentant un cation onium tel que défini dans l'une des revendications 3 à 17, et étant de préférence un cation triméthylalkylammonium, triéthylalkylammonium, tributylalkylphosphonium, N-méthylimidazolium ou pyridinium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁ et F₂ étant telles que définies ci-dessous :
- F₀ représente un groupe -OH,
- F₁ répond à la formule suivante : 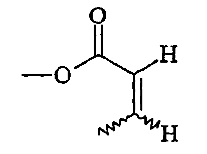
- F₂ répond à la formule suivante : 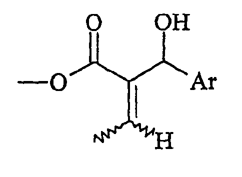 G répondant à la formule suivante :
| | |
|---|---|
| 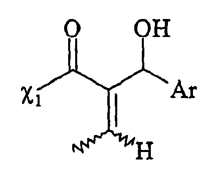 | χ₁ représentant un groupe -OH, ou un groupe -OR_{g}, R_{g} représentant un groupe alkyle, linéaire ou ramifié, comprenant de 1 à 20 atomes de carbone, |
Ar représentant un groupe aromatique ou hétéroaromatique, substitué ou non,
ArCHO étant notamment choisi parmi : 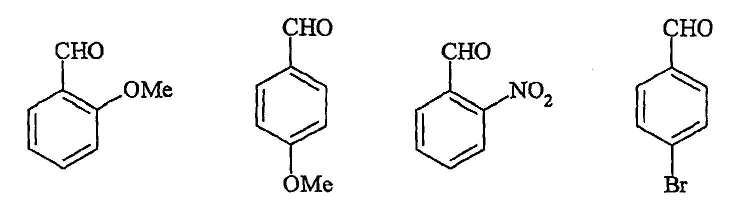 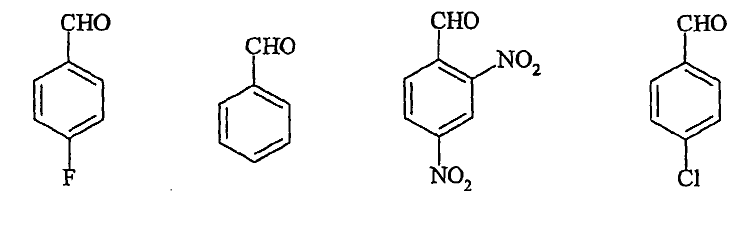
**b)** 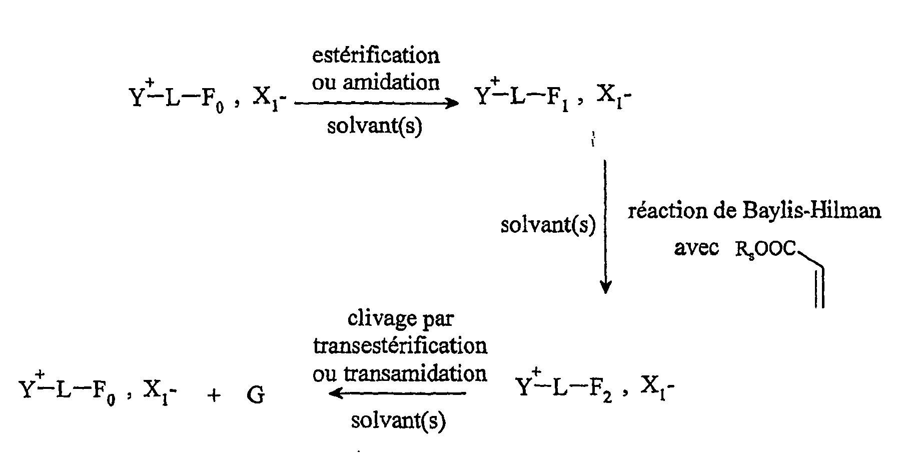 Y⁺- représentant un cation onium tel que défini dans l'une des revendications 3 à 17, et étant de préférence un cation triméthylalkylammonium, triéthylalkylammonium, tributylalkylphosphonium, N-méthylimidazolium, alkylpyridinium, diméthylalkylsulfonium ou diéthylalkyl-sulfonium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, -N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
Rₛ représentant un atome d'hydrogène ou un groupe alkyle comprenant de 1 à 20 atomes de carbone ou aralkyle ou alkaryle comprenant de 7 à 30 atomes de carbone,
les fonctions F₀, F₁ et F₂ étant telles que définies ci-dessous :
- F₀ correspond à un groupe -χ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante :
| | |
|---|---|
| 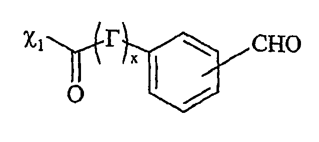 | χ₁ étant tel que défini ci-dessus, |
| | x étant égal à 0 ou 1, |
| | I⁻ représentant une chaîne alkyle comprenant de 1 à 20 atomes de carbone, alkaryle, aralkyle comprenant de 6 à 30 atomes de carbone, |
- F₂ répond à la formule suivante :
| | |
|---|---|
| 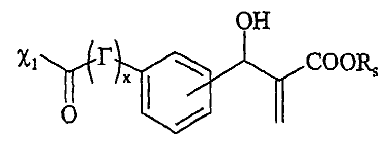 | χ₁, x, Rₛ et Γ étant tels que définis ci-dessus |
- G répondant à la formule suivante :
| | |
|---|---|
| 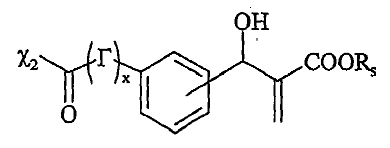 | χ₂, x, Rₛ et et Γ étant tels que définis ci-dessus |
dans laquelle χ₂ représente soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone, χ₂ représentant notamment un groupe OMe, OEt, OPr ou OBu.
**c)** 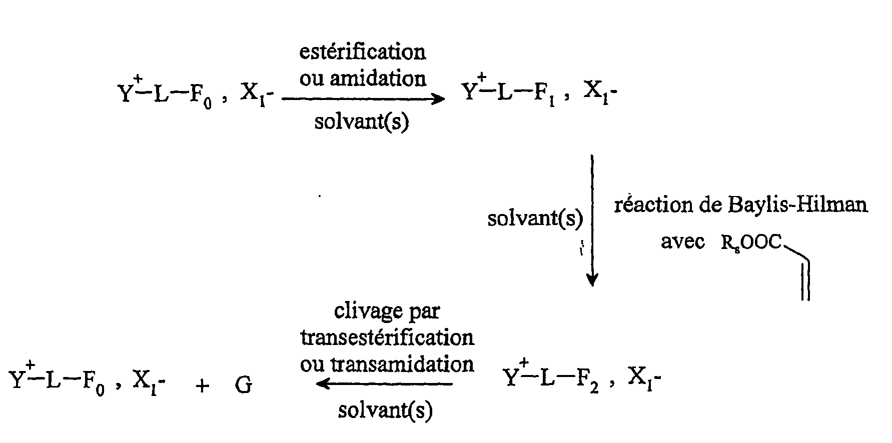 Y⁺- représentant un cation onium tel que défini dans l'une des revendications 3 à 17, et étant de préférence un cation triméthylalkylammonium, triéthylalkylammonium, tributylalkylphosphonium, N-méthylimidazolium ou pyridinium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
Rₛ représentant un atome d'hydrogène ou un groupe alkyle comprenant de 1 à 20 atomes de carbone ou aralkyle ou alkaryle comprenant de 7 à 30 atomes de carbone,
les fonctions F₀, F₁ et F₂ étant telles que définies ci-dessous :
- F₀ correspond à un groupe -COχ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante : 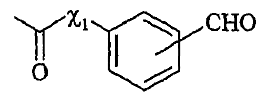
- F₂ répond à la formule suivante : 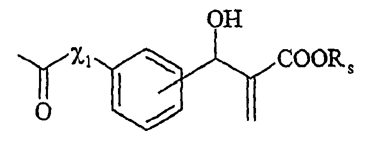
- G répondant à la formule suivante :

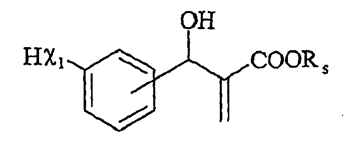

24. Utilisation selon l'une quelconque des revendications 1 à 17, pour la synthèse, éventuellement asymétrique, d'α-aminoacides, selon le schéma réactionnel suivant : 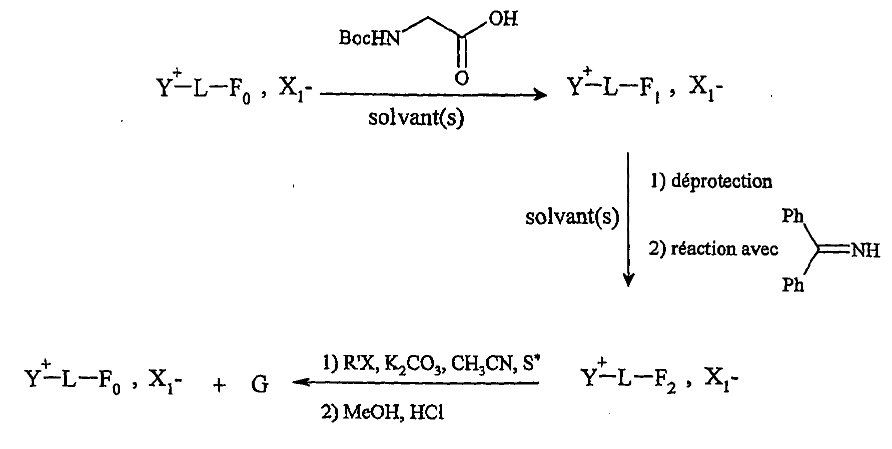 Y⁺- représentant un cation onium tel que défini dans l'une des revendications 3 à 17, et étant de préférence un cation triméthylalkylammonium, triéthylalkylammonium
ou tributylalkylphosphonium,
L représentant un bras, notamment un groupe alkyle linéaire ou ramifié comprenant de 1 à 20 atomes de carbone, ou un groupe aralkyle éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 3 à 6,
X₁⁻ étant tel que défini dans l'une des revendications 1 à 17, et étant notamment Cl⁻, Br⁻, I⁻, -N(SO₂CF₃)₂, BF₄⁻, PF₆⁻,
le ou les solvants étant choisis parmi : l'acétonitrile, le dichlorométhane, le tétrahydrofuranne, le dioxane, le toluène, le chlorobenzène ou un mélange de ces solvants,
R' représentant un groupe alkyle, linéaire ou ramifié, comprenant de 1 à 30 atomes de carbone, éventuellement fonctionnel,
S* représentant un agent chiral de transfert de phase tel que le bromure de O(9)-allyl-N-9-anthracényl-méthylcinchonidinium,
les fonctions F₀, F₁ et F₂ étant telles que définies ci-dessous :
- F₀ correspond à -OH,
- F₁ répond à la formule suivante : 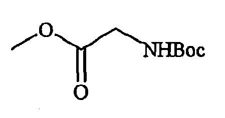
- F₂ répond à la formule suivante : 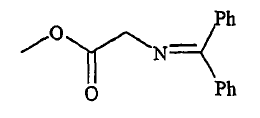
G répondant à la formule suivante :

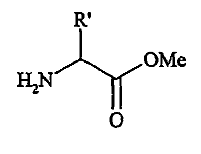

25. Utilisation selon l'une quelconque des revendications 1 à 17, pour la mise en oeuvre de réactions multi-composants, notamment pour la réaction de type Grieco selon l'un des schémas réactionnels suivants :
**a)** 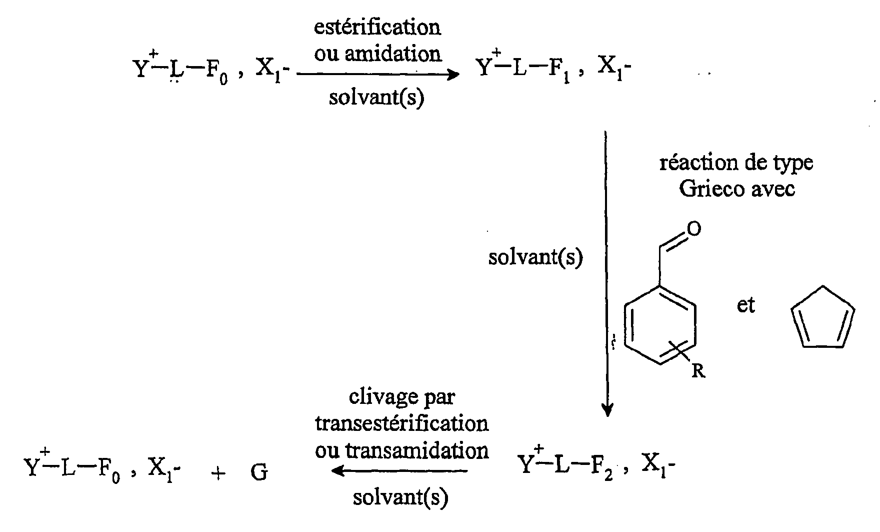 Y⁺- représentant un cation onium tel que défini dans l'une des revendications 3 à 17, et étant de préférence un cation triméthylalkylammonium, triéthylalkylammonium, tributylalkylphosphonium, N-méthyl-N'-alkylimidazolium, N-alkylpyridinium, diméthylalkylsulfonium ou diéthylalkylsulfonium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
R représentant un atome d'hydrogène, un groupe nitro en position para, un atome de chlore en para ou un groupe méthoxy en position ortho,
les fonctions F₀, F₁ et F₂ étant telles que définies ci-dessous :
- F₀ représente un groupe -OH,
- F₁ répond à la formule suivante : 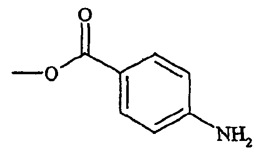
- F₂ répond à la formule suivante : 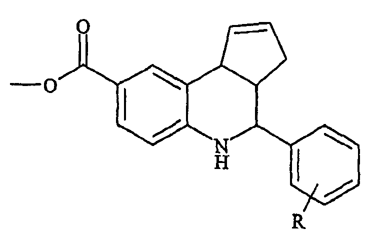
G répondant à la formule suivante :
| | |
|---|---|
| 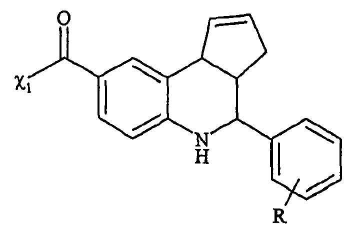 | χ₁ représentant un groupe -OH, ou un groupe -OR_{g}, R_{g} représentant un groupe alkyle, linéaire ou ramifié, comprenant de 1 à 20 atomes de carbone. |
**b)** 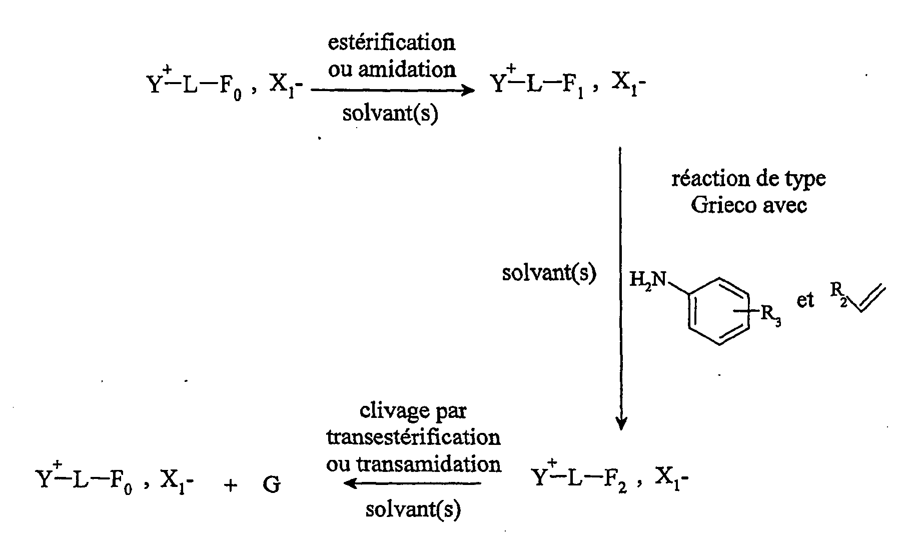 Y⁺- représentant un cation onium tel que défini dans l'une des revendications 3 à 17, et étant de préférence un cation triméthylalkylammonium, triéthylalkylammonium, tributylalkylphosphonium, N-méthyl-N'-alkylimidazolium, N-alkylpyridinium, diméthylalkylsulfonium ou diéthylallylsulfonium,
L représentant un bras, notamment un groupe alkyle linéaire ou ramifié comprenant de 1 à 20 atomes de carbone, ou un groupe aralkyl ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
R₂ représentant un groupe alkyle, fonctionnel ou non, comprenant de 1 à 20 atomes de carbone, ou un groupe aryle, fonctionnel ou non, comprenant de 6 à 30 atomes de carbone, ou un groupe aralkyle ou alkaryle, fonctionnel ou non, comprenant de 7 à 50 atomes de carbone,
R₃ représentant un atome d'hydrogène, un groupe alkyle, linéaire ou ramifié, comprenant de 1 à 20 atomes de carbone ou un groupe aryle comprenant de 6 à 30 atomes de carbone, ou un groupe aralkyle ou alkaryle, fonctionnel ou non, comprenant de 7 à 50 atomes de carbone, ou un groupe fonctionnel notamment choisi parmi NO₂, CN, COOR, OR, COR, NHCOR, NRR', SO₂R, I, Br, R et R' représentant indépendamment l'un de l'autre un groupe alkyle comprenant de 1 à 20 atomes de carbone ou un groupe aryle comprenant de 6 à 30 atomes de carbone,
les fonctions F₀, F₁ et F₂ étant telles que définies ci-dessous :
- F₀ représente un groupe -OH,
- F₁ répond à la formule suivante : 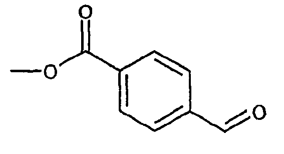
- F₂ répond à la formule suivante : 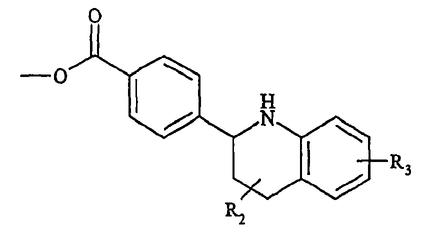
G répondant à la formule suivante :
| | |
|---|---|
| 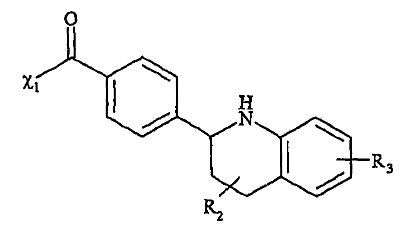 | χ₁ représentant un groupe -OH, ou un groupe -OR_{g}, R_{g} représentant un groupe alkyle, linéaire ou ramifié, comprenant de 1 à 20 atomes de carbone. |
**c)** 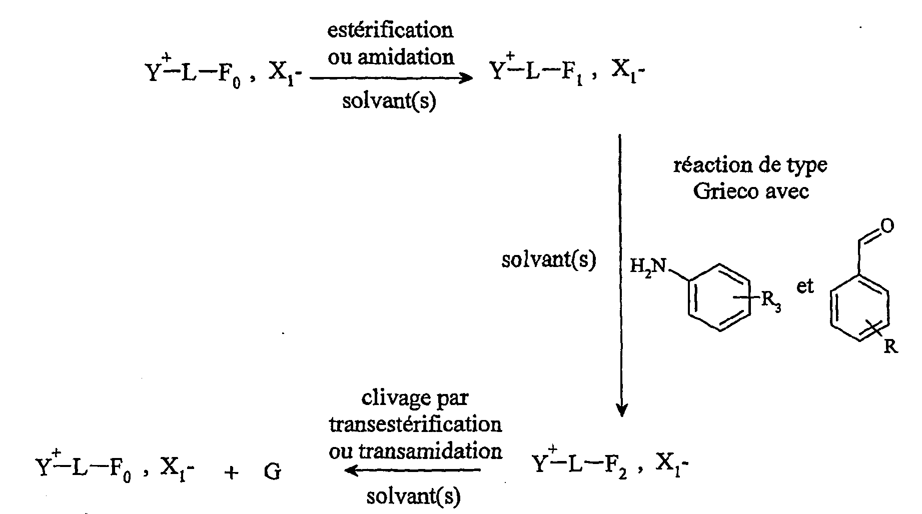 Y⁺- représentant un cation onium tel que défini dans l'une des revendications 3 à 17, et étant de préférence un cation triméthylalkylammonium, triéthylalkylammonium, tributylalkylphosphonium, N-méthyl-N'-alkylimidazolium, N-alkylpyridinium, diméthylalkylsulfonium ou diéthylalkylsulfonium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, -N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
R représentant un atome d'hydrogène ou un groupe fonctionnel tel qu'un groupe nitro en position para, un atome de chlore en para ou un groupe méthoxy en position ortho, ou un groupe alkyle, fonctionnel ou non, comprenant de 1 à 20 atomes de carbone, ou un groupe aryle, fonctionnel ou non, comprenant de 6 à 30 atomes de carbone, ou un groupe aralkyle ou alkaryle, fonctionnel ou non, comprenant de 7 à 50 atomes de carbone,
R₃ représentant un atome d'hydrogène, un groupe alkyle, linéaire ou ramifié, comprenant de 1 à 20 atomes de carbone ou un groupe aryle comprenant de 6 à 30 atomes de carbone, ou un groupe aralkyle ou alkaryle, fonctionnel ou non, comprenant de 7 à 50 atomes de carbone, ou un groupe fonctionnel notamment choisi parmi NO₂, CN, COOR, OR, COR, NHCOR, NRR', SO₂R, I, Br, R et R' représentant indépendamment l'un de l'autre un groupe alkyle comprenant de 1 à 20 atomes de carbone ou un groupe aryle comprenant de 6 à 30 atomes de carbone,
les fonctions F₀, F₁ et F₂ étant telles que définies ci-dessous :
- F₀ représente toute fonction permettant d'accrocher et de décrocher un reste portant une oléfine, de préférence un ester, ou un amide.
- F₁ répond à l'une des formules générales suivantes :
| | |
|---|---|
| 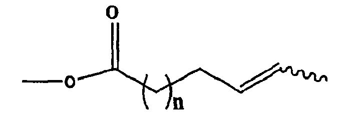 | n représentant un nombre entier variant de 1 à 10 |
| 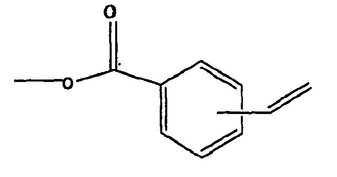 | |
- F₂ répond à l'une des formules générales suivantes : 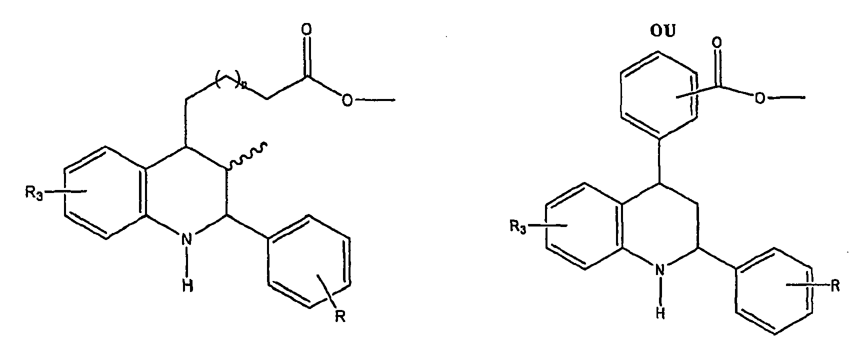
G répondant à l'une des formules générales suivantes : 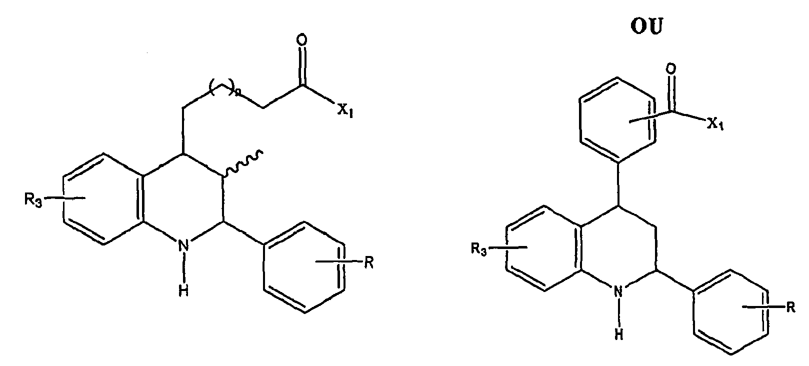 n, R et R₃ étant tels que définis ci-dessus, et
χ₁ représentant un groupe -OH, ou un groupe -OR_{g}, R_{g} représentant un groupe alkyle, linéaire ou ramifié, comprenant de 1 à 20 atomes de carbone.

26. Utilisation selon l'une quelconque des revendications 1 à 17, pour la mise en oeuvre de réactions multi-composants, notamment pour la synthèse d'oléfines tétrasubstituées selon le schéma réactionnel suivant : 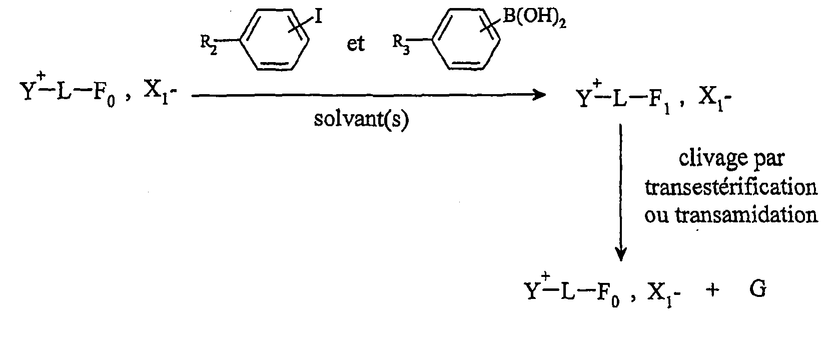 Y⁺- représentant un cation onium tel que défini dans l'une des revendications 3 à 17, et étant de préférence un cation triméthylalkylammonium, triéthylalkylammonium, tributylalkylphosphonium, N-méthyl-N'-alkylimidazolium, N-alkylpyridinium, diméthylalkylsulfonium ou diéthylalkylsulfonium,
L représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 1 à 10,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
R₂ et R₃, de préférence en para, représentant un atome d'hydrogène, un groupe alkyle, linéaire ou ramifié, éventuellement fonctionnel, comprenant de 1 à 30 atomes de carbone, un groupe aryle, éventuellement substitué et/ou fonctionnel, comprenant de 6 à 30 atomes de carbone, un groupe fonctionnel, de préférence un groupe méthoxy, mono-alkylamino, dialkylamino, arylamino, cyano, ester, nitro, cétone, sulfonyle, alkylthio, sulfoxyde,
les fonctions F₀ et F₁ étant telles que définies ci-dessous :
- F₀ répond à la formule suivante :
| | |
|---|---|
| 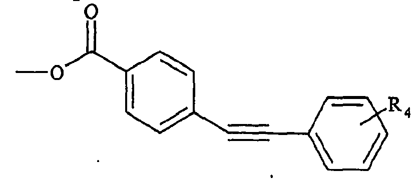 | R₄ représentant un groupe tel que défini pour R₂ et R₃ ci-dessus, |
- F₁ répond à l'une des formules suivantes : 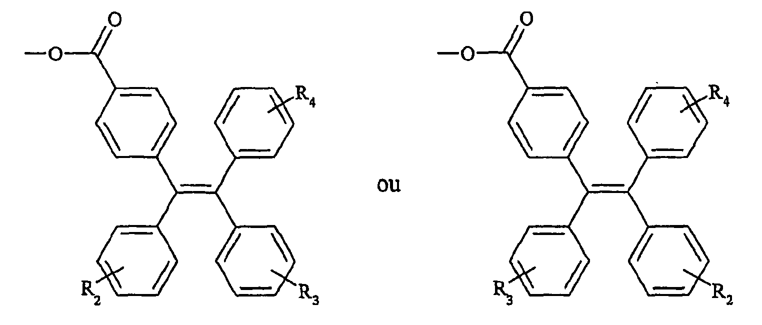
R₂, R₃ et R₄ étant tels que définis ci-dessus,
G répond à l'une des formules suivantes : 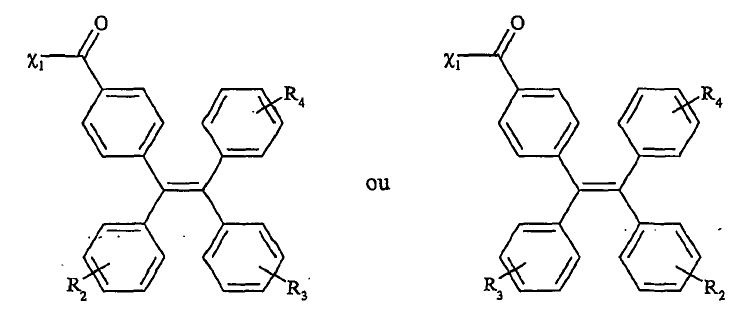 χ ₁ représentant un groupe -OH, ou un groupe -OR_{g}, R_{g} représentant un groupe alkyle, linéaire ou ramifié, comprenant de 1 à 20 atomes de carbone.

27. Utilisation selon l'une quelconque des revendications 1 à 17, pour la mise en oeuvre de réactions de cycloaddition, de préférence pour la mise en oeuvre de la réaction de Diels-Alder, selon le schéma réactionnel suivant : 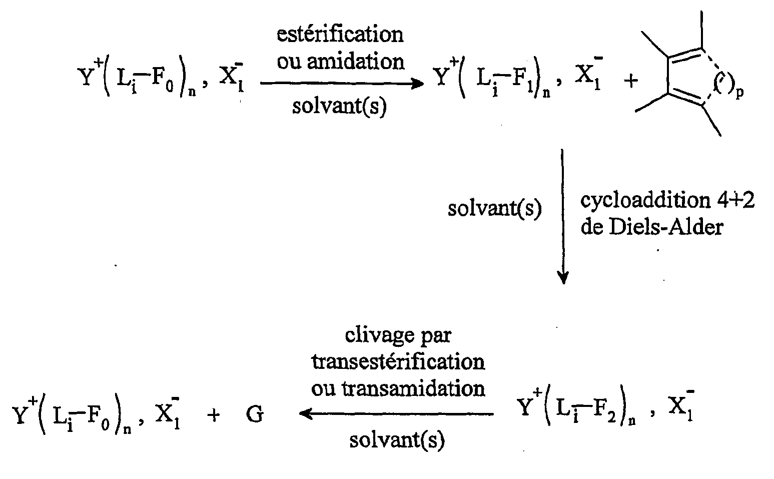 n étant un nombre entier variant de 2 à 4, tel que défini ci-dessous,
i étant un nombre entier variant de 1 à n,
p étant un nombre entier variant de 0 à 2,
Y⁺ représentant un cation onium tel que défini dans l'une des revendications 3 à 17, de formule (R_{b})ₓ₋ₙΛ⁺ dans laquelle x représente un nombre entier égal à 3 ou 4, n étant égal à 2, 3 ou 4 lorsque x est égal à 4 et n étant égal à 2 ou 3 lorsque x est égal à 3, R_{b} représente un groupe alkyle comprenant de 1 à 20 atomes de carbone, un groupe aryle comprenant de 6 à 30 atomes de carbone ou un groupe aralkyle ou alkaryle comprenant de 6 à 30 atomes de carbone, lesdits groupes alkyle, aryle, aralkyle ou alkaryle susmentionnés étant non fonctionnels, et dans laquelle Λ⁺ représente un cation ammonium, imidazolium, phosphonium ou sulfonium, Y⁺ représentant notamment un cation alkylammonium, alkylphosphonium ou alkylsulfonium, et étant de préférence un cation tétraalkylammonium, tétraalkylphosphonium, dialkylimidazolium, trialkylsulfonium,
Lᵢ représentant un bras, notamment un groupe alkyle linéaire ou ramifié comprenant de 1 à 20 atomes de carbone, ou un groupe aralkyle ou alkaryle éventuellement fonctionnel, comprenant de 6 à 30 atomes de carbone, et étant de préférence un groupe alkyle linéaire de préférence un groupe alkyle linéaire de type (CH₂)ᵣ, r variant de 1 à 20, et de préférence de 2 à 10, les bras Lᵢ pouvant être identiques ou différents,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁ et F₂ étant telles que définies ci-dessous :
- F₀ correspond à un groupe -χ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante :
| | |
|---|---|
| 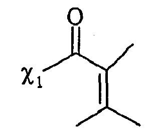 | χ₁ étant tel que défini ci-dessus, |
- F₂ répond à la formule suivante :
| | |
|---|---|
| 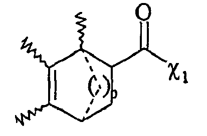 | χ₁ étant tel que défini ci-dessus, |
G répondant à la formule suivante : 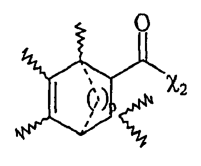 dans laquelle χ₂ représente soit un groupe OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone.

28. Utilisation selon la revendication 19, pour la mise en oeuvre de la réaction de Heck, selon le schéma réactionnel suivant : 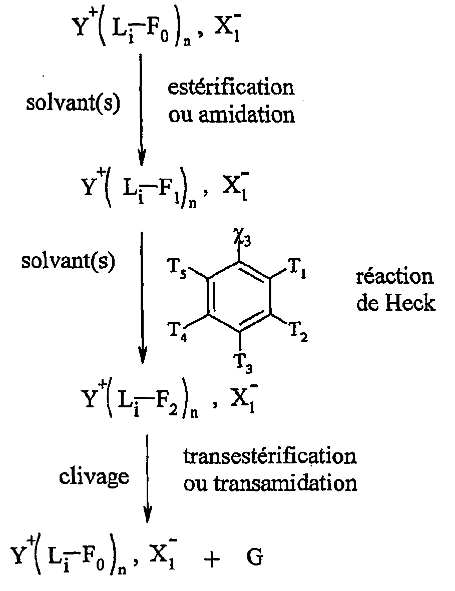 n étant un nombre entier variant de 2 à 4,
i étant un nombre entier variant de 1 à n,
Y⁺ représentant un cation onium tel que défini dans l'une des revendications 3 à 17, de formule (R_{b})ₓ₋ₙΛ⁺ dans laquelle x représente un nombre entier égal à 3 ou 4, n étant égal à 2, 3 ou 4 lorsque x est égal à 4 et n étant égal à 2 ou 3 lorsque x est égal à 3, R_{b} représente un groupe alkyle comprenant de 1 à 20 atomes de carbone, un groupe aryle comprenant de 6 à 30 atomes de carbone ou un groupe aralkyle ou alkaryle comprenant de 6 à 30 atomes de carbone, lesdits groupes alkyle, aryle, aralkyle ou alkaryle susmentionnés étant non fonctionnels, et dans laquelle Λ⁺ représente un cation ammonium, imidazolium, phosphonium ou sulfonium, Y⁺ représentant notamment un cation alkylammonium, alkylphosphonium ou alkylsulfonium, et étant de préférence un cation tétraalkylammonium, tétraalkylphosphonium, dialkylimidazolium, trialkylsulfonium,
Lᵢ représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 2 à 10, les bras Lᵢ pouvant être identiques ou différents,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁ et F₂ étant telles que définies ci-dessous :
- F₀ correspond à un groupe -χ₁H, dans lequel χ₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ répond à la formule suivante :
| | |
|---|---|
| 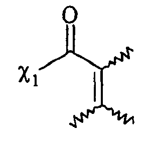 | χ₁ étant tel que défini ci-dessus, |
- F₂ répond à la formule suivante :
| | |
|---|---|
| 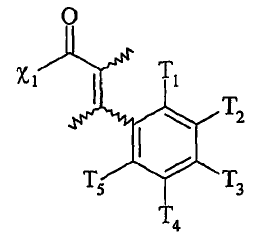 | χ₁ étant tel que défini ci-dessus, |
G répondant à la formule suivante : 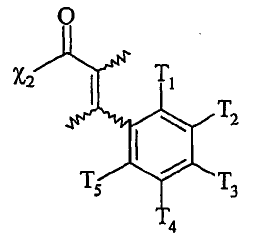 dans laquelle χ₂ représente soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone,
χ₃ représentant un groupement partant, notamment choisi parmi les halogénures I, Cl et Br, les groupes mésylate, tosylate, triflate, sulfonate, sulfate ou phosphate,
T₁, T₂, T₃, T₄ et T₅ représentant indépendamment les uns des autres un atome d'hydrogène, un groupe alkyle, linéaire ou ramifié, comprenant de 1 à 20 atomes de carbone ou un groupe aryle comprenant de 6 à 30 atomes de carbone, ou un groupe fonctionnel notamment choisi parmi NO₂, CN, COOR, OR, COR, NHCOR, NRR", SO₂R, I, Br, R et R" représentant indépendamment l'un de l'autre un groupe alkyle comprenant de 1 à 20 atomes de carbone ou un groupe aryle comprenant de 6 à 30 atomes de carbone,
l'entité 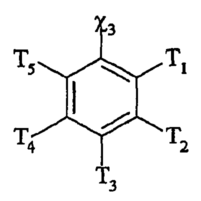 représentant notamment les groupes suivants :

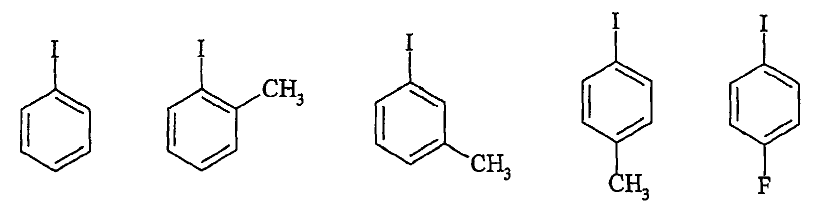

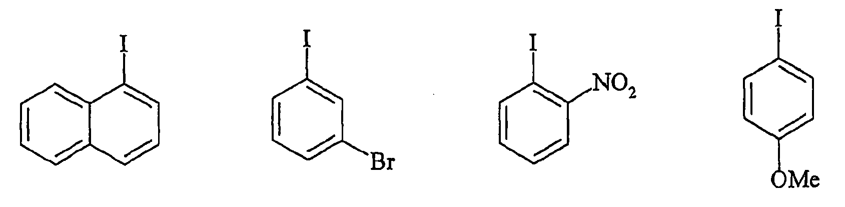

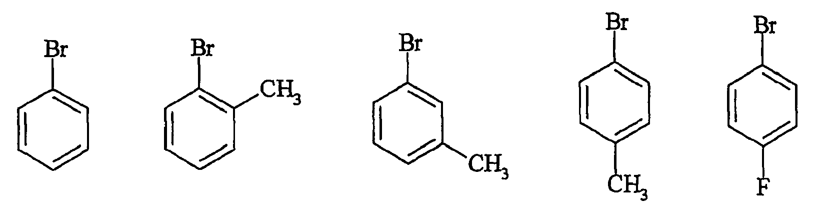

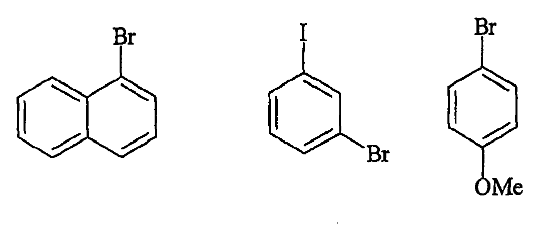

29. Utilisation selon la revendication 19, pour la mise en oeuvre du couplage de Suzuki, selon le schéma réactionnel suivant : 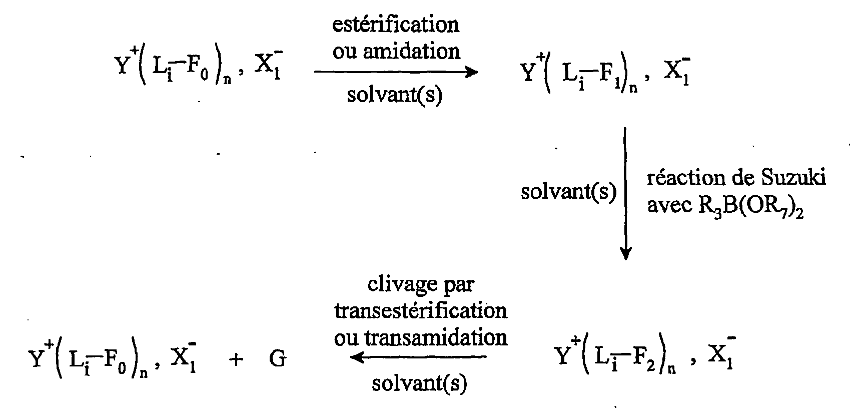 R₃ étant choisi parmi les groupes aryle, hétéroaryle, éthényle, diényle, allyle, éthynyle, substitués ou non, comprenant de 2 à 30 atomes de carbone,
R₇ représentant un atome d'hydrogène ou un groupe alkyle, ramifié ou linéaire ou un groupe cycloalkyle comprenant de 1 à 12 atomes de carbone,
n étant un nombre entier variant de 2 à 4,
i étant un nombre entier variant de 1 à n,
Y⁺ représentant un cation onium tel que défini dans l'une des revendications 3 à 17, de formule (R_{b})ₓ₋ₙΛ⁺ dans laquelle x représente un nombre entier égal à 3 ou 4, n étant égal à 2, 3 ou 4 lorsque x est égal à 4 et n étant égal à 2 ou 3 lorsque x est égal à 3, R_{b} représente un groupe alkyle comprenant de 1 à 20 atomes de carbone, un groupe aryle comprenant de 6 à 30 atomes de carbone ou un groupe aralkyle ou alkaryle comprenant de 6 à 30 atomes de carbone, lesdits groupes alkyle, aryle, aralkyle ou alkaryle susmentionnés étant non fonctionnels, et dans laquelle Λ⁺ représente un cation ammonium, imidazolium, phosphonium ou sulfonium, Y⁺ représentant notamment un cation alkylammonium, alkylphosphonium ou alkylsulfonium, et étant de préférence un cation tétraalkylammonium, tétraalkylphosphonium, dialkylimidazolium, trialkylsulfonium,
Lᵢ représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 2 à 10, les bras Lᵢ pouvant être identiques ou différents,
X₁⁻ étant tel que défini dans l'une des revendications 1 à 17, et étant notamment Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀, F₁ et F₂ étant telles que définies ci-dessous :
- F₀ est de la forme -χ₁H, χ₁ représentant un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁ est de la forme -Rₑ-χ, Rₑ représentant un groupe aromatique ou hétéroaromatique comprenant de 6 à 30 atomes de carbone, χ représentant un groupe partant choisi de préférence parmi Cl, Br, I, OTf, O-CO₂R⁵ ou OSO₃-R⁵, R⁵ représentant un groupe alkyle comprenant de 1 à 10 atomes de carbone ou un groupe aralkyle comprenant de 6 à 30 atomes de carbone, F₁ répondant de préférence à la formule suivante : 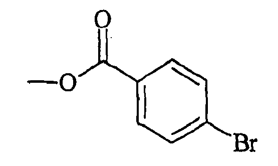
- F₂ est de la forme -Rₑ-R₂, Rₑ étant tel que défini ci-dessus et R₂ étant choisi parmi les groupes aryle, hétéroaryle, éthényle, diényle, allyle, éthynyle, substitués ou non, comprenant de 2 à 30 atomes de carbone, F₂ répondant de préférence à la formule suivante : 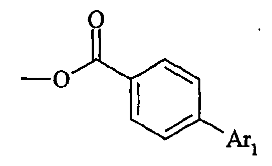 Ar₁ représentant un groupe aromatique choisi de préférence parmi : 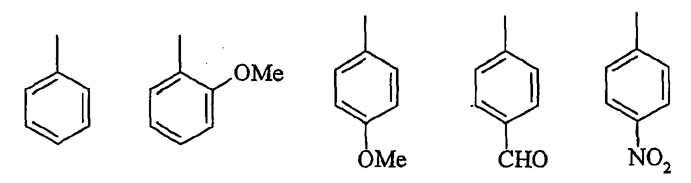 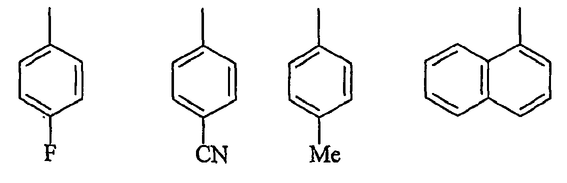 la molécule G étant de la forme R₂-R₃, R₂ et R₃ étant tels que définis ci-dessus, et répond notamment à la formule suivante : 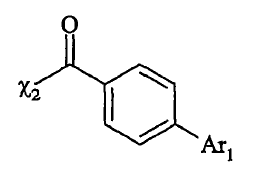
dans laquelle χ₂ représente soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone,
Ar₁ est tel que défini ci-dessus.

30. Utilisation selon la revendication 19, pour la mise en oeuvre de la réaction de Heck, selon le schéma réactionnel suivant : 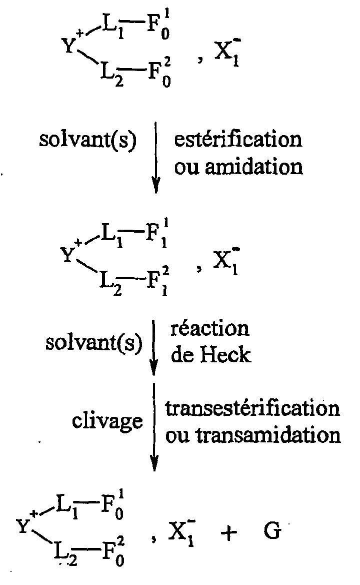 Y⁺- représentant un cation onium tel que défini dans l'une des revendications 3 à 17, de formule (R_{b})ₓ₋₂Λ⁺ dans laquelle x représente un nombre entier égal à 3 ou 4, R_{b} représente un groupe alkyle comprenant de 1 à 20 atomes de carbone, un groupe aryle comprenant de 6 à 30 atomes de carbone ou un groupe aralkyle ou alkaryle comprenant de 6 à 30 atomes de carbone, lesdits groupes alkyle, aryle, aralkyle ou alkaryle susmentionnés étant non fonctionnels, et dans laquelle Λ⁺ représente un cation ammonium, imidazolium, phosphonium ou sulfonium, Y⁺ représentant notamment un cation alkylammonium, alkylphosphonium ou alkylsulfonium, et étant de préférence un cation tétraalkylammonium, tétraalkylphosphonium, dialkylimidazolium, trialkylsulfonium, Λ⁺ représentant un cation ammonium ou phosphonium lorsque x = 4 et un cation sulfonium lorsque x = 3,
L₁ et L₂, identiques ou différents, représentant un bras, notamment un groupe alkyle linéaire ou ramifié comprenant de 1 à 20 atomes de carbone, ou un groupe aralkyle ou alkaryle, éventuellement fonctionnel comprenant de 1 à 20 atomes de carbone, et étant de préférence un groupe alkyle linéaire de préférence un groupe alkyle linéaire de type (CH₂)ᵣ, r variant de 1 à 20, et de préférence de 2 à 10,
X₁⁻ étant tel que défini dans l'une des revendications 1 à 17, et étant notamment Cₗ⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ représentant un groupe alkyle comprenant de 1 à 20 atomes de carbone,
le ou les solvants étant choisis parmi : le dichlorométhane, le tétrahydrofuranne, le dioxane, l'acétonitrile, le diméthylformamide, le diméthylacétamide, la N-méthylpyrrolidinone, le propionitrile, l'acétone, le toluène, le chlorobenzène, le nitrobenzène, le dichlorobenzène, le nitrométhane, le nitroéthane, ou un mélange de ces solvants,
les fonctions F₀¹, F₁¹, F₀² et F₁² étant telles que définies ci-dessous :
- F₀¹ correspond à un groupe -χ¹₁H, dans lequel χ¹₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₀² correspond à un groupe -χ²₁H, dans lequel χ²₁ représente un atome d'oxygène ou un groupe -NR_{f}, R_{f} correspondant à un groupe alkyle, linéaire ou ramifié, comprenant de 1 à 20 atomes de carbone, ou un groupe aryle comprenant de 6 à 30 atomes de carbone,
- F₁¹ répond à la formule suivante :
| | |
|---|---|
| 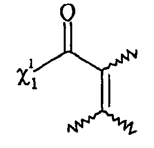 | χ¹₁ étant tel que défini ci-dessus, |
- F₁² répond à la formule suivante :
| | |
|---|---|
| 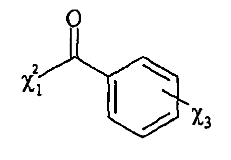 | χ²₁ étant tel que défini ci-dessus, et |
| | χ₃ représentant un groupement partant, notamment choisi parmi les halogénures I, Cl et Br, les groupes mésylate, tosylate, triflate, sulfonate, sulfate ou phosphate, |
G répondant à la formule suivante :
| | |
|---|---|
| 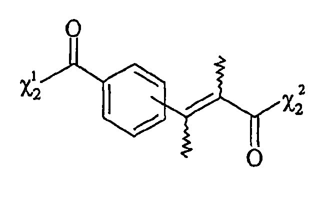 | dans laquelle χ₂¹ et χ₂², identiques ou différents, représentent soit un groupe -OR_{g}, R_{g} représentant un atome d'hydrogène ou un groupe alkyle comprenant de 1 à 20 atomes de carbone, soit un groupe -NRₕRᵤ, Rₕ et Rᵤ représentant indépendamment l'un de l'autre un atome d'hydrogène, un groupe alkyle comprenant de 1 à 20 atomes de carbone ou un groupe aryle comprenant de 6 à 30 atomes de carbone. |

## Claims

1. The use of an onium salt functionalized by at least one organic function, as a soluble support, in the presence of at least one organic solvent, for the organic synthesis of a molecule, in homogeneous phase, said organic synthesis comprising at least one conversion of said organic function, followed by a cleavage reaction releasing the synthesized molecule in solution in said solvent and the onium salt in its initial form, i. e. before the conversion of said organic function,
said onium salt being presented in liquid or solid form at ambient temperature, and corresponding to the formula A₁⁺, X₁⁻,
in which:
- A₁⁺ represents a cation,
- X₁⁻ represents an anion,
A₁⁺ being a functional or polyfunctional cation, and/or
X₁⁻ being a functional or polyfunctional anion,
the onium salt being such that in its initial form, i.e. before the first conversion of said organic function, A₁⁺ and X₁⁻ are not bound together by a covalent bond,
and when the anion and the cation respectively carry an organic function, these cannon react with each other before the first conversion of said organic function.

2. The use according to claim 1, **characterized in that** the onium salt is purified and/or recycled in its initial form after the release of the synthesized molecule.

3. The use according to claim 1 or 2, **characterized in that** the functional cations and anions correspond to an ionic entity, cationic Y⁺- and anionic Z⁻- respectively, optionally bound by means of an arm, L and M respectively, in particular an alkyl or aralkyl or alkaryl group comprising 1 to 30 carbon atoms, to at least one function Fᵢ and F'ᵢ respectively, Fᵢ varying from F₀ to Fₙ, F'ᵢ varying from F'₀ to F'ₙ, n being an integer varying from 1 to 10,
the functional cation A₁⁺ being able to be represented in the form Y⁺-L-Fᵢ, and
the functional anion X₁⁻ in the form Z⁻-(M)ₖ-F'ᵢ, k being equal to 0 or 1.

4. The use according to any one of claims 1 to 3, **characterized in that** the organic functions Fᵢ and F'ᵢ are chosen from the standard functions of organic chemistry, such as the hydroxyl functions, carboxylic acid, amide, sulphone, primary amine, secondary amine, aldehyde, ketone, ethenyl, ethynyl, dienyl, ether, epoxide, phosphine (primary, secondary or tertiary), azide, imine, ketene, cumulene, heterocumulene, thiol, thioether, sulphoxide, phosphorated groups, heterocycles, sulphonic acid, silane, stannane or functional aryl.

5. The use according to any one of claims 1 to 4, **characterized in that** the molecular weight of the functionalized onium salt is less than 1500 g.mol⁻¹, in particular less than 750 g.mol⁻¹, and is preferably comprised from 130 to 500 g.mol⁻¹.

6. The use according to any one of claims 1 to 5, **characterized in that** A₁⁺ is a functional cation and **in that** X₁⁻ is a non-functional anion.

7. The use according to claim 6, in which the onium salt A₁⁺, X₁⁻ has as its initial form Y⁺-L-F₀, X₁⁻, for obtaining a molecule G, by conversion of said initial function F₀ according to the diagram 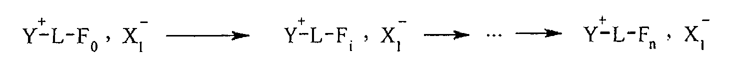 L being as defined in claim 3,
said molecule G being obtained by cleavage of the function Fₙ,
and the functionalized onium salt being able to be recovered or recycled in its initial form Y⁺-L-F₀, X₁⁻, after the release of G.

8. The use according to any one of claims 1 to 7, **characterized in that** the functional cation A₁⁺ is chosen from the pyridinium, imidazolium, ammonium, phosphonium or sulphonium cations, cyclic or non-cyclic, substituted or non-substituted, and preferably ammonium or phosphonium.

9. The use according to claim 8, **characterized in that** the functional cation A₁⁺ is chosen from the quaternary ammonium cations, cyclic or non-cyclic.

10. The use according to any one of claims 1 to 5, **characterized in that** X₁⁻ is a functional anion and A₁⁺ is a non-functional cation.

11. The use according to claim 10, in which the onium salt A₁⁺, X₁⁻ has as its initial form A₁⁺, Z⁻-(M)ₖ-F'₀, for obtaining a molecule G, by conversion of said initial function F'₀ according to the diagram 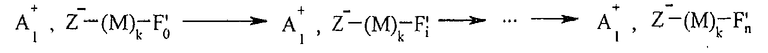 k and M being as defined in claim 3,
said molecule G being obtained by cleavage of the function F'ₙ,
and the functionalized onium salt being able to be recovered or recycled in its initial form A₁⁺, Z⁻-(M)ₖ-F'₀, after the release of G.

12. The use according to claim 10 or 11, **characterized in that** X₁⁻ is chosen from:
- the family of the phosphates: R₁PO₄²⁻, R₁R₂PO₄⁻,
- the family of the sulphates: R₁SO₄⁻,
- the family of the sulphonates: R₁SO₃⁻,
- the family of the carboxylates: R₁CO₂⁻,
or from the following anions:
R₁N̅SO₂R₂
R₂SO₂N̅SO₂R₂ 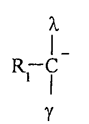 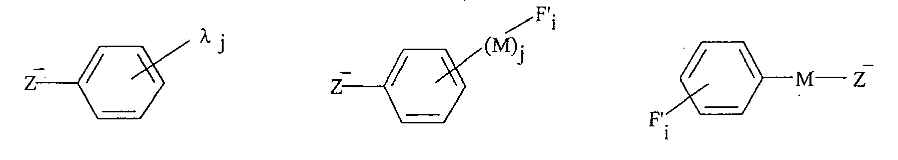
Z⁻, M and F'ᵢ being as defined in claim 3, Z⁻ representing in particular O⁻, SO₃⁻, CO₂⁻, R₁PO₃⁻ or R₁PO₂⁻,
j representing an integer comprised from 1 to 5,
R₁ and R₂ being able to represent independently of one another a functional alkyl group, a vinyl or alkynyl group, optionally functional, comprising from 1 to 20 carbon atoms, or being able to represent a functional aryl group comprising from 6 to 30 carbon atoms,
γ and λ representing an electroattractive group, in particular chosen from the groups: CO₂R', SO₂R', CN, NO₂, P(O)(OR')₂, C(O)R' and SO₃R',
R' representing an alkyl group, optionally functional, comprising from 1 to 20 carbon atoms, or an aryl group, optionally functional, comprising from 6 to 30 carbon atoms.

13. The use according to any one of claims 1 to 5, **characterized in that** A₁⁺ is a functional cation and X₁⁻ is a functional anion.

14. The use according to claim 13, in which the onium salt A₁⁺, X₁⁻ has as its initial form Y⁺-L-F₀, Z⁻-(M)ₖ-F'₀, for obtaining a molecule G, by conversion of said initial functions F₀ and F'₀ according to the diagram 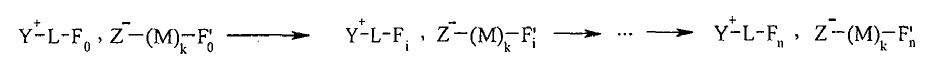 L, k and M being as defined in claim 3,
and by reaction of Fₙ on F'ₙ in the functionalized onium salt Y⁺-L-Fₙ, leading to the formation of an internal salt of formula: 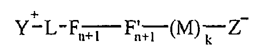 said molecule G being obtained by cleavage of the abovementioned internal salt and corresponding to the formula Fₙ₊₂-F'ₙ₊₂,
and the functionalized onium salt being able to be recovered or recycled in its initial form Y⁺-L-F₀, Z⁻-(M)ₖ-F'₀, after the release of G.

15. The use according to any one of claims 1 to 14, **characterized in that** the onium salt is chosen from the following salts: 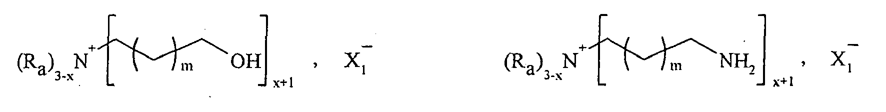 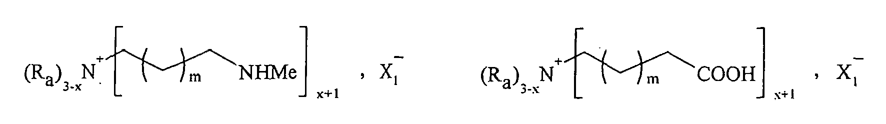 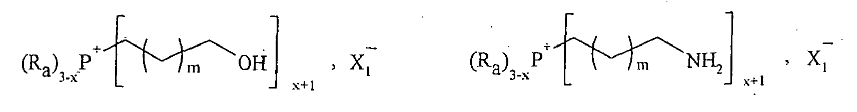 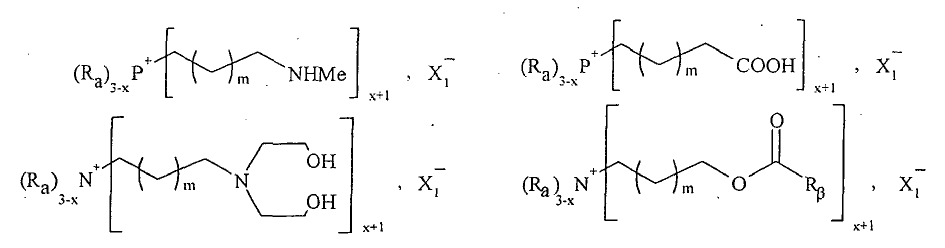 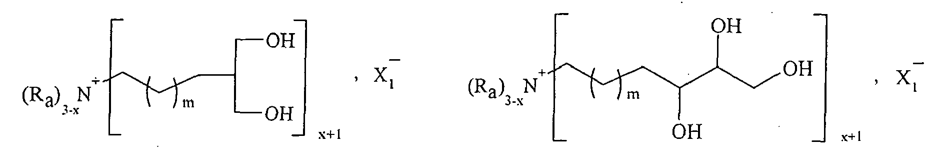 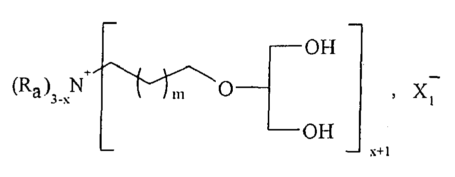 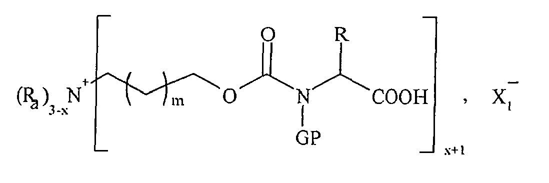 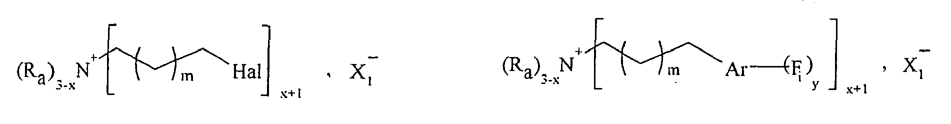 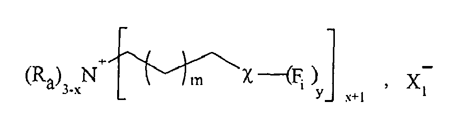 R representing a hydrogen atom, an alkyl group, functional or non-functional, comprising from 1 to 20 carbon atoms, or an aryl group, functional or non-functional, comprising from 6 to 30 carbon atoms,
x representing an integer comprised from 0 to 3,
y representing an integer comprised from 1 to 5,
Ar representing a functional or polyfunctional aromatic ring,
Fᵢ being as defined in claim 4,
Hal representing a halogen atom, in particular chosen from chlorine, bromine and iodine,
χ representing a carbocycle or a functional heterocycle,
X₁⁻ being chosen from: NTf₂⁻, PF₆⁻, BF₄⁻, Cl⁻, Br⁻, I⁻, CF₃SO₃⁻, MeSO₄⁻, EtSO₄⁻, MeSO₃⁻, C₆H₅SO₃⁻, pMeC₆H₄SO₃⁻,
m being an integer comprised from 0 to 20,
R_{β} representing a dienyl, vinyl group, substituted or non-substituted, functional, alkyl comprising from 1 to 20 carbon atoms, or functional aryl comprising from 6 to 30 carbon atoms, substituted or non-substituted alkynyl, and being in particular an alkylvinyl, alkylalkynyl, alkylaryl, alkyldienyl, alkylmalonyl, acyl group,
and Rₐ representing a branched or non-branched alkyl group comprising from 1 to 20 carbon atoms, in particular an ethyl, propyl, butyl, pentyl, hexyl, heptyl or octyl group.

16. The use according to any one of claims 1 to 15, **characterized in that** the solvent(s) used is/are a solvent aprotic, chosen from:
- solvents the dielectric constant ε of which is less than or equal to 2, such as the alkanes, the aromatic carbides such as benzene, toluene or xylene,
- solvents the dielectric constant s of which is comprised between approximately 2 and 15, such as the ethers, halogenobenzenes or dichloromethane, and
- solvents the dielectric constant ε of which is greater than 15, such as acetonitrile, nitromethane, DMF or dimethylacetamide.

17. The use according to any one of claims 1 to 16, for continuous, discontinuous, combinatorial or parallel organic synthesis, and/or for the preparation of banks of products.

18. The use according to any one of claims 1 to 17, for the implementation of cycloaddition reactions, preferably for the implementation of the Diels-Alder reaction, according to one of the following reaction diagrams:
a) 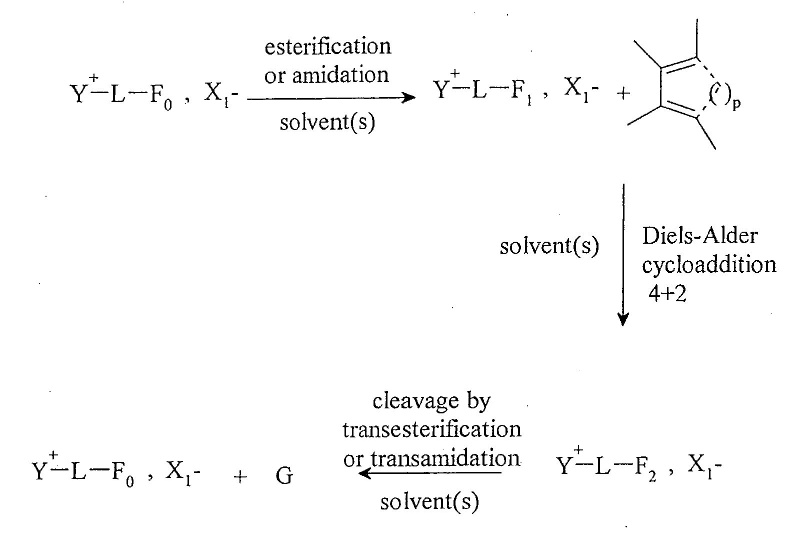 p being an integer varying from 0 to 2,
Y⁺- representing an onium cation as defined in one of claims 3 to 17, and preferably being a trimethylalkylammonium, triethylalkylammonium, tributylalkylphosphonium, N-methylimidazolium or pyridinium cation,
L representing an arm, in particular a linear or branched alkyl group comprising from 1 to 20 carbon atoms, or an optionally functional aralkyl or alkaryl group, comprising from 6 to 30 carbon atoms, and preferably being a linear alkyl group, preferably a linear alkyl group of type (CH₂)ᵣ, r varying from 1 to 20, and preferably from 2 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
the functions F₀, F₁ and F₂ being as defined below:
- F₀ corresponds to a -χ₁H group, in which χ₁ represents an oxygen atom or an -NR_{f} group, R_{f} corresponding to a linear or branched alkyl group, comprising from 1 to 20 carbon atoms, or an aryl group comprising from 6 to 30 carbon atoms,
- F₁ corresponds to the following formula:
| | |
|---|---|
| 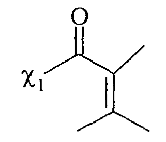 | χ₁ being as defined above, |
- F₂ corresponds to the following formula:
| | |
|---|---|
| 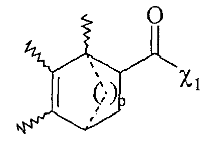 | χ₁ being as defined above, |
G corresponding to the following formula: 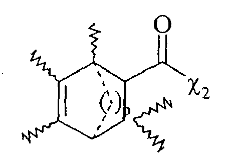 in which χ₂ represents either an OR_{g} group, Rg representing a hydrogen atom or an alkyl group comprising from 1 to 20 carbon atoms, or an -NRₕRᵤ group, Rₕ and Rᵤ representing independently of one another a hydrogen atom, an alkyl group comprising from 1 to 20 carbon atoms or an aryl group comprising from 6 to 30 carbon atoms,
b) 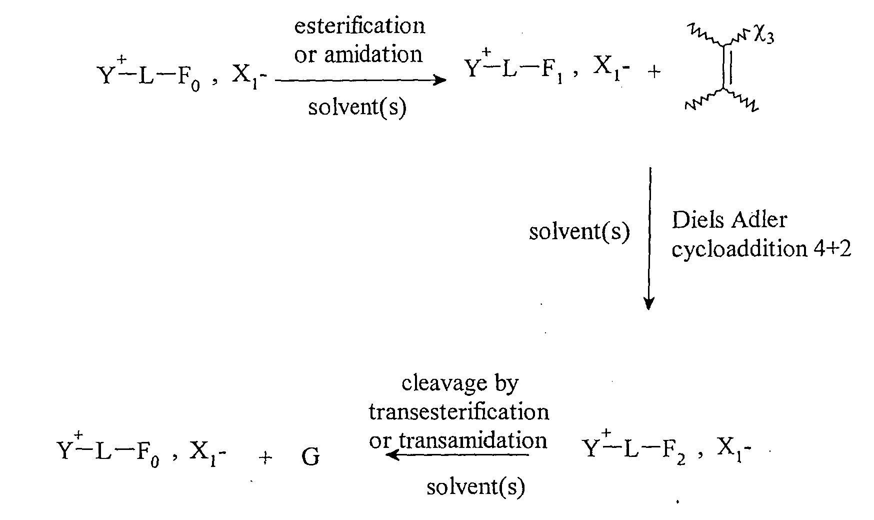 Y⁺-, L and X₁⁻ being as defined previously,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, or a mixture of these solvents,
the functions F₀, F₁ and F₂ being as defined below:
- F₀ represents any function making it possible to attach a 1,3-diene, and is in particular chosen from the carbonyl, amine, alkoxy, silane, stannane and borane functions, comprising from 1 to 20 carbon atoms,
- F₁ corresponds to the following formula:
| | |
|---|---|
| 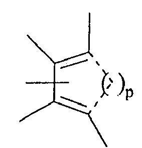 | p being an integer varying from 0 to 2, |
- F₂ corresponds to the following formula:
| | |
|---|---|
| 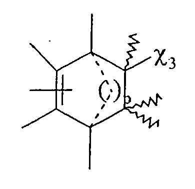 | χ₃ representing an electroattractive group, in particular chosen from the cyano, alkoxycarbonyl groups, comprising from 1 to 20 carbon atoms, acyl comprising from 2 to 20 carbon atoms, benzoyl, sulphonyl, dialkoxyphosphonyl comprising from 1 to 10 carbon atoms, |
G corresponding to the following formula:
| | |
|---|---|
| 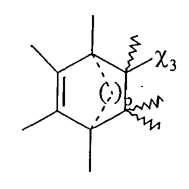 | χ₃ being as defined above. |
c) 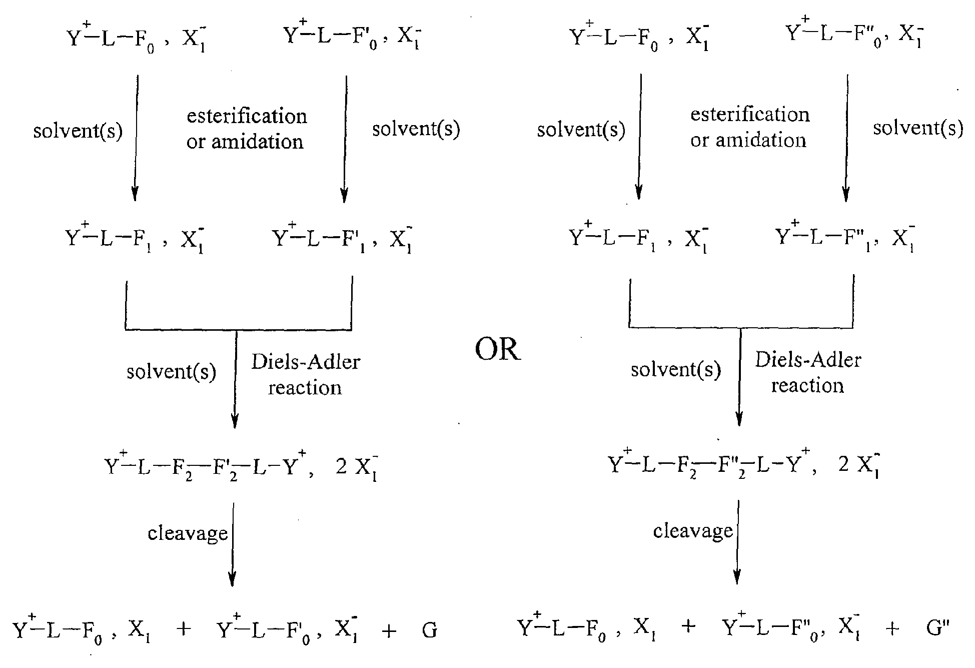 Y⁺-, L and X₁⁻ being as defined previously,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
the functions F₀, F'₀, F"₀, F₁, F'₁, F"₁, F₂, F'₂ and F"₂ being as defined below:
- F₀ and F'₀ correspond respectively to a -χ₁H and -χ'₁H group, in which χ₁ and χ'₁, identical or different, represent an oxygen atom or an -NR_{f} group, R_{f} corresponding to a linear or branched alkyl group, comprising from 1 to 20 carbon atoms, or an aryl group comprising from 6 to 30 carbon atoms,
- F"₀ corresponds to a -COOH function;
- F₁ corresponds to the following formula:
| | |
|---|---|
| 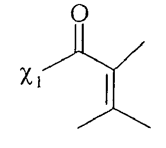 | χ₁ being as defined above, |
- F'₁ corresponds to the following formula:
| | |
|---|---|
| 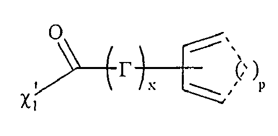 | p being an integer varying from 0 to 2, |
| | χ'₁ being as defined above, |
| | x being equal to 0 or 1, |
| | Γ representing an alkyl chain comprising from 1 to 30 carbon atoms, alkaryl, aralkyl, aryl comprising from 6 to 30 carbon atoms, |
- F"₁ corresponds to the following formula:
| | |
|---|---|
| 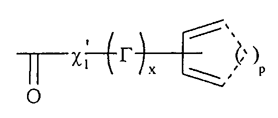 | p, x and Γ being as defined above, |
| | χ'₁ being as defined above, |
- F₂-F'₂ corresponds to the following formula:
| | |
|---|---|
| 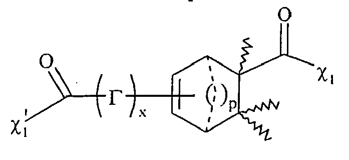 | p, χ₁, χ'₁, x and Γ being as defined above, |
- F₂-F"₂ corresponds to the following formula:
| | |
|---|---|
| 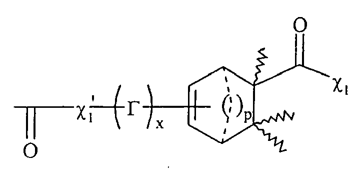 | p, χ₁, χ'₁, x and Γ being as defined above, |
- G corresponds to the following formula: 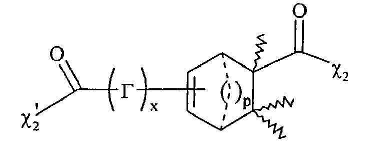
- G" corresponds to the following formula: 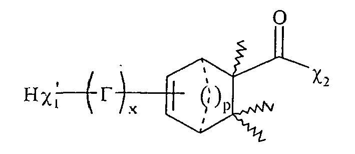 χ ₂ and χ'₂, identical or different, represent either an OR_{g} group, R_{g} representing a hydrogen atom or an alkyl group comprising from 1 to 20 carbon atoms, or an -NRₕRᵤ group, Rₕ and Rᵤ representing independently of one another a hydrogen atom, an alkyl group comprising from 1 to 20 carbon atoms or an aryl group comprising from 6 to 30 carbon atoms.

19. The use according to any one of claims 1 to 17, for the implementation of coupling reactions such as the Heck, Suzuki, Sonogashira or Ullmann reactions.

20. The use according to claim 19 for the implementation of the Heck reaction, according to one of the following reaction diagrams: 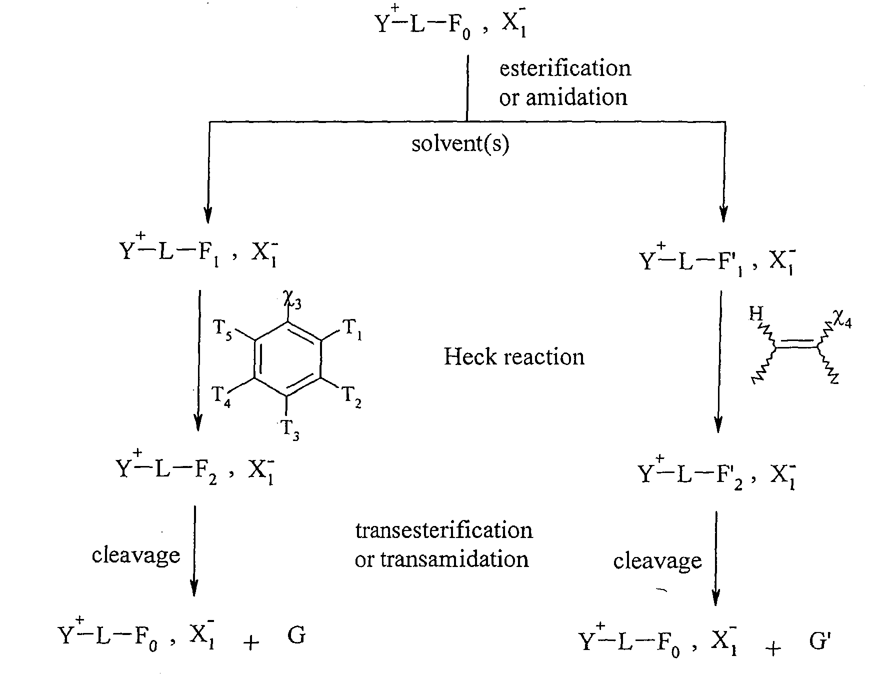 Y⁺- representing an onium cation as defined in one of claims 3 to 17, and preferably being a trimethylalkylammoniun, triethylalkylammonium, tributylalkylphosphonium, N-methylimidazolium or pyridinium,
L representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 2 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
the functions F₀, F₁, F'₁, F₂ and F'₂ being as defined below:
- F₀ corresponds to a -χ₁H group, in which χ₁ represents an oxygen atom or an -NR_{f} group, R_{f} corresponding to a linear or branched alkyl group, comprising from 1 to 20 carbon atoms, or an aryl group comprising from 6 to 30 carbon atoms,
- F₁ corresponds to one of the following formulae: 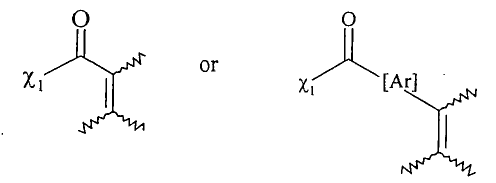 χ ₁ being as defined above,
[Ar] representing an aromatic ring, optionally substituted by a linear or branched alkyl group, comprising from 1 to 20 carbon atoms or an aryl group comprising from 6 to 30 carbon atoms, or a functional group in particular chosen from NO₂, CN, COOR, OR, COR, NHCOR, NRR', SO₂R, I, Br, R and R' representing independently of one another an alkyl group comprising from 1 to 20 carbon atoms or an aryl group comprising from 6 to 30 carbon atoms, [Ar] preferably corresponding to the following formula:
| | |
|---|---|
| 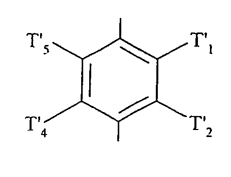 | in which T'₁, T'₂, T'₄ and T'₅ represent independently of one another a hydrogen atom, a linear or branched alkyl group, comprising from 1 to 20 carbon atoms or an aryl group comprising from 6 to 30 carbon atoms, or a functional group in particular chosen from NO₂, CN, COOR, OR, COR, NHCOR, NRR', SO₂R, I, Br, R and R' representing independently of one another an alkyl group comprising from 1 to 20 carbon atoms or an aryl group comprising from 6 to 30 carbon atoms, |
- F₂ corresponds to one of the following formulae: 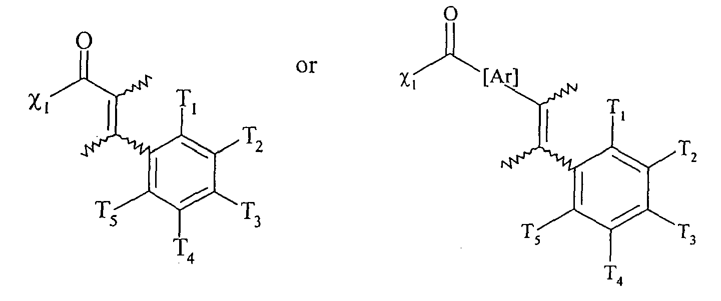 χ ₁ and Ar being as defined above, T₄
T₁, T₂, T₃, T₄ and T₅ corresponding to the definition given above for T'₁, T'₂, T'₄ and T'₅
G corresponding to one of the following formulae: 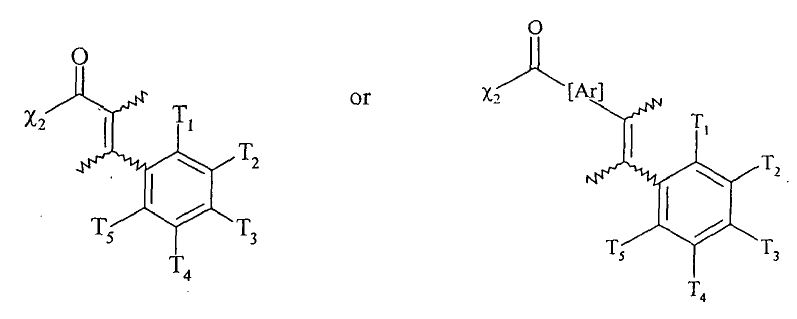 in which χ₂ represents either an -OR_{g} group, R_{g} representing a hydrogen atom or an alkyl group comprising from 1 to 20 carbon atoms, or an -NRₕRᵤ group, Rₕ and Rᵤ representing independently of one another a hydrogen atom, an alkyl group comprising from 1 to 20 carbon atoms or an aryl group comprising from 6 to 30 carbon atoms,
χ₃ representing a leaving group, in particular chosen from the I, Cl and Br halides, the mesylate, tosylate, triflate, sulphonate, sulphate or phosphate groups,
the entity 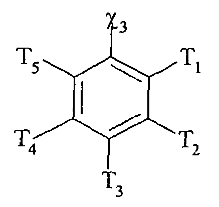 representing in particular the following groups: 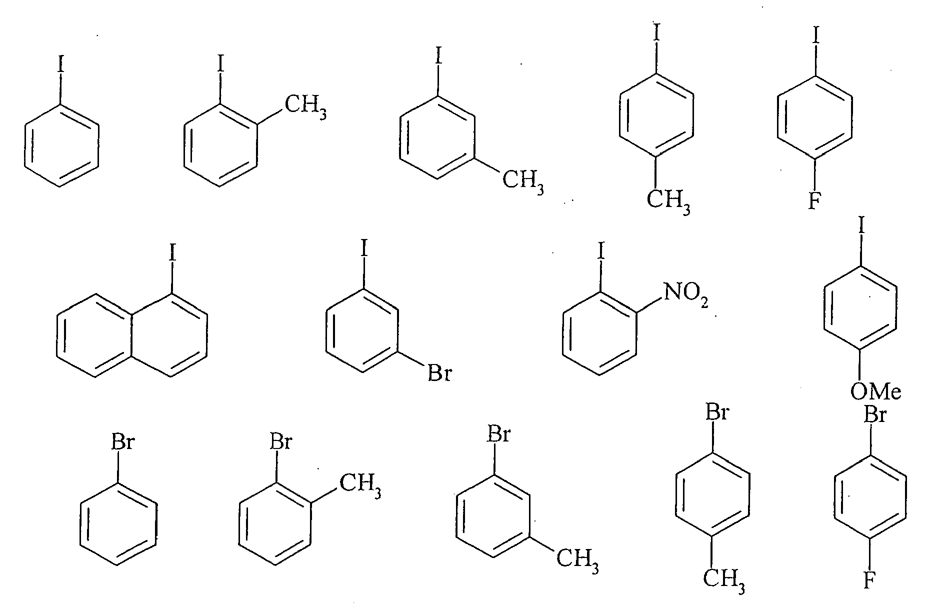 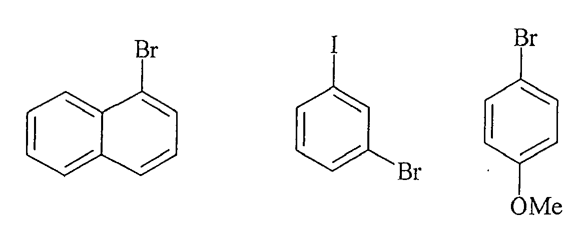
- F'₁ corresponds to the following formula:
| | |
|---|---|
| 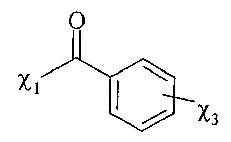 | χ₁ and χ₃ being as defined above, |
- F'₂ corresponds to the following formula:
| | |
|---|---|
| 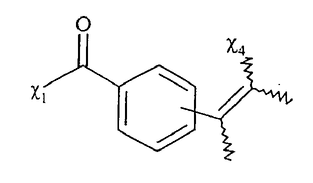 | χ₁ being as defined above, |
| | χ₄ representing a functional group of ester, amide, sulphone, phosphonate, silane, borane type, or a functional or non-functional alkyl group, comprising from 1 to 20 carbon atoms, or a functional or non-functional aryl group, comprising from 6 to 30 carbon atoms, |
G' corresponding to the following formula:
| | |
|---|---|
| 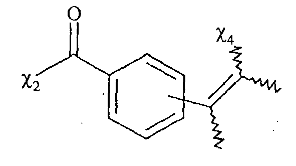 | χ₂ and χ₄ being as defined above. |

21. The use according to claim 19 for the implementation of Suzuki coupling, according to one of the following reaction diagrams:
a) 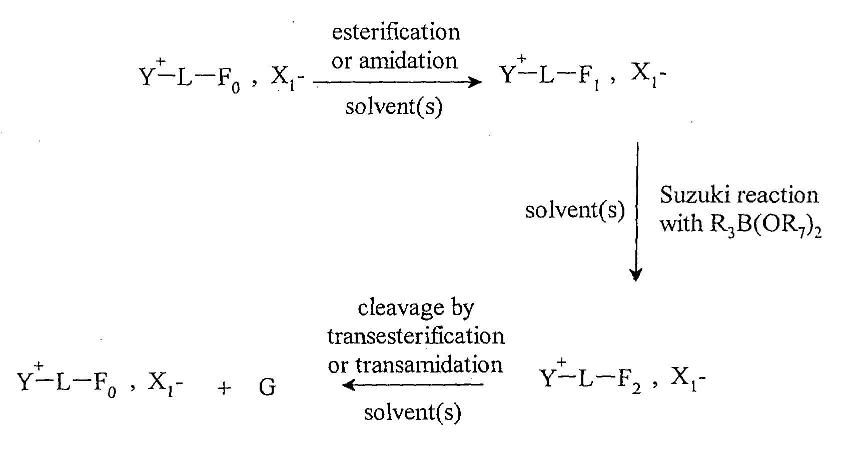 R₃ being chosen from the aryl, heteroaryl, ethenyl, dienyl, allyl, ethynyl groups, substituted or non-substituted, comprising from 2 to 30 carbon atoms,
R₇ representing a hydrogen atom or a branched or linear alkyl group, or a cycloalkyl group comprising from 1 to 12 carbon atoms,
Y⁺- representing an onium cation as defined in one of claims 3 to 17, and preferably being a trimethylalkylammonium, triethylalkylammonium, tributylalkylphosphonium, N-methylimidazolium or pyridinium cation,
L representing an arm, in particular a linear or branched alkyl group comprising from 1 to 20 carbon atoms, or an optionally functional aralkyl group comprising from 6 to 30 carbon atoms, and preferably being a linear alkyl group, preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
the functions F₀, F₁ and F₂ being as defined below:
- F₀ is in the form -χ₁H, χ₁ representing an oxygen atom or an -NR_{f} group, R_{f} corresponding to a linear or branched alkyl group, comprising from 1 to 20 carbon atoms, or an aryl group comprising from 6 to 30 carbon atoms,
- F₁ is in the form -Rₑ-χ, Rₑ representing an aromatic or heteroaromatic group comprising from 6 to 30 carbon atoms, χ representing a leaving group preferably chosen from Cl, Br, I, OTf, O-CO₂R⁵ or OSO₃-R⁵, R⁵ representing an alkyl group comprising from 1 to 10 carbon atoms or an aralkyl group comprising from 6 to 30 carbon atoms, F₁ preferably corresponding to the following formula: 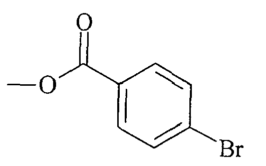
- F₂ is in the form -Rₑ-R₂, Rₑ being as defined above and R₂ being chosen from the aryl, heteroaryl, ethenyl, dienyl, allyl, ethynyl groups, substituted or non-substituted, comprising from 2 to 30 carbon atoms, F₂ preferably corresponding to the following formula: 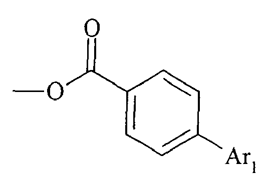 Ar₁ representing an aromatic group preferably chosen from: 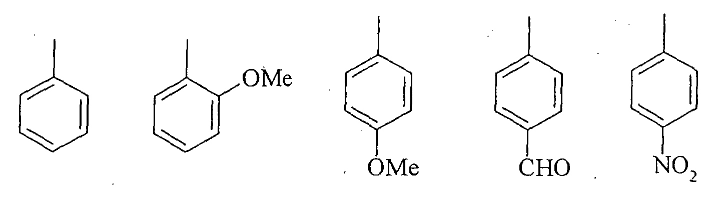 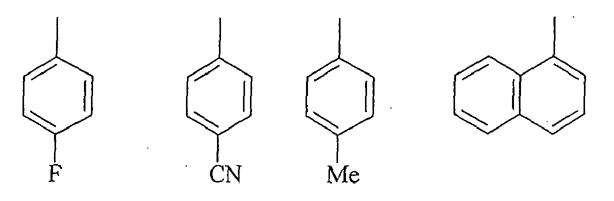 the molecule G being in the form R₂-R₃, R₂ and R₃ being as defined above, and corresponding in particular to the following formula: 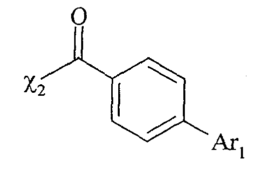
in which χ₂ represents either an -OR_{g} group, R_{g} representing a hydrogen atom or an alkyl group comprising from 1 to 20 carbon atoms, or an -NRₕRᵤ group, Rₕ and Rᵤ representing independently of one another a hydrogen atom, an alkyl group comprising from 1 to 20 carbon atoms or an aryl group comprising from 6 to 30 carbon atoms,
Ar₁ is as defined above,
b) 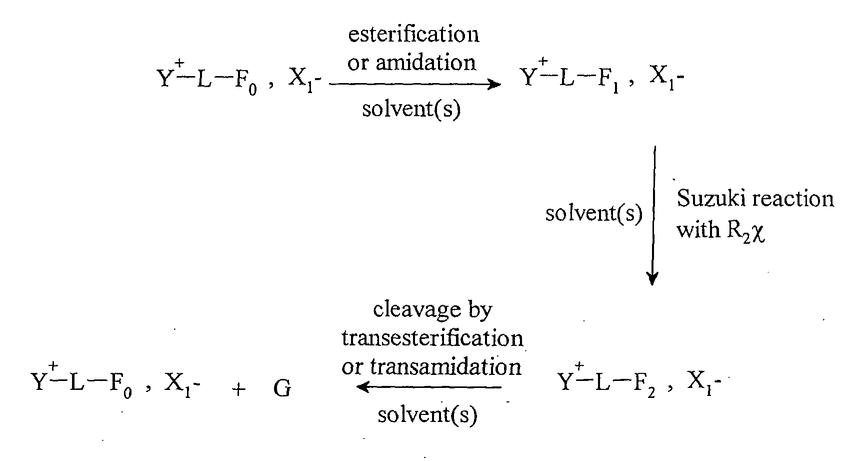 Y⁺- representing an onium cation as defined in one of claims 3 to 17, and preferably being a trimethylalkylammonium, triethylalkylammonium, tributylalkylphosphonium, N-methylimidazolium or pyridinium cation,
L representing an ann, in particular a linear or branched alkyl group comprising from 1 to 20 carbon atoms, or an optionally functional aralkyl group comprising from 6 to 30 carbon atoms, and preferably being a linear alkyl group, preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
R₂ being chosen from the aryl, heteroaryl, ethenyl, dienyl, allyl, ethynyl groups, substituted or non-substituted, comprising from 2 to 30 carbon atoms,
the functions F₀, F₁ and F₂ being as defined below:
- F₀ is in the form -χ₁H, χ₁ being as defined above,
- F₁ is in the form -R_{q}-B(OR₇)₂, R₇ being as defined above, and R_{q} corresponding to an aryl group comprising from 6 to 30 carbon atoms, heteroaryl comprising from 4 to 20 carbon atoms, ethenyl comprising from 2 to 20 carbon atoms, dienyl comprising from 3 to 20 carbon atoms, allyl comprising from 3 to 20 carbon atoms, ethynyl comprising from 2 to 20 carbon atoms, substituted or non-substituted, F₁ preferably corresponding to the following formula: 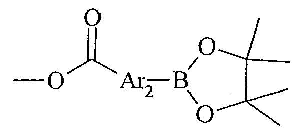 Ar₂ corresponding to an aryl group, substituted or non-substituted, comprising from 6 to 30 carbon atoms,
- F₂ is in the form -R_{q}-Rₑ, Rq and Rₑ being as defined above, F₂ preferably corresponding to the following formula: 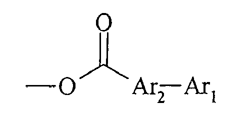 Ar₁ representing an aromatic group preferably chosen from: 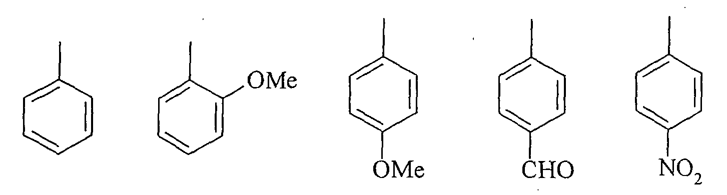 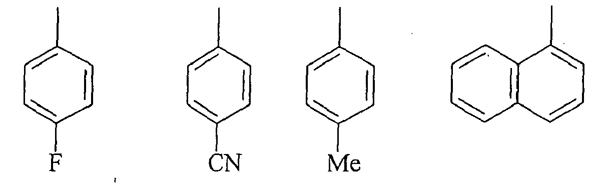 the molecule G being in the form R₂-R₃, R₂ and R₃ being as defined above, and corresponding in particular to the following formula: 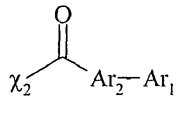
in which χ₂, Ar₁ and Ar₂ are as defined above,
c) 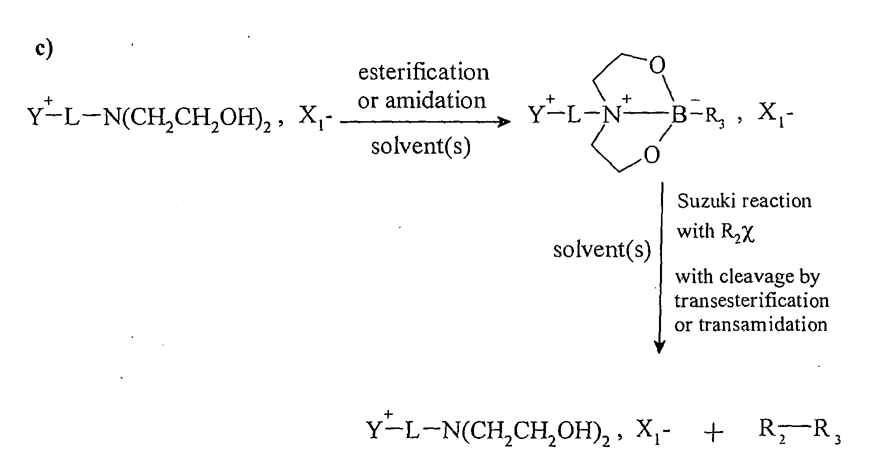 Y⁺-, L, X₁⁻, R₂ and R₃ being as defined above,
R₃ preferably being a phenyl group,
the solvent or solvents being chosen from: dichloromethane, tetrahydrofuran, dioxane, acetonitrile, dimethylformamide, dimethylacetamide, N-methylpyrrolidinone; propionitrile, acetone, toluene, chlorobenzene, nitrobenzene, dichlorobenzene, nitromethane, nitroethane, or a mixture of these solvents,
d) 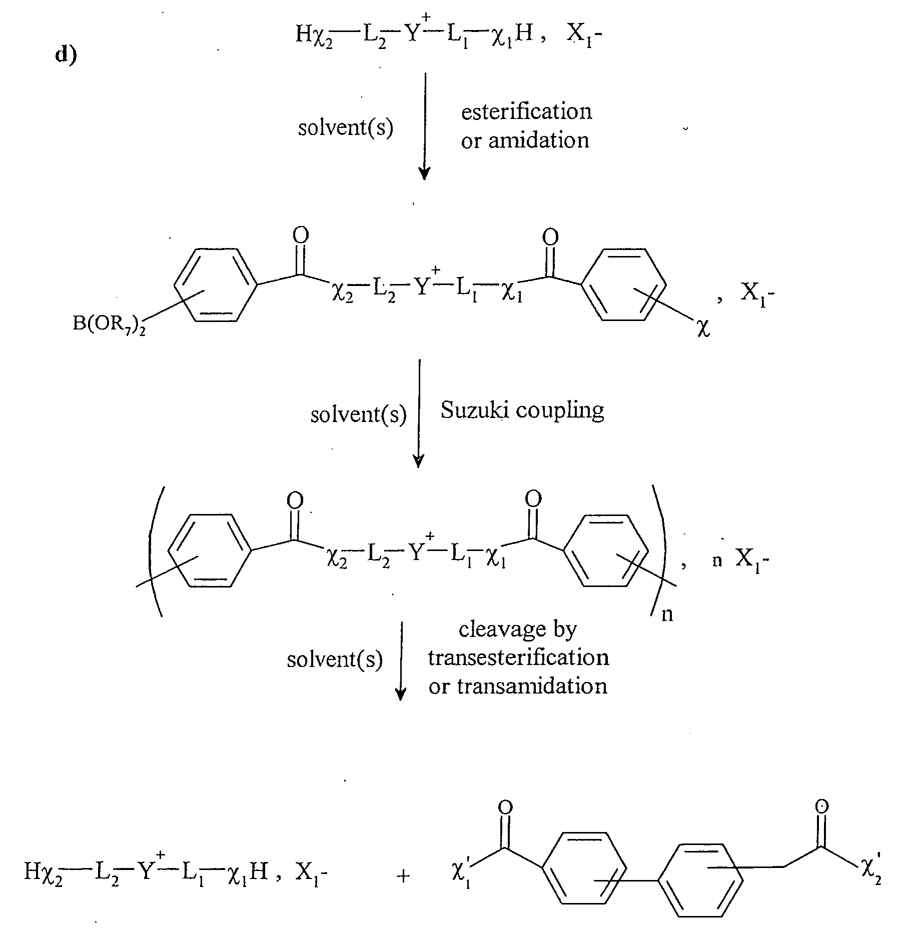 n representing an integer comprised between 1 and 50,
Y⁺- representing an onium cation as defined in one of claims 3 to 17, and preferably being a trimethylalkylammonium, triethylalkylammonium, tributylalkylphosphonium, N-methylimidazolium or pyridinium cation,
L₁ and L₂ representing an arm, identical or different, in particular a linear or branched alkyl group comprising from 1 to 20 carbon atoms, or an optionally functional aralkyl group comprising from 6 to 30 carbon atoms, and preferably being a linear alkyl group preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpylpyrrolidinone, propionitrile, acetone, toluene, chlorobenzene, nitrobenzene, dichlorobenzene, nitromethane, nitroethane, or a mixture of these solvents,
χ₁ and χ₂, identical or different, representing an oxygen atom or an -NR_{f} group, R_{f} corresponding to a linear or branched alkyl group, comprising from 1 to 20 carbon atoms, or an aryl group comprising from 6 to 30 carbon atoms,
χ representing a leaving group preferably chosen from Cl, Br, I, OTf, O-CO₂R⁵ or OSO₃-R⁵, R⁵ representing an alkyl group comprising from 1 to 10 carbon atoms or an aralkyl group comprising from 6 to 30 carbon atoms,
R₇ representing a hydrogen atom, a branched or non-branched alkyl group, or cycloalkyl, comprising from 1 to 12 carbon atoms, or an aryl group, comprising from 6 to 30 carbon atoms,
χ'₁ and χ'₂, identical or different, representing either an -OR_{g} group, R_{g} representing a hydrogen atom or an alkyl group comprising from 1 to 20 carbon atoms, or an -NRₕRᵤ group, Rₕ and Rᵤ representing independently of one another a hydrogen atom, an alkyl group comprising from 1 to 20 carbon atoms or an aryl group comprising from 6 to 30 carbon atoms.

22. The use according to claim 19 for the implementation of Sonogashira coupling, according to one of the following reaction diagrams:
a) 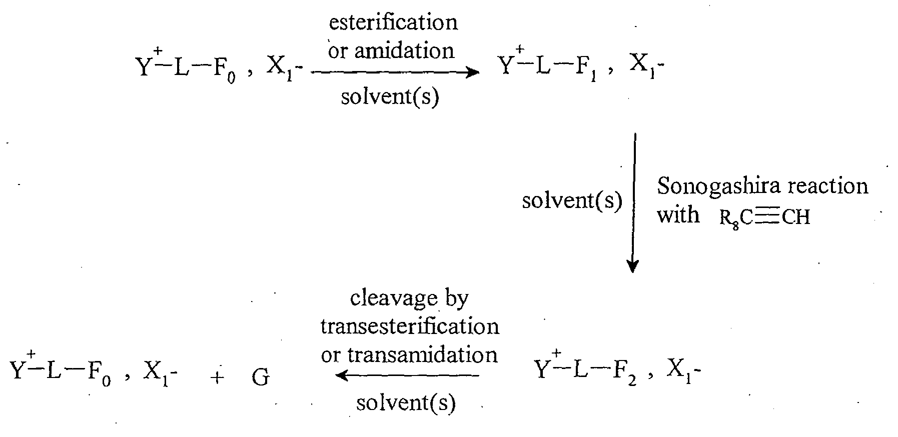 Y⁺- representing an onium cation as defined in one of claims 3 to 17, and preferably being a trimethylalkylammonium, triethylalkylammonium, tributylalkylphosphonium, N-methylimidazolium or pyridinium cation,
L representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
R₈ representing an ORₕ, NRₕRᵤ, CORₕ, CN, SO₂Rₕ, SRₕ group, an alkenyl, ethynyl, dienyl group, Rₕ and Rᵤ representing, independently of one another, a hydrogen atom, an alkyl group comprising from 1 to 20 carbon atoms or an aryl group comprising from 6 to 30 carbon atoms,
or R₈ representing an alkyl group, branched or linear, optionally functional, comprising from 1 to 20 carbon atoms, or an aryl group, or an alkaryl or aralkyl group, comprising from 6 to 30 carbon atoms, substituted or non-substituted, said alkyl or aryl groups being able to be substituted by one of the following functional groups: a halogen atom, in particular Cl, an ORₕ, NRₕRᵤ, CORₕ, CN, SO₂Rₕ, SRₕ group, an alkenyl, ethynyl, dienyl, vinyl, alkynyl group, Rₕ and Rᵤ being as defined previously,
R₈ being in particular one of the following groups:
-(CH₂)ₛ-CH₃, -(CH₂)ₛ-CH₂OH, -(CH₂)ₛ-CH₂OMe,
s representing an integer comprised between 0 and 10, 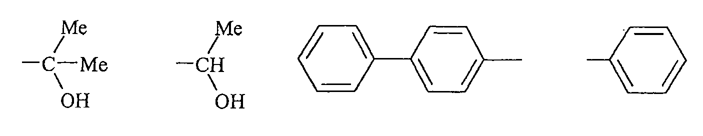 Me₃Si-
the functions F₀, F₁ and F₂ being as defined below:
- F₀ corresponds to a -χ₁N group, in which χ₁ represents an oxygen atom or an -NR_{f} group, R_{f} corresponding to a linear or branched alkyl group, comprising from 1 to 20 carbon atoms, or an aryl group comprising from 6 to 30 carbon atoms,
- F₁ corresponds to the following formula:
| | |
|---|---|
| 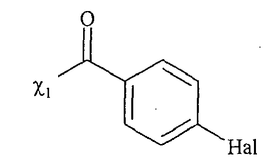 | χ₁ being as defined above, and Hal representing a halogen, and preferably being iodine, |
- F₂ corresponds to the following formula:
| | |
|---|---|
| 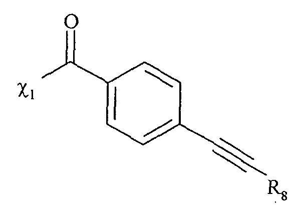 | χ₁ and R₈ being as defined above, |
G corresponding to the following formula: 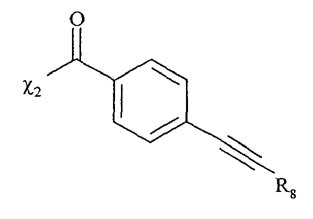 in which χ₂ represents either an -OR_{g} group, R_{g} representing a hydrogen atom or an alkyl group comprising from 1 to 20 carbon atoms, or an -NRₕRᵤ group, Rₕ and Rᵤ representing independently of one another a hydrogen atom, an alkyl group comprising from 1 to 20 carbon atoms or an aryl group comprising from 6 to 30 carbon atoms, χ₂ representing in particular an OMe, OEt, OPr or OBu group.
**b)** 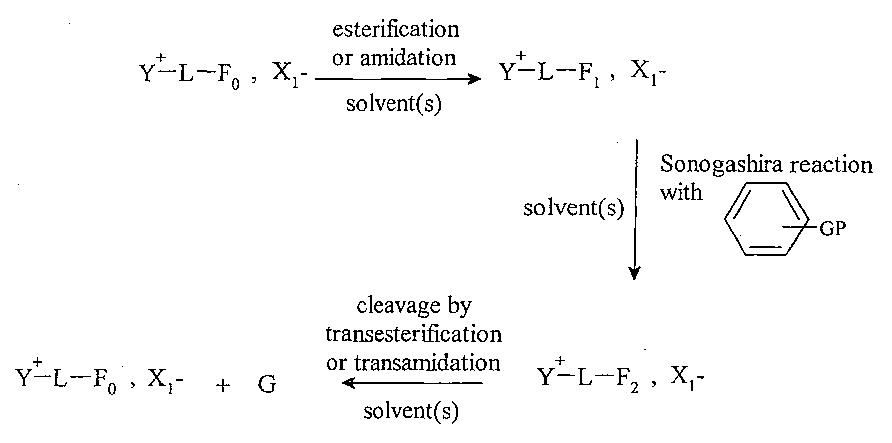 Y⁺- representing an onium cation as defined in one of claims 3 to 17, and preferably being a trimethylalkylammonium, triethylalkylammonium, tributylalkylphosphonium, N-methylimidazolium, alkylpyridinium, dimethylalkylsulphonium or diethylalkyl-sulphonium cation,
L representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
GP representing a leaving group, and being in particular Cl, Br, I or OTf,
the functions F₀, F₁ and F₂ being as defined below:
- F₀ corresponds to a -COOH group,
- F₁ corresponds to the following formula: 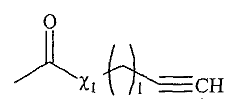 in which 1 represents an integer varying from 1 to 20, and χ₁ represents an oxygen atom or an -NR_{f} group, R_{f} corresponding to a linear or branched alkyl group, comprising from 1 to 20 carbon atoms, or an aryl group comprising from 6 to 30 carbon atoms,
- F₂ corresponds to the following formula:
| | |
|---|---|
| 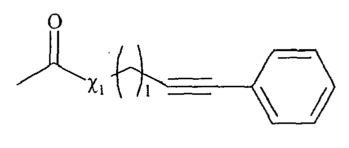 | χ₁ and 1 being as defined above, |
G corresponding to the following formula: 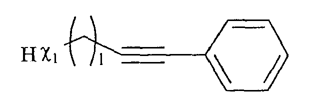
in which χ₁ and 1 are as defined above.

23. The use according to any one of claims 1 to 17, for the implementation of the Baylis-Hilman reaction, according to one of the following reaction diagrams:
**a)** 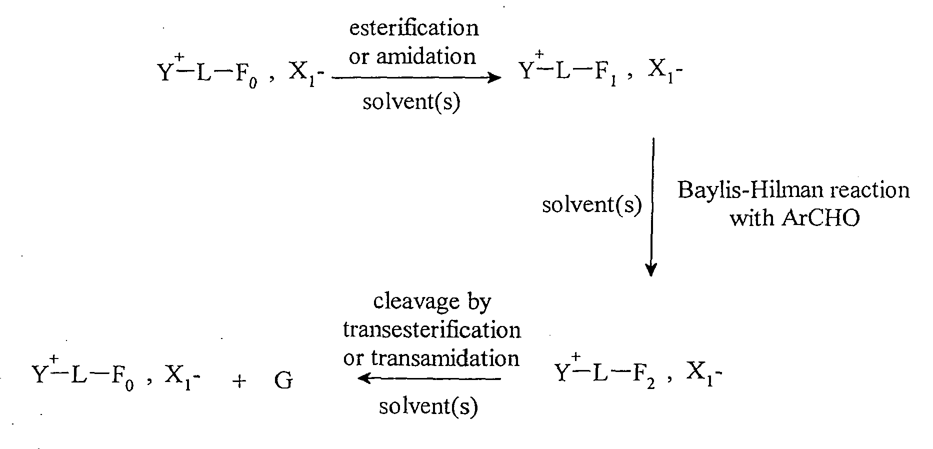 Y⁺- representing an onium cation as defined in one of claims 3 to 17, and preferably being a trimethylalkylammonium, triethylalkylammonium, tributylalkylphosphonium, N-methylimidazolium or pyridinium cation,
L representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
the functions F₀, F₁ and F₂ being as defined below:
- F₀ represents an -OH group,
- F₁ corresponds to the following formula: 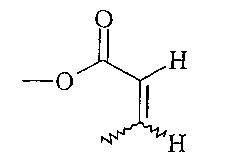
- F₂ corresponds to the following formula: 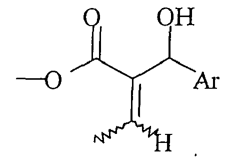
G corresponding to the following formula:
| | |
|---|---|
| 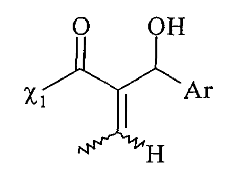 | χ₁ representing an -OH group, or an -OR_{g} group, R_{g} representing a linear or branched alkyl group, comprising from 1 to 20 carbon atoms, |
Ar representing an aromatic or heteroaromatic group, substituted or non-substituted,
ArCHO being in particular chosen from: 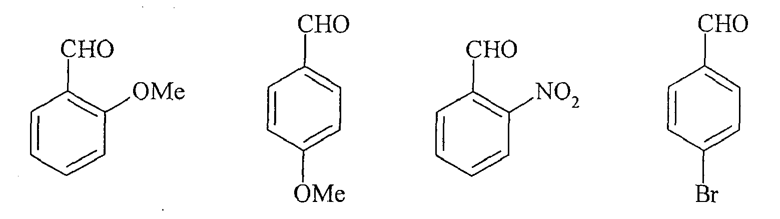 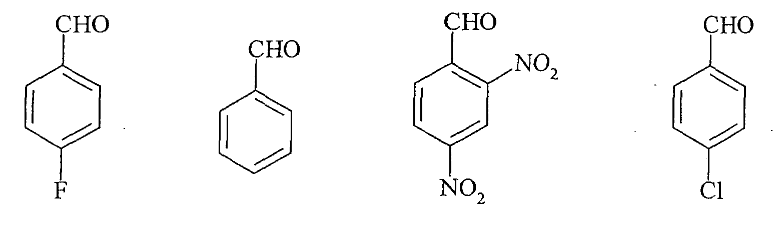
**b)** 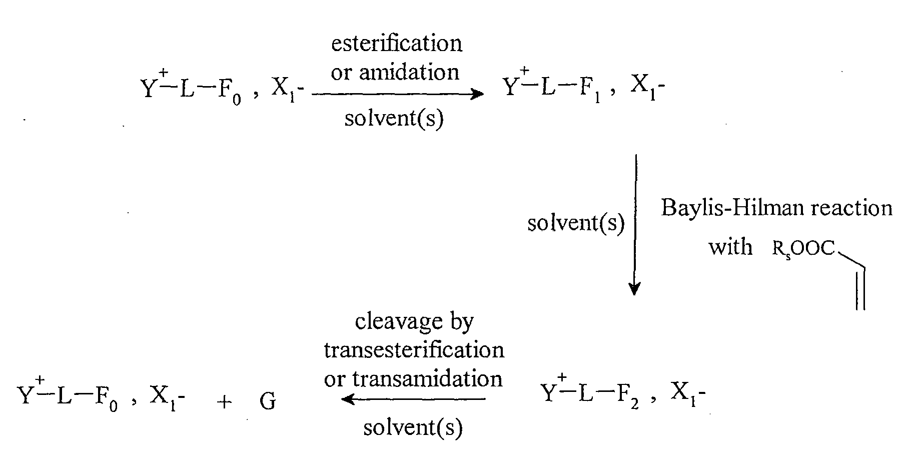 Y⁺- representing an onium cation as defined in one of claims 3 to 17, and preferably being a trimethylalkylammonium, triethylalkylammonium, tributylalkylphosphonium, N-methylimidazolium, alkylpyridinium, dimethylalkylsulphonium or diethylalkyl-sulphonium cation,
L representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
Rₛ representing a hydrogen atom or an alkyl group comprising from 1 to 20 carbon atoms or aralkyl or alkaryl comprising from 7 to 30 carbon atoms,
the functions F₀, F₁ and F₂ being as defined below:
- F₀ corresponds to a -χ₁H group, in which χ₁ represents an oxygen atom or an -NR_{f} group, R_{f} corresponding to a linear or branched alkyl group, comprising from 1 to 20 carbon atoms, or an aryl group comprising from 6 to 30 carbon atoms,
- F₁ corresponds to the following formula:
| | |
|---|---|
| 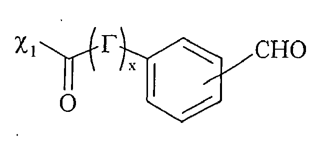 | χ₁ being as defined above, |
| | x being equal to 0 or 1, |
| | Γ representing an alkyl chain comprising from 1 to 20 carbon atoms, alkaryl, aralkyl comprising from 6 to 30 carbon atoms, |
- F₂ corresponds to the following formula:
| | |
|---|---|
| 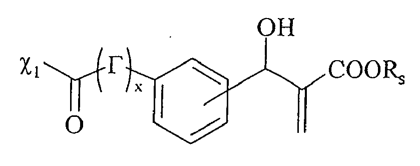 | χ₁, x, Rₛ and Γ being as defined above |
- G corresponding to the following formula:
| | |
|---|---|
| 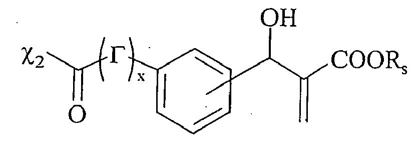 | χ₂, x, Rₛ and Γ being as defined above |
in which χ₂ represents either an -OR_{g} group, R_{g} representing a hydrogen atom or an alkyl group comprising from 1 to 20 carbon atoms, or an -NRₕRᵤ group, Rₕ and Rᵤ representing independently of one another a hydrogen atom, an alkyl group comprising from 1 to 20 carbon atoms or an aryl group comprising from 6 to 30 carbon atoms, χ₂ representing in particular an OMe, OEt, OPr or OBu group.
**c)** 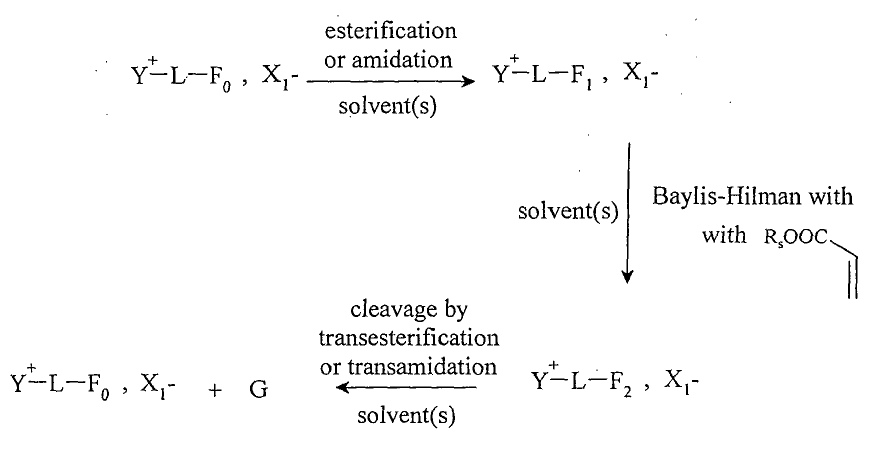 Y⁺- representing an onium cation as defined in one of claims 3 to 17, and preferably being a trimethylalkylammonium, triethylalkylammonium, tributylalkylphosphonium, N-methylimidazolium or pyridinium cation,
L representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
Rₛ representing a hydrogen atom or an alkyl group comprising from 1 to 20 carbon atoms or aralkyl or alkaryl comprising from 7 to 30 carbon atoms,
the functions F₀, F₁ and F₂ being as defined below:
- F₀ corresponds to a -COχ₁H group, in which χ₁ represents an oxygen atom or an -NR_{f} group, R_{f} corresponding to a linear or branched alkyl group, comprising from 1 to 20 carbon atoms, or an aryl group comprising from 6 to 30 carbon atoms,
- F₁ corresponds to the following formula: 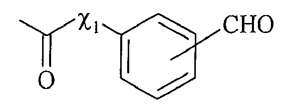
- F₂ corresponds to the following formula: 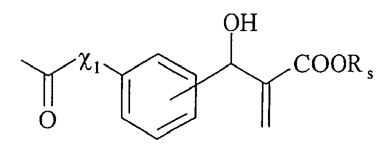
- G corresponding to the following formula:

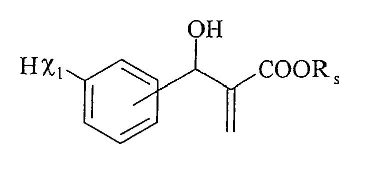

24. The use according to any one of claims 1 to 17, for the synthesis, optionally asymmetrical, of α-amino acids, according to the following reaction diagram: 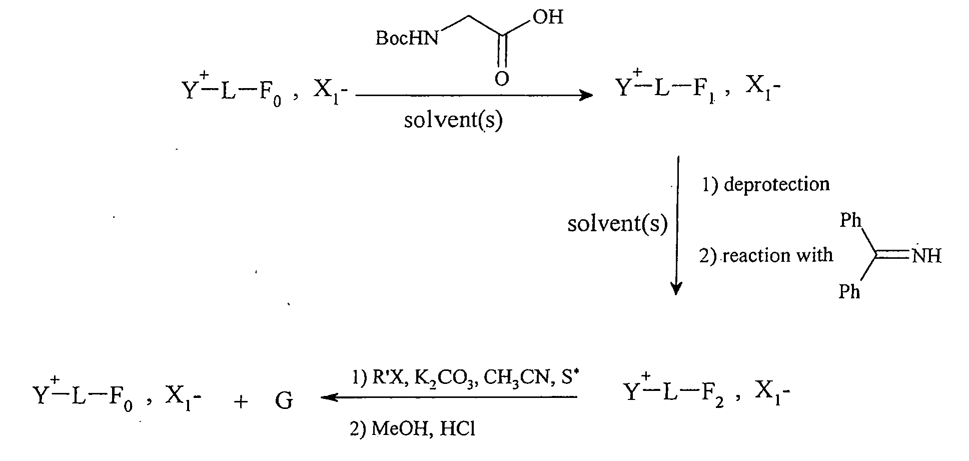 Y⁺- representing an onium cation as defined in one of claims 3 to 17, and preferably being a trimethylalkylammonium, triethylalkylammonium or tributylalkylphosphonium cation,
L representing an arm, in particular a linear or branched alkyl group comprising from 1 to 20 carbon atoms, or an optionally functional aralkyl group comprising from 1 to 20 carbon atoms, and preferably being a linear alkyl group, preferably a linear alkyl group of type (CH₂)ᵣ, r varying from 1 to 20, and preferably from 3 to 6,
X₁⁻ being as defined in one of claims 1 to 17, and being in particular Cl⁻, Br⁻, I⁻, ⁻N(SO₂CF₃)₂, BF₄⁻, PF₆⁻,
the solvent or solvents being chosen from: acetonitrile, dichloromethane, tetrahydrofuran, dioxane, toluene, chlorobenzene or a mixture of these solvents,
R' representing a linear or branched alkyl group, comprising from 1 to 30 carbon atoms, optionally functional,
S* representing a chiral phase transfer agent such as O(9)-allyl-N-9-anthracenylmethylcinchonidinium bromide,
the functions F₀, F₁ and F₂ being as defined below:
- F₀ corresponds to -OH,
- F₁ corresponds to the following formula: 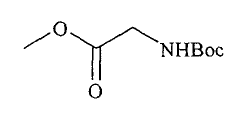
- F₂ corresponds to the following formula: 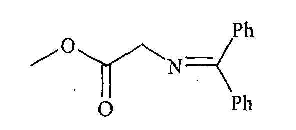
G corresponding to the following formula:

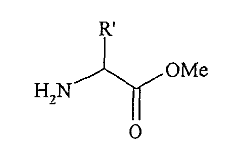

25. The use according to any one of claims 1 to 17, for the implementation of multi-component reactions, in particular for the Grieco-type reaction according to one of the following reaction diagrams:
**a)** 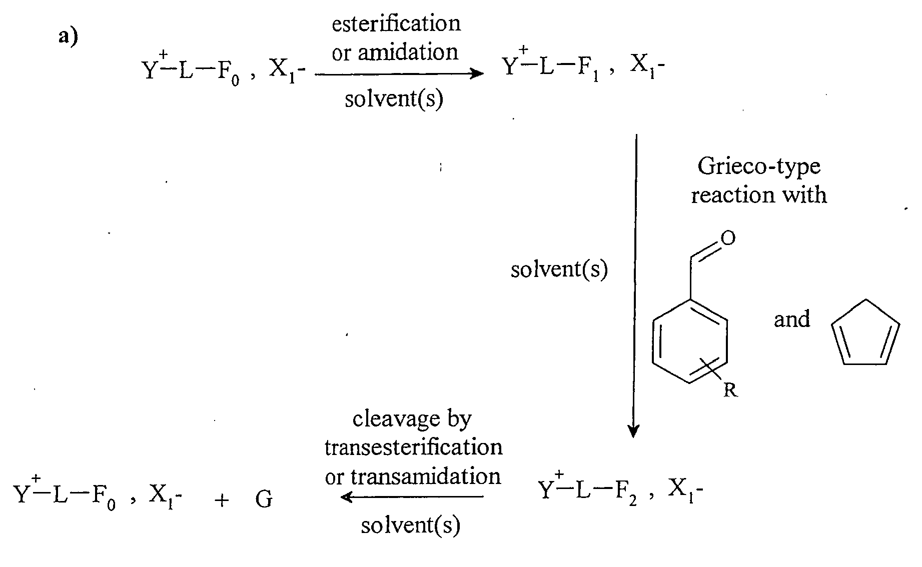 Y⁺- representing an onium cation as defined in one of claims 3 to 17, and preferably being a trimethylalkylammonium, triethylalkylammonium, tributylalkylphosphonium, N-methyl-N'-alkylimidazolium, N-alkylpyridinium, dimethylalkylsulphonium or diethylalkylsulphonium cation,
L representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
R representing a hydrogen atom, a nitro group in para position, a chlorine atom in para position or a methoxy group in ortho position,
the functions F₀, F₁ and F₂ being as defined below:
- F₀ represents an -OH group,
- F₁ corresponds to the following formula: 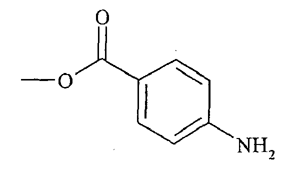
- F₂ corresponds to the following formula: 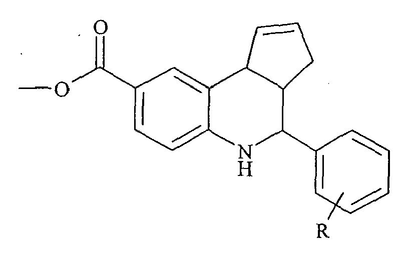
G corresponding to the following formula:
| | |
|---|---|
| 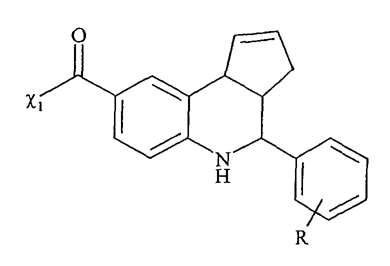 | χ₁ representing an -OH group, or an -OR_{g} group, R_{g} representing a linear or branched alkyl group, comprising from 1 to 20 carbon atoms, |
**b)** 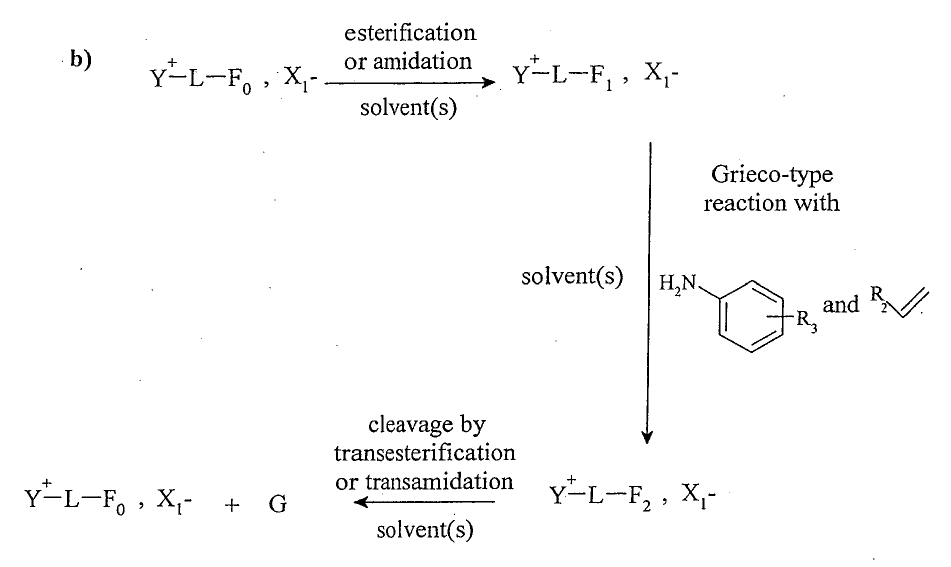 Y⁺- representing an onium cation as defined in one of claims 3 to 17, and preferably being a trimethylalkylammonium, triethylalkylammonium, tributylalkylphosphonium, N-methyl-N'-alkylimidazolium, N-alkylpyridinium, dimethylalkylsulphonium or diethylalkylsulphonium cation,
L representing an arm, in particular a linear or branched alkyl group comprising from 1 to 20 carbon atoms, or an optionally functional aralkyl or alkaryl group, comprising from I to 20 carbon atoms, and preferably being a linear alkyl group, preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
R₂ representing a functional or non-functional alkyl group, comprising from 1 to 20 carbon atoms, or a functional or non-functional aryl group, comprising from 6 to 30 carbon atoms, or an aralkyl or alkaryl group, functional or non-functional, comprising from 7 to 50 carbon atoms,
R₃ representing a hydrogen atom, a linear or branched alkyl group, comprising from 1 to 20 carbon atoms or an aryl group comprising from 6 to 30 carbon atoms, or an aralkyl or alkaryl group, functional or non-functional, comprising from 7 to 50 carbon atoms, or a functional group in particular chosen from NO₂, CN, COOR, OR, COR, NHCOR, NRR', SO₂R, I, Br, R and R' representing independently of one another an alkyl group comprising from 1 to 20 carbon atoms or an aryl group comprising from 6 to 30 carbon atoms,
the functions F₀, F₁ and F₂ being as defined below:
- F₀ represents an -OH group,
- F₁ corresponds to the following formula: 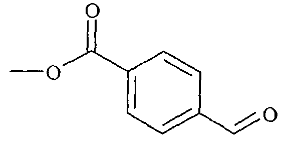
- F₂ corresponds to the following formula: 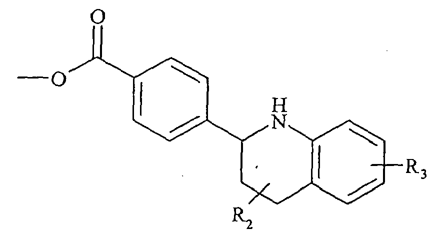
G corresponding to the following formula:
| | |
|---|---|
| 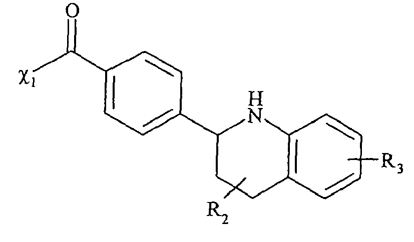 | χ₁ representing an -OH group, or an -OR_{g} group, R_{g} representing a linear or branched alkyl group, comprising from 1 to 20 carbon atoms. |
**c)** 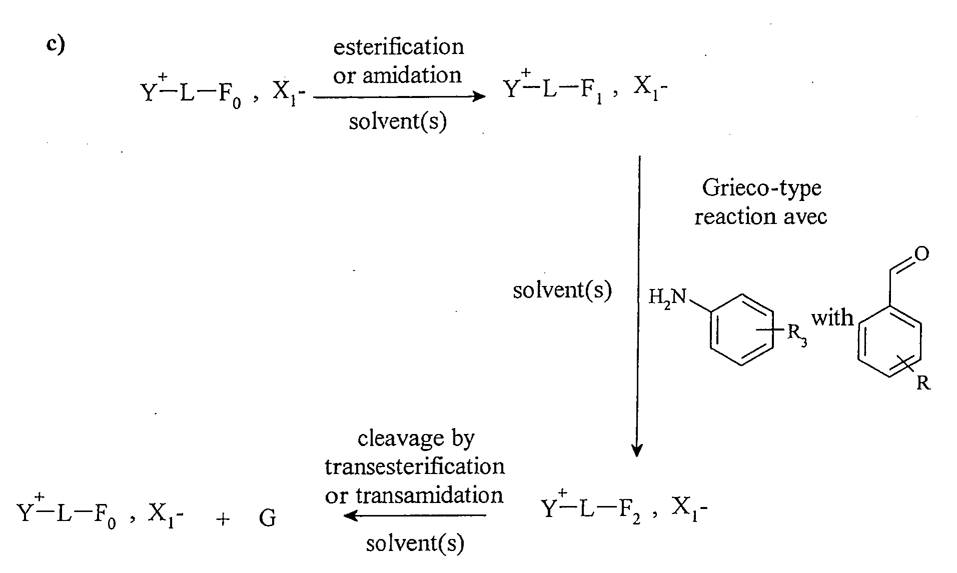 Y⁺- representing an onium cation as defined in one of claims 3 to 17, and preferably being a trimethylalkylammonium, triethylalkylammonium, tributylalkylphosphonium, N-methyl-N'-alkylimidazolium, N-alkylpyridinium, dimethylalkylsulphonium or diethylalkylsulphonium cation,
L representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
R representing a hydrogen atom or a functional group such as a nitro group in para position, a chlorine atom in para position or a methoxy group in ortho position, or a functional or non-functional alkyl group, comprising from 1 to 20 carbon atoms, or a functional or non-functional aryl group, comprising from 6 to 30 carbon atoms, or an aralkyl or alkaryl group, functional or non-functional, comprising from 7 to 50 carbon atoms,
R₃ representing a hydrogen atom, a linear or branched alkyl group, comprising from 1 to 20 carbon atoms or an aryl group comprising from 6 to 30 carbon atoms, or an aralkyl or alkaryl group, functional or non-functional, comprising from 7 to 50 carbon atoms, or a functional group in particular chosen from NO₂, CN, COOR, OR, COR, NHCOR, NRR', SO₂R, I, Br, R and R' representing independently of one another an alkyl group comprising from 1 to 20 carbon atoms or an aryl group comprising from 6 to 30 carbon atoms,
the functions F₀, F₁ and F₂ being as defined below:
- F₀ represents any function making it possible to attach and release a radical carrying an olefin, preferably an ester, or an amide.
- F₁ corresponds to one of the following general formulae:
| | |
|---|---|
| 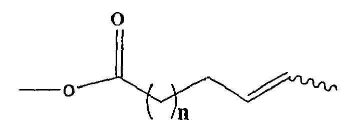 | n representing an integer varying from 1 to 10 |
| 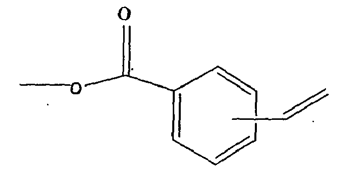 | |
- F₂ corresponds to one of the following general formulae: 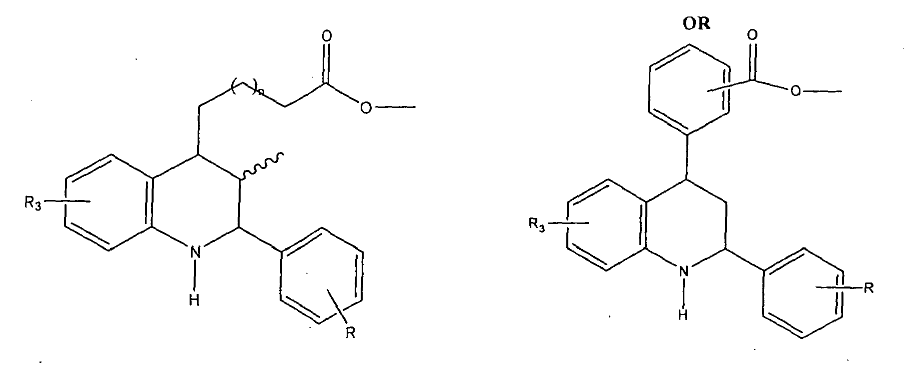
G corresponding to one of the following general formulae: 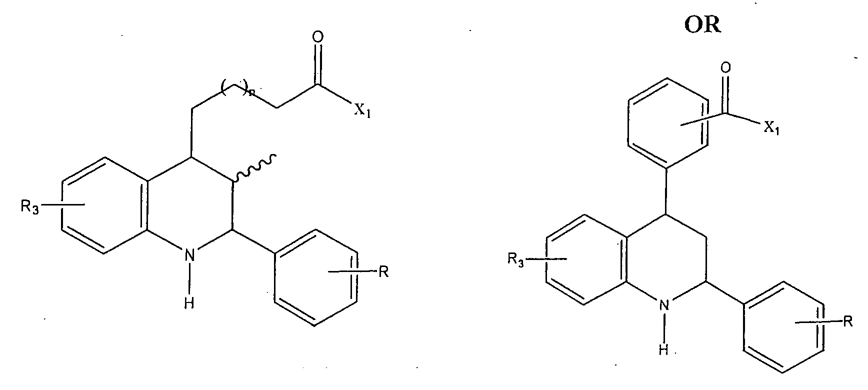 n, R and R₃ being as defined above, and
χ₁ representing an -OH group, or an -OR_{g} group, R_{g} representing a linear or branched alkyl group, comprising from 1 to 20 carbon atoms.

26. The use according to any one of claims 1 to 17, for the implementation of multi-component reactions, in particular for the synthesis of tetrasubstituted olefins, according to the following reaction diagram: 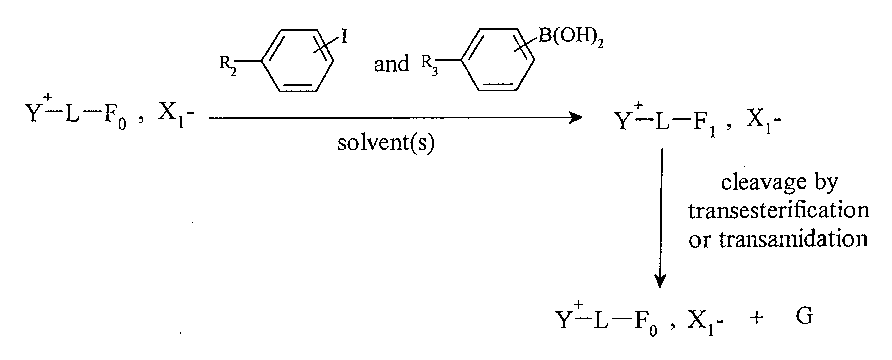 Y+- representing an onium cation as defined in one of claims 3 to 17, and preferably being a trimethylalkylammonium, triethylalkylammonium, tributylalkylphosphonium, N-methyl-N'-alkylimidazolium, N-alkylpyridinium, dimethylalkylsulphonium or diethylalkylsulphonium cation,
L representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 1 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
R₂ and R₃, preferably in para position, representing a hydrogen atom, a linear or branched, optionally functional alkyl group comprising from 1 to 30 carbon atoms, an optionally substituted and/or functional aryl group, comprising from 6 to 30 carbon atoms, a functional group, preferably a methoxy, mono-alkylamino, dialkylamino, arylamino, cyano, ester, nitro, ketone, sulphonyl, alkylthio, sulphoxide group,
the functions F₀ and F₁ being as defined below:
- F₀ corresponds to the following formula:
| | |
|---|---|
| 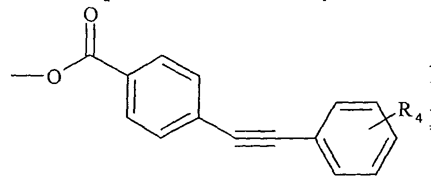 | R₄ representing a group as defined for R₂ and R₃ above, |
- F₁ corresponds to one of the following formulae: 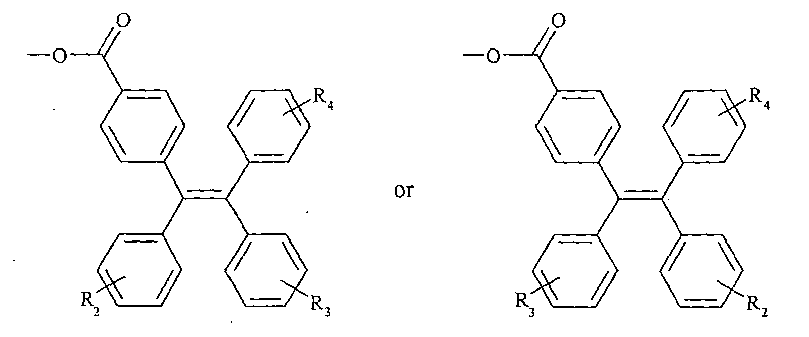 R₂, R₃ and R₄ being as defined above,
G corresponds to one of the following formulae: 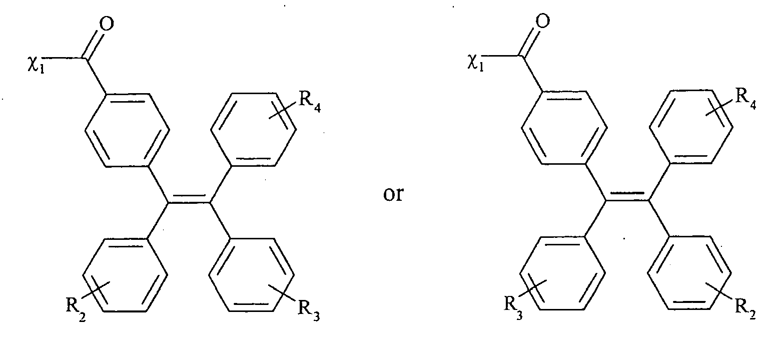 χ ₁ representing an -OH group, or an -OR_{g} group, R_{g} representing a linear or branched alkyl group, comprising from 1 to 20 carbon atoms.

27. The use according to any one of claims 1 to 17, for the implementation of cycloaddition reactions, preferably for the implementation of the Diels-Alder reaction, according to the following reaction diagram: 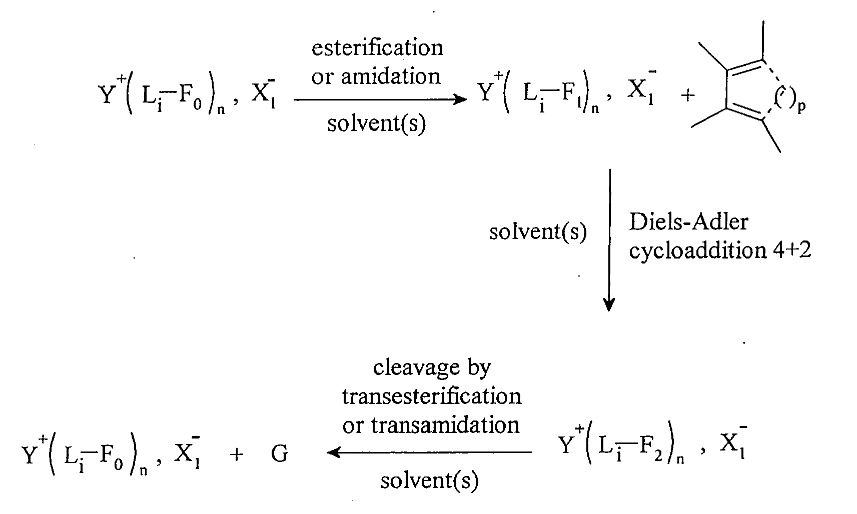 n being an integer varying from 2 to 4, as defined below,
i being an integer varying from 1 to n,
p being an integer varying from 0 to 2,
Y⁺ representing an onium cation as defined in one of claims 3 to 17, of formula (R_{b})ₓ₋ₙΛ⁺ in which x represents an integer equal to 3 or 4, n being equal to 2, 3 or 4 when x is equal to 4 and n being equal to 2 or 3 when x is equal to 3, R_{b} represents an alkyl group comprising from 1 to 20 carbon atoms, an aryl group comprising from 6 to 30 carbon atoms or an aralkyl or alkaryl group comprising from 6 to 30 carbon atoms, said abovementioned alkyl, aryl, aralkyl or alkaryl groups being non-functional, and in which Λ⁺ represents an ammonium, imidazolium, phosphonium or sulphonium cation, Y⁺ representing in particular an alkylammonium, alkylphosphonium or alkylsulphonium cation, and preferably being a tetraalkylammonium, tetraalkylphosphonium, dialkylimidazolium, trialkylsulphonium cation,
Lᵢ representing an arm, in particular a linear or branched alkyl group comprising from 1 to 20 carbon atoms, or an optionally functional aralkyl or alkaryl group, comprising from 6 to 30 carbon atoms, and preferably being a linear alkyl group, preferably a linear alkyl group of type (CH₂)ᵣ, r varying from 1 to 20, and preferably from 2 to 10, the arms Lᵢ being able to be identical or different,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
the functions F₀, F₁ and F₂ being as defined below:
- F₀ corresponds to a -χ₁H group, in which χ₁ represents an oxygen atom or an -NR_{f} group, R_{f} corresponding to a linear or branched alkyl group, comprising from 1 to 20 carbon atoms, or an aryl group comprising from 6 to 30 carbon atoms,
- F₁ corresponds to the following formula:
| | |
|---|---|
| 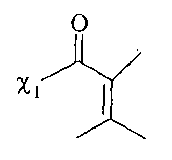 | χ₁ being as defined above, |
- F₂ corresponds to the following formula:
| | |
|---|---|
| 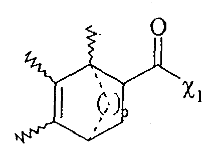 | χ₁ being as defined above, |
G corresponding to the following formula: 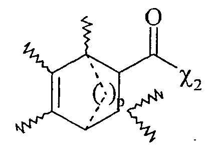 in which χ₂ represents either an OR_{g} group, R_{g} representing a hydrogen atom or an alkyl group comprising from 1 to 20 carbon atoms, or an -NRₕRᵤ group, Rₕ and Rᵤ representing independently of one another a hydrogen atom, an alkyl group comprising from 1 to 20 carbon atoms or an aryl group comprising from 6 to 30 carbon atoms.

28. The use according to claim 19, for the implementation of the Heck reaction, according to the following reaction diagram: 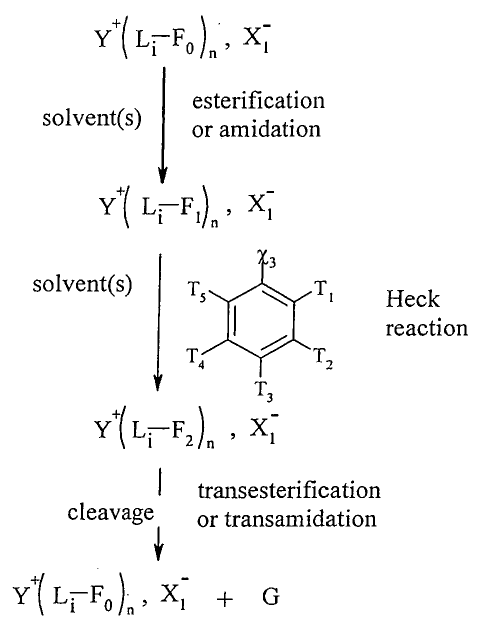 n being an integer varying from 2 to 4,
i being an integer varying from 1 to n,
Y⁺ representing an onium cation as defined in one of claims 3 to 17, of formula (R_{b})ₓ₋ₙΛ⁺ in which x represents an integer equal to 3 or 4, n being equal to 2, 3 or 4 when x is equal to 4 and n being equal to 2 or 3 when x is equal to 3, R_{b} represents an alkyl group comprising from 1 to 20 carbon atoms, an aryl group comprising from 6 to 30 carbon atoms or an aralkyl or alkaryl group comprising from 6 to 30 carbon atoms, said abovementioned alkyl, aryl, aralkyl or alkaryl groups being non-functional, and in which Λ⁺ represents an ammonium, imidazolium, phosphonium or sulphonium cation, Y⁺ representing in particular an alkylammonium, alkylphosphonium or alkylsulphonium cation, and preferably being a tetraalkylammonium, tetraalkylphosphonium, dialkylimidazolium, trialkylsulphonium cation,
Lᵢ representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 2 to 10, the arm Lᵢ being able to be identical or different,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
the functions F₀, F₁ and F₂ being as defined below:
- F₀ corresponds to a -χ₁H group, in which χ₁ represents an oxygen atom or an -NR_{f} group, R_{f} corresponding to a linear or branched alkyl group, comprising from 1 to 20 carbon atoms, or an aryl group comprising from 6 to 30 carbon atoms,
- F₁ corresponds to the following formula:
| | |
|---|---|
| 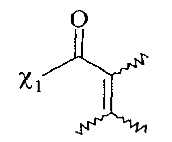 | χ₁ being as defined above, |
- F₂ corresponds to the following formula:
| | |
|---|---|
| 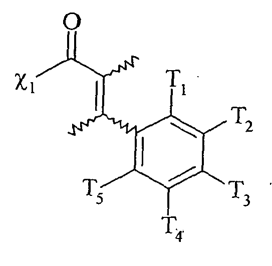 | χ₁ being as defined above, |
G corresponding to the following formula: 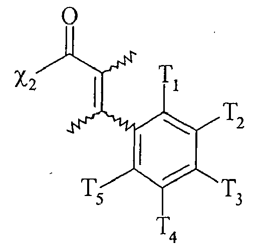 in which χ₂ represents either an -OR_{g} group, R_{g} representing a hydrogen atom or an alkyl group comprising from 1 to 20 carbon atoms, or an -NRₕRᵤ group, Rₕ and Rᵤ representing independently of one another a hydrogen atom, an alkyl group comprising from 1 to 20 carbon atoms or an aryl group comprising from 6 to 30 carbon atoms,
χ₃ representing a leaving group, in particular chosen from the I, Cl and Br halides, the mesylate, tosylate, triflate, sulphonate, sulphate or phosphate groups,
T₁, T₂, T₃, T₄ and T₅ representing independently of one another a hydrogen atom, a linear or branched alkyl group, comprising from 1 to 20 carbon atoms or an aryl group comprising from 6 to 30 carbon atoms, or a functional group in particular chosen from NO₂, CN, COOR, OR, COR, NHCOR, NRR", SO₂R, I, Br, R and R" representing independently of one another an alkyl group comprising from 1 to 20 carbon atoms or an aryl group comprising from 6 to 30 carbon atoms,
the entity 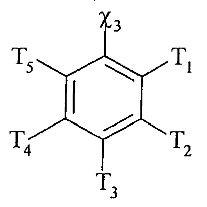 representing in particular the following groups:

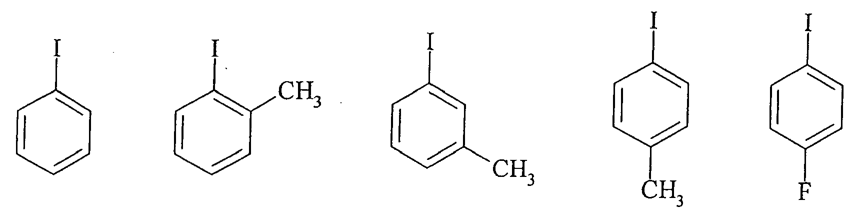

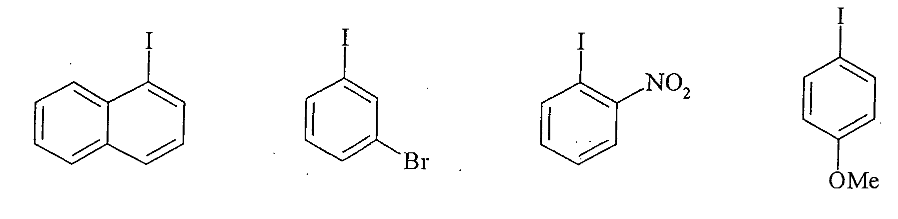

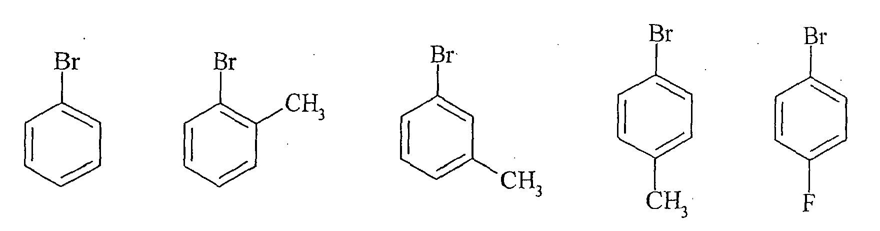

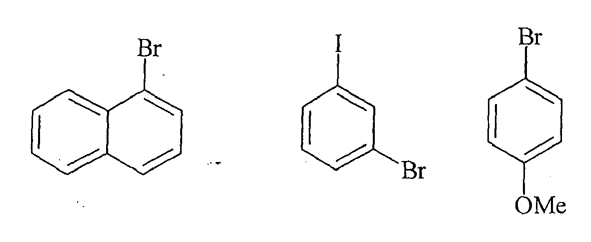

29. The use according to claim 19, for the implementation of Suzuki coupling, according to the following reaction diagram: 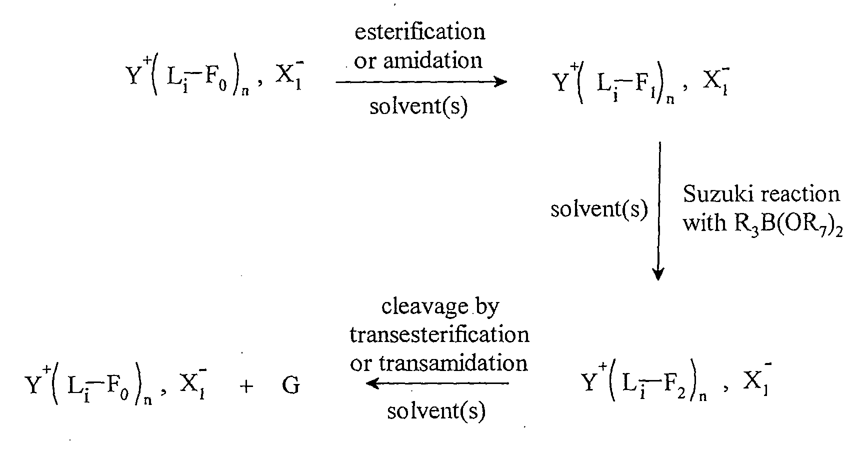 R₃ being chosen from the substituted or non-substituted aryl, heteroaryl, ethenyl, dienyl, allyl, ethynyl groups, comprising from 2 to 30 carbon atoms,
R₇ representing a hydrogen atom or a branched or linear alkyl group, or a cycloalkyl group comprising from 1 to 12 carbon atoms,
n being an integer varying from 2 to 4,
i being an integer varying from 1 to n,
Y⁺ representing an onium cation as defined in one of claims 3 to 17, of formula (R_{b})ₓ₋ₙΛ⁺ in which x represents an integer equal to 3 or 4, n being equal to 2, 3 or 4 when x is equal to 4 and n being equal to 2 or 3 when x is equal to 3, R_{b} represents an alkyl group comprising from 1 to 20 carbon atoms, an aryl group comprising from 6 to 30 carbon atoms or an aralkyl or alkaryl group comprising from 6 to 30 carbon atoms, said abovementioned alkyl, aryl, aralkyl or alkaryl groups being non-functional, and in which Λ⁺ represents an ammonium, imidazolium, phosphonium or sulphonium cation, Y⁺ representing in particular an alkylammonium, alkylphosphonium or alkylsulphonium cation, and preferably being a tetraalkylammonium, tetraalkylphosphonium, dialkylimidazolium, trialkylsulphonium cation,
Lᵢ representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 2 to 10, the arms Lᵢ being able to be identical or different,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms,
the solvent or solvents being chosen from: dichloromethane, tetrahydrofuran, dioxane, acetonitrile, dimethylfonnamide, dimethylacetamide, N-methylpyrrolidinone, propionitrile, acetone, toluene, chlorobenzene, nitrobenzene, dichlorobenzene, nitromethane, nitroethane, or a mixture of these solvents,
the functions F₀, F₁ and F₂ being as defined below:
- F₀ is in the form -χ₁H, χ₁ representing an oxygen atom or an -NR_{f} group, R_{f} corresponding to a linear or branched alkyl group, comprising from 1 to 20 carbon atoms, or an aryl group comprising from 6 to 30 carbon atoms,
- F₁ is in the form -Rₑ-χ, Rₑ representing an aromatic or heteroaromatic group comprising from 6 to 30 carbon atoms, χ representing a leaving group preferably chosen from Cl, Br, I, OTf, O-CO₂R⁵ or OSO₃-R⁵, R⁵ representing an alkyl group comprising from 1 to 10 carbon atoms or an aralkyl group comprising from 6 to 30 carbon atoms, F₁ preferably corresponding to the following formula: 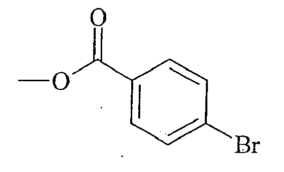
- F₂ is in the form Rₑ-R₂, Rₑ being as defined above and R₂ being chosen from the aryl, heteroaryl, ethenyl, dienyl, allyl, ethynyl groups, substituted or non-substituted, comprising from 2 to 30 carbon atoms, F₂ preferably corresponding to the following formula: 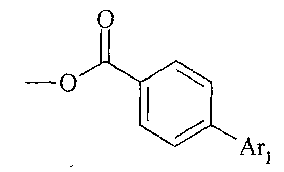 Ar₁ representing an aromatic group preferably chosen from: 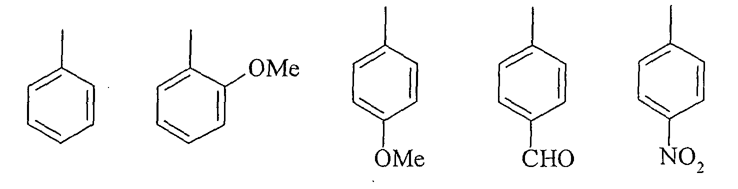 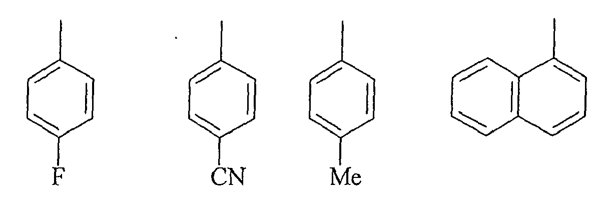
the molecule G being in the form R₂-R₃, R₂ and R₃ being as defined above, and corresponding in particular to the following formula: 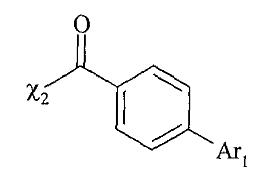 in which χ₂ represents either an -OR_{g} group, R_{g} representing a hydrogen atom or an alkyl group comprising from 1 to 20 carbon atoms, or an -NRₕRᵤ group, Rₕ and Rᵤ representing independently of one another a hydrogen atom, an alkyl group comprising from 1 to 20 carbon atoms or an aryl group comprising from 6 to 30 carbon atoms,
Ar₁ is as defined above.

30. The use according to claim 19, for the implementation of the Heck reaction, according to the following reaction diagram: 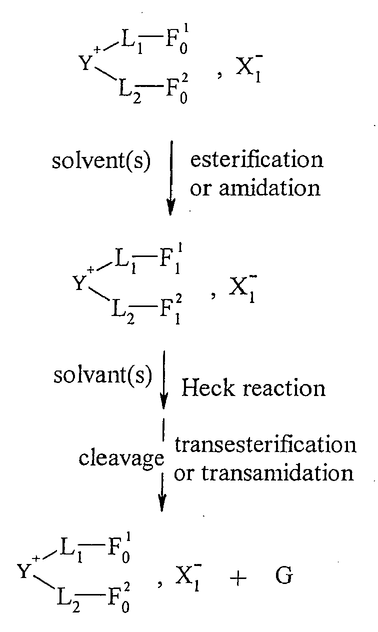 Y⁺ representing an onium cation as defined in one of claims 3 to 17, of formula (R_{b})ₓ₋₂Λ⁺ in which x represents an integer equal to 3 or 4, R_{b} represents an alkyl group comprising from 1 to 20 carbon atoms, an aryl group comprising from 6 to 30 carbon atoms or an aralkyl or alkaryl group comprising from 6 to 30 carbon atoms, said abovementioned alkyl, aryl, aralkyl or alkaryl groups being non-functional, and in which Λ⁺ represents an ammonium, imidazolium, phosphonium or sulphonium cation, Y⁺ representing in particular an alkylammonium, alkylphosphonium or alkylsulphonium cation, and preferably being a tetraalkylammonium, tetraalkylphosphonium, dialkylimidazolium, trialkylsulphonium cation, Λ⁺ representing an ammonium or phosphonium cation when x = 4 and a sulphonium cation when x = 3,
L₁ and L₂, identical or different, representing an arm, in particular a linear or branched alkyl group comprising from 1 to 20 carbon atoms, or an optionally functional aralkyl or alkaryl group, comprising from 1 to 20 carbon atoms, and preferably being a linear alkyl group, preferably a linear alkyl group of type (CH₂)ᵣ, r varying from 1 to 20, and preferably from 2 to 10,
X₁⁻ being as defined in one of claims 1 to 17, and being in particular Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻, R₁ representing an alkyl group comprising from 1 to 20 carbon atoms, the solvent or solvents being chosen from: dichloromethane, tetrahydrofuran, dioxane, acetonitrile, dimethylformamide, dimethylacetamide, N-methylpyrrolidinone, propionitrile, acetone, toluene, chlorobenzene, nitrobenzene, dichlorobenzene, nitromethane, nitroethane, or a mixture of these solvents,
the functions F₀¹, F₁¹, F₀² and F₁² being as defined below:
- F₀¹ corresponds to a -χ¹₁H group, in which χ¹₁ represents an oxygen atom or an -NR_{f} group, R_{f} corresponding to a linear or branched alkyl group, comprising from 1 to 20 carbon atoms, or an aryl group comprising from 6 to 30 carbon atoms,
- F₀² corresponds to a -χ²₁H group, in which χ²₁ represents an oxygen atom or an -NR_{f} group, R_{f} corresponding to a linear or branched alkyl group, comprising from 1 to 20 carbon atoms, or an aryl group comprising from 6 to 30 carbon atoms,
- F₁¹ corresponds to the following formula:
| | |
|---|---|
| 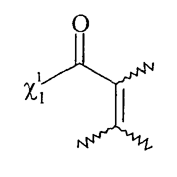 | χ¹₁ being as defined above, |
- F₁² corresponds to the following formula:
| | |
|---|---|
| 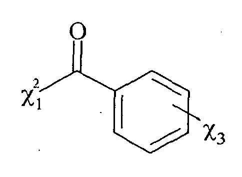 | χ²₁ being as defined above, and |
| | χ₃ representing a leaving group, in particular chosen from the I, Cl and Br halides, the mesylate, tosylate, triflate, sulphonate, sulphate or phosphate groups, |
G corresponding to the following formula:
| | |
|---|---|
| 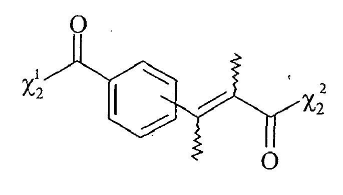 | in which χ₂¹ and χ₂², identical or different, represent either an -OR_{g} group, R_{g} representing a hydrogen atom or an alkyl group comprising from 1 to 20 carbon atoms, or an -NRₕRᵤ group, Rₕ and Rᵤ representing independently of one another a hydrogen atom, an alkyl group comprising from 1 to 20 carbon atoms or an aryl group comprising from 6 to 30 carbon atoms. |

## Patentansprüche

1. Verwendung eines Oniumsalzes, das durch mindestens eine organische Funktion funktionalisiert wurde, als löslichen Träger im Beisein mindestens eines organischen Lösungsmittels für die organische Synthese eines Moleküls in homogener Phase, wobei die organische Synthese mindestens eine Umwandlung der organischen Funktion, gefolgt von einer Spaltungsreaktion, die das synthetisierte Molekül in Lösung in dem Lösungsmittel und das Oniumsalz in seiner ursprünglichen Form, d.h. vor der Umwandlung der organischen Funktion, freisetzt,
wobei das Oniumsalz in flüssiger oder fester Form bei Raumtemperatur vorhanden ist und der Formel A₁⁺, X₁⁻ entspricht,
wobei:
- A₁⁺ ein Kation darstellt,
- X₁⁻ ein Anion darstellt,
wobei
A₁⁺ ein funktionelles oder polyfunktionelles Kation ist, und/oder
X₁⁻ ein funktionelles oder polyfunktionelles Anion ist,
wobei das Oniumsalz derart ist, dass in seiner ursprünglichen Form, d.h. vor der ersten Umwandlung der organischen Funktion, A₁⁺ und X₁⁻ nicht durch eine kovalente Verbindung miteinander verbunden sind,
und wenn das Anion und das Kation jeweils eine organische Funktion tragen, diese nicht miteinander vor der ersten Umwandlung der organischen Funktion reagieren können.

2. Verwendung nach Anspruch 1, **dadurch gekennzeichnet, dass** das Oniumsalz nach der Freisetzung des synthetisierten Moleküls gereinigt und/oder in seine ursprüngliche Form rezykliert wird.

3. Verwendung nach Anspruch 1 oder 2, **dadurch gekennzeichnet, dass** die funktionellen Kationen und Anionen einer ionischen Einheit entsprechen, einer kationischen Y⁺- und einer anionischen Z⁻-, die eventuell über einen Arm L bzw. M insbesondere eine Alkyl- oder Aralkyl- oder Alkarylgruppe mit 1 bis 30 Kohlenstoffatomen mit mindestens einer Funktion Fᵢ und F'ᵢ verbunden ist, wobei Fᵢ von F₀ bis Fₙ variiert, wobei F'ᵢ von F'₀ bis F'ₙ variiert, wobei n eine ganze Zahl zwischen 1 und 10 ist,
wobei das funktionelle Kation A₁⁺ in der Form Y⁺-L-Fᵢ dargestellt werden kann, und
das funktionelle Anion X₁⁻ in der Form Z⁻-(M)ₖ-F'ᵢ dargestellt werden kann, wobei k gleich 0 oder 1 ist.

4. Verwendung nach einem der Ansprüche 1 bis 3, **dadurch gekennzeichnet, dass** die organischen Funktionen Fᵢ und F'ᵢ unter den herkömmlichen Funktionen der organischen Chemie ausgewählt werden, wie beispielsweise den Hydroxyl-, Carboxylsäure-, Amid-, Sulfon-, Primäramin-, Sekundäramin-, Aldehyd-, Keton-, Ethenyl-, Ethynyl-, Dienyl-, Ether-, Epoxyd-, Phosphin- (primär, sekundär oder tertiär), Azotur-, Imin-, Keten-, Kumulen-, Heterokumulen-, Thiol-, Thioether-, Sulfoxyd-, Phosphorgruppen-, Heterozyklen-, Sulfosäure-, Silan-, Stannan- oder funktionellen Arylfunktionen.

5. Verwendung nach einem der Ansprüche 1 bis 4, **dadurch gekennzeichnet, dass** das Molekulargewicht des funktionalisierten Oniumsalzes geringer als 1500 g.mol⁻¹, insbesondere geringer als 750 g.mol⁻¹ ist und vorzugsweise zwischen 130 und 500 g.mol⁻¹ liegt.

6. Verwendung nach einem der Ansprüche 1 bis 5, **dadurch gekennzeichnet, dass** A₁⁺ ein funktionelles Kation ist, und dass X₁⁻ ein nicht funktionelles Anion ist.

7. Verwendung nach Anspruch 6, bei der das Oniumsalz A₁⁺, X₁⁻ als ursprüngliche Form Y⁺-L-F₀, X₁⁻ zur Gewinnung eines Moleküls G durch Umwandlung der ursprünglichen Funktion F₀ nach folgendem Schema hat: 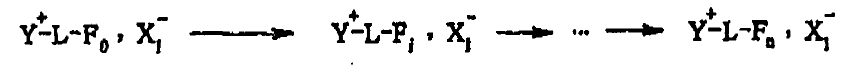 wobei L wie in Anspruch 3 definiert ist,
wobei das Molekül G durch Spaltung der Funktion Fₙ erhalten wird,
und das funktionalisierte Oniumsalz in seiner ursprünglichen Form Y⁺-L-F₀, X₁⁻ nach Freisetzung von G wiedergewonnen oder rezykliert werden kann.

8. Verwendung nach einem der Ansprüche 1 bis 7, **dadurch gekennzeichnet, dass** das funktionelle Kation A₁⁺ unter den Kationen Pyridinium, Imidazolium, Ammonium, Phosphonium oder Sulfonium, zyklisch oder nicht, substituiert oder nicht, und vorzugsweise Ammonium oder Phosphonium ausgewählt wird.

9. Verwendung nach Anspruch 8, **dadurch gekennzeichnet, dass** das funktionelle Kation A₁⁺ unter den quaternären Ammoniumkationen, zyklisch oder nicht, ausgewählt wird.

10. Verwendung nach einem der Ansprüche 1 bis 5, **dadurch gekennzeichnet, dass** X₁⁻ ein funktionelles Anion und A₁⁺ ein nicht funktionelles Kation ist.

11. Verwendung nach Anspruch 10, bei der das Oniumsalz A₁⁺, X₁⁻ als ursprüngliche Form A₁⁺, Z⁻(M)ₖ- F'₀ für die Gewinnung eines Moleküls G durch Umwandlung der ursprünglichen Funktion F'₀ nach folgendem Schema hat: 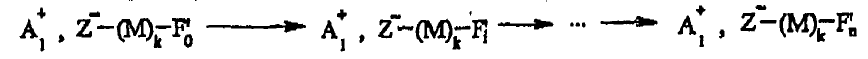 wobei k und M wie in Anspruch 3 definiert sind,
wobei das Molekül G durch Spaltung der Funktion F'ₙ erhalten wird,
und das funktionalisierte Oniumsalz in seiner ursprünglichen Form A₁⁺, Z⁻(M)ₖ- F'₀ nach Freisetzung von G wiedergewonnen oder rezykliert werden kann.

12. Verwendung nach Anspruch 10 oder 11, **dadurch gekennzeichnet, dass** X₁⁻ ausgewählt wird unter:
- der Familie der Phosphate: P₁PO₄²⁻, R₁R₂PO₄⁻
- der Familie der Sulfate: P₁SO₄⁻
- der Familie der Sulfonate: R₁SO₃⁻
- der Familie der Carboxylate: R₁CO₂⁻
oder unter den folgenden Anionen:
**R₁N̅SO₂R₂**
**R₂SO₂N̅SO₂R₂** 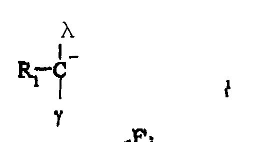 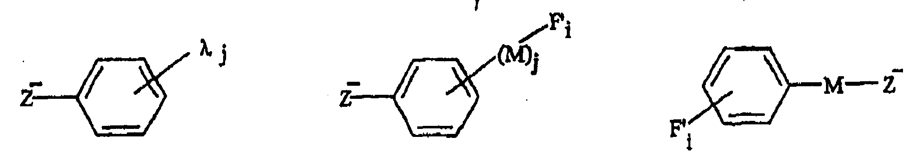
wobei Z⁻, M und F'ᵢ wie in Anspruch 3 definiert sind,
wobei Z⁻ insbesondere O⁻, SO₃⁻, CO₂⁻, R₁PO₃⁻ oder R₁PO₂⁻ darstellt,
wobei j eine ganze Zahl zwischen 1 und 5 darstellt,
wobei R₁ und R₂ unabhängig voneinander eine funktionelle Alkylgruppe, eine Vinyl- oder Alcynylgruppe, eventuell funktionell, umfassend 1 bis 20 Kohlenstoffatome darstellen können oder eine funktionelle Arylgruppe darstellen können, umfassend 6 bis 30 Kohlenstoffatome,
wobei γ und λ eine elektrisch anziehende Gruppe darstellen, die insbesondere ausgewählt wird unter den Gruppen: CO₂R', SO₂R', CN, NO₂, P(O)(OR')₂, C(O)R' und SO₃R',
wobei R' eine Alkylgruppe, eventuell funktionell, umfassend 1 bis 20 Kohlenstoffatome oder eine Arylgruppe, eventuell funktionell, umfassend 6 bis 30 Kohlenstoffatome darstellt.

13. Verwendung nach einem der Ansprüche 1 bis 5, **dadurch gekennzeichnet, dass** A₁⁺ ein funktionelles Kation und X₁⁻ ein funktionelles Anion ist.

14. Verwendung nach Ansprüche 13, bei der das Oniumsalz A₁⁺, X₁⁻ als ursprüngliche Form Y⁺-L-F₀, Z⁻-(M)ₖ-F'₀ zur Gewinnung eines Moleküls G durch Umwandlung der ursprünglichen Funktionen F₀ und F'₀ nach folgendem Schema hat: 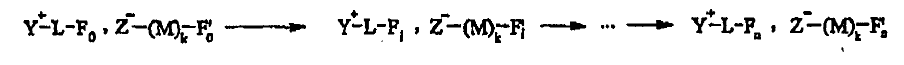 wobei L, k und M wie in Anspruch 3 definiert sind, und durch Reaktion von Fₙ auf F'ₙ im funktionalisierten Oniumsalz 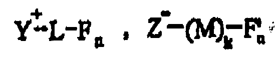 zur Bildung eines internen Salzes mit folgender Formel führen: 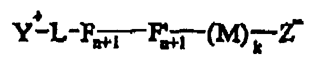 wobei das Molekül G durch Spaltung des oben erwähnten internen Salzes entsprechend der Formel Fₙ₊₂-F'ₙ₊₂ erhalten wird,
und das funktionalisierte Oniumsalz in seiner ursprünglichen Form Y⁺-L-F₀, Z⁻-(M)ₖ-F'₀ nach Freisetzung von G wiedergewonnen oder rezykliert werden kann.

15. Verwendung nach einem der Ansprüche 1 bis 14, **dadurch gekennzeichnet, dass** das Oniumsalz unter den folgenden Salzen ausgewählt wird: 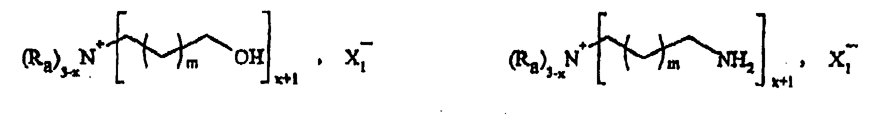 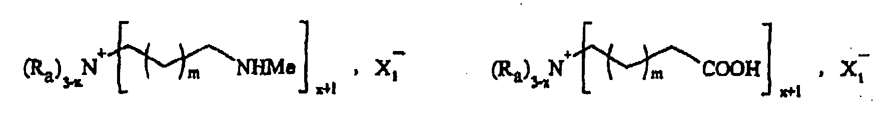 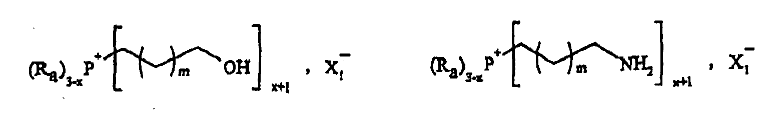 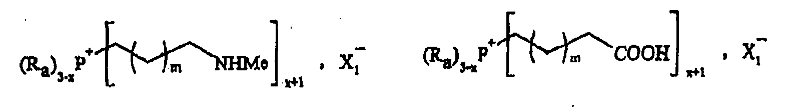 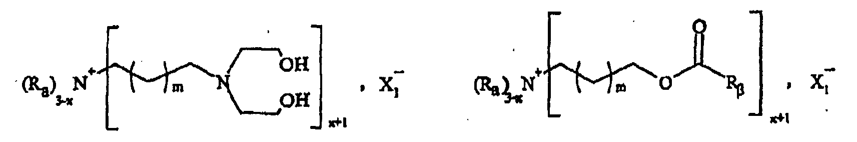 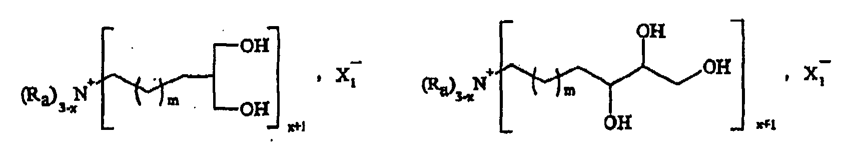 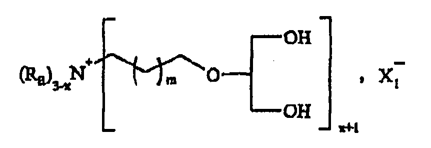 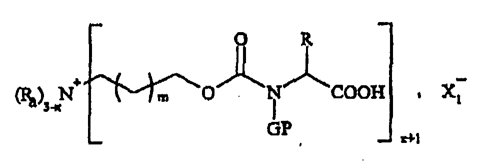 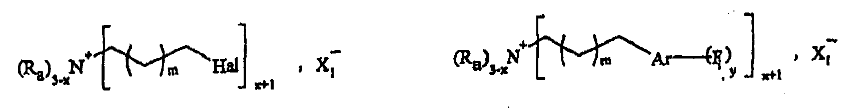 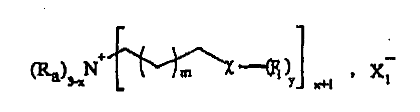 wobei R ein Wasserstoffatom, eine Alkylgruppe, funktionell oder nicht, umfassend 1 bis 20 Kohlenstoffatome, oder eine Arylgruppe, funktionell oder nicht, umfassend 6 bis 30 Kohlenstoffatome, darstellt,
wobei x eine ganze Zahl zwischen 0 und 3 darstellt,
wobei y eine ganze Zahl zwischen 1 und 5 darstellt,
wobei Ar einen funktionellen oder polyfunktionellen aromatischen Kern darstellt,
wobei Fᵢ wie in Anspruch 4 definiert ist,
wobei Hal ein Halogenatom darstellt, das insbesondere unter Chlor, Brom und Jod ausgewählt wird,
wobei X einen Carbozyklus oder einen funktionellen Heterozyklus darstellt,
wobei X₁⁻ ausgewählt wird unter:
**NTf₂⁻, PF₆⁻, BF₄⁻, Cl⁻, Br⁻, I⁻, CF₃SO₃⁻, MeSO₄⁻, EtSO₄⁻, MeSO₃⁻, C₆H₅SO₃⁻, pMeC₆H₄SO₃⁻,**
wobei m eine ganze Zahl zwischen 0 und 20 ist,
wobei R_{β} eine Dienyl-, Vinylgruppe, substituiert oder nicht, eine funktionelle Alkylgruppe, umfassend 1 bis 20 Kohlenstoffatome, oder eine funktionelle Arylgruppe, umfassend 6 bis 30 Kohlenstoffatome, eine Alcynylgruppe, substituiert oder nicht, darstellt und insbesondere eine Alkylvinyl-, Alkylalcynyl-, Alkylaryl-, Alkyldienyl-, Alkylmalonyl-, Acylgruppe darstellt,
und Rₐ eine Alkylgruppe, verzweigt oder nicht, umfassend 1 bis 20 Kohlenstoffatome, insbesondere eine Ethyl-, Propyl-, Butyl-, Pentyl-, Hexyl-, Heptyl- oder Oktylgruppe darstellt.

16. Verwendung nach einem der Ansprüche 1 bis 15, **dadurch gekennzeichnet, dass** das oder die verwendeten Lösungsmittel ein aprotisches Lösungsmittel sind, das ausgewählt wird unter:
- den Lösungsmitteln, deren Dielektrizitätskonstante ε kleiner oder gleich 2 ist, wie den Alkanen, den aromatischen Karbiden, wie Benzol, Toluol oder Xylol,
- den Lösungsmitteln, deren Dielektrizitätskonstante ε zwischen ungefähr 2 und 15 beträgt, wie den Ether, Halogenbenzolen oder Dichlormethanen, und
- den Lösungsmitteln, deren Dielektrizitätskonstante ε größer als 15 ist, wie Azetonitril, Nitromethan, DMF oder Dimethylazetamid.

17. Verwendung nach einem der Ansprüche 1 bis 16 für die kontinuierliche, diskontinuierliche, kombinatorische oder parallele organische Synthese und/oder für die Herstellung von Produktbanken.

18. Verwendung nach einem der Ansprüche 1 bis 17 für den Einsatz von Zykloadditions-Reaktionen, vorzugsweise für den Einsatz der Diels-Alder-Reaktion, nach einem der folgenden Reaktionsschemata:
a) 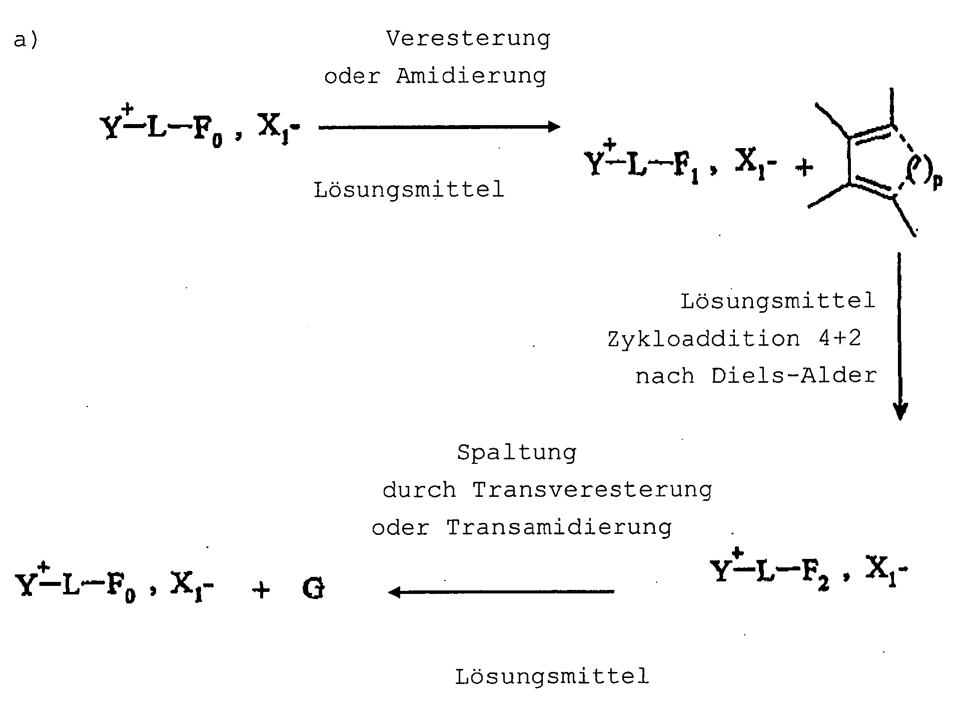 wobei p eine ganze Zahl zwischen 0 und 2 ist,
wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methylimidazolium- oder Pyridinium-Kation darstellt,
wobei L einen Arm darstellt, insbesondere eine lineare oder verzweigte Alkylgruppe, umfassend 1 bis 20 Kohlenstoffatome, oder eine eventuell funktionelle Aralkyl- oder Alkarylgruppe, umfassend 6 bis 30 Kohlenstoffatome, vorzugsweise eine lineare Alkylgruppe, vorzugsweise eine lineare Alkylgruppe des Typs (CH₂)ᵣ, wobei r zwischen 1 und 20, vorzugsweise zwischen 2 und 10, variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻_{,} R₁SO₃⁻, FSO₃⁻, PO₄³⁻,** wobei R₁ eine Alkylgruppe darstellt, umfassend 1 bis 20 Kohlenstoffatome, wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei die Funktionen F₀, F₁ und F₂ wie unten definiert sind:
- F₀ entspricht einer Gruppe -χ₁H, bei der χ₁ ein Sauerstoffatom darstellt, oder eine Gruppe -NR_{f}, wobei R_{f} einer linearen oder verzweigten Alkylgruppe, umfassend 1 bis 20 Kohlenstoffatome, oder einer Arylgruppe entspricht, umfassend 6 bis 30 Kohlenstoffatome,
- F₁ entspricht der folgenden Formel: 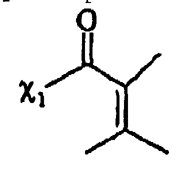 wobei χ₁ wie oben definiert ist
- F₂ entspricht der folgenden Formel: 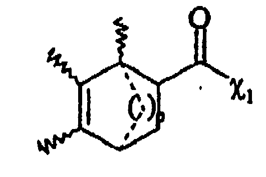 wobei χ₁ wie oben definiert ist
wobei G der folgenden Formel entspricht: 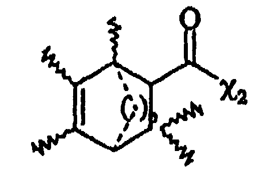 wobei χ₂ entweder eine Gruppe OR_{g} darstellt, wobei R_{g} ein Wasserstoffatom oder eine Alkylgruppe, umfassend 1 bis 20 Kohlenstoffatome, darstellt, oder eine Gruppe - NRₕRᵤ darstellt, wobei Rₕ und Rᵤ unabhängig voneinander ein Wasserstoffatom, eine Alkylgruppe, umfassend 1 bis 20 Kohlenstoffatome, oder eine Arylgruppe darstellt, umfassend 6 bis 30 Kohlenstoffatome,
b) 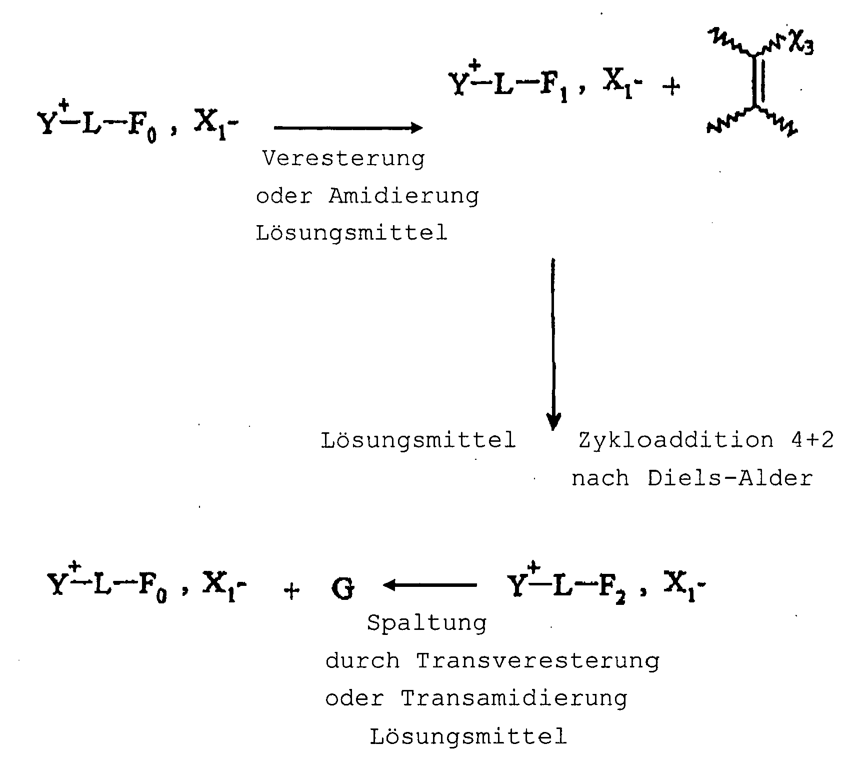 wobei Y⁺-, L und X₁⁻ wie vorher definiert sind,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei die Funktionen F₀, F₁ und F₂ wie unten definiert sind:
- F₀ stellt jede Funktion dar, die es ermöglicht, ein Dien-1,3 anzuheften, und wird insbesondere unter den Carbonyl-, Amin-, Alcoxy-, Silan-, Stannan- und Boranfunktionen, umfassend 1 bis 20 Kohlenstoffatome, ausgewählt,
- F₁ entspricht der folgenden Formel: 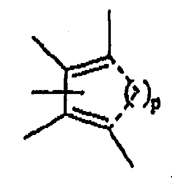 wobei p eine ganze Zahl zwischen 0 und 2 ist
- F₂ entspricht der folgenden Formel: 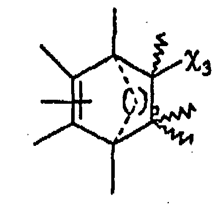 wobei χ₃ eine elektrisch anziehende Gruppe darstellt, die insbesondere unter den Zyan-, Alkoxycarbonylgruppen, umfassend 1 bis 20 Kohlenstoffatome, den Acylgruppen, umfassend 2 bis 20 Kohlenstoffatome, den Benzoyl-, Sulfonyl-, Dialkoxyphosphonylgruppen, umfassend 1 bis 10 Kohlenstoffatome, ausgewählt wird,
wobei G der folgenden Formel entspricht: 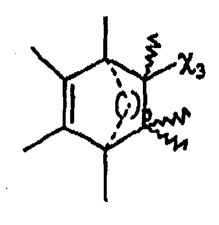 wobei χ₃ wie oben definiert ist.
c) 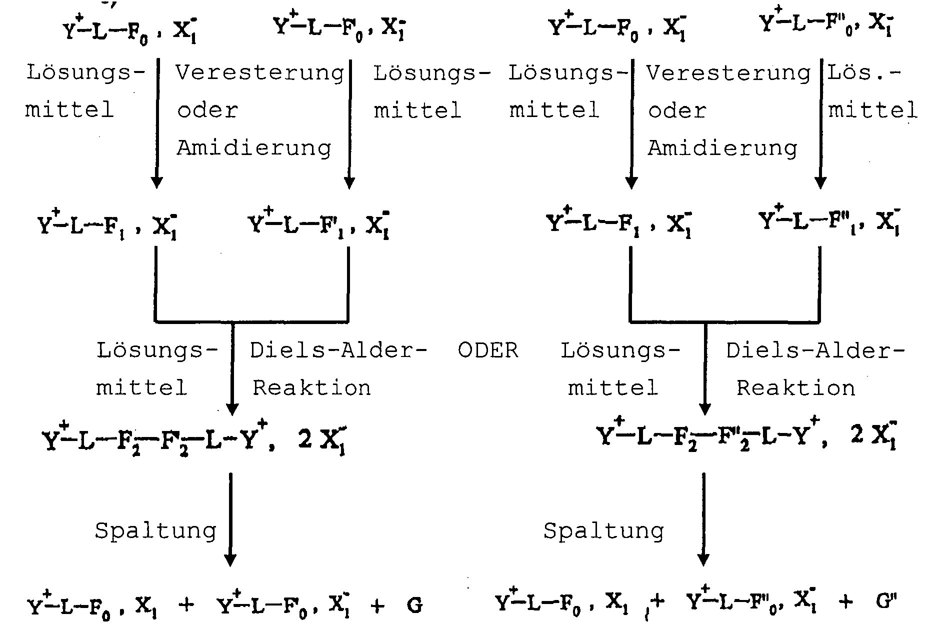 wobei Y⁺-, L und X₁⁻ wie vorher definiert sind,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei die Funktionen F_{0,} F'_{0,} F"_{0,} F_{1,} F'_{1,} F"_{1,} F_{2,} F'₂ und F"₂ wie unten definiert sind:
- F₀ und F'₀ entsprechen einer Gruppe -χ₁H bzw. -χ'₁H, wobei χ₁ und χ'₁ identisch oder unterschiedlich sind, ein Sauerstoffatom oder eine Gruppe -NR_{f} aufweisen, wobei R_{f} einer linearen oder verzweigten Alkylgruppe, umfassend 1 bis 20 Kohlenstoffatome, oder einer Arylgruppe, umfassend 6 bis 30 Kohlenstoffatome, entspricht,
- F"₀ entspricht einer Funktion -COOH;
- F₁ entspricht der folgenden Formel: 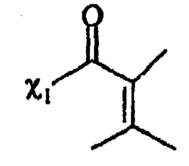 wobei χ₁ wie oben definiert ist,
- F'₁ entspricht der folgenden Formel: 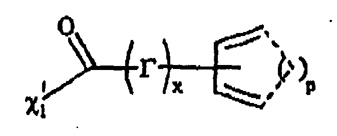 wobei p eine ganze Zahl zwischen 0 und 2 ist,
wobei χ'₁ wie oben definiert ist,
wobei x gleich 0 der 1 ist,
wobei Γ́ eine Alkylkette, umfassend 1 bis 30
Kohlenstoffatome, eine Alkaryl-, Aralkyl-, Arylkette darstellt, umfassend 6 bis 30 Kohlenstoffatome,
- F"₁ entspricht der folgenden Formel: 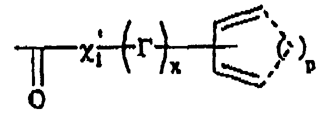 wobei p, x und Γ́ wie oben definiert sind,
wobei χ'₁ wie oben definiert ist,
- F₂-F'₂ entspricht folgender Formel: 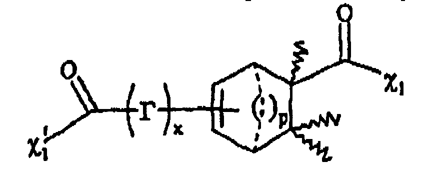 wobei p, χ₁, χ'₁, x und Γ́ wie oben definiert sind,
- F₂-F"₂ entspricht folgender Formel: 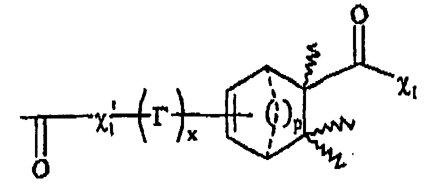 wobei p, χ₁, χ'₁, x und Γ́ wie oben definiert sind,
- G entspricht folgender Formel: 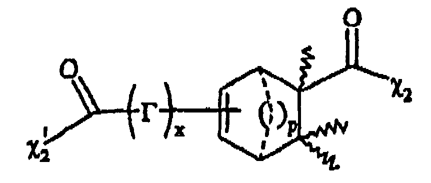
- G" entspricht folgender Formel: 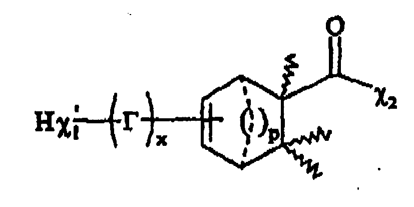 wobei χ₂ und χ'₂ identisch oder unterschiedlich sind, entweder eine Gruppe ORg aufweisen, wobei Rg ein Wasserstoffatom oder eine Alkylgruppe, umfassend 1 bis 20 Kohlenstoffatome, oder eine Gruppe -NRₕRᵤ darstellen, wobei Rₕ und Rᵤ unabhängig voneinander ein Wasserstoffatom, eine Alkylgruppe, umfassend 1 bis 20 Kohlenstoffatome, oder eine Arylgruppe, umfassend 6 bis 30 Kohlenstoffatome, darstellen.

19. Verwendung nach einem der Ansprüche 1 bis 17 für den Einsatz von Kopplungsreaktionen, wie der Reaktionen Heck, Suzuki, Sonogashira oder Ullmann.

20. Verwendung nach Anspruch 19 für den Einsatz der Heck-Reaktion nach einem der folgenden Reaktionsschemata: 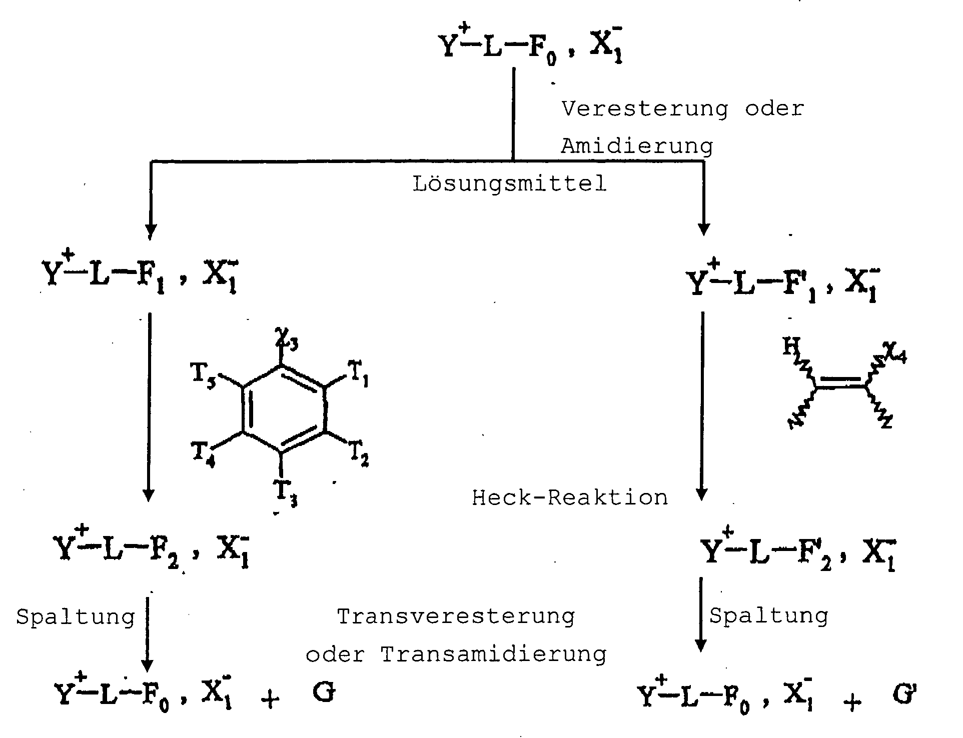 wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methylimidazolium- oder Pyridinium-Kation ist,
wobei L einen Arm darstellt, insbesondere eine lineare oder verzweigte Alkylgruppe, umfassend 1 bis 20 Kohlenstoffatome, oder eine eventuell funktionelle Aralkyl- oder Alkarylgruppe, umfassend 1 bis 20 Kohlenstoffatome, und vorzugsweise eine lineare Alkylgruppe, vorzugsweise eine lineare Alkylgruppe des Typs (CH₂)ᵣ ist, wobei r von 1 bis 20 und vorzugsweise von 2 bis 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe darstellt, umfassend 1 bis 20 Kohlenstoffatome, wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei die Funktionen F_{0,} F_{1,} F'_{1,} F₂ und F'₂ wie unten definiert sind:
- F₀ entspricht einer Gruppe -χ₁H, wobei χ₁ ein Sauerstoffatom oder eine Gruppe -NR_{f} darstellt, wobei R_{f} einer linearen oder verzweigten Alkylgruppe, umfassend 1 bis 20 Kohlenstoffatome, oder einer Arylgruppe, umfassend 6 bis 30 Kohlenstoffatome, entspricht,
- F₁ entspricht der folgenden Formel: 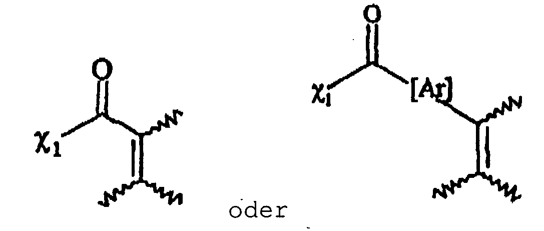 wobei χ₁ wie oben definiert ist,
wobei [Ar] einen aromatischen Kern darstellt, der eventuell durch eine lineare oder verzweigte Alkylgruppe, umfassend 1 bis 20 Kohlenstoffatome, oder eine Arylgruppe, umfassend 6 bis 30 Kohlenstoffatome, oder eine funktionelle Gruppe substituiert ist, die insbesondere unter NO₂, CN, COOR, OR, COR, NHCOR, NRR', SO₂R, I, Br, R und R' ausgewählt wird, die unabhängig voneinander eine Alkylgruppe, umfassend 1 bis 20 Kohlenstoffatome, oder eine Arylgruppe, umfassend 6 bis 30 Kohlenstoffatome, darstellen, wobei [Ar] vorzugsweise folgender Formel entspricht: 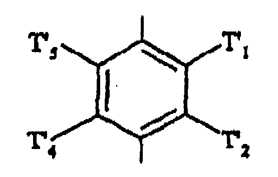 wobei T'₁, T'₂, T'₄ und T'₅ unabhängig voneinander ein Wasserstoffatom, eine lineare oder verzweigte Alkylgruppe, umfassend 1 bis 20 Kohlenstoffatome, oder eine Arylgruppe, umfassend 6 bis 30 Kohlenstoffatome, oder eine funktionelle Gruppe darstellen, die insbesondere unter NO₂, CN, COOR, OR, COR, NHCOR, NRR', SO₂R, I, Br, R und R' ausgewählt wird, die unabhängig voneinander eine Alkylgruppe, umfassend 1 bis 20 Kohlenstoffatome, oder eine Arylgruppe, umfassend 6 bis 30 Kohlenstoffatome, darstellen,
- F₂ entspricht einer der folgenden Formeln: 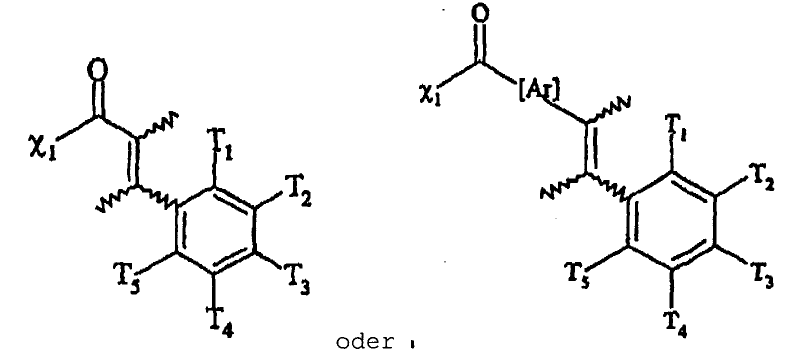 wobei χ₁ und Ar wie oben definiert sind,
wobei T₁, T₂, T₃, T₄ und T₅ der oben für T'₁, T'₂, T'₄ und T'₅ gegebenen Definition entsprechen,
wobei G einer der folgenden Formeln entspricht: 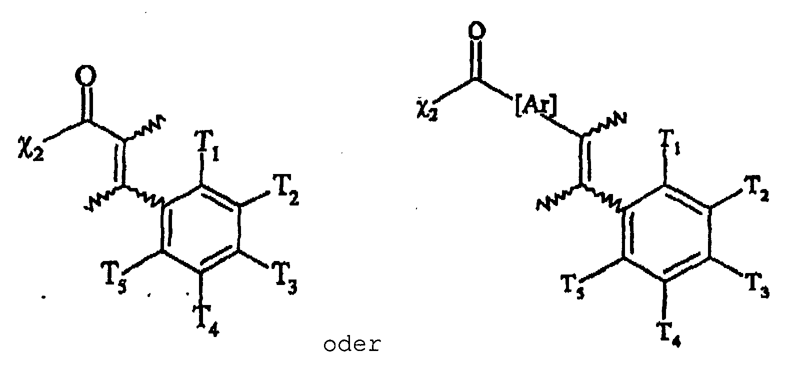 wobei χ₂ entweder eine Gruppe -OR_{g} darstellt, wobei Rg ein Wasserstoffatom oder eine Alkylgruppe, umfassend 1 bis 20 Kohlenstoffatome, oder eine Gruppe -NRₕRᵤ darstellt, wobei Rₕ und Rᵤ unabhängig voneinander ein Wasserstoffatom, eine Alkylgruppe, umfassend 1 bis 20 Kohlenstoffatome, oder eine Arylgruppe, umfassend 6 bis 30 Kohlenstoffatome, darstellen,
wobei χ₃ eine weggehende Gruppe darstellt, die insbesondere unter den Halogeniden I, Cl und Br, den Mesylat-, Tosylat-, Triflat-, Sulfonat-, Sulfat- oder Phosphatgruppen ausgewählt wird,
wobei die Einheit 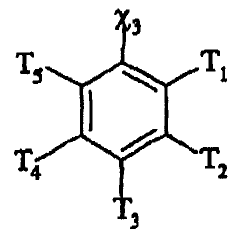 insbesondere die folgenden Gruppen darstellt: 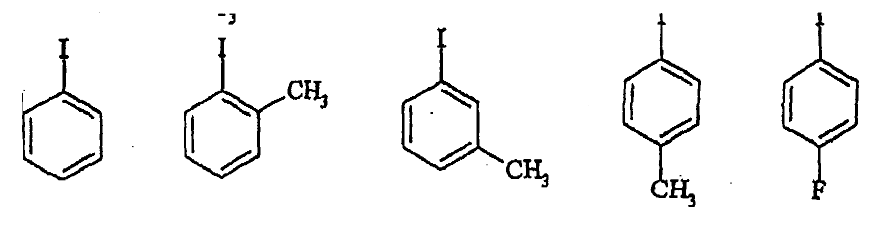 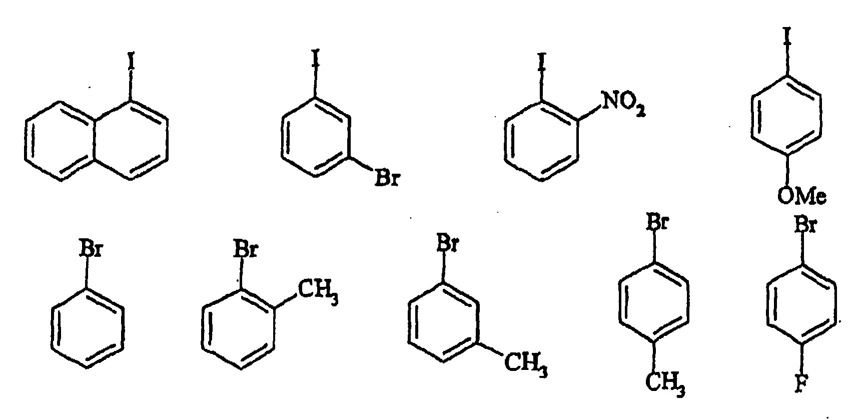 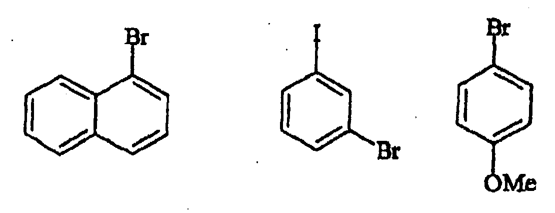
- F'₁ entspricht der folgenden Formel: 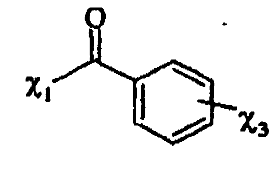 wobei χ₁ und χ₃ wie oben definiert sind,
- F'₂ entspricht der folgenden Formel: 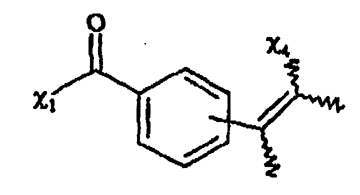 wobei χ₁ wie oben definiert ist,
wobei χ₄ eine funktionelle Gruppe des Typs Ester, Amid, Sulfon, Phosphonat, Silan, Boran oder eine Alkylgruppe, funktionell oder nicht, umfassend 1 bis 20 Kohlenstoffatome, oder eine Arylgruppe, funktionell oder nicht, umfassend 6 bis 30 Kohlenstoffatome, darstellt,
wobei G' der folgenden Formel entspricht: 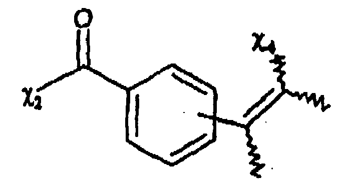 wobei χ₂ und χ₄ wie oben definiert sind.

21. Verwendung nach Anspruch 19 für den Einsatz der Suzuki-Kopplung nach einem der folgenden Reaktionsschemata
a) 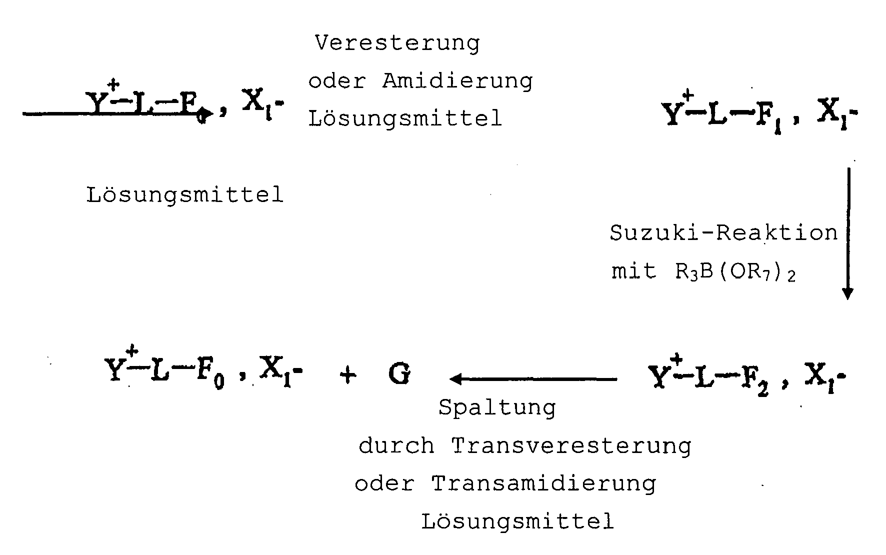 wobei R₃ unter den Aryl-, Heteroaryl-, Ethenyl-, Dienyl-, Allyl-, Ethynylgruppen ausgewählt wird, die substituiert sind oder nicht und 2 bis 20 Kohlenstoffatome umfassen,
wobei R₇ ein Wasserstoffatom oder eine Alkylgruppe, verzweigt oder linear, oder eine Zykloalkylgruppe, umfassend 1 bis 12 Kohlenstoffatome, darstellt,
wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methylimidazolium- oder Pyridinium-Kation ist,
wobei L einen Arm darstellt, insbesondere eine lineare oder verzweigte Alkylgruppe, umfassend 1 bis 20 Kohlenstoffatome, oder eine eventuell funktionelle Aralkylgruppe, umfassend 6 bis 30 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel, wobei die Funktionen F_{0,} F₁ und F₂ wie unten definiert sind:
- F₀ ist von der Form -χ₁H, wobei χ₁ ein Sauerstoffatom oder eine Gruppe NR_{f} darstellt, wobei R_{f} einer linearen oder verzweigten Alkylgruppe, umfassend 1 bis 20 Kohlenstoffatome, oder einer Arylgruppe, umfassend 6 bis 30 Kohlenstoffatome, entspricht,
- F₁ ist von der Form -Rₑ-χ₀, wobei Rₑ eine aromatische oder heteroaromatische Gruppe, umfassend 6 bis 30 Kohlenstoffatome, darstellt, wobei χ eine weggehende Gruppe darstellt, die vorzugsweise ausgewählt wird unter Cl, Br, I, OTf, O-CO₂R⁵ oder OSO₃R⁵, wobei R⁵ eine Alkylgruppe, umfassend 1 bis 10 Kohlenstoffatome, oder eine Aralkylgruppe, umfassend 6 bis 30 Kohlenstoffatome, darstellt, wobei F₁ vorzugsweise der folgenden Formel entspricht: 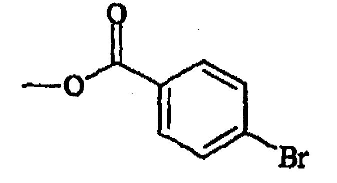
- F₂ ist von der Form -Rₑ-R₂, wobei Rₑ wie oben definiert ist, und wobei R₂ unter den Aryl-, Heteroaryl-, Ethenyl-, Dienyl-, Allyl-, Ethynylgruppen ausgewählt wird, die substituiert sind oder nicht und 2 bis 30 Kohlenstoffatome umfassen, wobei F₂ vorzugsweise der folgenden Formel entspricht: 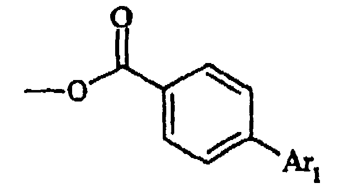 wobei Ar₁ eine aromatische Gruppe darstellt, die vorzugsweise ausgewählt wird unter: 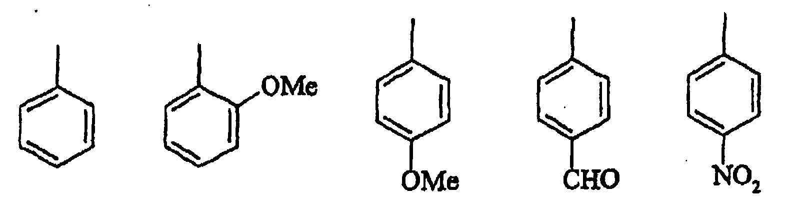 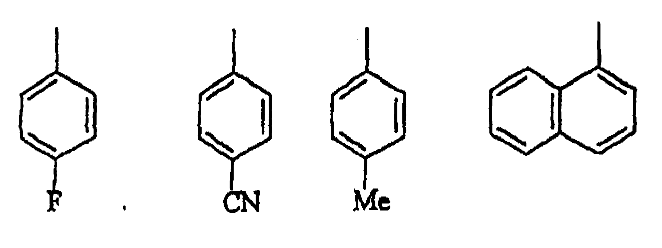 wobei das Molekül G von der Form R₂-R₃ ist, wobei R₂ und R₃ wie oben definiert sind, und insbesondere der folgenden Formel entspricht, 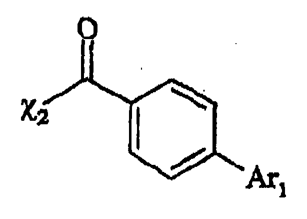 wobei χ₂ entweder eine Gruppe -OR_{g} darstellt, wobei Rg ein Wasserstoffatom oder eine Alkylgruppe, umfassend 1 bis 20 Kohlenstoffatome, oder eine Gruppe -NRₕRᵤ darstellt, wobei Rₕ und Rᵤ unabhängig voneinander ein Wasserstoffatom, eine Alkylgruppe, umfassend 1 bis 20 Kohlenstoffatome, oder eine Arylgruppe, umfassend 6 bis 30 Kohlenstoffatome, darstellen,
wobei Ar₁ wie oben definiert ist,
b) 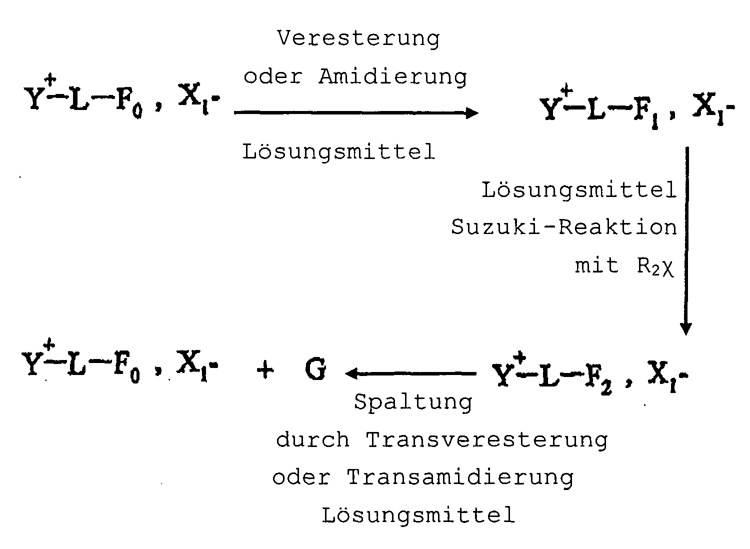 wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methylimidazolium- oder Pyridinium-Kation ist,
wobei L einen Arm darstellt, insbesondere eine lineare oder verzweigte Alkylgruppe, umfassend 1 bis 20 Kohlenstoffatome, oder eine eventuell funktionelle Aralkylgruppe, umfassend 6 bis 30 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei R₂ unter den Aryl-, Heteroaryl-, Ethenyl-, Dienyl-, Allyl-, Ethynylgruppen ausgewählt wird, die substituiert sind oder nicht und 2 bis 30 Kohlenstoffatome umfassen,
wobei die Funktionen F_{0,} F₁ und F₂ wie unten definiert sind:
- F₀ ist von der Form -χ₁H, wobei χ₁ wie oben definiert ist,
- F₁ ist von der Form -R_{q}-B(OR₇)₂, wobei R₇ wie oben definiert ist und Rq einer Arylgruppe, umfassend 6 bis 30 Kohlenstoffatome, einer Heteroarylgruppe, umfassend 4 bis 20 Kohlenstoffatome, einer Ethenylgruppe, umfassend 2 bis 20 Kohlenstoffatome, einer Dienylgruppe, umfassend 3 bis 20 Kohlenstoffatome, einer Allylgruppe, umfassend 3 bis 20 Kohlenstoffatome, einer Ethynylgruppe, umfassend 2 bis 20 Kohlenstoffatome, die substituiert sind oder nicht, entspricht, wobei F₁ vorzugsweise der folgenden Formel entspricht: 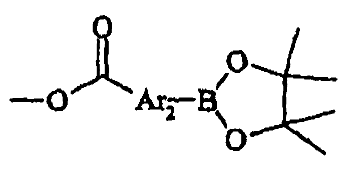 wobei Ar₂ einer Arylgruppe, substituiert oder nicht, umfassend 6 bis 30 Kohlenstoffatome, entspricht,
- F₂ ist von der Form -R_{q}-Rᵤ, wobei Rq und Rᵤ wie oben definiert sind, wobei F₂ vorzugsweise der folgenden Formel entspricht: 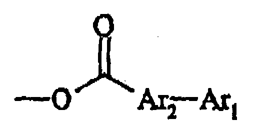 wobei Ar₁ eine aromatische Gruppe darstellt, die vorzugsweise ausgewählt wird unter: 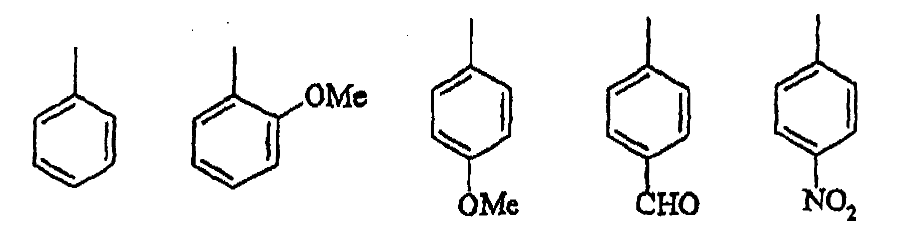 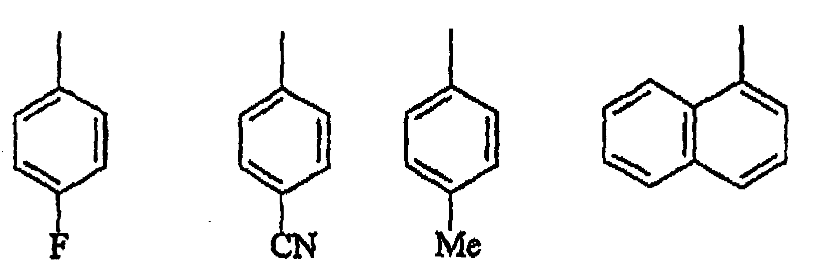 wobei das Molekül G von der Form R₂-R₃, wobei R₂ und R₃ wie oben definiert sind und insbesondere folgender Formel entsprechen: 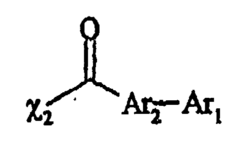 wobei χ₂, Ar₁ und Ar₂ wie oben definiert sind,
c) 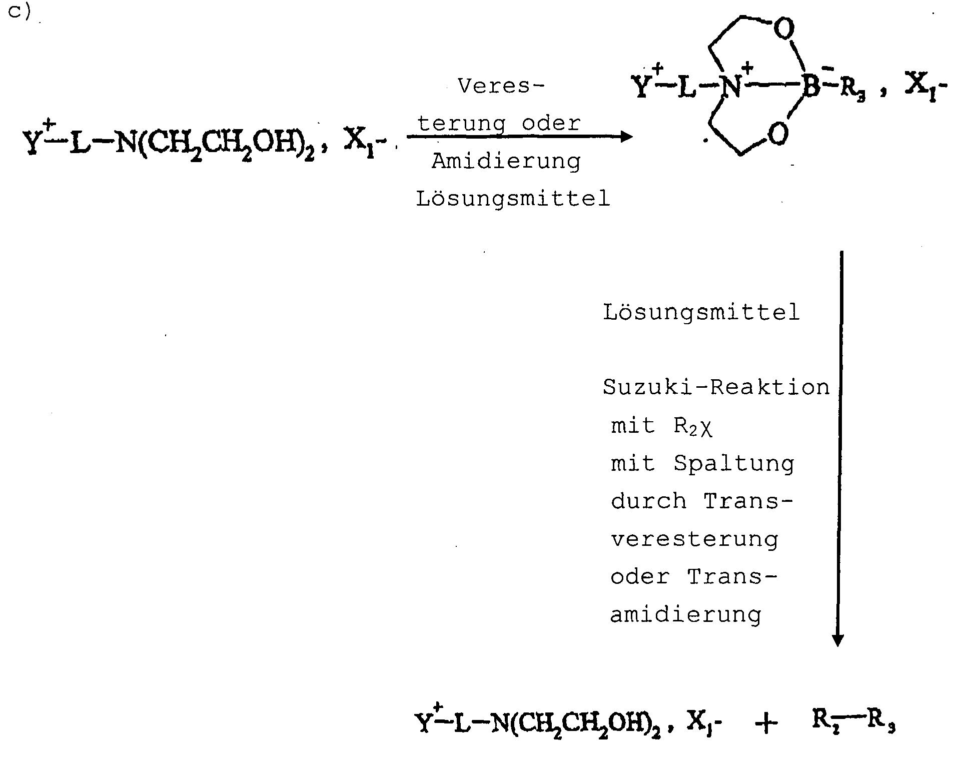 wobei Y⁺-, L, X₁⁻, R₂ und R₃ wie oben definiert sind,
wobei R₃ vorzugsweise eine Phenylgruppe ist,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
d) 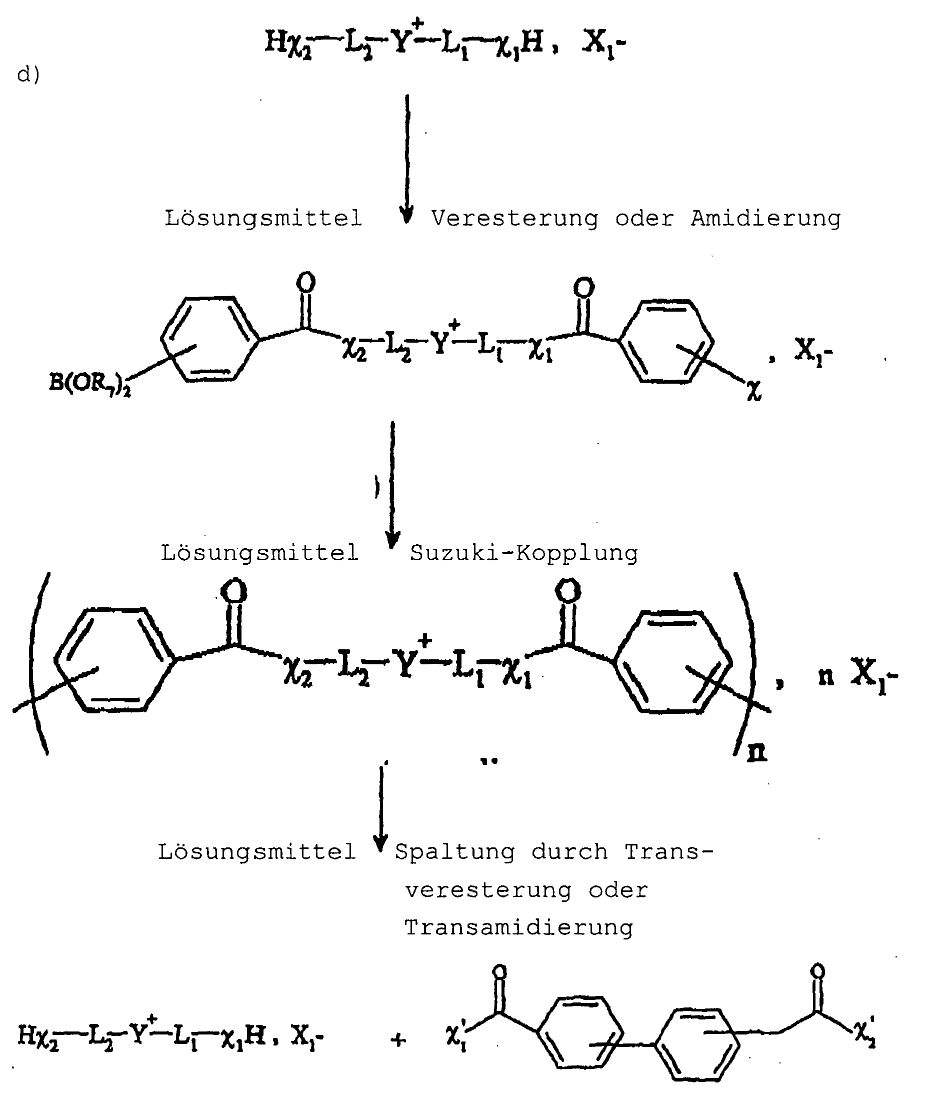 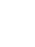
wobei n eine ganze Zahl zwischen 1 und 50 darstellt,
wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methylimidazolium- oder Pyridinium-Kation ist,
wobei L einen Arm darstellt, insbesondere eine lineare oder verzweigte Alkylgruppe, umfassend 1 bis 20 Kohlenstoffatome, oder eine eventuell funktionelle Aralkylgruppe, umfassend 6 bis 30 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei χ₁ und χ₂ identisch oder unterschiedlich sind, ein Sauerstoffatom oder eine Gruppe NR_{f} darstellen, wobei R_{f} eine lineare oder verzweigte Alkylgruppe, umfassend 1 bis 20 Kohlenstoffatome, oder eine Arylgruppe, umfassend 6 bis 30 Kohlenstoffatome, darstellt,
wobei χ eine weggehende Gruppe darstellt, die vorzugsweise unter Cl, Br, I, OTf, OCO₂R⁵ oder OSO₃-R⁵ darstellt, wobei R⁵ eine Alkylgruppe, umfassend 1 bis 10 Kohlenstoffatome, oder eine Aralkylgruppe, umfassend 6 bis 30 Kohlenstoffatome, darstellt,
wobei R₇ ein Wasserstoffatom, eine Alkylgruppe, verzweigt oder nicht, oder eine Zykloalkylgruppe, umfassend 1 bis 12 Kohlenstoffatome, oder eine Arylgruppe, umfassend 6 bis 30 Kohlenstoffatome, darstellt,
wobei χ'₁ und χ'₂ identisch oder unterschiedlich sind, entweder eine Gruppe -OR_{g} darstellen, wobei R_{g} ein Wasserstoffatom oder eine Alkylgruppe, umfassend 1 bis 20 Kohlenstoffatome, oder eine Gruppe -NRₕRᵤ darstellen, wobei Rₕ und Rᵤ unabhängig voneinander ein Wasserstoffatom, eine Alkylgruppe, umfassend 1 bis 20 Kohlenstoffatome, oder eine Arylgruppe, umfassend 6 bis 30 Kohlenstoffatome, darstellen.

22. Verwendung nach Anspruch 19 für den Einsatz der Sonogashira-Kopplung nach einem der folgenden Reaktionsschemata:
a) 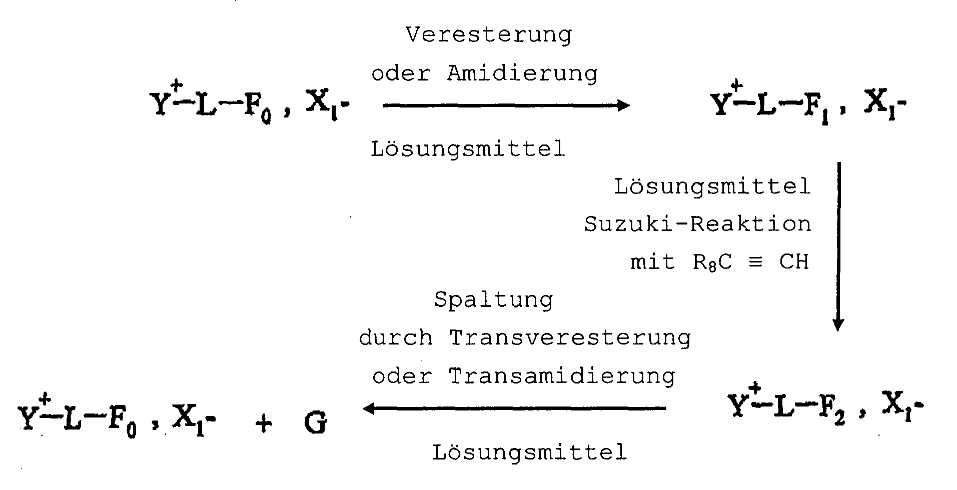 wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methylimidazolium- oder Pyridinium-Kation ist,
wobei L einen Arm darstellt, insbesondere eine lineare oder verzweigte Alkylgruppe, umfassend 1 bis 20 Kohlenstoffatome, oder eine eventuell funktionelle Aralkylgruppe, umfassend 6 bis 30 Kohlenstoffatome, und vorzugsweise eine lineare Alkylgruppe, vorzugsweise eine lineare Alkylgruppe des Typs (CH₂)ᵣ,
wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻,I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei R₈ eine Gruppe ORₕ, NRₕRᵤ, CORₕ, CN, SO₂Rₕ, SRₕ, eine Alcenyl-, Ethynyl-, Dienylgruppe darstellt, wobei Rₕ und Rᵤ unabhängig voneinander ein Wasserstoffatom, eine Alkylgruppe, umfassend 1 bis 20 Kohlenstoffatome, oder eine Arylgruppe, umfassend 6 bis 30 Kohlenstoffatome darstellen,
oder wobei R₈ eine verzweigte oder lineare eventuell funktionelle Alkylgruppe, umfassend 1 bis 20 Kohlenstoffatome, oder eine Arylgruppe, oder eine Alkaryl- oder Aralkylgruppe, umfassend 6 bis 30 Kohlenstoffatome, substituiert oder nicht, darstellt, wobei die Alkyl- oder Arylgruppen durch eine der folgenden funktionellen Gruppen substituiert sein können: ein Halogenatom, insbesondere Cl, eine Gruppe ORₕ, NRₕRᵤ, CORₕ, CN, SO₂Rₕ, SRₕ, eine Alcenyl-, Ethynyl-, Dienyl-, Vinyl-, Alcynylgruppe, wobei Rₕ und Rᵤ wie vorher definiert sind,
wobei R₈ insbesondere eine der folgenden Gruppen ist:
**-(CH₂)₈-CH₃, -(CH₂)₃-CH₂OH, -(CH₂)₃-CH₂OMe,**
wobei s eine ganze Zahl zwischen 0 und 10 ist 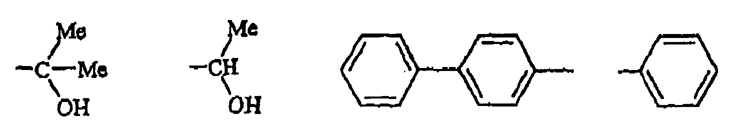 **Me₃Si**- wobei die Funktionen F₀, F₁ und F₂ wie unten definiert sind:
- F₀ entspricht einer Gruppe -χ₁H, wobei χ₁ ein Sauerstoffatom oder eine Gruppe -NR_{f}, wobei R_{f} einer linearen oder verzweigten Alkylgruppe, umfassend 1 bis 20 Kohlenstoffatome, oder einer Arylgruppe, umfassend 6 bis 30 Kohlenstoffatome, entspricht,
- F₁ entspricht der folgenden Formel: 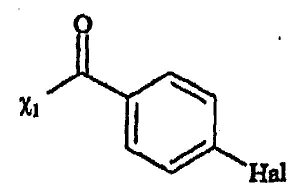 wobei χ₁ wie oben definiert ist, und Hal ein Halogen darstellt und vorzugsweise Jod ist,
- F₂ entspricht der folgenden Formel: 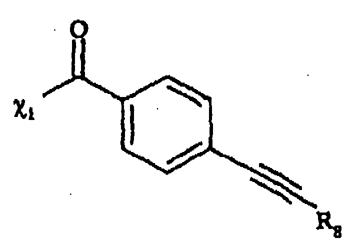 wobei χ₁ und R₈ wie oben definiert sind,
wobei G der folgenden Formel entspricht: 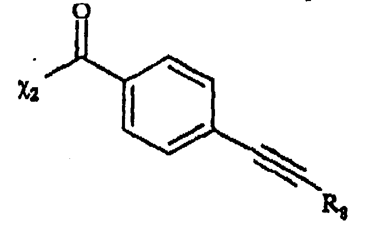 wobei χ₂ entweder eine eine Gruppe -OR_{g} darstellt,
wobei Rg ein Wasserstoffatom oder eine Alkylgruppe, umfassend 1 bis 20 Kohlenstoffatome, oder eine Gruppe -NRₕRᵤ darstellen, wobei Rₕ und Rᵤ unabhängig voneinander ein Wasserstoffatom, eine Alkylgruppe, umfassend 1 bis 20 Kohlenstoffatome, oder eine Arylgruppe, umfassend 6 bis 30 Kohlenstoffatome, darstellt, wobei χ₂ insbesondere eine Gruppe OMe, OEt, OPr oder OBu darstellt.
b) 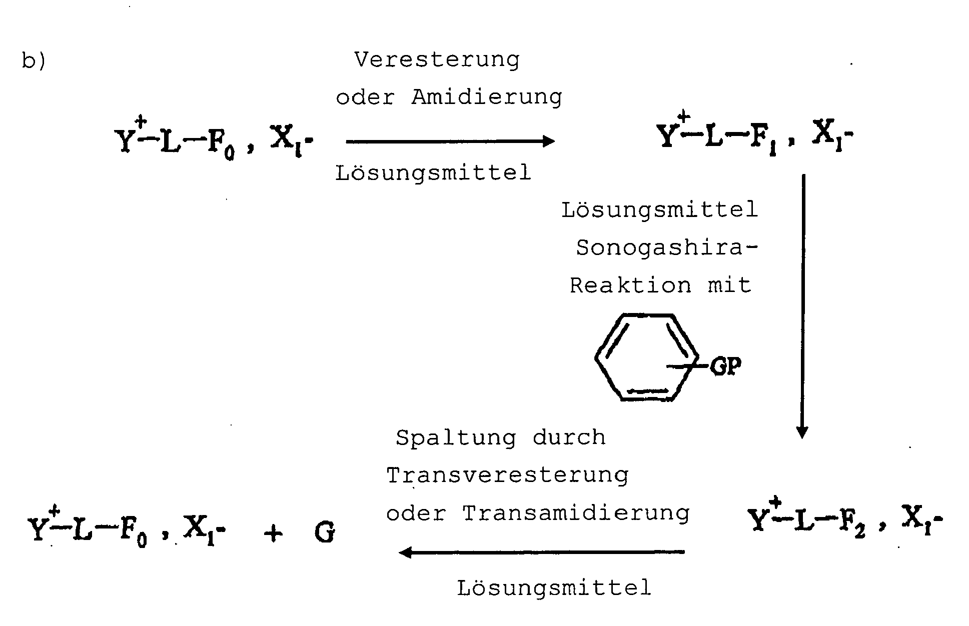 wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methylimidazolium- oder Pyridinium-Kation ist,
wobei L einen Arm darstellt, insbesondere eine lineare oder verzweigte Alkylgruppe, umfassend 1 bis 20 Kohlenstoffatome, oder eine eventuell funktionelle Aralkylgruppe, umfassend 6 bis 30 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻,I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei GP eine weggehende Gruppe darstellt und insbesondere Cl, Br, I oder OTf,
wobei die Funktionen F₀, F₁ und F₂ wie unten definiert sind:
- F₀ entspricht einer Gruppe -COOH,
- F₁ entspricht der folgenden Formel: 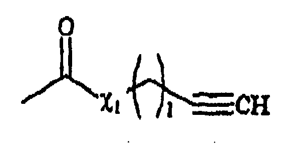 wobei 1 eine ganze Zahl zwischen 1 und 20 darstellt und χ₁ ein Sauerstoffatom oder eine Gruppe -NR_{f} darstellt, wobei R_{f} einer linearen oder verzweigten Alkylgruppe, umfassend 1 bis 20 Kohlenstoffatome, oder einer Arylgruppe, umfassend 6 bis 30 Kohlenstoffatome, entspricht,
- F₂ entspricht der folgenden Formel: 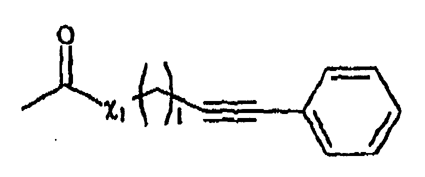
wobei χ₁ und 1 wie oben definiert sind,
wobei G der folgenden Formel entspricht: 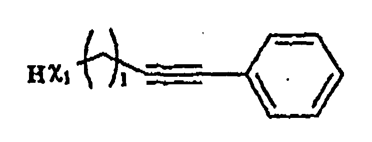 wobei χ₁ und 1 wie oben definiert sind.

23. Verwendung nach einem der Ansprüche 1 bis 17 für den Einsatz der Baylis-Hilman-Reaktion nach einem der folgenden Reaktionsschemata:
a) 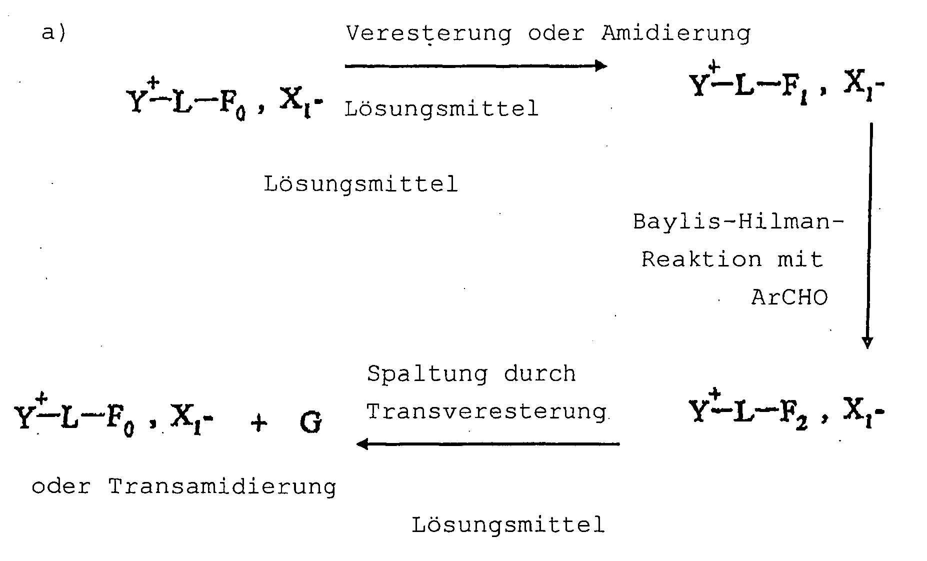 wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methylimidazolium- oder Pyridinium-Kation ist,
wobei L einen Arm darstellt, insbesondere eine lineare oder verzweigte Alkylgruppe, umfassend 1 bis 20 Kohlenstoffatome, oder eine eventuell funktionelle Aralkylgruppe, umfassend 6 bis 30 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei die Funktionen F₀, F₁ und F₂ wie unten definiert sind:
- F₀ entspricht einer Gruppe -OH,
- F₁ entspricht der folgenden Formel: 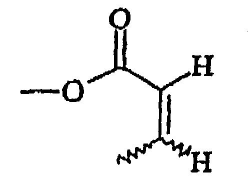
- F₂ entspricht der folgenden Formel: 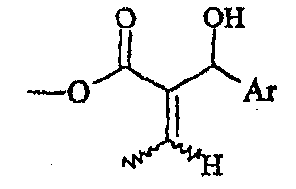 wobei G der folgenden Formel entspricht: 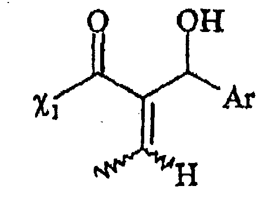 wobei χ₁ eine Gruppr -OH oder eine Gruppe ORg darstellt, wobei R_{g} eine lineare oder verzweigte Alkylgruppe, umfassend 1 bis 20 Kohlenstoffatome darstellt,
wobei Ar eine aromatische oder heteroaromatische Gruppe, substituiert oder nicht, darstellt, wobei ArCHO insbesondere ausgewählt wird unter: 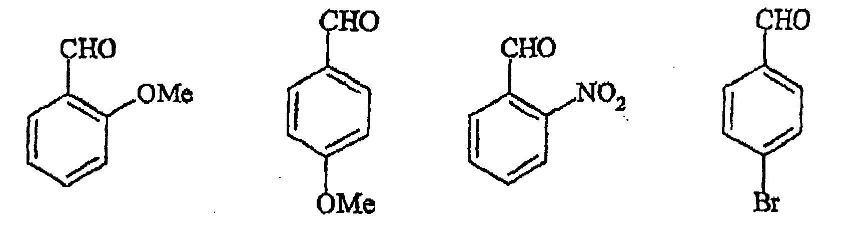 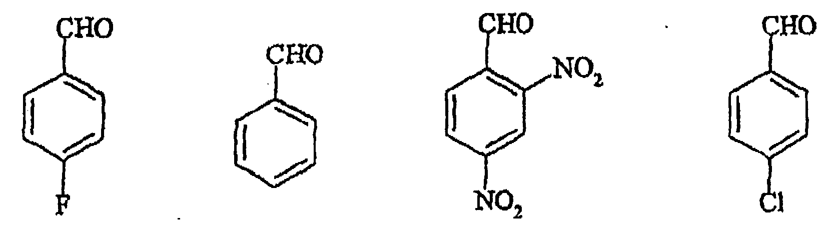
b) 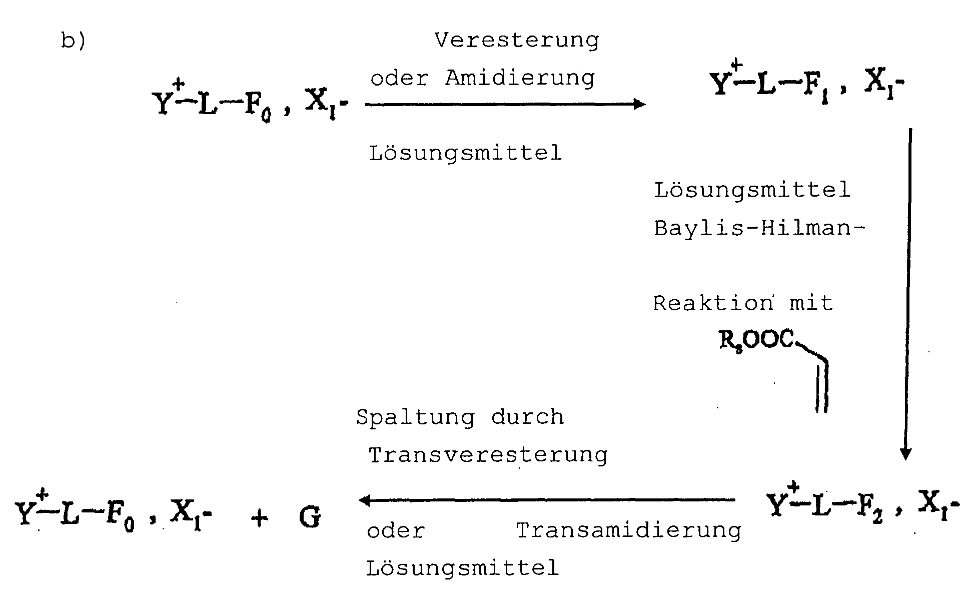 wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methylimidazolium- oder Pyridinium-Kation ist,
wobei L einen Arm darstellt, insbesondere eine lineare oder verzweigte Alkylgruppe, umfassend 1 bis 20 Kohlenstoffatome, oder eine eventuell funktionelle Aralkylgruppe, umfassend 6 bis 30 Kohlenstoffatome, und vorzugsweise eine lineare Alkylgruppe, vorzugsweise eine lineare Alkylgruppe des Typs (CH₂)ᵣ,
wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻_{,} R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei R₈ ein Wasserstoffatom oder eine Alkylgruppe, umfassend 1 bis 20 Kohlenstoffatome, oder eine Aralkyl- oder Alkarylgruppe, umfassend 7 bis 30 Kohlenstoffatome, darstellt,
wobei die Funktionen F₀, F₁ und F₂ wie unten definiert sind:
- F₀ entspricht einer Gruppe -χ₁H, wobei χ₁ ein Sauerstoffatom oder eine Gruppe -NR_{f}, wobei R_{f} einer linearen oder verzweigten Alkylgruppe, umfassend 1 bis 20 Kohlenstoffatome, oder einer Arylgruppe, umfassend 6 bis 30 Kohlenstoffatome, entspricht,
- F₁ entspricht der folgenden Formel: 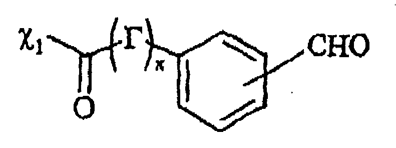 wobei χ₁ wie oben definiert ist,
wobei x gleich 0 oder 1 ist,
wobei Γ́ eine Alkylkette, umfassend 1 bis 20 Kohlenstoffatome, eine Alkaryl-, Aralkylkette, umfassend 6 bis 30 Kohlenstoffatome, darstellt,
- F₂ entspricht der folgenden Formel: 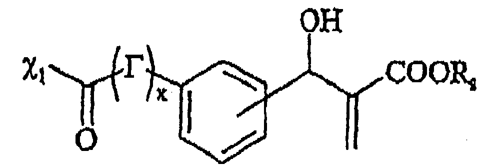 wobei χ₁, x, R₅ und Γ́ wie oben definiert sind,
wobei G der folgenden Formel entspricht: 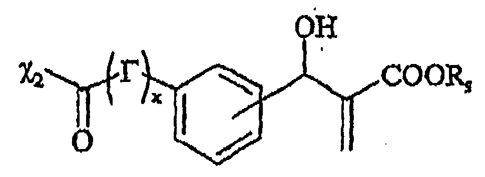 wobei χ₂, x, R_{g} und Γ́ wie oben definiert sind,
wobei χ₂ entweder eine eine Gruppe -OR_{g} darstellt, wobei Rg ein Wasserstoffatom oder eine Alkylgruppe, umfassend 1 bis 20 Kohlenstoffatome, oder eine Gruppe -NRₕRᵤ darstellen, wobei Rₕ und Rᵤ unabhängig voneinander ein Wasserstoffatom, eine Alkylgruppe, umfassend 1 bis 20 Kohlenstoffatome, oder eine Arylgruppe, umfassend 6 bis 30 Kohlenstoffatome, darstellt, wobei χ₂ insbesondere eine Gruppe OMe, OEt, OPr oder OBu darstellt.
c) 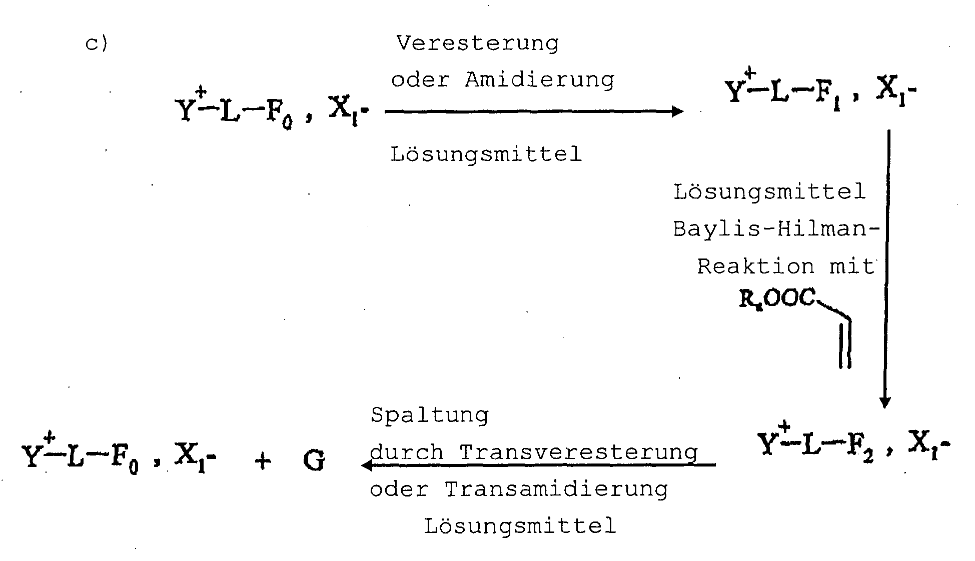 wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methylimidazolium- oder Pyridinium-Kation ist,
wobei L einen Arm darstellt, insbesondere eine lineare oder verzweigte Alkylgruppe, umfassend 1 bis 20 Kohlenstoffatome, oder eine eventuell funktionelle Aralkylgruppe, umfassend 6 bis 30 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei R₅ ein Wasserstoffatom oder eine Alkylgruppe, umfassend 1 bis 20 Kohlenstoffatome, oder eine Aralkyl- oder Alkarylgruppe, umfassend 7 bis 30 Kohlenstoffatome, darstellt,
wobei die Funktionen F₀, F₁ und F₂ wie unten definiert sind:
- F₀ entspricht einer Gruppe -COχ₁H, wobei χ₁ ein Sauerstoffatom oder eine Gruppe -NR_{f}, wobei R_{f} einer linearen oder verzweigten Alkylgruppe, umfassend 1 bis 20 Kohlenstoffatome, oder einer Arylgruppe, umfassend 6 bis 30 Kohlenstoffatome, entspricht,
- F₁ entspricht der folgenden Formel: 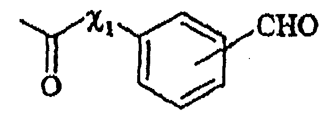
- F₂ entspricht der folgenden Formel: 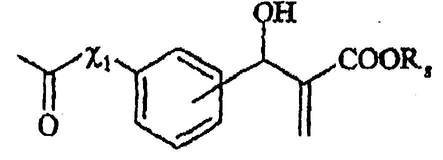
- wobei G der folgenden Formel entspricht:

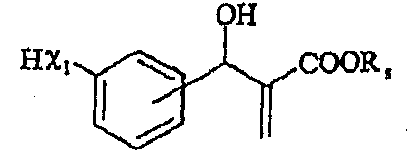

24. Verwendung nach einem der Ansprüche 1 bis 17 für die eventuell asymmetrische Synthese von α-Aminosäuren nach dem folgenden Reaktionsschema: 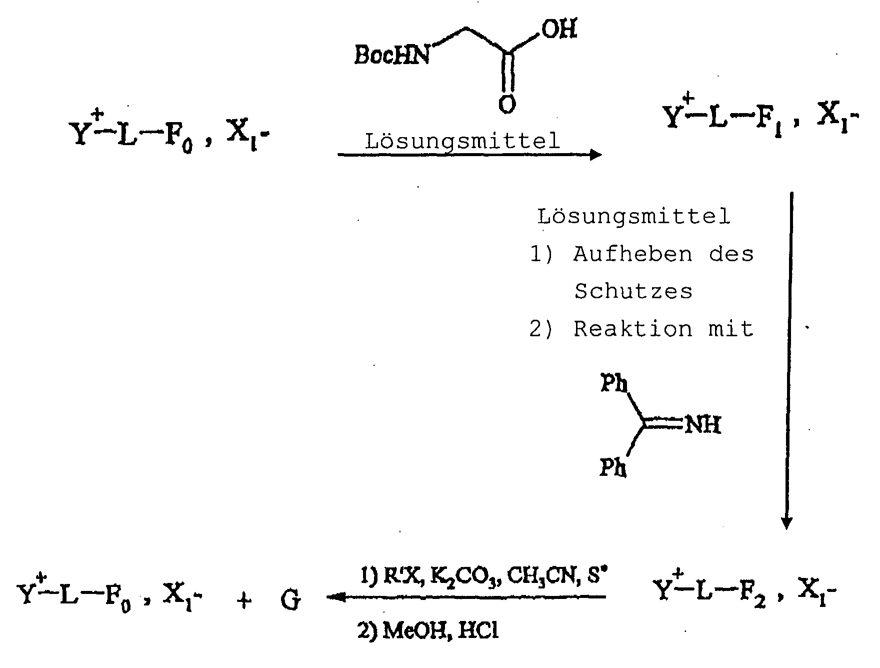 wobei Y⁺- ein Oniumkation, wie in einem der Ansprüche 3 bis 17 definiert, darstellt und vorzugsweise ein Trimethylalkylammonium, Triethylalkylammonium, Tributylalkylphosphonium-Kation ist,
wobei L einen Arm darstellt, insbesondere eine lineare oder verzweigte Alkylgruppe, umfassend 1 bis 20 Kohlenstoffatome, oder eine eventuell funktionelle Aralkylgruppe, umfassend 1 bis 20 Kohlenstoffatome, und vorzugsweise eine lineare Alkylgruppe, vorzugsweise eine lineare Alkylgruppe des Typs (CH₂)ᵣ,
wobei r zwischen 1 und 20 und vorzugsweise zwischen 3 und 6 variiert,
wobei X₁⁻ wie in einem der Ansprüche 1 bis 17 definiert ist, und insbesondere **Cl⁻, Br⁻, I⁻, ⁻N(SO₂CF₃)₂, BF₄⁻, PF₆⁻,**
wobei das oder die Lösungsmittel ausgewählt werden unter: Azetonitril, Dichlormethan, Tetrahydrofuran, Dioxan, Toluol, Chlorbenzol oder einem Gemisch dieser Lösungsmittel,
wobei R' eine lineare oder verzweigte eventuell funktionelle Alkylgruppe, umfassend 1 bis 30 Kohlenstoffatome, darstellt,
wobei S* einen chiralen Phasenübertragungswirkstoff, wie beispielsweise O(9)-Allyl-N-9-Anthracenyl-Methylcinchonidinium-Bromid, darstellt,
wobei die Funktionen F₀, F₁ und F₂ wie unten definiert sind:
- F₀ entspricht -OH,
- F₁ entspricht der folgenden Formel: 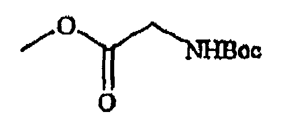
- F₂ entspricht der folgenden Formel: 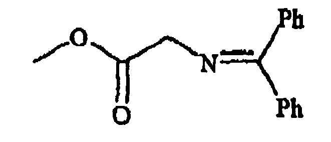 wobei G der folgenden Formel entspricht:

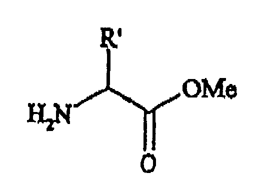

25. Verwendung nach einem der Ansprüche 1 bis 17 für den Einsatz von Mehrkomponentenreaktionen, insbesondere für die Reaktion des Typs Grieco, nach einem der folgenden Reaktionsschemata:
a) 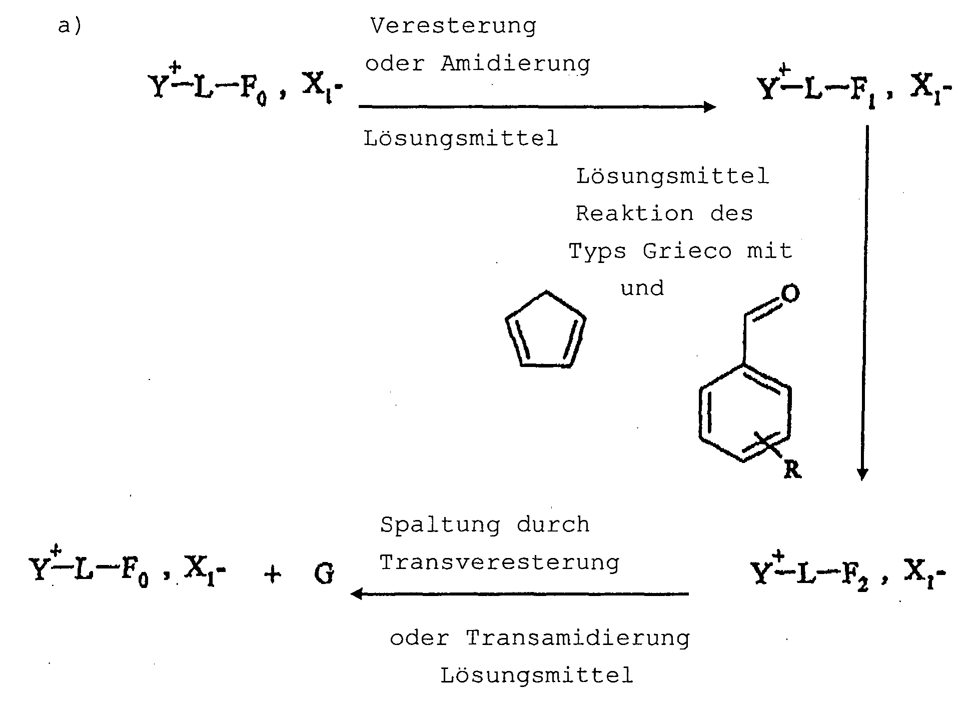 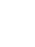 wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methyl-N'-Alkylimidazolium- N-Alkylpyridinium-, Dimethylalkylsulfonium- oder Diethylalkylsulfonium-Kation ist,
wobei L einen Arm darstellt, insbesondere eine lineare oder verzweigte Alkylgruppe, umfassend 1 bis 20 Kohlenstoffatome, oder eine eventuell funktionelle Aralkyl- oder Alkarylgruppe, umfassend 1 bis 20 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei R ein Wasserstoffatom, eine Nitrogruppe in Para-Position, ein Chloratom in Para-Position oder eine Methoxygruppe in Ortho-Position darstellt,
wobei die Funktionen F₀, F₁ und F₂ wie unten definiert sind:
- F₀ stellt eine Gruppe -OH dar,
- F₁ entspricht der folgenden Formel: 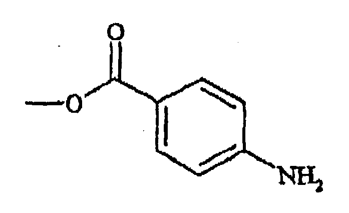
- F₂ entspricht der folgenden Formel: 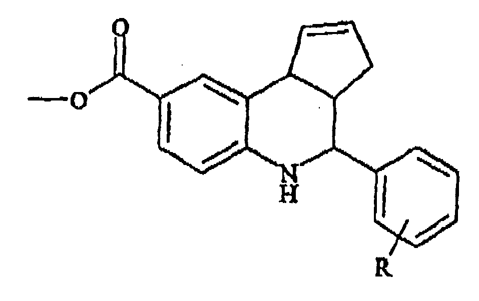 wobei G der folgenden Formel entspricht: 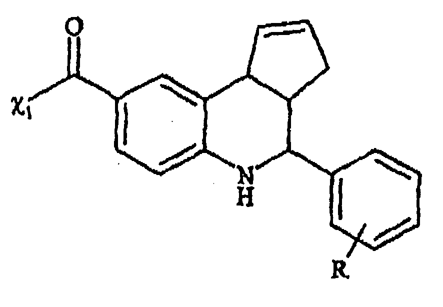 wobei χ₁ eine Gruppe -OH oder eine Gruppe -OR_{g}, wobei R_{g} eine lineare oder verzweigte Alkylgruppe, umfassend 1 bis 20 Kohlenstoffatome, darstellt.
b) 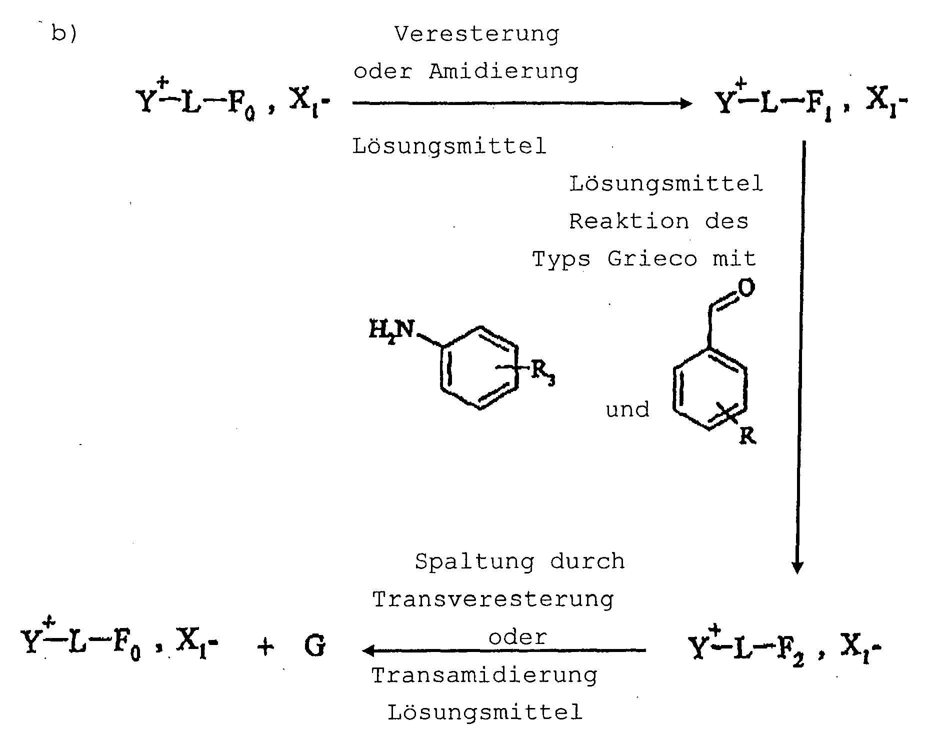 wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methyl-N'-Alkylimidazolium- N-Alkylpyridinium-, Dimethylalkylsulfonium- oder Diethylalkylsulfonium-Kation ist,
wobei L einen Arm darstellt, insbesondere eine lineare oder verzweigte Alkylgruppe, umfassend 1 bis 20 Kohlenstoffatome, oder eine eventuell funktionelle Aralkyl- oder Alkarylgruppe, umfassend 1 bis 20 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei R₂ eine Alkylgruppe, funktionell oder nicht, umfassend 1 bis 20 Kohlenstoffatome, oder eine Arylgruppe, funktionell oder nicht, umfassend 6 bis 30 Kohlenstoffatome, oder eine Aralkyl- oder Alkarylgruppe, funktionell oder nicht, umfassend 7 bis 50 Kohlenstoffatome, darstellt,
wobei R₃ ein Wasserstoffatom, eine lineare oder verzweigte Alkylgruppe, umfassend 1 bis 20 Kohlenstoffatome, oder eine Arylgruppe, umfassend 6 bis 30 Kohlenstoffatome, oder eine Aralkyl- oder Alkarylgruppe, funktionell oder nicht, umfassend 7 bis 50 Kohlenstoffatome, oder eine funktionelle Gruppe darstellt, die insbesondere ausgewählt wird unter NO₂, CN, COOR, OR, COR, NHCOR, NRR', SO₂R, I, Br, R und R', die unabhängig voneinander eine Alkylgruppe, umfassend 1 bis 20 Kohlenstoffatome, oder eine Arylgruppe, umfassend 6 bis 30 Kohlenstoffatome, darstellen,
wobei die Funktionen F₀, F₁ und F₂ wie unten definiert sind:
- F₀ stellt eine Gruppe -OH dar,
- F₁ entspricht der folgenden Formel: 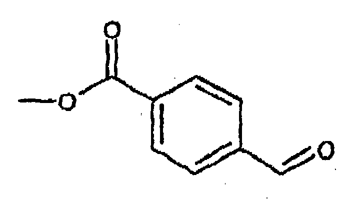
- F₂ entspricht der folgenden Formel: 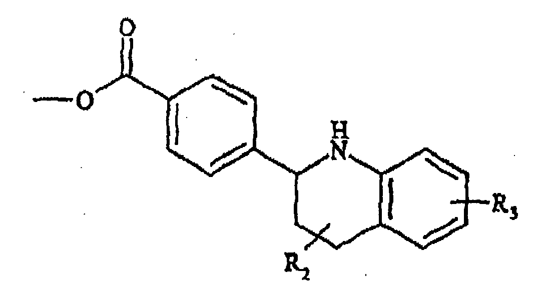 wobei G der folgenden Formel entspricht: 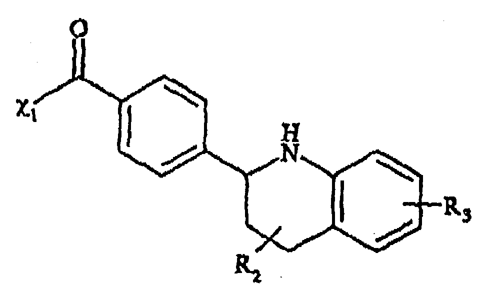 wobei χ₁ eine Gruppe -OH oder eine Gruppe -OR_{g}, wobei R_{g} eine lineare oder verzweigte Alkylgruppe, umfassend 1 bis 20 Kohlenstoffatome, darstellt.
c) 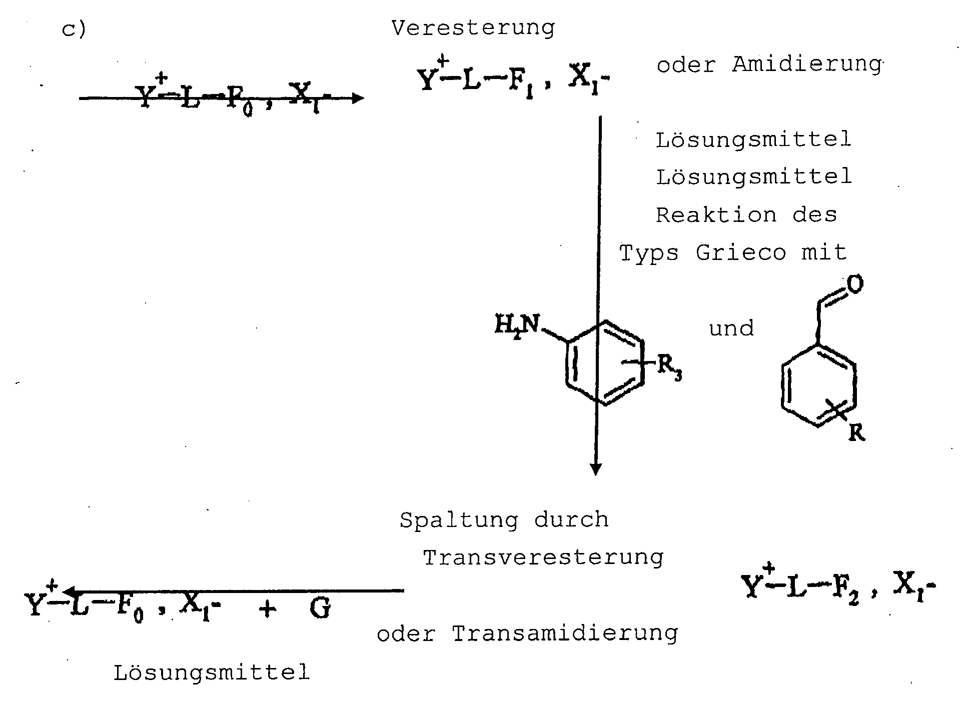
wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methyl-N'-Alkylimidazolium- N-Alkylpyridinium-, Dimethylalkylsulfonium- oder Diethylalkylsulfonium-Kation ist,
wobei L einen Arm darstellt, insbesondere eine lineare oder verzweigte Alkylgruppe, umfassend 1 bis 20 Kohlenstoffatome, oder eine eventuell funktionelle Aralkyl- oder Alkarylgruppe, umfassend 1 bis 20 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei R ein Wasserstoffatom oder eine funktionelle Gruppe, wie beispielsweise eine Nitrogruppe in Para-Position, ein Chloratom in Para-Position oder eine Methoxygruppe in Ortho-Position, oder eine Alkylgruppe, funktionell oder nicht, umfassend 1 bis 20 Kohlenstoffatome, oder eine Arylgruppe, funktionell oder nicht, umfassend 6 bis 30 Kohlenstoffatome, oder eine Aralkyl- oder Alkarylgruppe, funktionell oder nicht, umfassend 7 bis 50 Kohlenstoffatome, darstellt,
wobei R₃ ein Wasserstoffatom, eine lineare oder verzweigte Alkylgruppe, umfassend 1 bis 20 Kohlenstoffatome, oder eine Arylgruppe, umfassend 6 bis 30 Kohlenstoffatome, oder eine Aralkyl- oder Alkarylgruppe, funktionell oder nicht, umfassend 7 bis 50 Kohlenstoffatome, oder eine funktionelle Gruppe darstellt, die insbesondere ausgewählt wird unter NO_{2,} CN, COOR, OR, COR, NHCOR, NRR', SO₂R, I, Br, R und R', die unabhängig voneinander eine Alkylgruppe, umfassend 1 bis 20 Kohlenstoffatome, oder eine Arylgruppe, umfassend 6 bis 30 Kohlenstoffatome, darstellen,
wobei die Funktionen F₀, F₁ und F₂ wie unten definiert sind:
- F₀ stellt jede Funktion dar, die es ermögliche, einen ein Olefin tragenden Rest, vorzugsweise ein Ester oder ein Amid, anzuheften oder abzulösen,
- F₁ entspricht einer der folgenden allgemeinen Funktionen: 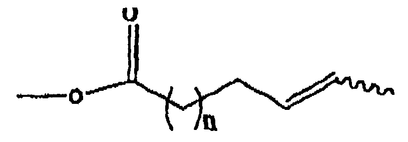 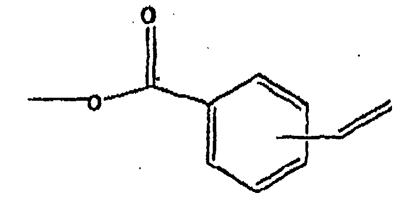 wobei n eine ganze Zahl zwischen 1 und 10 darstellt,
- F₂ entspricht einer der folgenden allgemeinen Formeln: 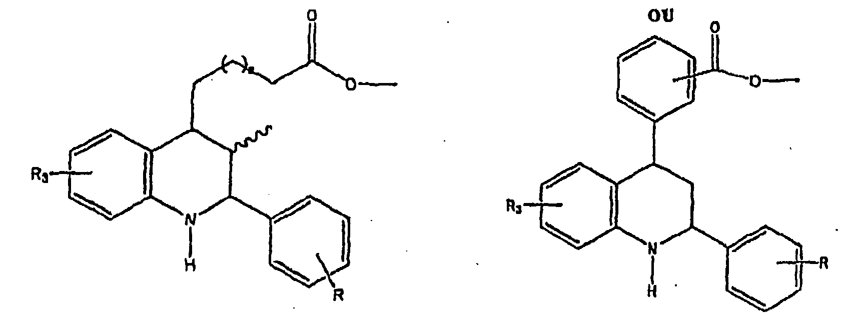 wobei G einer der folgenden allgemeinen Formeln entspricht: ODER 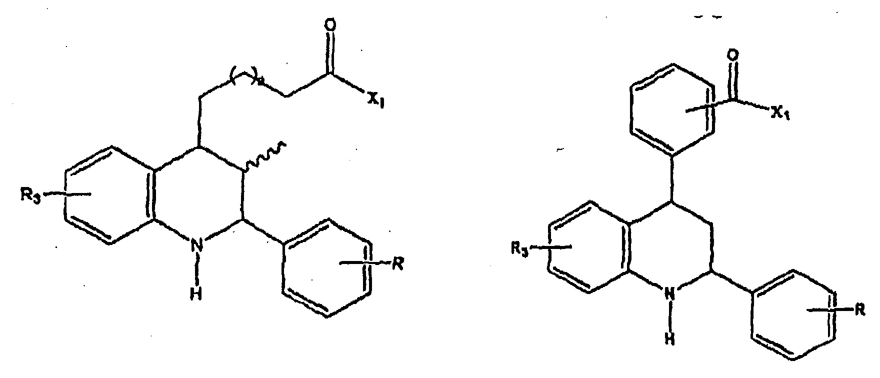 wobei n, R und R₃ wie oben definiert sind, und
wobei χ₁ eine Gruppe -OH oder eine Gruppe -OR_{g}, wobei R_{g} eine lineare oder verzweigte Alkylgruppe, umfassend 1 bis 20 Kohlenstoffatome, darstellt.

26. Verwendung nach einem der Ansprüche 1 bis 17 für den Einsatz von Mehrkomponenten-Reaktionen, insbesondere für die Synthese von tetrasubstituierten Olefinen nach dem folgenden Reaktionsschema: 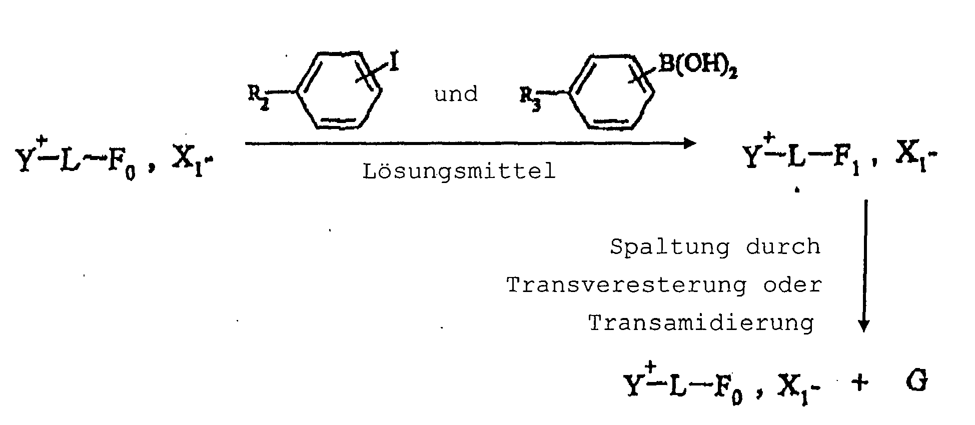 wobei Y⁺- ein Oniumkation, wie in einem der Ansprüche 3 bis 17 definiert, darstellt und vorzugsweise ein Trimethylalkylammonium-, Triethylalkylammonium-, Tributylalkylphosphonium-, N-Methyl-N'-Alkylimidazolium- N-Alkylpyridinium-, Dimethylalkylsulfonium- oder Diethylalkylsulfonium-Kation ist,
wobei L einen Arm darstellt, insbesondere eine lineare oder verzweigte Alkylgruppe, umfassend 1 bis 20 Kohlenstoffatome, oder eine eventuell funktionelle Aralkyl- oder Alkarylgruppe, umfassend 1 bis 20 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 1 und 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO³⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei R₂ und R₃ vorzugsweise in Para-Position ein Wasserstoffatom, eine lineare oder verzweigte Alkylgruppe, umfassend 1 bis 10 Kohlenstoffatome, eine Arylgruppe, eventuell substituiert und/oder funktionell, umfassend 6 bis 30 Kohlenstoffatome, eine funktionelle Gruppe, vorzugsweise eine Methoxy-, Monoalkylamin-, Dialkylamin-, Arylamin-, Zyan-, Ester-, Nitro-, Keton-, Sulfonyl-, Alkylthio-, Sulfoxidgruppe, darstellen,
wobei die Funktionen F₀ und F₁ wie unten definiert sind:
- F₀ entspricht der folgenden Formel: 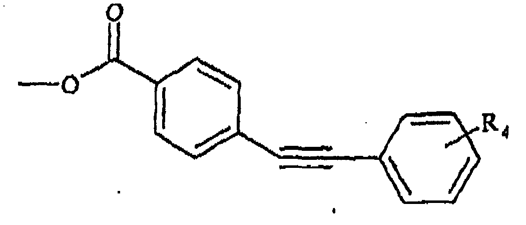 wobei R₄ eine Gruppe, wie für R₂ und R₃ oben definiert, darstellt,
- F₁ entspricht einer der folgenden Formeln: 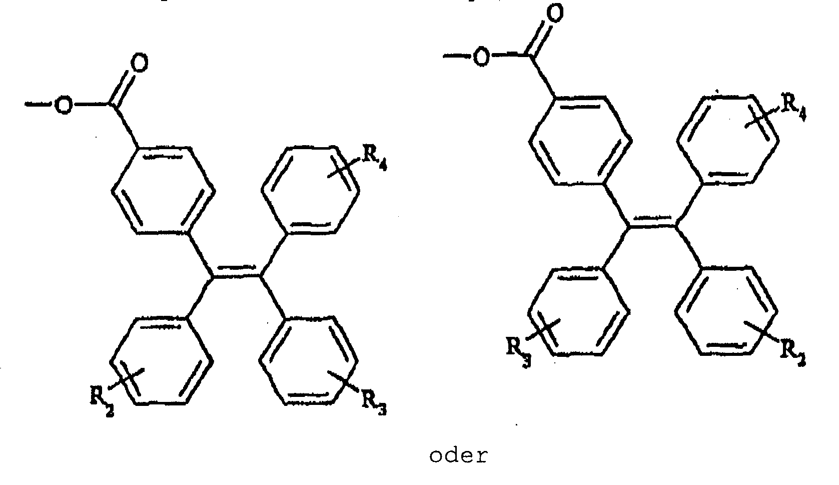 wobei R₂, R₃ und R₄ wie oben definiert sind,
wobei G einer der folgenden Formeln entspricht: 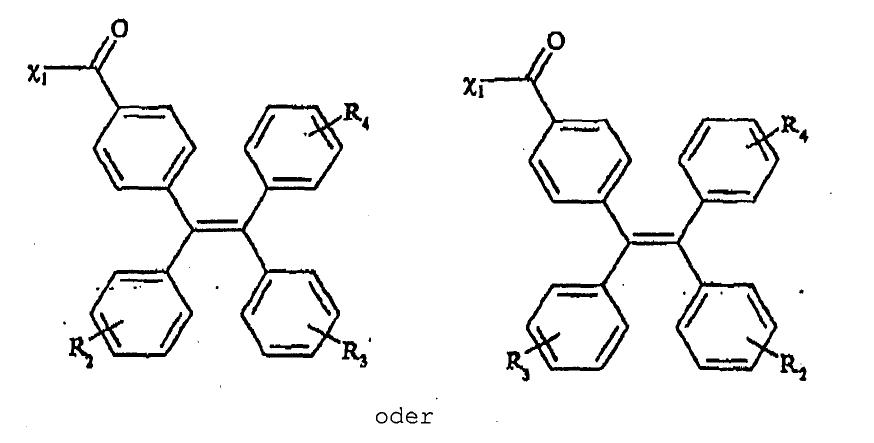 wobei X₁ eine Gruppe -OH oder eine Gruppe -OR_{g}, wobei R_{g} eine lineare oder verzweigte Alkylgruppe, umfassend 1 bis 20 Kohlenstoffatome, darstellt.

27. Verwendung nach einem der Ansprüche 1 bis 17 für den Einsatz von Zykloadditionsreaktionen, vorzugsweise für den Einsatz der Diels-Alder-Reaktion nach dem folgenden Reaktionsschema: 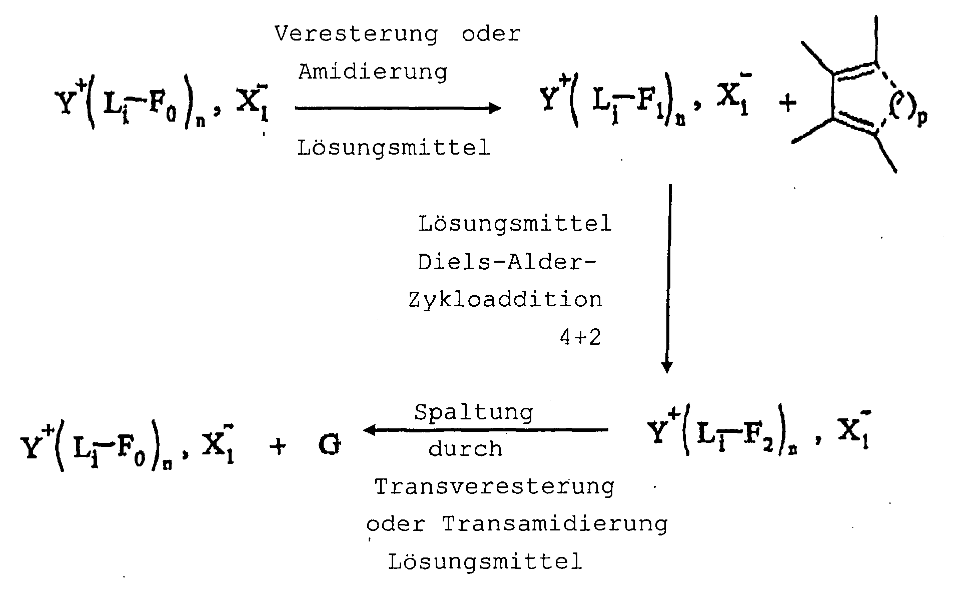 wobei n eine ganze Zahl zwischen 2 und 4 ist, wie unten definiert,
wobei i eine ganze Zahl zwischen 1 und n ist,
wobei p eine ganze Zahl zwischen 0 und 2 ist,
wobei Y⁺ ein Oniumkation, wie in einem der Ansprüche 3 bis 17 definiert, mit der Formel (R_{b})_{z-n}Λ⁺ darstellt, wobei x eine ganze Zahl gleich 3 oder 4 ist, n gleich 2, 3 oder 4 ist, wenn x gleich 4 ist, und n gleich 2 oder 3 ist, wenn x gleich 3 ist, R_{b} eine Alkylgruppe, umfassend 1 bis 20 Kohlenstoffatome, eine Arylgruppe, umfassend 6 bis 30 Kohlenstoffatome, oder eine Aralkyl- oder Alkarylgruppe, umfassend 6 bis 30 Kohlenstoffatome, darstellt, wobei die oben erwähnten Alkyl-, Aryl-, Aralkyl- oder Alkarylgruppen nicht funktionell sind, und wobei Λ⁺ ein Ammonium-, Imidazolium-, Phosphonium- oder Sulfoniumkation darstellt, wobei Y⁺ insbesondere ein Alkylammonium-, Alkylphosphonium- oder Alkylsulfoniumkation darstellt und vorzugsweise ein Tetraalkylammonium-, Tetraalkylphosphonium-, Dialkylimidazolium-, Trialkylsulfonium-Kation ist,
wobei Lᵢ einen Arm darstellt, insbesondere eine lineare oder verzweigte Alkylgruppe, umfassend 1 bis 20 Kohlenstoffatome, oder eine Aralkyl- oder Alkarylgruppe, eventuell funktionell, umfassend 6 bis 30 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 2 und 10 variiert, wobei die Arme Lᵢ identisch oder unterschiedlich sein können,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei die Funktionen F₀, F₁ und F₂ wie unten definiert sind:
- F₀ entspricht einer Gruppe -χ₁H, wobei χ₁ ein Sauerstoffatom oder eine Gruppe -NR_{f}, wobei R_{f} einer linearen oder verzweigten Alkylgruppe, umfassend 1 bis 20 Kohlenstoffatome, oder einer Arylgruppe, umfassend 6 bis 30 Kohlenstoffatome, entspricht,
- F₁ entspricht der folgenden Formel: 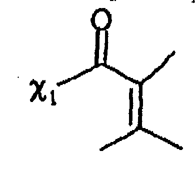 wobei χ₁ wie oben definiert ist,
- F₂ entspricht der folgenden Formel: 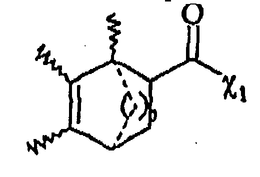 wobei χ₁ wie oben definiert ist,
wobei G der folgenden Formel entspricht: 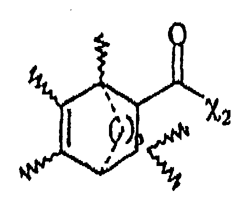 wobei χ₂ entweder eine eine Gruppe -OR_{g} darstellt, wobei R_{g} ein Wasserstoffatom oder eine Alkylgruppe, umfassend 1 bis 20 Kohlenstoffatome, oder eine Gruppe -NRₕRᵤ darstellt, wobei Rₕ und Rᵤ unabhängig voneinander ein Wasserstoffatom, eine Alkylgruppe, umfassend 1 bis 20 Kohlenstoffatome, oder eine Arylgruppe, umfassend 6 bis 30 Kohlenstoffatome, darstellen.

28. Verwendung nach Anspruch 19 für den Einsatz der Heck-Reaktion nach dem folgenden Reaktionsschema: 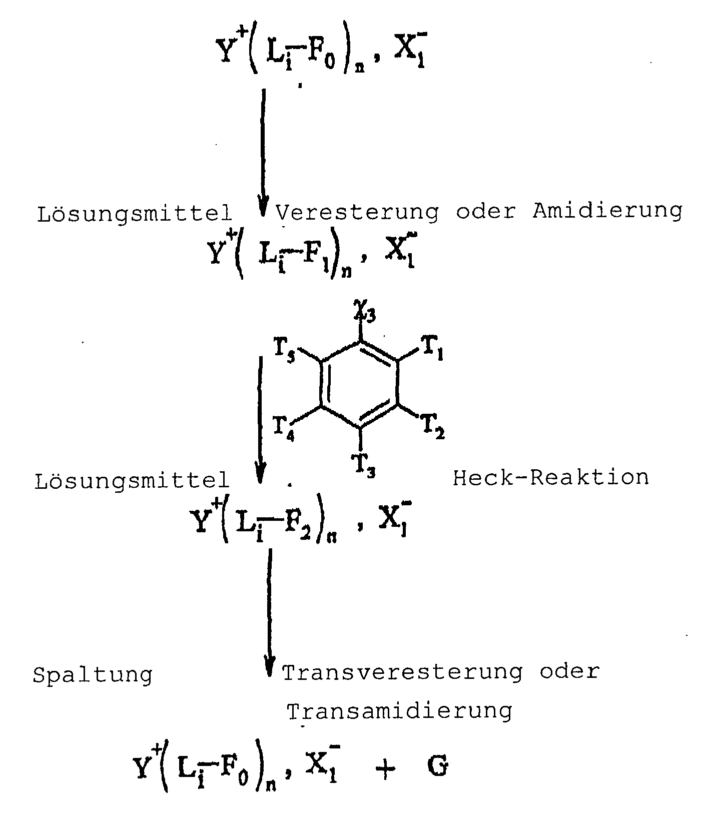 wobei eine ganze Zahl zwischen 2 und 4 ist,
wobei i eine ganze Zahl zwischen 1 und n ist,
wobei Y⁺ ein Oniumkation, wie in einem der Ansprüche 3 bis 17 definiert, mit der Formel (R_{b})_{z-n}Λ⁺ darstellt,
wobei x eine ganze Zahl gleich 3 oder 4 ist, n gleich 2, 3 oder 4 ist, wenn x gleich 4 ist, und n gleich 2 oder 3 ist, wenn x gleich 3 ist, R_{b} eine Alkylgruppe, umfassend 1 bis 20 Kohlenstoffatome, eine Arylgruppe, umfassend 6 bis 30 Kohlenstoffatome, oder eine Aralkyl- oder Alkarylgruppe, umfassend 6 bis 30 Kohlenstoffatome, darstellt, wobei die oben erwähnten Alkyl-, Aryl-, Aralkyl- oder Alkarylgruppen nicht funktionell sind, und wobei Λ⁺ ein Ammonium-, Imidazolium-, Phosphonium- oder Sulfoniumkation darstellt, wobei Y⁺ insbesondere ein Alkylammonium-, Alkylphosphonium- oder Alkylsulfoniumkation darstellt und vorzugsweise ein Tetraalkylammonium-, Tetraalkylphosphonium-, Dialkylimidazolium-, Trialkylsulfonium-Kation ist,
wobei Lᵢ einen Arm darstellt, insbesondere eine lineare oder verzweigte Alkylgruppe, umfassend 1 bis 20 Kohlenstoffatome, oder eine Aralkyl- oder Alkarylgruppe, eventuell funktionell, umfassend 6 bis 30 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 2 und 10 variiert, wobei die Arme Lᵢ identisch oder unterschiedlich sein können,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei die Funktionen F₀, F₁ und F₂ wie unten definiert sind:
- F₀ entspricht einer Gruppe -χ₁H, wobei X₁ ein Sauerstoffatom oder eine Gruppe -NR_{f}, wobei R_{f} einer linearen oder verzweigten Alkylgruppe, umfassend 1 bis 20 Kohlenstoffatome, oder einer Arylgruppe, umfassend 6 bis 30 Kohlenstoffatome, entspricht,
- F₁ entspricht der folgenden Formel: 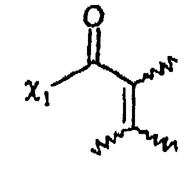 wobei χ₁ wie oben definiert ist,
- F₂ entspricht der folgenden Formel: 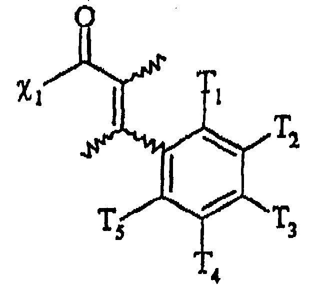 wobei χ₁ wie oben definiert ist,
wobei G der folgenden Formel entspricht: 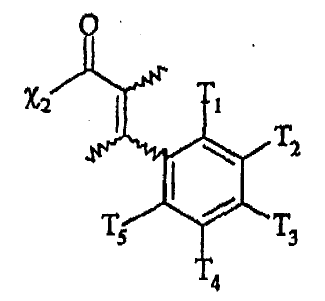 wobei χ₂ entweder eine eine Gruppe -OR_{g} darstellt, wobei R_{g} ein Wasserstoffatom oder eine Alkylgruppe, umfassend 1 bis 20 Kohlenstoffatome, oder eine Gruppe -NRₕRᵤ darstellt, wobei Rₕ und Rᵤ unabhängig voneinander ein Wasserstoffatom, eine Alkylgruppe, umfassend 1 bis 20 Kohlenstoffatome, oder eine Arylgruppe, umfassend 6 bis 30 Kohlenstoffatome, darstellen,
wobei χ₃ eine weggehende Gruppierung darstellt, die insbesondere ausgewählt wird unter den Halogeniden I, Cl und Br, den Mesylat-, Tosylat-, Triflat-, Sulfonat-,
Sulfat- oder Phosphatgruppen.
wobei T₁, T₂, T₃, T₄ und T₅ unabhängig voneinander ein Wasserstoffatom, eine lineare oder verzweigte Alkylgruppe, umfassend 1 bis 20 Kohlenstoffatome, oder eine Arylgruppe, umfassend 6 bis 30 Kohlenstoffatome, oder eine funktionelle Gruppe darstellen, die insbesondere unter NO₂, CN, COOR, OR, COR, NHCOR, NRR", SO₂R, I, Br, R und R" ausgewählt wird, die unabhängig voneinander eine Alkylgruppe, umfassend 1 bis 20 Kohlenstoffatome, oder eine Arylgruppe, umfassend 6 bis 30 Kohlenstoffatome, darstellen,
wobei die Einheit 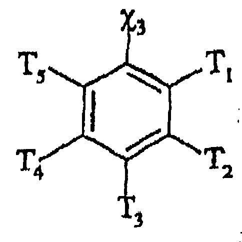 insbesondere die folgenden Gruppen darstellt:

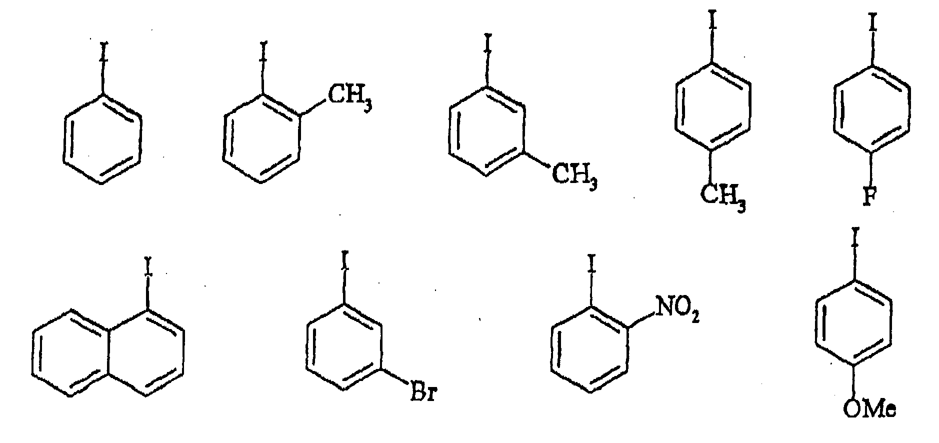

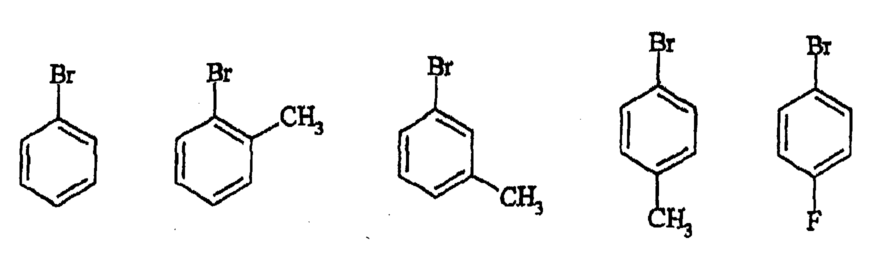

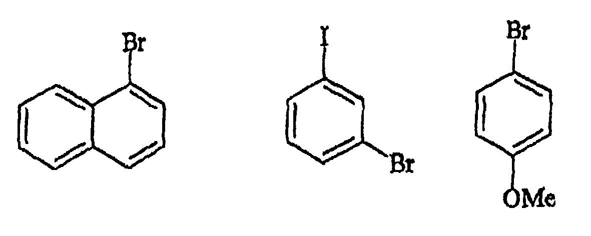

29. Verwendung nach Anspruch 19 für den Einsatz der Suzuki-Kopplung nach dem folgenden Reaktionsschema: 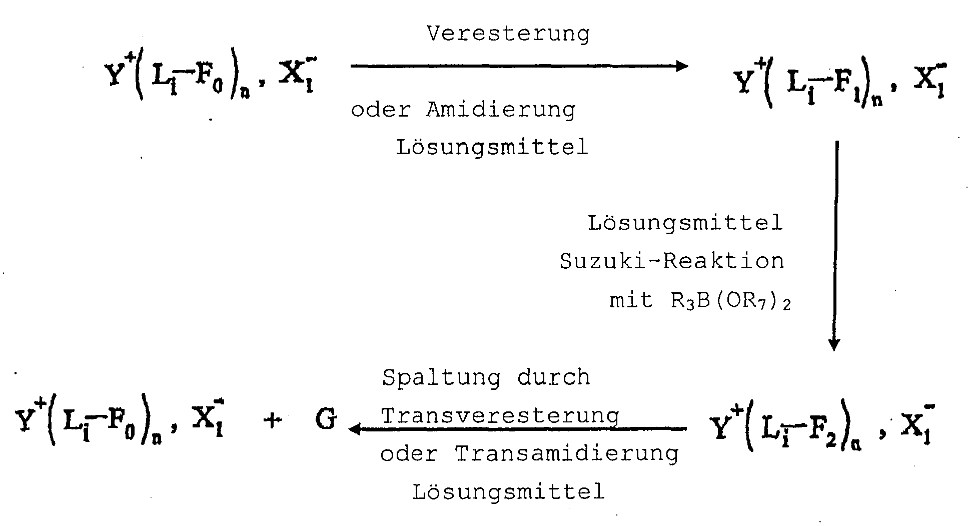 wobei R₃ unter den Aryl-, Heteroaryl-, Ethenyl-, Dienyl-, Allyl-, Ethynylgruppen, die substituiert sind oder nicht und 2 bis 30 Kohlenstoffatome aufweisen, ausgewählt wird,
wobei R₇ ein Wasserstoffatom oder eine Alkylgruppe, verzweigt oder linear, oder eine Zykloalkylgruppe, umfassend 1 bis 12 Kohlenstoffatome, darstellt,
wobei n eine ganze Zahl zwischen 2 und 4 ist,
wobei i eine ganze Zahl zwischen 1 und n ist,
wobei Y⁺ ein Oniumkation, wie in einem der Ansprüche 3 bis 17 definiert, mit der Formel (R_{b})_{z-n}Λ⁺ darstellt, wobei x eine ganze Zahl gleich 3 oder 4 ist, n gleich 2, 3 oder 4 ist, wenn x gleich 4 ist, und n gleich 2 oder 3 ist, wenn x gleich 3 ist, R_{b} eine Alkylgruppe, umfassend 1 bis 20 Kohlenstoffatome, eine Arylgruppe, umfassend 6 bis 30 Kohlenstoffatome, oder eine Aralkyl- oder Alkarylgruppe, umfassend 6 bis 30 Kohlenstoffatome, darstellt, wobei die oben erwähnten Alkyl-, Aryl-, Aralkyl- oder Alkarylgruppen nicht funktionell sind, und wobei Λ⁺ ein Ammonium-, Imidazolium-, Phosphonium- oder Sulfoniumkation darstellt, wobei Y⁺ insbesondere ein Alkylammonium-, Alkylphosphonium- oder Alkylsulfoniumkation darstellt und vorzugsweise ein Tetraalkylammonium-, Tetraalkylphosphonium-, Dialkylimidazolium-, Trialkylsulfonium-Kation ist,
wobei Lᵢ einen Arm darstellt, insbesondere eine lineare oder verzweigte Alkylgruppe, umfassend 1 bis 20 Kohlenstoffatome, oder eine Aralkyl- oder Alkarylgruppe, eventuell funktionell, umfassend 6 bis 30 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 2 und 10 variiert, wobei die Arme Lᵢ identisch oder unterschiedlich sein können,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂GF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei die Funktionen F₀, F₁ und F₂ wie unten definiert sind:
- F₀ entspricht einer Gruppe -χ₁H, wobei χ₁ ein Sauerstoffatom oder eine Gruppe -NR_{f}, wobei R_{f} einer linearen oder verzweigten Alkylgruppe, umfassend 1 bis 20 Kohlenstoffatome, oder einer Arylgruppe, umfassend 6 bis 30 Kohlenstoffatome, entspricht,
- F₁ ist von der Form -Rₑ-χ₀, wobei Rₑ eine aromatische oder heteroaromatische Gruppe, umfassend 6 bis 30 Kohlenstoffatome, darstellt, wobei χ eine weggehende Gruppe darstellt, die vorzugsweise ausgewählt wird unter Cl, Br, I, OTf, O-CO₂R⁵ oder OSO₃R⁵, wobei R⁵ eine Alkylgruppe, umfassend 1 bis 10 Kohlenstoffatome, oder eine Aralkylgruppe, umfassend 6 bis 30 Kohlenstoffatome, darstellt, wobei F₁ vorzugsweise der folgenden Formel entspricht: 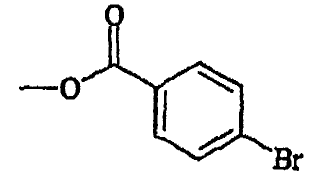
- F₂ ist von der Form -Rₑ-R₂, wobei Rₑ wie oben definiert ist, und wobei R₂ unter den Aryl-, Heteroaryl-, Ethenyl-, Dienyl-, Allyl-, Ethynylgruppen ausgewählt wird, die substituiert sind oder nicht und 2 bis 30 Kohlenstoffatome umfassen, wobei F₂ vorzugsweise der folgenden Formel entspricht: 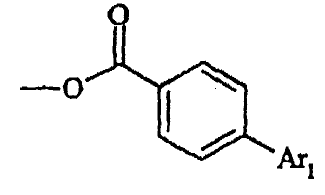 wobei Ar₁ eine aromatische Gruppe darstellt, die vorzugsweise ausgewählt wird unter: 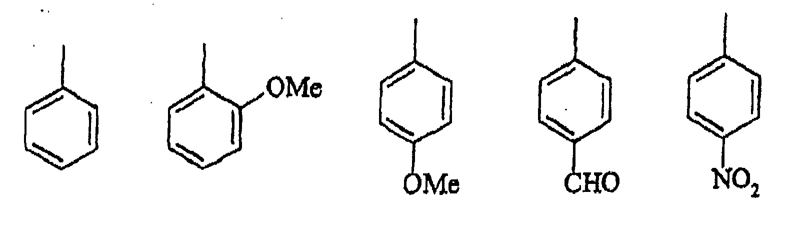 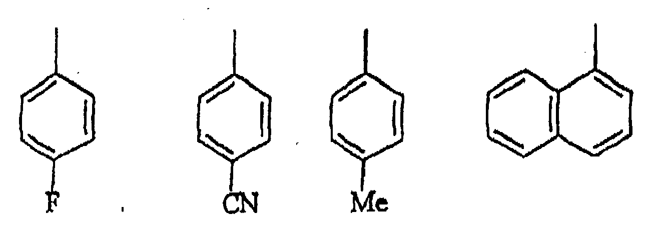 wobei das Molekül G von der Form R₂-R₃ ist, wobei R₂ und R₃ wie oben definiert sind, und insbesondere der folgenden Formel entspricht, 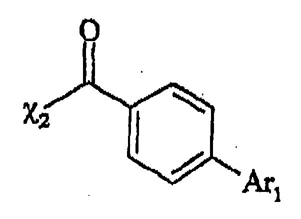 wobei χ₂ entweder eine Gruppe -OR_{g} darstellt, wobei R_{g} ein Wasserstoffatom oder eine Alkylgruppe, umfassend 1 bis 20 Kohlenstoffatome, oder eine Gruppe -NRₕRᵤ darstellt, wobei Rₕ und Rᵤ unabhängig voneinander ein Wasserstoffatom, eine Alkylgruppe, umfassend 1 bis 20 Kohlenstoffatome, oder eine Arylgruppe, umfassend 6 bis 30 Kohlenstoffatome, darstellen,
wobei Ar₁ wie oben definiert ist.

30. Verwendung nach Anspruch 19 für den Einsatz der Heck-Reaktion nach dem folgenden Reaktionsschema: 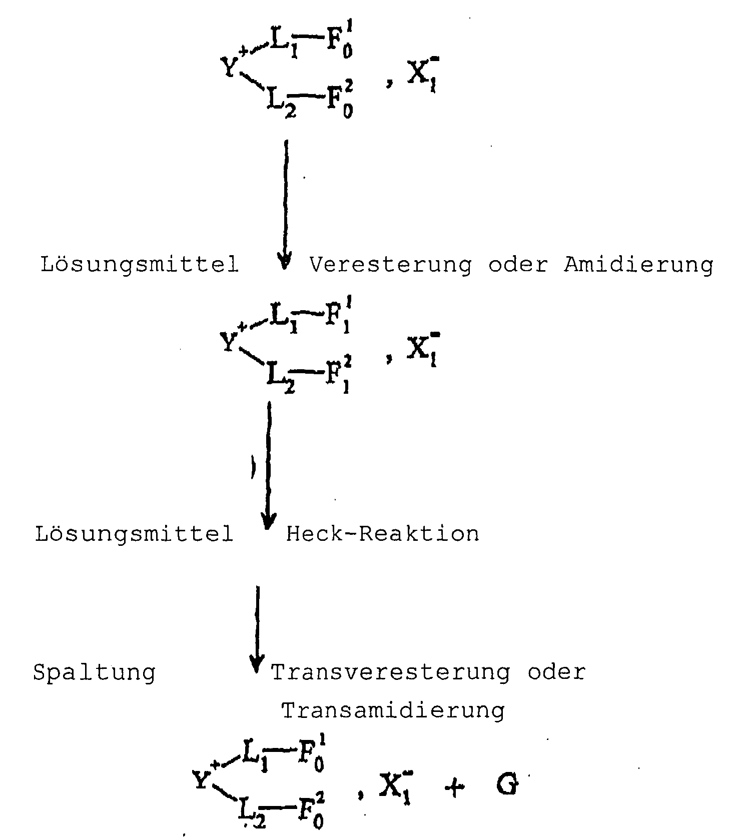 wobei Y⁺ ein Oniumkation, wie in einem der Ansprüche 3 bis 17 definiert, mit der Formel (R_{b})_{z-2}Λ⁺ darstellt, wobei x eine ganze Zahl gleich 3 oder 4 ist, R_{b} eine Alkylgruppe, umfassend 1 bis 20 Kohlenstoffatome, eine Arylgruppe, umfassend 6 bis 30 Kohlenstoffatome, oder eine Aralkyl- oder Alkarylgruppe, umfassend 6 bis 30 Kohlenstoffatome, darstellt, wobei die oben erwähnten Alkyl-, Aryl-, Aralkyl- oder Alkarylgruppen nicht funktionell sind, und wobei Λ⁺ ein Ammonium-, Imidazolium-, Phosphonium- oder Sulfoniumkation darstellt, wobei Y⁺ insbesondere ein Alkylammonium-, Alkylphosphonium- oder Alkylsulfoniumkation darstellt und vorzugsweise ein Tetraalkylammonium-, Tetraalkylphosphonium-, Dialkylimidazolium-, Trialkylsulfonium-Kation ist, wobei Λ⁺ ein Ammonium- oder Phosphoniumkation darstellt, wenn x = 4, und ein Sulfoniumkation darstellt, wenn x = 3,
wobei L₁ und L₂, die identisch oder unterschiedlich sind, einen Arm darstellen, insbesondere eine lineare oder verzweigte Alkylgruppe, umfassend 1 bis 20 Kohlenstoffatome, oder eine Aralkyl- oder Alkarylgruppe, eventuell funktionell, umfassend 1 bis 20 Kohlenstoffatome, und vorzugsweise eine lineare Alkylgruppe, vorzugsweise eine lineare Alkylgruppe des Typs (CH₂)ᵣ, wobei r zwischen 1 und 20 und vorzugsweise zwischen 2 und 10 variiert,
wobei X₁⁻ wie in einem der Ansprüche 1 bis 17 definiert ist, und insbesondere **Cl⁻, Br⁻, I⁻, CF₃CO₂⁻, CH₃CO₂⁻, BF₄⁻, PF₆⁻, CF₃SO₃⁻, ⁻N(SO₂CF₃)₂, SO₄²⁻, R₁SO₄⁻, SbF₆⁻, R₁SO₃⁻, FSO₃⁻, PO₄³⁻,** wobei R₁ eine Alkylgruppe, umfassend 1 bis 20 Kohlenstoffatome, darstellt,
wobei das oder die Lösungsmittel ausgewählt werden unter: Dichlormethan, Tetrahydrofuran, Dioxan, Azetonitril, Dimethylformamid, Dimethylazetamid, N-Methylpyrrolidinon, Propionitril, Azeton, Toluol, Chlorbenzol, Nitrobenzol, Dichlorbenzol, Nitromethan, Nitroethan oder einem Gemisch dieser Lösungsmittel,
wobei die Funktionen F₀¹, F₁¹, F₀² und F₁² wie unten definiert sind:
- F₀¹ entspricht einer Gruppe -χ²₁H, wobei χ²₁ ein Sauerstoffatom oder eine Gruppe -NR_{f}, wobei R_{f} einer linearen oder verzweigten Alkylgruppe, umfassend 1 bis 20 Kohlenstoffatome, oder einer Arylgruppe, umfassend 6 bis 30 Kohlenstoffatome, entspricht,
- F₁¹ entspricht der folgenden Formel: 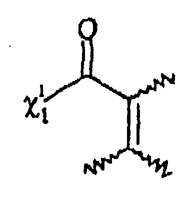 wobei χ¹₁ wie oben definiert ist,
- F₁² entspricht der folgenden Formel: 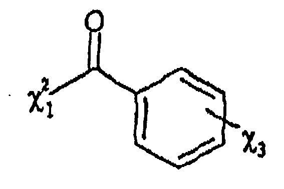
wobei χ²₁ wie oben definiert ist,
wobei χ₃ eine weggehende Gruppierung darstellt, die insbesondere unter den Halogeniden I, Cl und Br, den Mesylat-, Tosylat-, Triflat-, Sulfonat-, Sulfat- oder Phosphatgruppen ausgewählt wird,
wobei G der folgenden Formel entspricht: 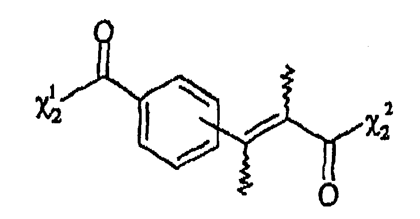 wobei χ₂¹ und χ₂² identisch oder unterschiedlich sind und entweder eine Gruppe -OR_{g} darstellen, wobei Rg ein Wasserstoffatom oder eine Alkylgruppe, umfassend 1 bis 20 Kohlenstoffatome, oder eine Gruppe -NRₕRᵤ darstellt,
wobei Rₕ und Rᵤ unabhängig voneinander ein Wasserstoffatom, eine Alkylgruppe, umfassend 1 bis 20 Kohlenstoffatome, oder eine Arylgruppe, umfassend 6 bis 30 Kohlenstoffatome, darstellen.
